(19) Europäisches Patentamt
European Patent Office
Office européen des brevets

(11) EP 3 433 610 B1

(12) EUROPEAN PATENT SPECIFICATION

(45) Date of publication and mention of the grant of the patent:
**25.12.2024 Bulletin 2024/52**

(21) Application number: **17771216.3**

(22) Date of filing: **24.03.2017**

(51) International Patent Classification (IPC):
***G01N 33/48*** *(2006.01)* ***C07H 21/04*** *(2006.01)*
***C12Q 1/6883*** *(2018.01)* ***C12N 15/12*** *(2006.01)*
***A61D 99/00*** *(2006.01)*

(52) Cooperative Patent Classification (CPC):
**A61D 99/00; C12Q 1/6883;** C12Q 2600/124; C12Q 2600/156; G01N 2800/10

(86) International application number:
**PCT/US2017/023969**

(87) International publication number:
**WO 2017/165733 (28.09.2017 Gazette 2017/39)**

# (54) METHOD OF DETECTING INHERITED EQUINE MYOPATHY

VERFAHREN ZUM NACHWEIS VERERBTER PFERDEMYOPATHIE

MÉTHODE DE DÉPISTAGE D’UNE MYOPATHIE ÉQUINE HÉRÉDITAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.03.2016 US 201662313272 P**
**14.11.2016 US 201662421625 P**

(43) Date of publication of application:
**30.01.2019 Bulletin 2019/05**

(73) Proprietors:
- **UNM Rainforest Innovations**
  **Albuquerque, NM 87102 (US)**
- **Szauter, Paul**
  **Albuquerque, NM 87110 (US)**
- **Sinclair, Robert B.**
  **Asheville, NC 28805 (US)**

(72) Inventors:
- **EDWARDS, Jeremy, Scott**
  **Albuquerque, NM 87106 (US)**
- **SZAUTER, Paul**
  **Albuquerque, NM 87110 (US)**
- **SINCLAIR, Robert B.**
  **Asheville, NC 28805 (US)**

(74) Representative: **Potter Clarkson**
**Chapel Quarter**
**Mount Street**
**Nottingham NG1 6HQ (GB)**

(56) References cited:
**WO-A1-2008/100313**

- **VALBERG STEPHANIE J. ET AL: "Commercial genetic testing for type 2 polysaccharide storage myopathy and myofibrillar myopathy does not correspond to a histopathological diagnosis", EQUINE VETERINARY JOURNAL., vol. 53, no. 4, 29 October 2020 (2020-10-29), GB, pages 690 - 700, XP55982607, ISSN: 0425-1644, Retrieved from the Internet <URL:https://onlinelibrary. wiley.com/doi/full-xml/10.1111/evj.13345> DOI: 10.1111/evj.13345**
- **MYKKANEN A K ET AL: "MCT1, MCT4 and CD147 gene polymorphisms in healthy horses and horses with myopathy", RESEARCH IN VETERINARY SCIENCE, vol. 91, no. 3, December 2011 (2011-12-01), pages 473 - 477, XP002793025**
- **T. FOROUD ET AL: "A mutation in myotilin causes spheroid body myopathy", NEUROLOGY, vol. 65, no. 12, 27 December 2005 (2005-12-27), US, pages 1936 - 1940, XP055606385, ISSN: 0028-3878, DOI: 10.1212/ 01.wnl.0000188872.28149.9a**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **S AURINO ET AL: "Candidate-gene testing for orphan limb-girdle muscular dystrophies", ACTA MYOLOGICA : MYOPATHIES AND CARDIOMYOPATHIES : OFFICIAL JOURNAL OF THE MEDITERRANEAN SOCIETY OF MYOLOGY, 1 December 2008 (2008-12-01), Italy, pages 90 - 97, XP055606381, Retrieved from the Internet <URL:https://www.ncbi.nlm.nih. gov/pmc/arti cles/PMC2858943/pdf/1128-2460.27.090.pdf>**
- **FERLINI A. ET AL.: "The medical genetics of dystrophinopathies: molecular genetic diagnosis and its impact on clinical practice", NEUROMUSCULAR DISORDERS, vol. 23, no. 1, 2013, pages 4 - 14, XP055578841**
- **SELCEN D. ET AL.: "Mutations in myotilin cause myofibrillar myopathy", NEUROLOGY, vol. 62, no. 8, 2004, pages 1363 - 1371, XP055578842**
- **KLEY RA. ET AL.: "Impairment of protein degradation in myofibrillar myopathy caused by FLNC/filamin C mutations", AUTOPHAGY, vol. 9, no. 3, 2013, pages 422 - 423t, XP055578844**
- **PERNILLA VON NANDELSTADH ET AL.: "A class III PDZ binding motif in the myotilin and FATZ families binds enigma family proteins: a common link for Z-disc myopathies", MOLECULAR AND CELLULAR BIOLOGY, vol. 29, no. 3, 2009, pages 822 - 834, XP055578845**


Remarks:

The file contains technical information submitted after the application was filed and not included in this specification

## Description

CROSS-REFERENCE TO RELATED APPLICATION

This application claims priority to U.S. Provisional Patent Application No. 62/313,272, filed March 25, 2016, and U.S. Provisional Patent Application No. 62/421,625, filed November 14, 2016.

SUMMARY

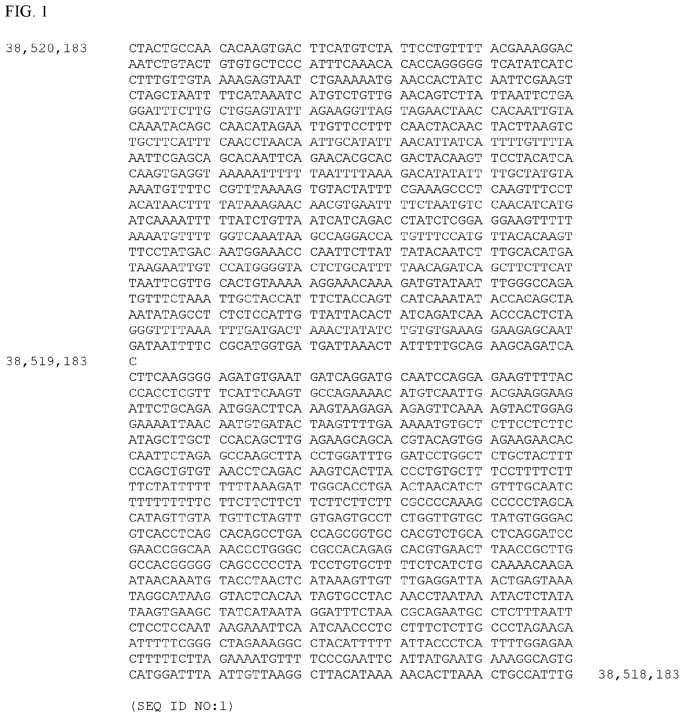

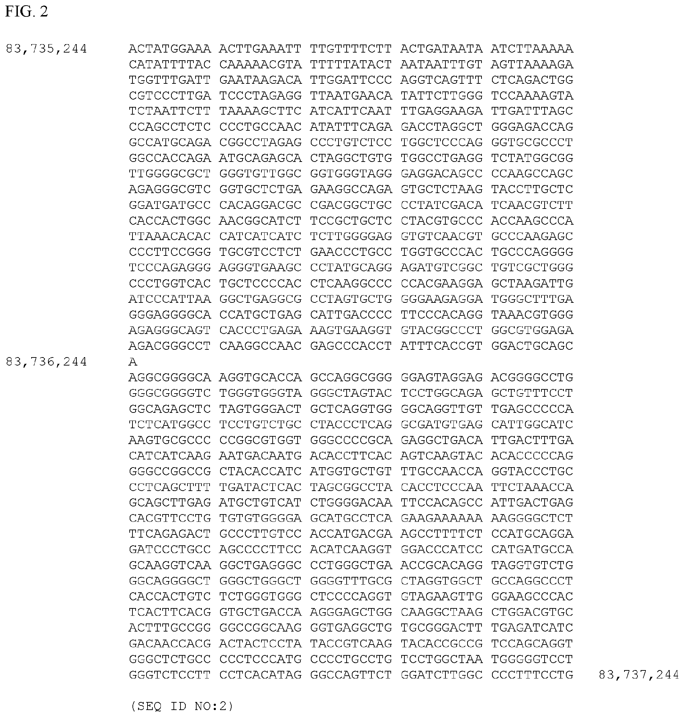

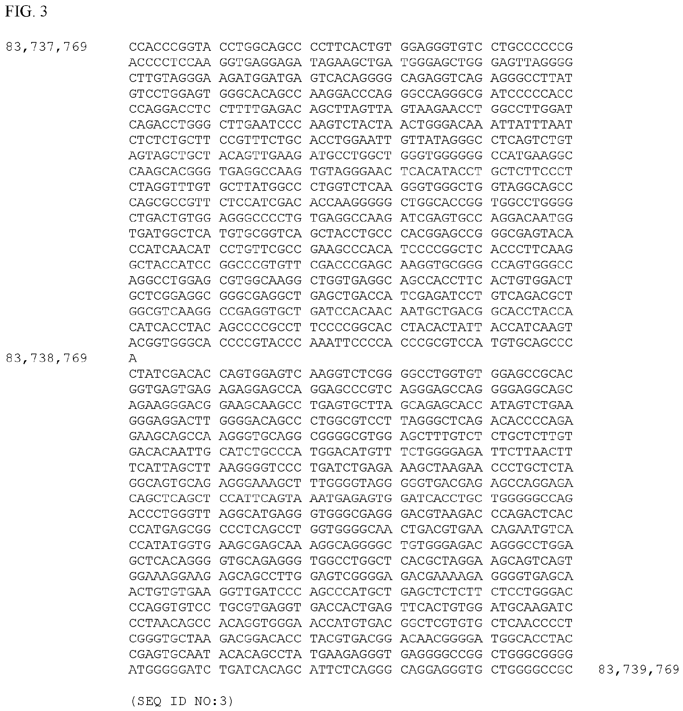

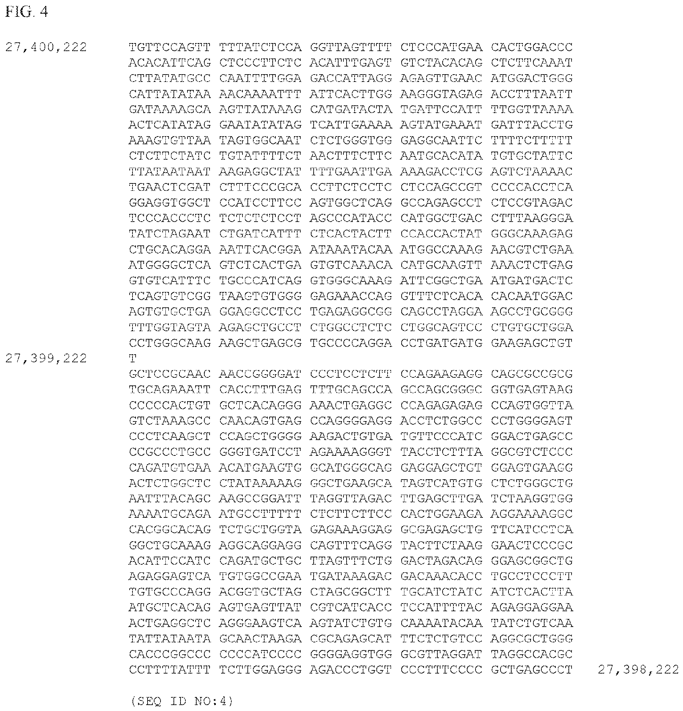

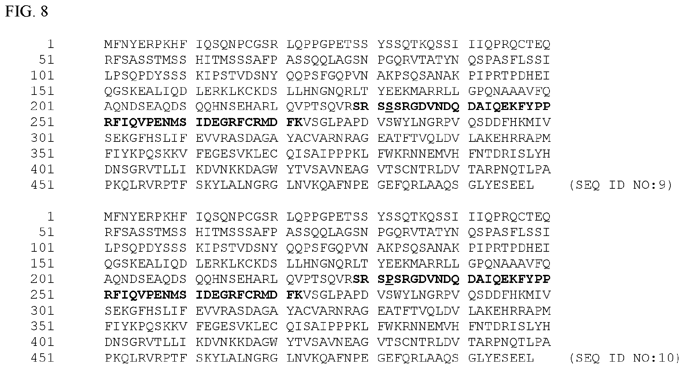

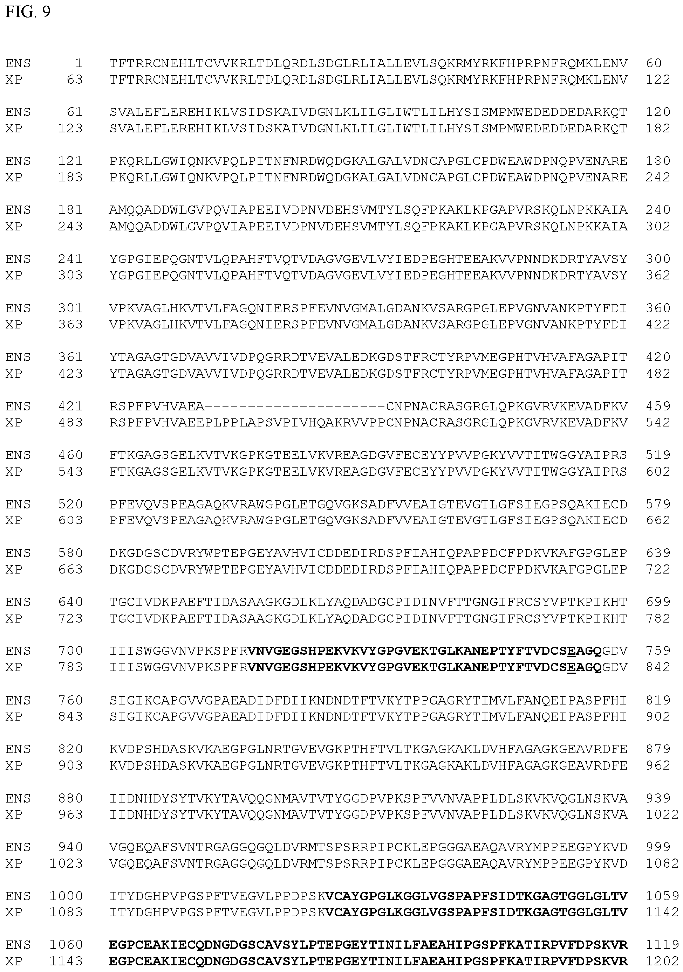

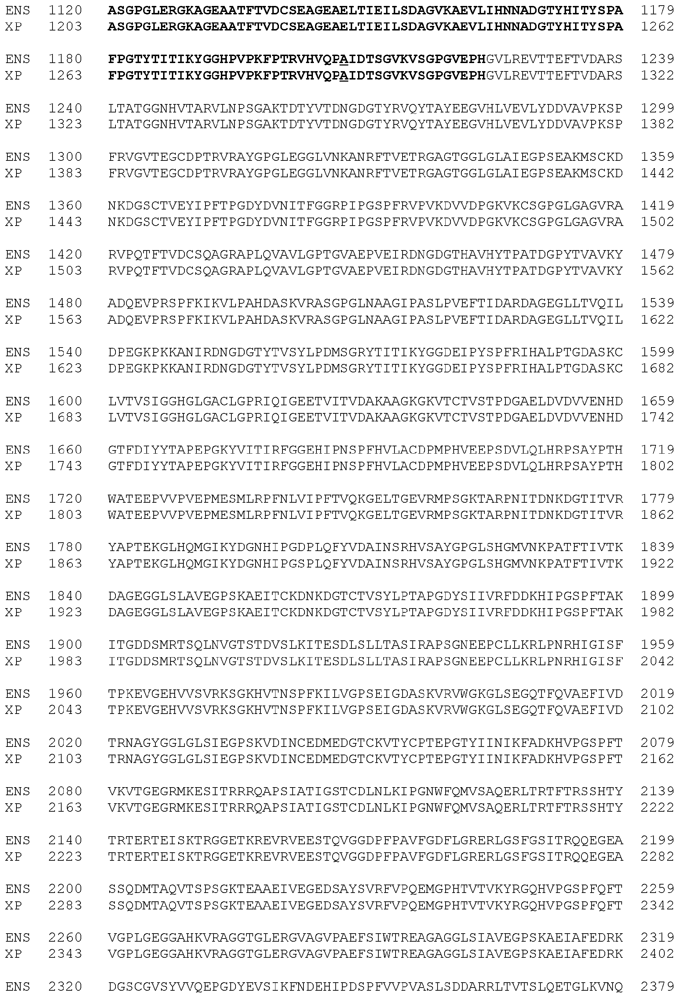

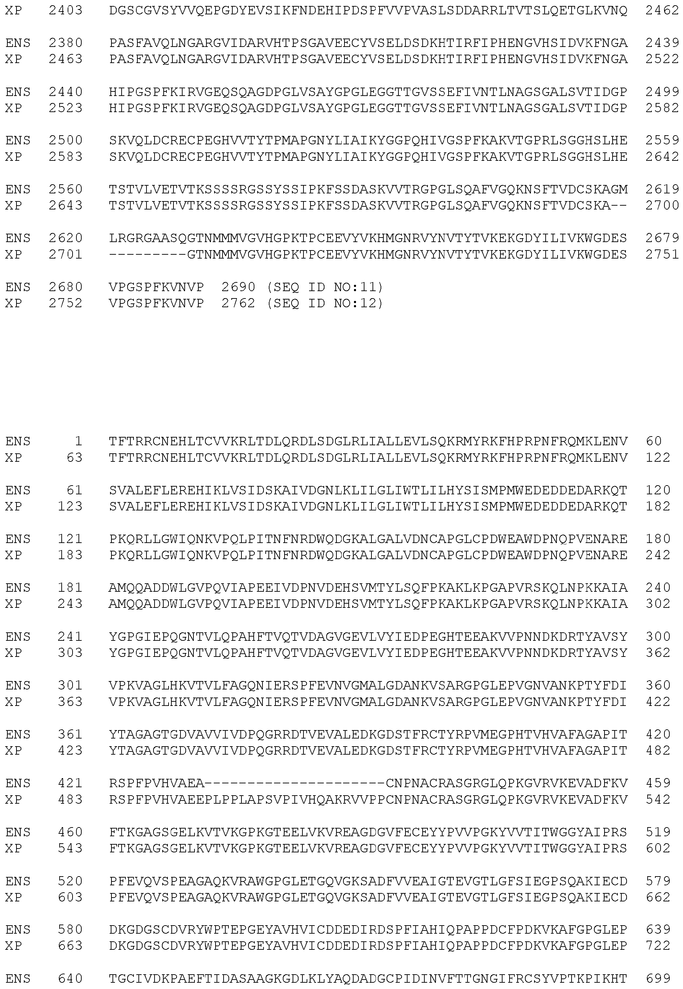

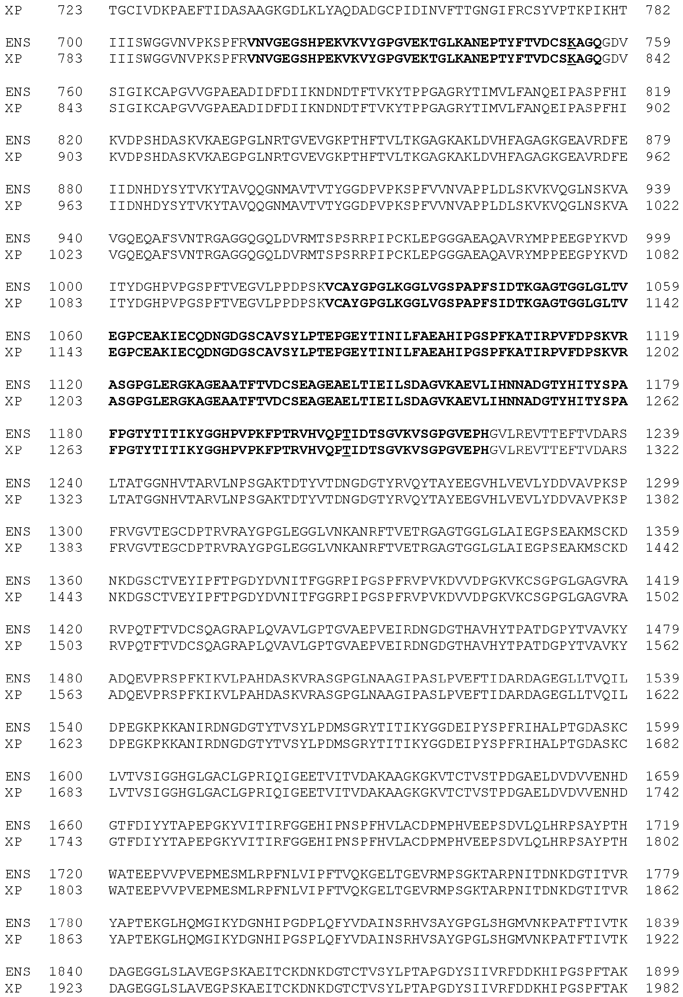

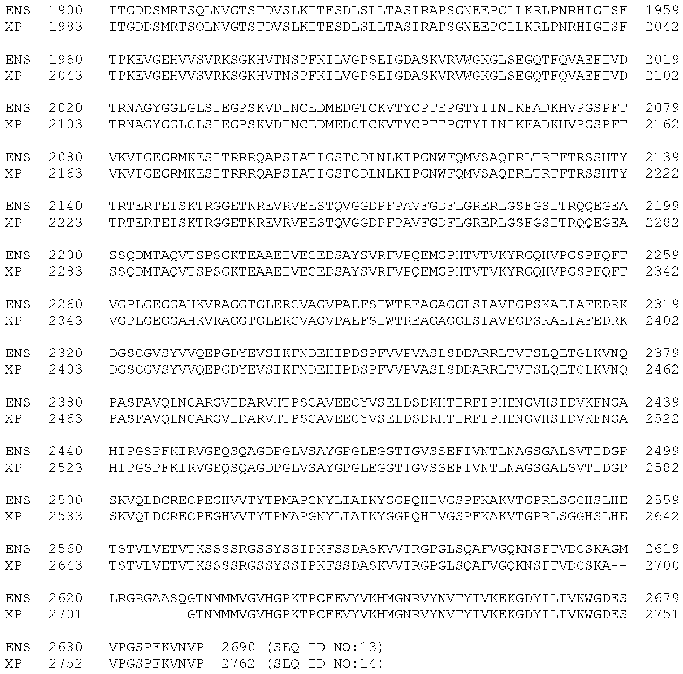

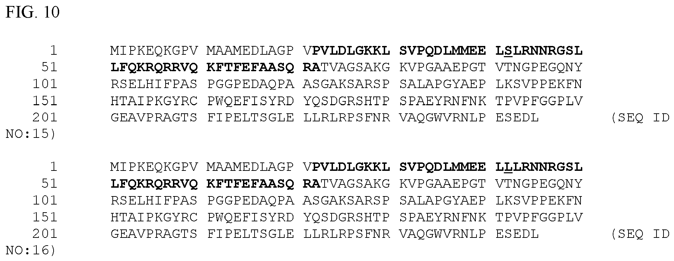

The invention is as defined by the claims. In particular, the invention provides a method for detecting the presence or absence of biomarkers in a horse, the method comprising:
detecting in a biological sample from a horse, the biological sample comprising a nucleic acid that includes the coding regions for myotilin (MYOT), filamin-C (FLNC) and myozenin-3 (MYOZ3), the presence or absence of a base substitution corresponding to guanine (G) substituted for an adenine (A) at nucleotide chr14:38519183 of the forward strand of SEQ ID NO: 1, a base substitution corresponding to an adenine (A) substituted for a guanine (G) at nucleotide chr4:83736244 of the forward strand of SEQ ID NO:2, a base substitution corresponding to an adenine (A) substituted for a guanine (G) at nucleotide chr4:83738769 of the forward strand of SEQ ID NO:3, and a base substitution corresponding to an adenine (A) substituted for a guanine (G) at nucleotide chr14:27399222 of SEQ ID NO:4, or the complement thereof, wherein the presence of any of said substitutions in said nucleic acid is indicative of inherited equine myopathy in said horse.
The invention also provides a method for detecting the presence or absence of biomarkers in a horse, the method comprising:
detecting in a biological sample from a horse, the biological sample comprising a nucleic acid encoding a myotilin polypeptide, a filamin-C polypeptide, and a myozenin-3 polypeptide, the presence or absence of a nucleic acid that encodes a myotilin polypeptide having a proline residue (P) substituted for serine (S) at position 232 of SEQ ID NO: 10, a nucleic acid that encodes a filamin-C polypeptide having a lysine residue (K) substituted for glutamate (E) at position 753, a nucleic acid that encodes a filamin-C polypeptide having a threonine residue (T) substituted for alanine (A) at position 1207 of SEQ ID NO: 13, and a nucleic acid that encodes a myozenin-3 polypeptide having a leucine residue (L) substituted for serine (S) at position 42 of SEQ ID NO: 16, wherein the presence of any of said nucleic acids in said sample is indicative of inherited equine myopathy in said horse.
To the extent that other methods including methods for detecting the presence or absence of biomarkers in a horse are described herein, or other aspects are described herein, they are included solely for reference purposes.
WO 2008/100313 A1 describes a method of detecting equine polysaccharide storage myopathy.
Mykkanen et al (Research in veterinary science, 91(3): 473-477) describes MCT1, MCT4 and CD147 gene polymorphisms in healthy horses and horses with myopathy.
Foroud et al (Neurology, 65(12): 1936-1940) describes how a mutation in myotilin causes spheroid body myopathy.
Aurino et al (Official journal of the Mediterranean Society of Myology, 1 December 2008, 90-97) describes candidate gene testing for organ limb-girdle muscular dystrophies.
This disclosure describes, in one aspect, a method for detecting the presence or absence of a set of biomarkers in a horse. In the context of the present invention, the method includes detecting in a biological sample from a horse that includes a nucleic acid that includes the coding regions for myotilin (MYOT), filamin-C (FLNC), and myozenin-3 (MYOZ3), the presence or absence of specific substitutions as follows: (1) a guanine (G) substituted for an adenine (A) at chr14:38,519,183 of the current horse genome assembly (EquCab2, GCA_000002305.1) as displayed in the UCSC Genome Browser and as shown in FIG. 1, or the equivalent substitution in the complement thereof; (2) an adenine (A) substituted for a guanine (G) at chr4:83,736,244 and an adenine (A) substituted for a guanine (G) at chr4:83,738,769 of the current horse genome assembly (EquCab2, GCA_000002305.1) as displayed in the UCSC Genome Browser and as shown in FIG. 2 and FIG. 3, respectively, or the equivalent substitution in the complement thereof; and (3) an adenine (A) substituted for a guanine (G) at chr14:27,399,222 of the current horse genome assembly (EquCab2, GCA_000002305.1) as displayed in the UCSC Genome Browser and as shown in FIG. 4, or the equivalent substitution in the complement thereof. These base substitutions, corresponding to position 38,519,183 in SEQ ID NO:1, position 83,736,244 in SEQ ID NO:2, position 83,738,769 in SEQ ID NO:3, and position 27,399,222 in SEQ ID NO:4, result in nonconservative amino acid substitutions in the myotilin (MYOT), filamin-C (FLNC), and myozenin3 (MYOZ3) proteins, respectively. The amino acid substitutions caused by these base substitutions are shown in FIG. 8, FIG. 9, and FIG. 10. FIG. 8 shows an altered myotilin with proline (P) substituted for serine (S) at position 232, with SEQ ID NO:9 showing the protein sequence encoded by the wild-type or common allele and SEQ ID NO: 10 showing the protein sequence encoded by the variant. FIG. 9 shows an altered filamin-C (FLNC) with lysine (K) substituted for glutamic acid (E) in filamin repeat 6 at position 753 in SEQ ID NO: 11 and position 836 in SEQ ID NO: 12 and threonine (T) substituted for alanine (A) filamin repeat 11 at position 1207 in SEQ ID NO: 11 and position 1290 in SEQ ID NO: 12, with SEQ ID NO: 11 and SEQ ID NO: 12 showing the protein sequence encoded by the wild-type or common allele and SEQ ID NO: 13 and SEQ ID NO: 14 showing the protein sequence encoded by the variants; both variants as typically seen as a single haplotype. FIG. 10 shows an altered myozenin-3 (MYOZ3) with leucine (L) substituted for serine (S) at position 42, with SEQ ID NO: 15 showing the wild-type or common allele and SEQ ID NO: 16 showing the protein sequence encoded by the variant.

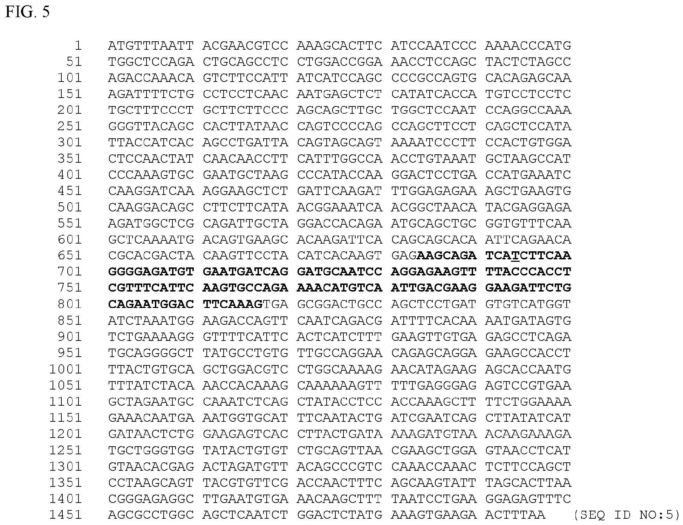

In some embodiments, the method further includes amplifying at least a portion of the MYOT, FLNC, or MYOZ3 coding regions. In some of these embodiments, exon 6 of the MYOT coding region, exons 15 and 21 of the FLNC coding region, and exon 3 of the MYOZ3 coding region are amplified. These specified exons correspond to the gene models presented in FIG. 5, FIG. 6, FIG. 7, FIG. 8, FIG. 9, and FIG. 10 in this disclosure; the specific base substitutions detected are presented in FIG. 1, FIG. 2, FIG. 3 and FIG. 4, even if alternative gene models or different isoforms result in these exons being numbered differently. In another aspect, this disclosure describes a method for detecting the presence or absence of a biomarker in a physiological sample. Generally, the method includes detecting in a physiological sample from a horse that includes a nucleic acid that includes at least a portion of SEQ ID NO: 1 that includes nucleotide 38,519,183 of SEQ ID NO:1, whether the nucleic acid has a guanine (G) at nucleotide 38,519,183 of the forward strand of SEQ ID NO:1, at least a portion of SEQ ID NO:2 that includes nucleotide 83,736,244 of SEQ ID NO:2, whether the nucleic acid has an adenine (A) at 83,736,244 of the forward strand of SEQ ID NO:2; at least a portion of SEQ ID NO:3 that includes nucleotide 83,738,769 of SEQ ID NO:3, whether the nucleic acid has an adenine (A) at 83,738,769 of the forward strand of SEQ ID NO:3; and at least a portion of SEQ ID NO:4 that includes nucleotide 27,399,222 of SEQ ID NO:4, whether the nucleic acid has an adenine (A) at 27,399,222 of the forward strand of SEQ ID NO:4. In all cases, the nucleotide at the specified position of the forward strand may be inferred by the determination of the nucleotide at the specified position on the reverse (complementary) strand. In some embodiments, the method further includes amplifying at least a portion of the nucleic acid.

In another aspect, this disclosure describes a method for detecting the presence or absence of a biomarker in a physiological sample. Generally, the method includes detecting in a physiological sample from a horse that includes a nucleic acid encoding a myotilin, filamin-C, or myozenin-3 polypeptide, whether the nucleic acid encodes a myotilin, filamin-C, or myozenin-3 polypeptide altered as described as follows: (1) a myotilin polypeptide having the amino acid sequence of SEQ ID NO:9 or a myotilin polypeptide having a proline (P) substituted for serine (S) at position 232 as shown in SEQ ID NO: 10, (2) a filamin-C polypeptide having the amino acid sequence of SEQ ID NO: 11 (equivalent to SEQ ID NO:12) or a filamin-C polypeptide having a lysine (K) substituted for glutamic acid (E) at position 753 in SEQ ID NO: 11 (equivalent to position 836 in SEQ ID NO:12) as shown in SEQ ID NO: 13 (equivalent to SEQ ID NO:14) or a filamin-C polypeptide having the amino acid sequence of SEQ ID NO: 11 (equivalent to SEQ ID NO:12) or a filamin-C polypeptide having a threonine (T) substituted for alanine (A) at position 1207 in SEQ ID NO: 11 (equivalent to position 1290 in SEQ ID NO: 12), as shown in SEQ ID NO: 13 (equivalent to SEQ ID NO: 14), or (3) a myozenin-3 polypeptide having the amino acid sequence of SEQ ID NO: 15 or a myozenin-3 polypeptide having a leucine (L) substituted for a serine (S) at position 42 in SEQ ID NO: 15 as shown in SEQ ID NO: 16.

The above summary is not intended to describe each disclosed embodiment or every implementation. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

## BRIEF DESCRIPTION OF THE FIGURES

FIG. 1. A portion of the current horse genome assembly (EquCab2, GCA 000002305.1) with coordinates as displayed in the UCSC Genome Browser centered on the chr14:38,519,183 position, the site of a substitution of a guanine (G) for an adenine (A) that results in the substitution of a proline (P) for serine (S) at amino acid position 232 in myotilin as shown in FIG. 8. The reverse complement sequence is shown (SEQ ID NO:1), with the site of a substitution of a cytosine (C) for a thymine (T) indicated.

FIG. 2. A portion of the current horse genome assembly (EquCab2, GCA 000002305.1) with coordinates as displayed in the UCSC Genome Browser centered on the chr4:83,736,244 position, the site of a substitution of an adenine (A) for a guanine (G) that results in the substitution of a lysine (K) for glutamic acid (E) in filamin-C, at amino acid position 753 in filamin-C as shown in FIG. 9. The forward strand sequence is shown (SEQ ID NO:2).

FIG. 3. A portion of the current horse genome assembly (EquCab2, GCA 000002305.1) with coordinates as displayed in the UCSC Genome Browser centered on the chr4:83,738,769 position, the site of a substitution of an adenine (A) for a guanine (G) that results in the substitution of a threonine (T) for alanine (A) in filamin-C, at amino acid position 1207 as shown in FIG. 9. The forward strand sequence is shown (SEQ ID NO:3).

FIG. 4. A portion of the current horse genome assembly (EquCab2, GCA 000002305.1) with coordinates as displayed in the UCSC Genome Browser centered on the chr14:27,399,222 position, the site of a substitution of an adenine (A) for a guanine (G) that results in the substitution of a leucine (L) for a serine (S) in myozenin-3, at amino acid position 42 as shown in FIG. 10. The reverse complement sequence is shown (SEQ ID NO:4), with the site of a substitution of a thymine (T) for a cytosine (C) indicated.

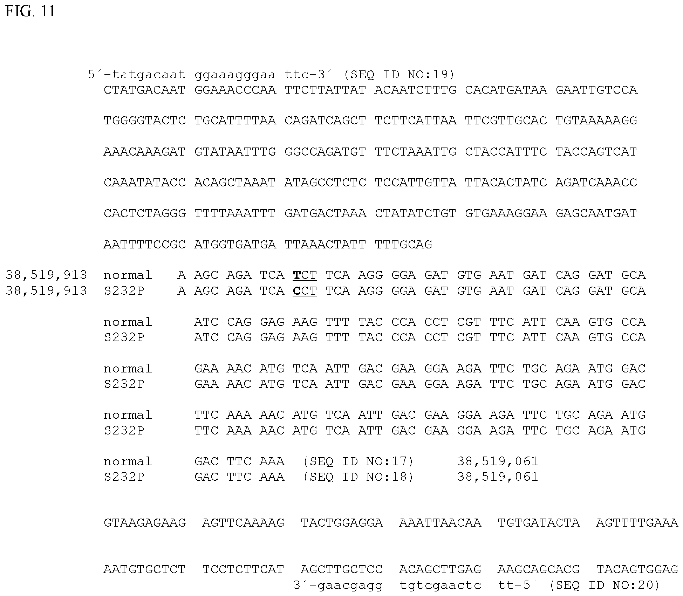

FIG. 5. Normal equine MYOT Coding DNA Sequence (SEQ ID NO:5), also known as XM_014730661.1. Exon 6 is indicated in bold. The site of a T to C mutation site at nucleotide position 694 in SEQ ID NO:5 (38,519,183 in SEQ ID NO:1, as shown in FIG. 1) is underlined. The region of sequence comprising exon 6 is displayed as codons in the correct reading frame in FIG. 11.

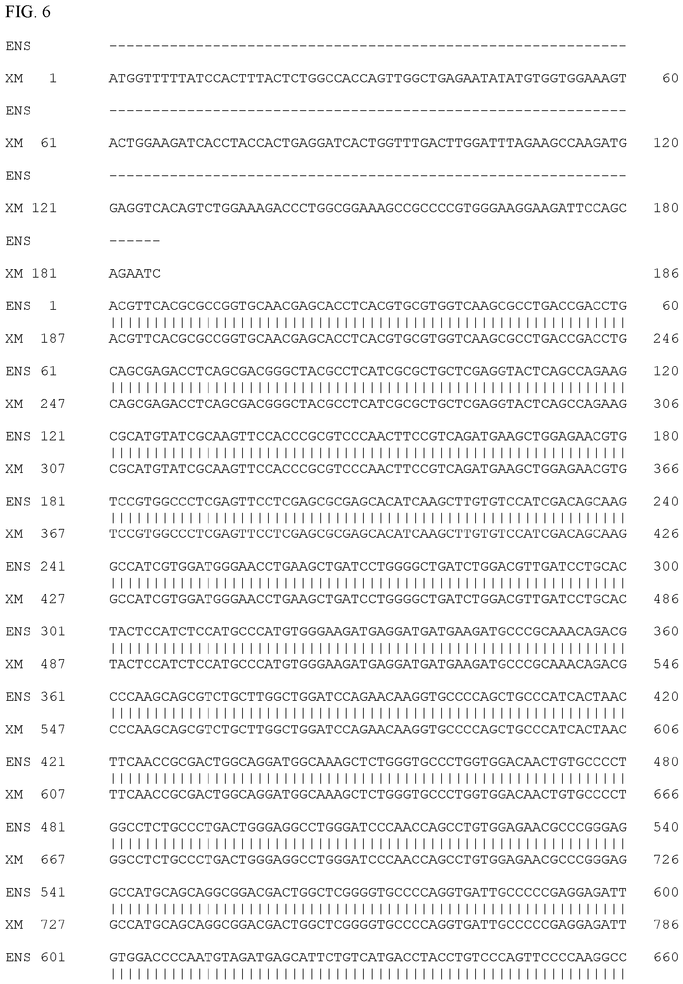

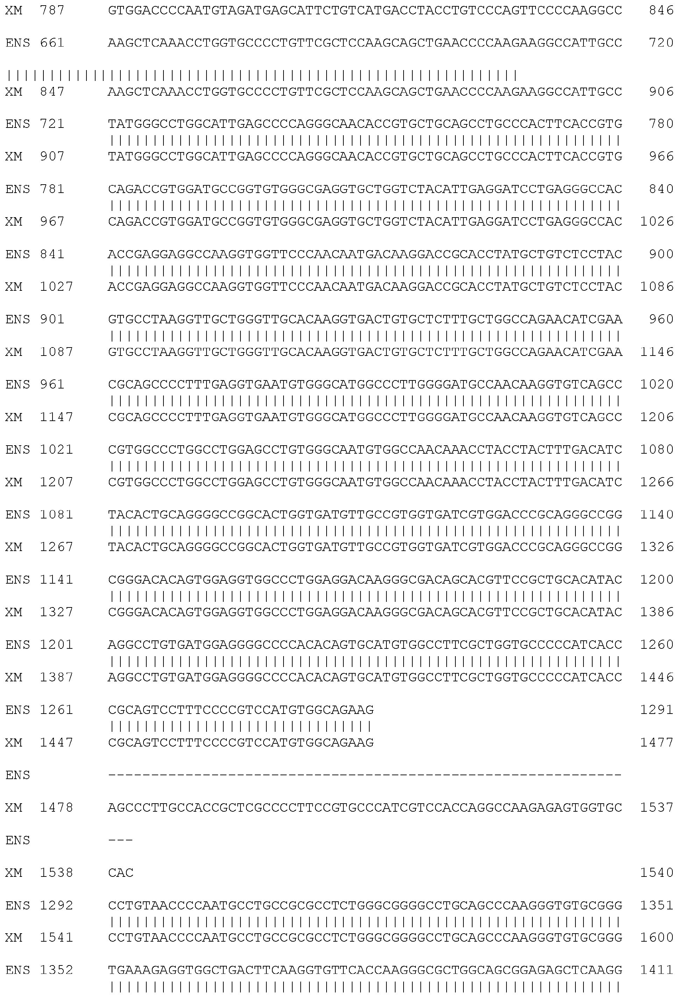

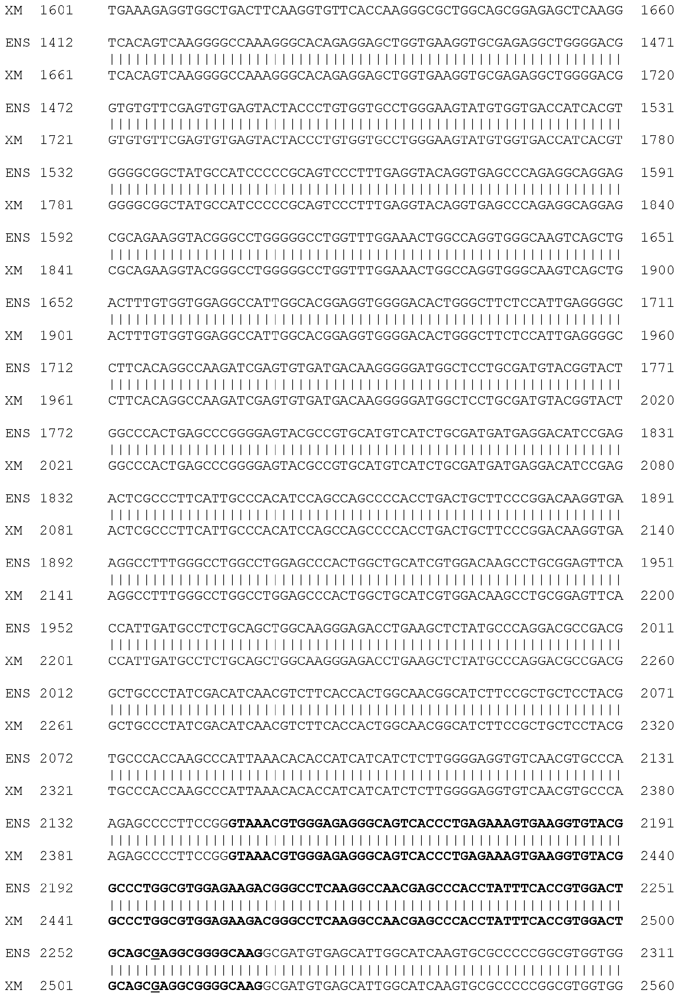

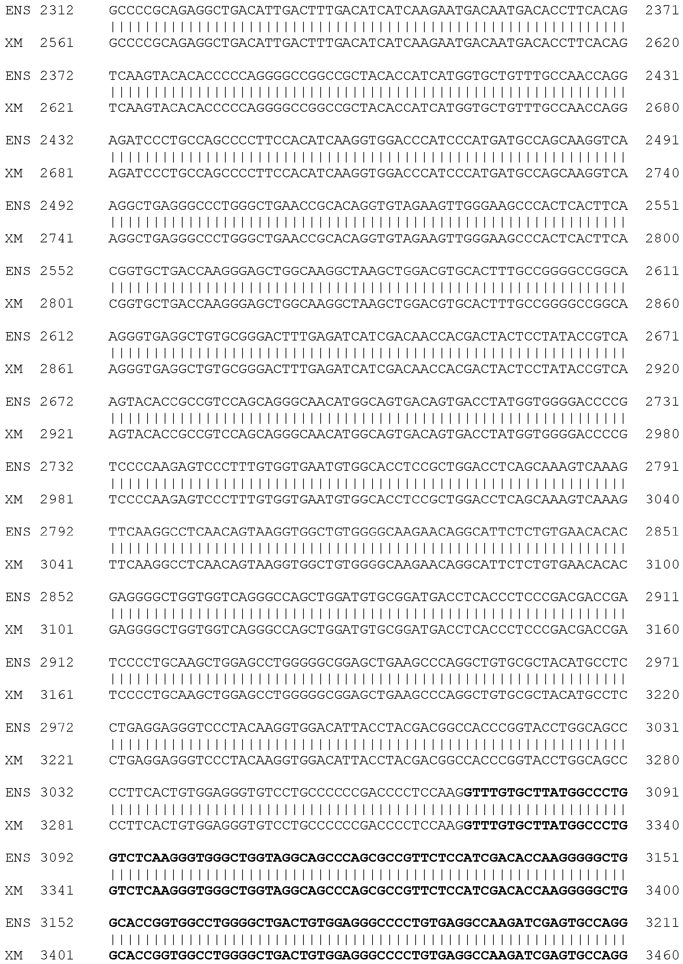

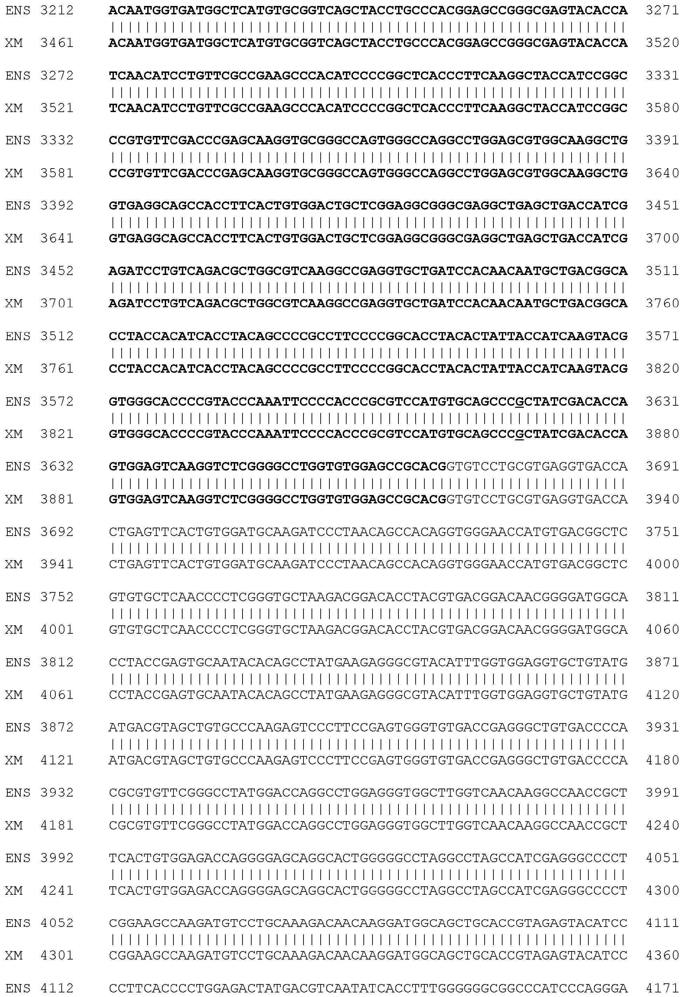

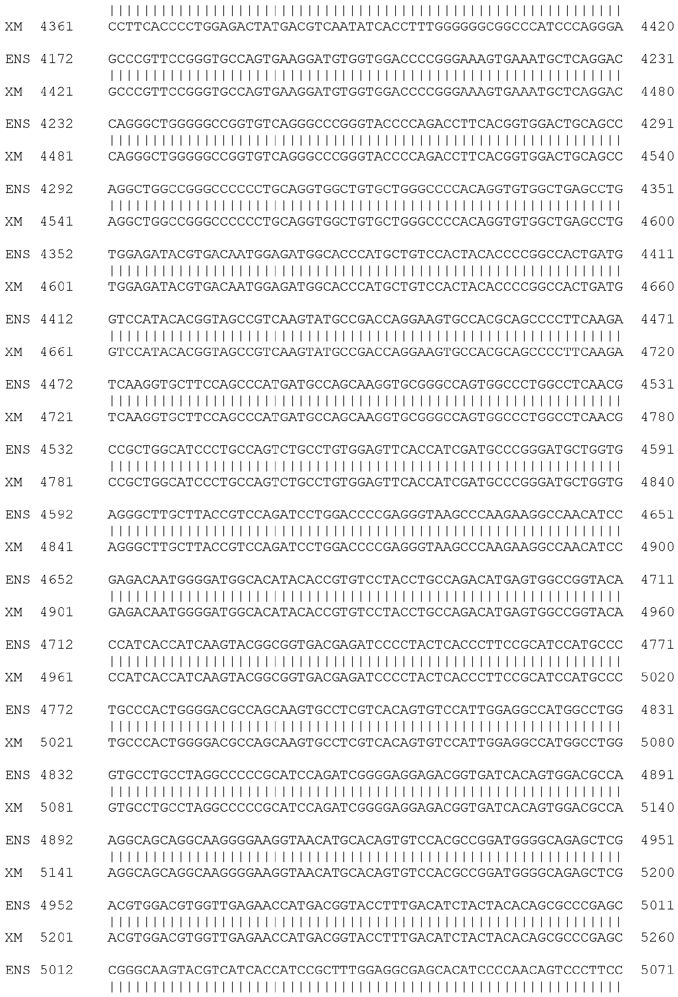

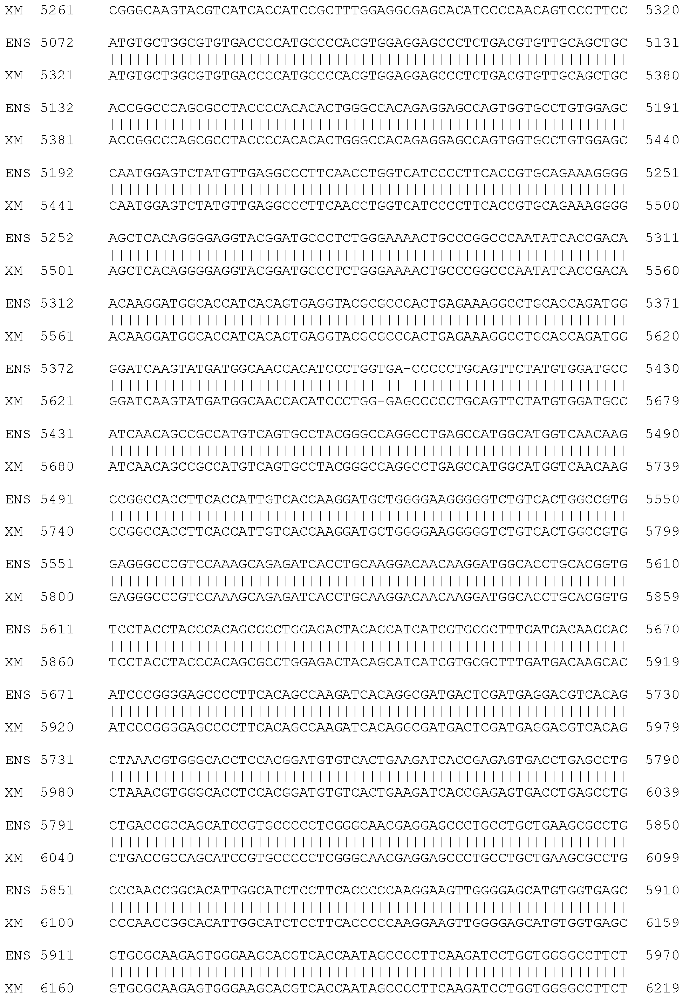

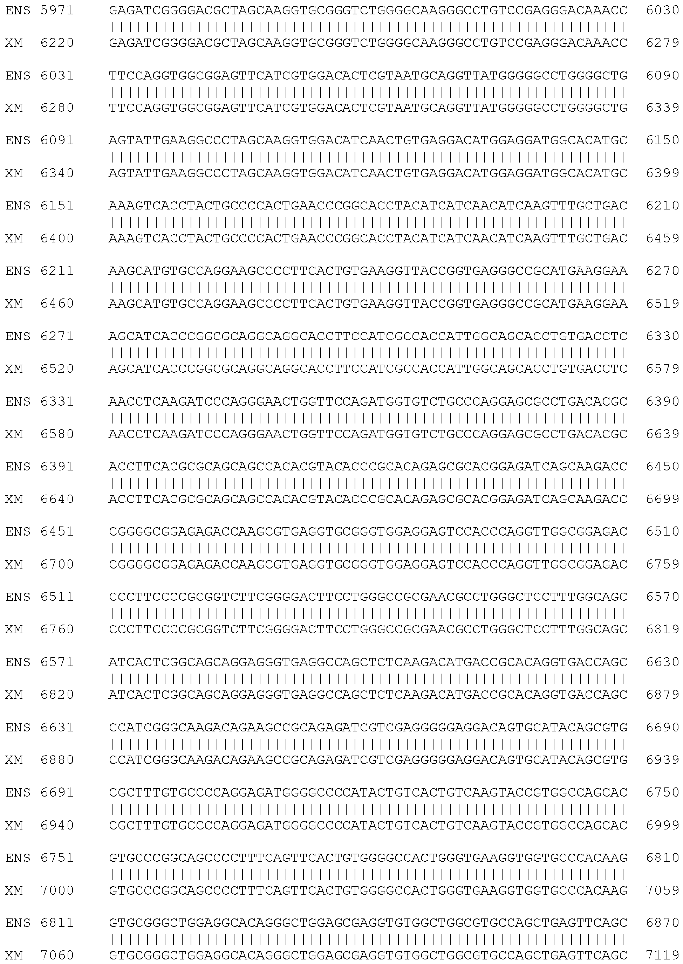

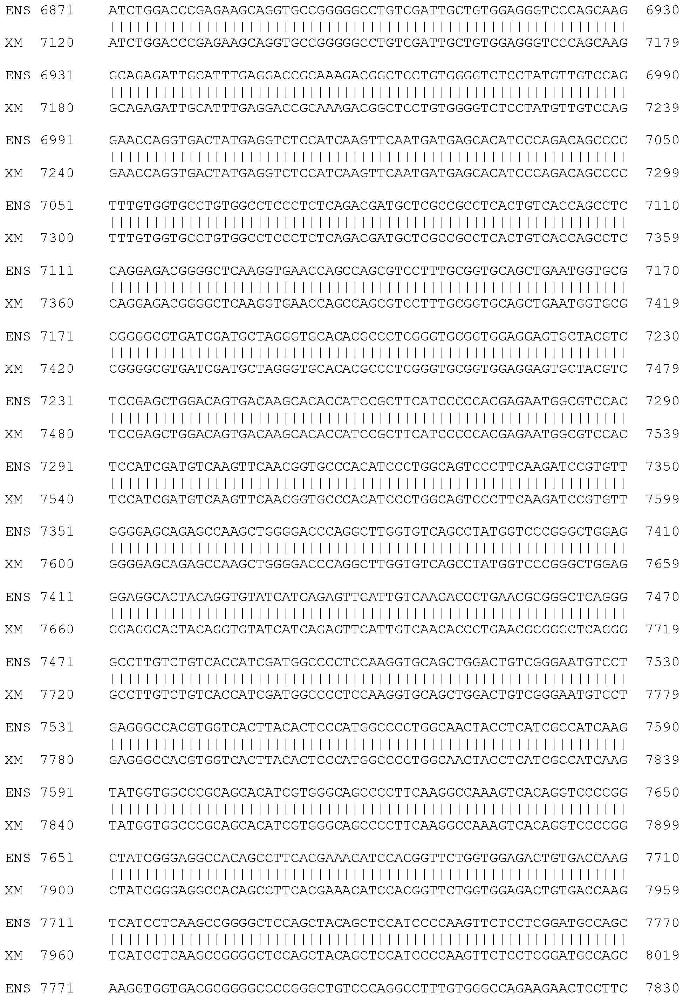

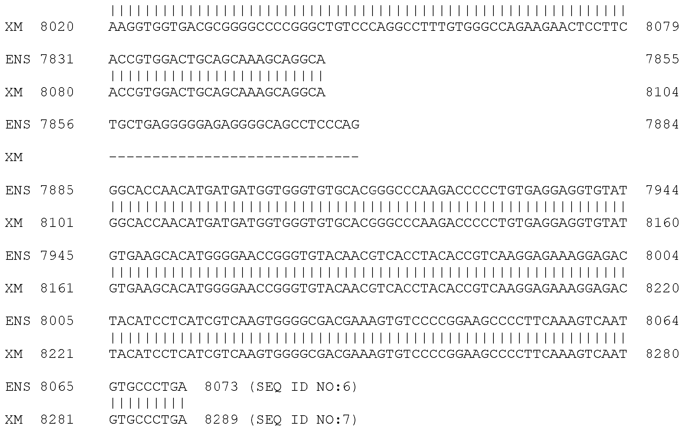

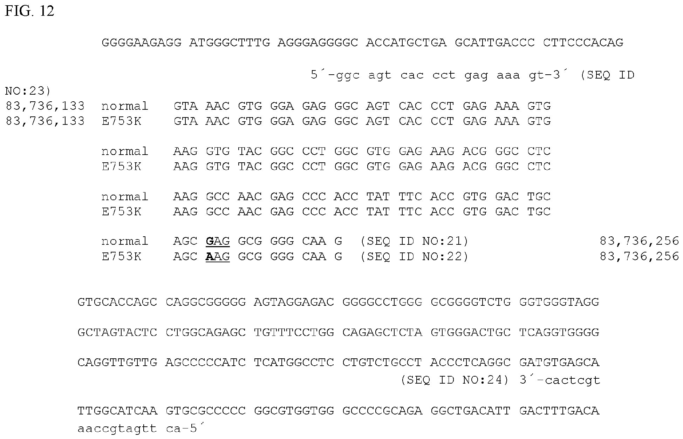

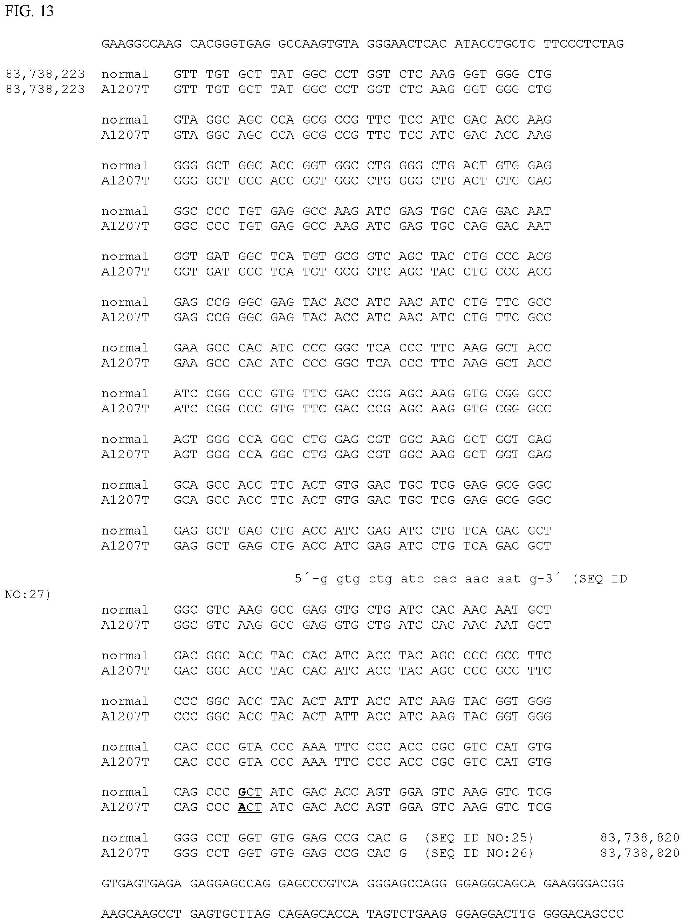

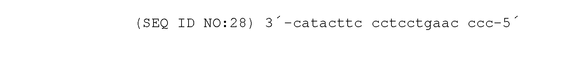

FIG. 6. Alignment of normal equine FLNC Coding DNA Sequence (SEQ ID NO:6), also known as Ensembl CDS 00000012220, and normal equine FLNC Coding DNA Sequence (SEQ ID NO:7), also known as XM_014739030.1. The sequences are identified in FIG. 6 as ENS and XM, respectively. Exons 15 and 21 are shown in bold. The sites of two G to A mutation sites, (83,736,244 in SEQ ID NO:2 as shown in FIG. 2 and 83,738,769 in SEQ ID NO:3 as shown in FIG. 3) in exons 15 and 21, at nucleotides 2257 and 3619 of SEQ ID NO:6 and nucleotides 2506 and 3868 of SEQ ID NO:7, are underlined. The regions of sequence comprising exons 15 and 21 are displayed as codons in the correct reading frame in FIG. 12 and FIG. 13.

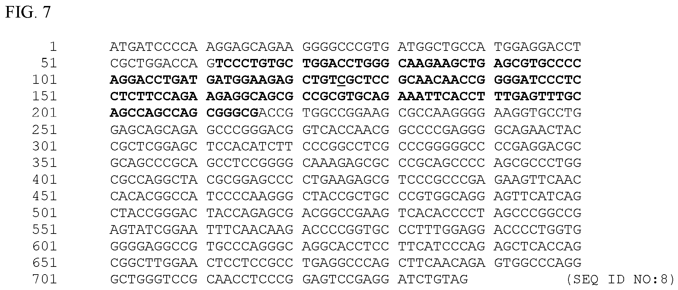

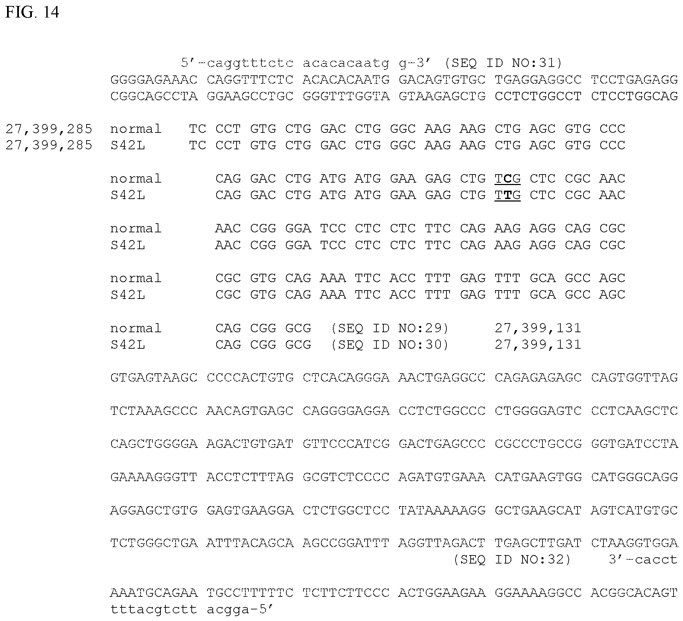

FIG. 7. Normal equine MYOZ3 Coding DNA Sequence (SEQ ID NO:8), derived from XM_014730574.1. Exon 3 is indicated in bold. The site of a C to T mutation site at nucleotide position 125 in SEQ ID NO:8 (27,399,222 of SEQ ID NO:4, as shown in FIG. 4) is underlined. The region of sequence comprising exon 3 is displayed as codons in the correct reading frame in FIG. 14.

FIG. 8. The entire MYOT coding nucleotide sequence shown in FIG. 5 was translated to give the wild-type amino acid sequence (SEQ ID NO:9) also known as XP_ 014586147.1. The amino acids encoded by exon 6 are in bold, with the site of the serine (S) to proline (P) mutation at codon 232 underlined. The MYOT-S232P amino acid sequence is also shown (SEQ ID NO:10), with the amino acids encoded by exon 6 shown in bold and the site of the serine (S) to proline (P) mutation at codon 232 underlined.

FIG. 9. The entire FLNC coding nucleotide sequences shown in FIG. 6 were translated to give these amino acid sequences (SEQ ID NO:11, also known as F6ZWZ3, and SEQ ID NO:12, also known as XP_014594516.1). The amino acids encoded by exons 15 and 21 are shown in bold. Underlining indicates the sites of the substitution of a lysine (K) for a glutamic acid (E) at position 753 in SEQ ID NO:11 and at position 836 in SEQ ID NO:12 and the substitution of a threonine (T) for an alanine (A) at position 1207 in SEQ ID NO:11 and at position 1290 in SEQ ID NO:12. The amino acid sequences of proteins with both of these amino acid substitutions are shown as SEQ ID NO:13 and SEQ ID NO:14, with the amino acids encoded by exons 15 and 21 shown in bold, and the sites of the FLNC-E753K and FLNC-A1207T substitutions (positions 753 and 1207 in SEQ ID NO:13 and positions 836 and 1290 in SEQ ID NO:14) indicated by underlining.

FIG. 10. The entire MYOZ3 coding nucleotide sequence shown in FIG. 7 was translated to give the wild-type amino acid sequence (SEQ ID NO:15), also known as XP_014586060.1 (identical to XP_005599348.1 and XP_014586061.1). The amino acids encoded by exon 3 are in bold, with the site of the serine (S) to leucine (L) mutation at codon 42 underlined. The MYOZ3-S42L amino acid sequence is shown (SEQ ID NO:16), with the amino acids encoded by exon 3 shown in bold and the site of the serine (S) to leucine (L) mutation at codon 42 underlined.

FIG. 11. Horse MYOT exon 6 and flanking genomic DNA sequence from which PCR primers to amplify genomic DNA containing the site of the MYOT-S232P mutation would be most appropriately derived. Genomic coordinates are as in FIG. 1. Exon 6 from chr14:38,519,913 to chr14:38,519,061 is shown broken into codons in the correct reading frame for the wild-type allele (SEQ ID NO:17) and the MYOT-S232P allele (SEQ ID NO:18). Only the reference sequence from the assembly is shown for the flanking sequences. The site of a A to G mutation site at nucleotide position chr14:38,519,183 is shown in bold (T to C in the reverse complement as shown). This changes the underlined three base codon from one coding for a serine (TCT) to one coding for a proline (CCT). Example primers used experimentally to amplify genomic DNA containing the mutation site are shown in lower case (SEQ ID NO: 19 and SEQ ID NO:20).

FIG. 12. Horse FLNC exon 15 and flanking genomic DNA sequence from which PCR primers to amplify genomic DNA containing the site of the FLNC-E753K mutation would be most appropriately derived. Genomic coordinates are as in FIG. 2. Exon 15 from chr4:83,736,133 to chr4:83,736,256 is shown broken into codons in the correct reading frame for the wild-type allele (SEQ ID NO:21) and the FLNC-E753K allele (SEQ ID NO:22). Only the reference sequence from the assembly is shown for the flanking sequences. The site of a G to A mutation site at nucleotide position chr4:83,736,244 is shown in bold. This mutation changes the underlined three base codon from one coding for a glutamic acid (GAG) to one coding for a lysine (AAG). Example primers used experimentally to amplify genomic DNA containing the mutation site are shown in lower case (SEQ ID NO:23 and SEQ ID NO:24).

FIG. 13. Horse FLNC exon 21 and flanking genomic DNA sequence from which PCR primers to amplify genomic DNA containing the site of the FLNC-A1207T mutation would be most appropriately derived. Genomic coordinates are as in FIG. 3. Exon 21 from chr4:83,738,223 to chr4:83,738,820 is shown broken into codons in the correct reading frame for the wild-type allele (SEQ ID NO:25) and the FLNC-A1207T allele (SEQ ID NO:26). Only the reference sequences from the assembly are shown for the flanking sequences. The site of a G to A mutation site at nucleotide position chr4:83,738,769 is shown in bold. This mutation changes the underlined three base codon from one coding for an alanine (GCT) to one coding for a threonine (ACT). Example primers used experimentally to amplify genomic DNA containing the mutation site are shown in lower case (SEQ ID NO:27 and SEQ ID NO:28).

FIG. 14. Horse MYOZ3 exon 3 and flanking genomic DNA sequence from which PCR primers to amplify genomic DNA containing the site of the MYOZ3-S42L mutation would be most appropriately derived. Genomic coordinates are as in FIG. 4. Exon 3 from chr14:27,399,285 to chr14:27,399,131 is shown broken into codons in the correct reading frame for the wild-type allele (SEQ ID NO:29) and the MYOZ3-S42L allele (SEQ ID NO:30). Only the reference sequences from the assembly are shown for the flanking sequences. The site of a G to A mutation site at nucleotide position chr14:27,399,222 is shown in bold (C to T in the reverse complement as shown). This mutation changes the underlined three base codon from one coding for a serine (TCG) to one coding for a leucine (TTG). Example primers used experimentally to amplify genomic DNA containing the mutation site are shown in lower case (SEQ ID NO:31 and SEQ ID NO:32).

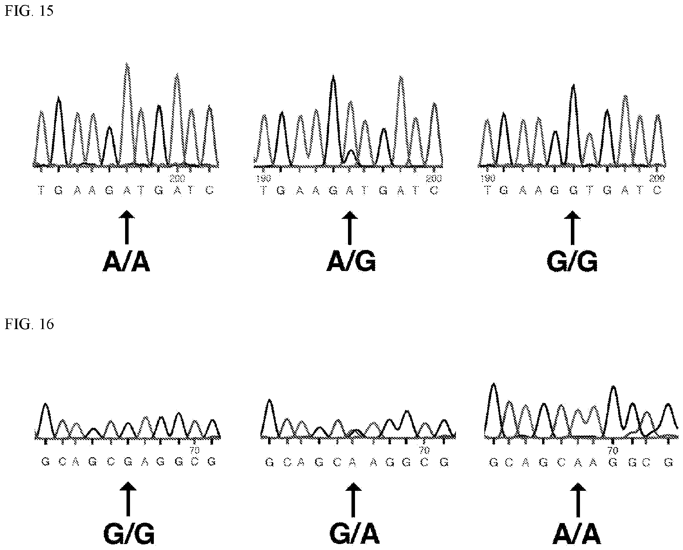

FIG. 15. Traces from Sanger DNA sequencing of amplified MYOT genomic DNA using primers shown in FIG. 11 (SEQ ID NO:19 and SEQ ID NO:20). The sequence of the forward strand is shown (SEQ ID NO:47). The arrows in the figure indicate nucleotide position chr14:38,519,183, the site of a substitution of a guanine (G) for an adenine (A) in this position that creates the MYOT-S232P variant. The traces show, from left to right, results for a horse homozygous for the wild-type or common allele, results for a horse heterozygous for the substitution, and results for a horse homozygous for the substitution.

FIG. 16. Traces from Sanger DNA sequencing of amplified FLNC genomic DNA using primers shown in FIG. 12 (SEQ ID NO:23 and SEQ ID NO:24). The sequence of the forward strand is shown (SEQ ID NO:48). The arrows in the figure indicate nucleotide position chr4:83,736,244, the site of a substitution of an adenine (A) for a guanine (G) in this position that creates the FLNC-E753K variant. The traces show, from left to right, results for a horse homozygous for the wild-type or common allele, results for a horse heterozygous for the substitution, and results for a horse homozygous for the substitution.

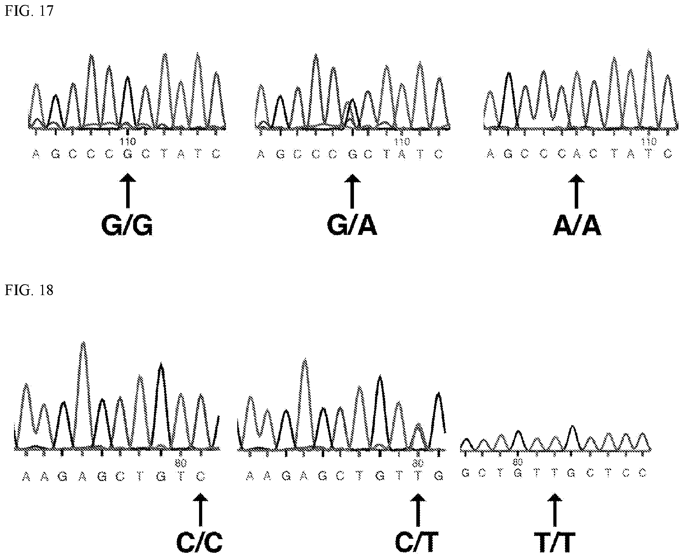

FIG. 17. Traces from Sanger DNA sequencing of amplified FLNC genomic DNA using primers shown in FIG. 13 (SEQ ID NO:27 and SEQ ID NO:28). The sequence of the forward strand is shown (SEQ ID NO:49). The arrows in the figure indicate nucleotide position chr4:83,738,769, the site of a substitution of an adenine (A) for a guanine (G) in this position that creates the FLNC-A1207T variant. The traces show, from left to right, results for a horse homozygous for the wild-type or common allele, results for a horse heterozygous for the substitution, and results for a horse homozygous for the substitution.

FIG. 18. Traces from Sanger DNA sequencing of amplified MYOZ3 genomic DNA using primers shown in FIG. 14 (SEQ ID NO:31 and SEQ ID NO:32). The sequence of the reverse strand is shown (SEQ ID NO:50). The arrows in the figure indicate nucleotide position chr14:27,399,222, the site of a substitution of a thymine (T) for a cytosine (C) in this position that creates the MYOZ3-S42L variant. The traces show, from left to right, results for a horse homozygous for the wild-type or common allele, results for a horse heterozygous for the substitution, and results for a horse homozygous for the substitution.

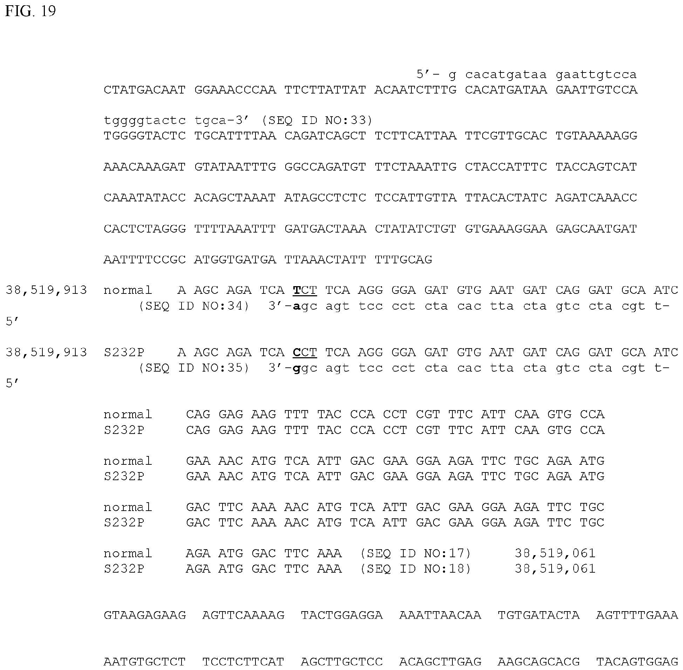

FIG. 19. Horse MYOT exon 6 and flanking genomic DNA sequence from which allele-specific PCR primers to amplify genomic DNA containing the site of the MYOT-S232P mutation would be most appropriately derived. Genomic coordinates are as in FIG. 1. Exon 6 from chr14:38,519,913 to chr14:38,519,061 is shown broken into codons in the correct reading frame for the wild-type allele (SEQ ID NO:17) and the MYOT-S232P allele (SEQ ID NO:18). Only the reference sequence from the assembly is shown for the flanking sequences. The site of a A to G mutation site at nucleotide position chr14:38,519,183 is shown in bold (T to C in the reverse complement as shown). This changes the underlined three base codon from one coding for a serine (TCT) to one coding for a proline (CCT). Example primers used experimentally to amplify genomic DNA containing the mutation site are shown in lower case. SEQ ID NO:33 is the common primer that is not allele-specific; the allele-specific primers SEQ ID NO:34 and SEQ ID NO:35 preferentially amplify the wild-type and MYOT-S232P alleles, respectively. Reaction conditions are described in the text.

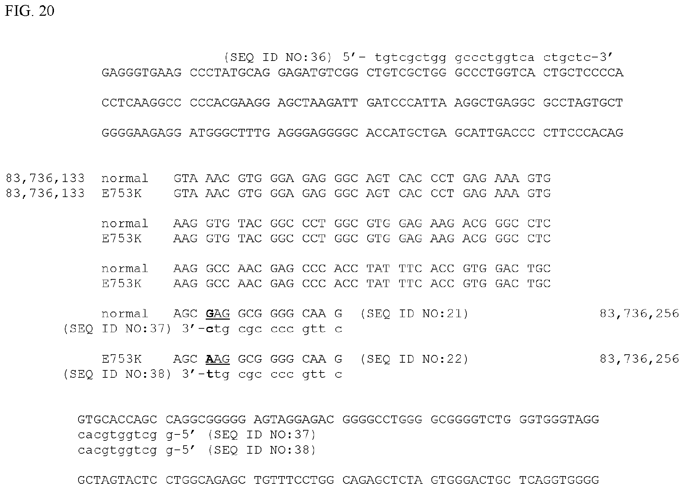

FIG. 20. Horse FLNC exon 15 and flanking genomic DNA sequence from which allele-specific PCR primers to amplify genomic DNA containing the site of the FLNC-E753K mutation would be most appropriately derived. Genomic coordinates are as in FIG. 2. Exon 15 from chr4:83,736,133 to chr4:83,736,256 is shown broken into codons in the correct reading frame for the wild-type allele (SEQ ID NO:21) and the FLNC-E753K allele (SEQ ID NO:22). Only the reference sequence from the assembly is shown for the flanking sequences. The site of a G to A mutation site at nucleotide position chr4:83,736,244 is shown in bold. This mutation changes the underlined three base codon from one coding for a glutamic acid (GAG) to one coding for a lysine (AAG). Example primers used experimentally to amplify genomic DNA containing the mutation site are shown in lower case. SEQ ID NO:36 is the common primer that is not allele-specific; the allele-specific primers SEQ ID NO:37 and SEQ ID NO:38 preferentially amplify the wild-type and FLNC-E753K alleles, respectively. Note that both allele-specific primers span the exon-intron boundary. Note also that additional mismatches have been introduced into both allele-specific primers. Reaction conditions are described in the text.

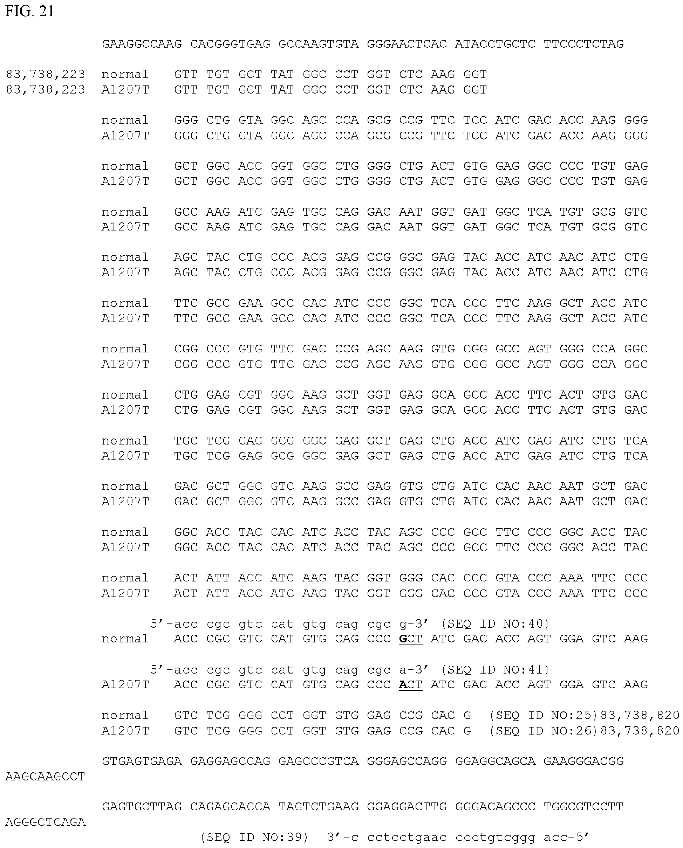

FIG. 21. Horse FLNC exon 21 and flanking genomic DNA sequence from which allele-specific PCR primers to amplify genomic DNA containing the site of the FLNC-A1207T mutation would be most appropriately derived. Genomic coordinates are as in FIG. 3. Exon 21 from chr4:83,738,223 to chr4:83,738,820 is shown broken into codons in the correct reading frame for the wild-type allele (SEQ ID NO:25) and the FLNC-A1207T allele (SEQ ID NO:26). Only the reference sequences from the assembly are shown for the flanking sequences. The site of a G to A mutation site at nucleotide position chr4:83,738,769 is shown in bold. This mutation changes the underlined three base codon from one coding for an alanine (GCT) to one coding for a threonine (ACT). Example primers used experimentally to amplify genomic DNA containing the mutation site are shown in lower case. SEQ ID NO:39 is the common primer that is not allele-specific; the allele-specific primers SEQ ID NO:40 and SEQ ID NO:41 preferentially amplify the wild-type and FLNC-A1207T alleles, respectively. Note that additional mismatches have been introduced into both allele-specific primers. Reaction conditions are described in the text.

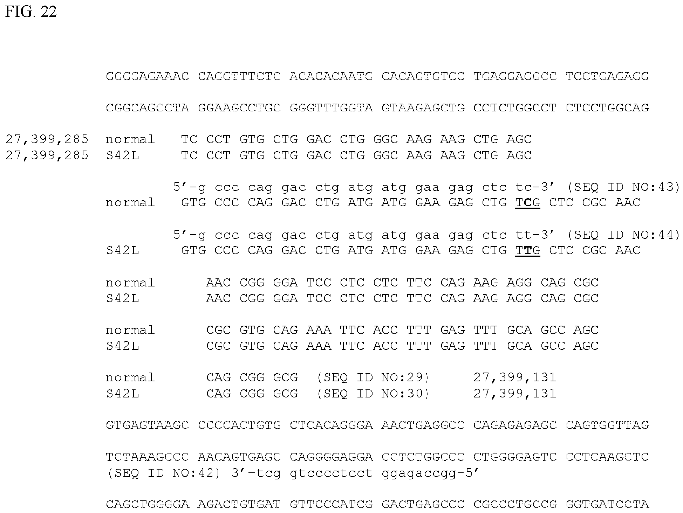

FIG. 22. Horse MYOZ3 exon 3 and flanking genomic DNA sequence from which allele-specific PCR primers to amplify genomic DNA containing the site of the MYOZ3-S42L mutation would be most appropriately derived. Genomic coordinates are as in FIG. 4. Exon 3 from chr14:27,399,285 to chr14:27,399,131 is shown broken into codons in the correct reading frame for the wild-type allele (SEQ ID NO:29) and the MYOZ3-S42L allele (SEQ ID NO:30). Only the reference sequences from the assembly are shown for the flanking sequences. The site of a G to A mutation site at nucleotide position chr14:27,399,222 is shown in bold (C to T in the reverse complement as shown). This mutation changes the underlined three base codon from one coding for a serine (TCG) to one coding for a leucine (TTG). Example primers used experimentally to amplify genomic DNA containing the mutation site are shown in lower case. SEQ ID NO:42 is the common primer that is not allele-specific; the allele-specific primers SEQ ID NO:43 and SEQ ID NO:44 preferentially amplify the wild-type and MYOZ3-S42L alleles, respectively. Note that additional mismatches have been introduced into both allele-specific primers. Reaction conditions are described in the text.

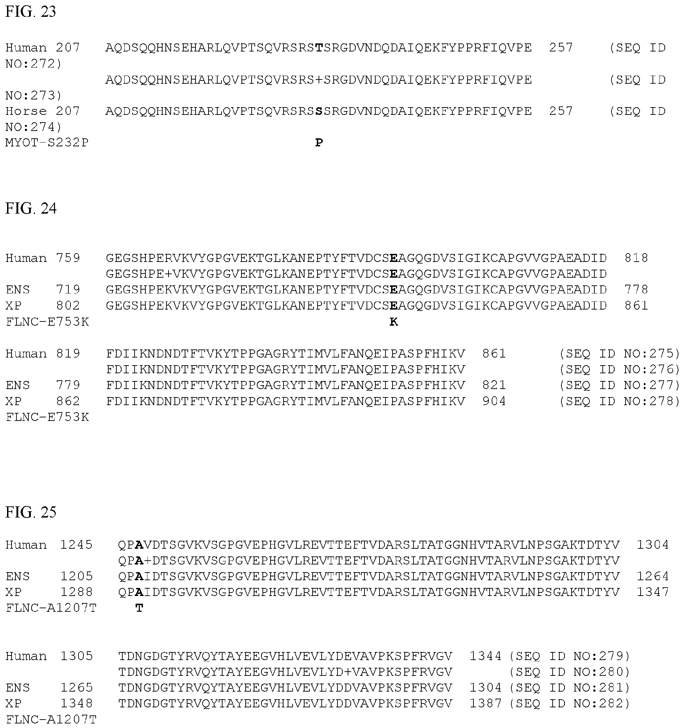

FIG. 23. Alignment of the sequence of a portion of the human MYOT protein with the horse protein sequence SEQ ID NO:9 shown in FIG. 8. The top line (indicated as Human; SEQ ID NO:45) corresponds to a portion of the human myotilin protein (MYOT) from UniProt Q9UBF9. The second line shows the alignment of the human sequence to the horse sequence (SEQ ID NO:46). A single conservative amino acid substitution is seen at amino acid 232. The last line, indicated as MYOT-S232P, shows the position of the S232P nonconservative substitution, at the same position as the conservative substitution between human and horse.

FIG. 24. Alignment of the sequence of filamin repeat 6 of the human FLNC protein with the horse protein sequences SEQ ID NO:11 and SEQ ID NO:12 shown in FIG. 9. The top line (indicated as Human; SEQ ID NO:272) corresponds to filamin repeat 6 of human filamin-C protein (FLNC) from UniProt Q14315. The second line (indicated as ENS; SEQ ID NO:273) corresponds to filamin repeat 6 of horse filamin-C protein (FLNC) with the numbering of amino acid positions as in SEQ ID NO: 11. A single conservative amino acid substitution between human and horse is seen at amino acid 766 in the human sequence. The third line (indicated as XP; SEQ ID NO: 274) corresponds to filamin repeat 6 of horse filamin-C protein (FLNC) with the numbering of amino acid positions as in SEQ ID NO:12. The last line (indicated as E753K) shows the position of the E753K substitution.

FIG. 25. Alignment of the sequence of filamin repeat 11 of the human FLNC protein with the horse protein sequences SEQ ID NO:11 and SEQ ID NO:12 shown in FIG. 9. The top line (indicated as Human; SEQ ID NO:275) corresponds to filamin repeat 11 of human filamin-C protein (FLNC) from UniProt Q14315. The second line (indicated as ENS; SEQ ID NO:276) corresponds to filamin repeat 11 of horse filamin-C protein (FLNC) with the numbering of amino acid positions as in SEQ ID NO:11. Two conservative amino acid substitutions between human and horse are seen at amino acids 1248 and 1332 in the human sequence. The third line (indicated as XP; SEQ ID NO:277) corresponds to filamin repeat 11 of horse filamin-C protein (FLNC) with the numbering of amino acid positions as in SEQ ID NO:12. The last line (indicated as A1207T) shows the position of the A1207T substitution.

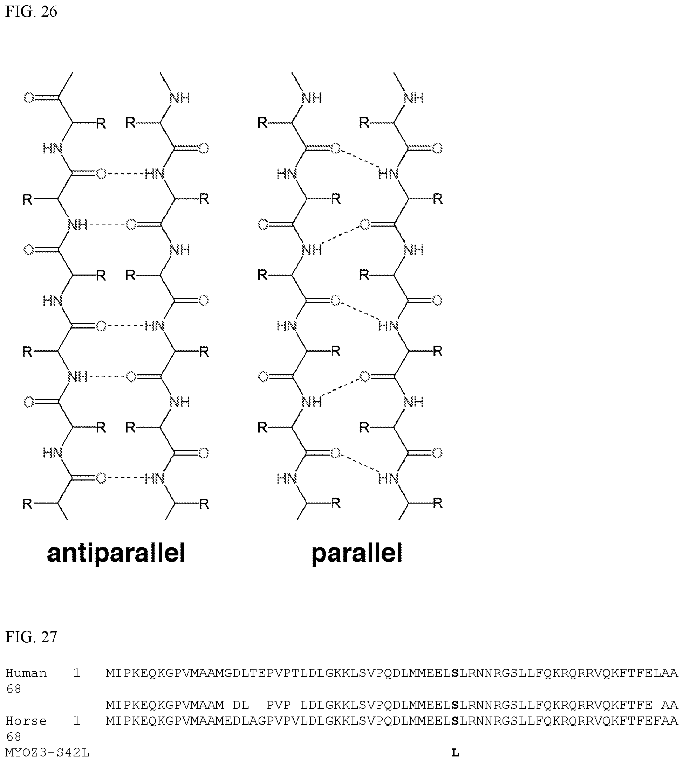

FIG. 26. Comparison of antiparallel and parallel beta sheet protein structures. Beta sheets are held together by hydrogen bonding between N-H groups in the backbone of one strand and the C=O groups in the backbone of the adjacent strand. In an antiparallel beta sheet, the adjacent strands have opposite polarity with respect to the N- and C-termini. In a parallel beta sheet, the adjacent strands have the same polarity with respect to the N- and C-termini. Comparison of the two structures shows that R groups are in close opposition in an antiparallel beta sheet, while R groups in a parallel beta sheet occupy the space between the N-H group and the C=O group of the adjacent strand.

FIG. 27. Alignment of the sequence of a portion of the human MYOZ3 protein with the horse protein sequence SEQ ID NO:15 shown in FIG. 10. The top line (indicated as Human; SEQ ID NO:278) corresponds to a portion of the human myozenin-3 protein (MYOZ3) from UniProt Q8TDC0. The second line shows the alignment of the human sequence to the horse sequence (SEQ ID NO:279). Five nonconservative substitutions are seen at positions 14, 17, 18, 22, and 66. The last line, indicated as MYOZ3-S42L, shows the position of the S42L nonconservative substitution.

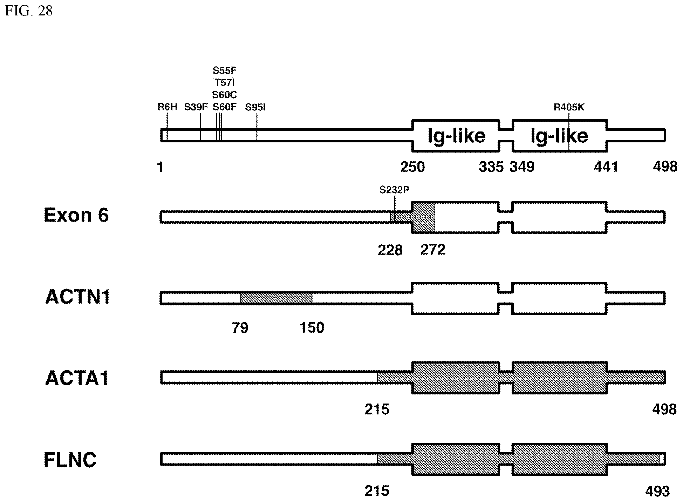

FIG. 28. Features of the human MYOT protein. The top line shows a linear representation of the 498 amino acid human myotilin protein (UniProt Q9UBF9). The locations of pathogenic amino acid substitutions summarized in TABLE 1 are indicated. The second line shows the amino acids encoded by exon 6 (228 to 272), with the position of the equine MYOT-S323P mutation indicated. The third line shows the region (79 to 150) that has been shown to interact with alpha-actinin (ACTN1). The fourth line shows the region (215 to 498) that has been shown to interact with actin (ACTA1). The last line shows the region (215 to 493) that has been shown to interact with filamin-C (FLNC).

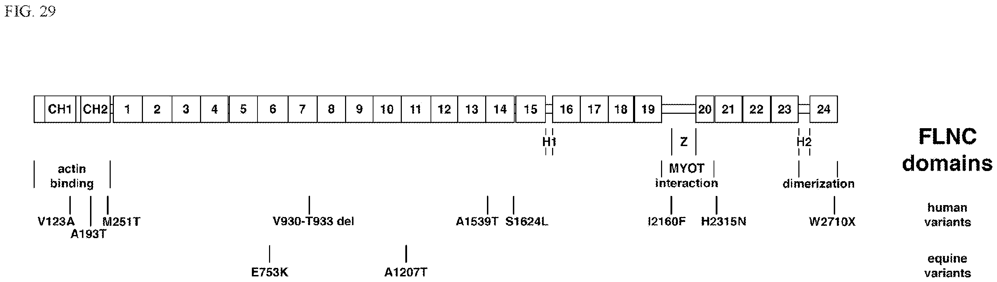

FIG. 29. Features of the human FLNC protein. The top line shows a linear representation of the 2725 amino acid human filamin-C protein (UniProt Q14315) with key features indicated. The actin binding domain with domains CH1 and CH2 is located at the amino terminus. Most of the molecule consists of filamin repeats, numbered 1-24. There are two hinge domains, H1 and H2. Between filamin repeat 19 and the partial filamin repeat 20 is an 82 amino acid region not found in filamin A or filamin B that is required for localization to the Z disc and for interaction with myotilin. The carboxy-terminal region including H2 and filamin repeat 24 is required for dimerization. The locations of pathogenic amino acid substitutions found in human patients and summarized in TABLE 2 are indicated (human variants). The locations of amino acid substitutions found in horses with Polysaccharide Storage Myopathy type 2 (PSSM2), also known as Myofibrillar Myopathy (MFM), are shown in the second line (equine variants). The substitution shown in FIG. 24 is indicated as E753K while the substitution shown in FIG. 25 is indicated as A1207T. The amino acid positions affected by the E753K and A1207T variants in horse correspond to positions 793 and 1247 in the human FLNC sequence represented by Q14315.

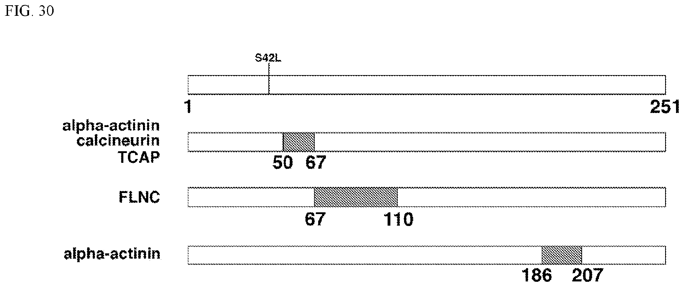

FIG. 30. Features of the human MYOZ3 protein. The top line shows a linear representation of the 251 amino acid human myozenin-3 protein (UniProt Q8TDC0). No pathogenic human alleles are known. The location of the equine MYOZ3-S42L is shown. The second line shows a region of the human MYOZ3 protein shown to bind the alpha-actinin (ACTN1), calcineurin, and telethonin (TCAP) proteins. Calcineurin is a calcium- and calmodulin-dependent serine/-threonine protein phosphatase made up of one calmodulin-binding catalytic subunit encoded by three different genes (PPP3CA, PPP3CB, and PPP3CC) and a one regulatory subunit encoded by two different genes (PPP3R1 and PPP3R2). The third line shows a region of the human MYOZ3 protein shown to bind filamin-C (FLNC) protein. The fourth line shows a second region of the human MYOZ3 protein shown to bind alpha-actinin (ACTN1) protein.

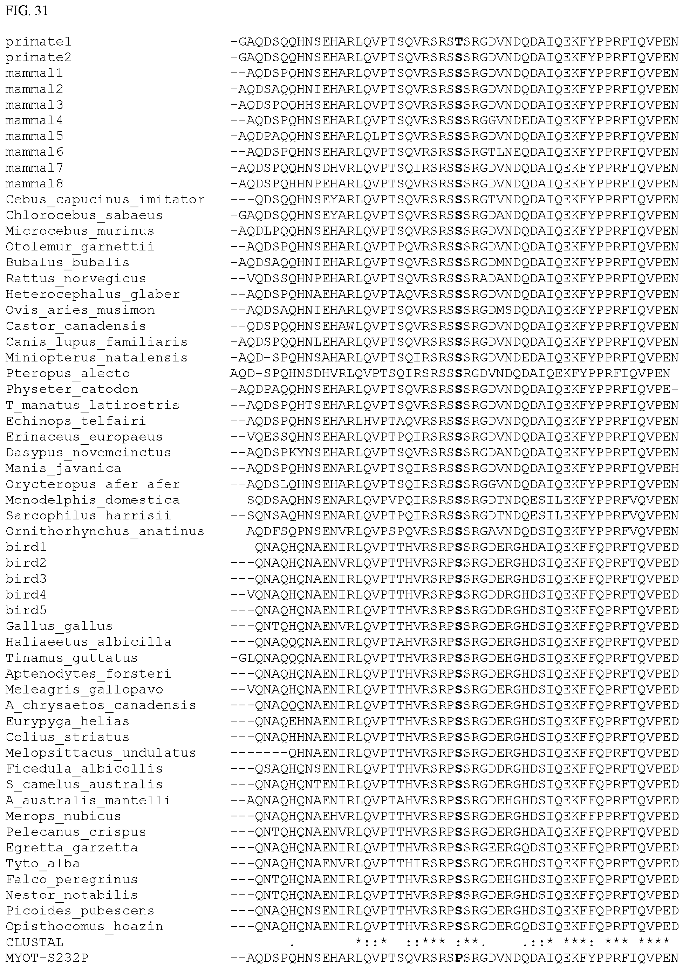

FIG. 31. Amino acid sequences (SEQ ID NO: 51-108) of proteins encoded by MYOT genes, centered on the position of the equine MYOT-S232P substitution. Species included in the analysis are described in the text. The next to the last line (labeled CLUSTAL) shows the consensus sequence, where positions with fully conserved amino acids are represented by an asterisk (*), positions with strongly conserved amino acids are indicated by a colon (:), positions with weakly conserved amino acids are indicated are indicated by period (.), and nonconserved positions are indicated by a blank space ( ). The last line shows the sequence of myotilin in horse with the MYOT-S232P substitution shown and highlighted in bold. The position of the MYOT-S232P substitution is indicated in bold in all of the sequences.

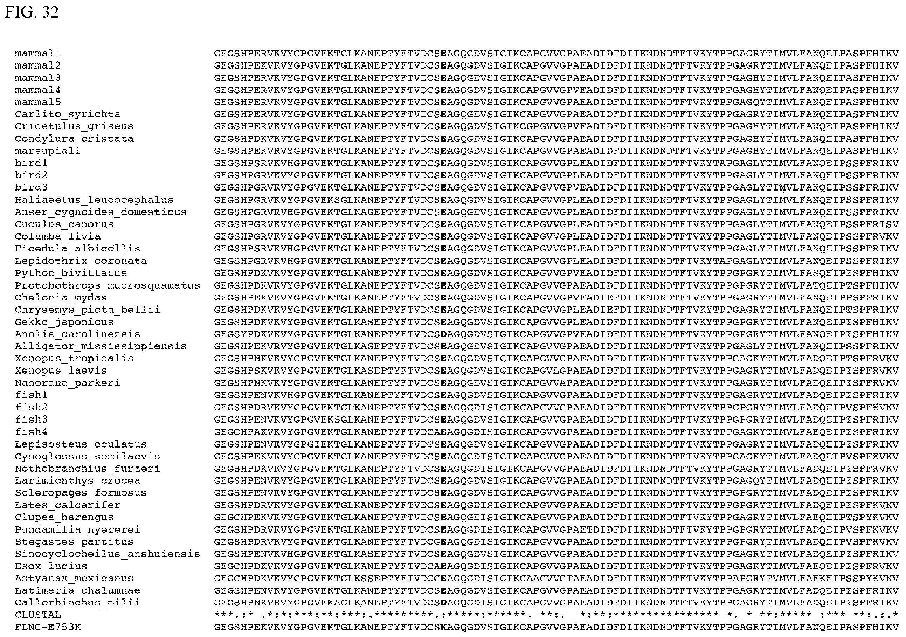

FIG. 32. Amino acid sequences (SEQ ID NO:109-155) of proteins encoded by FLNC genes, showing filamin repeat 6, which contains the equine FLNC-E753K substitution. Species included in the analysis are described in the text. The next to the last line (labeled CLUSTAL) shows the consensus sequence, where positions with fully conserved amino acids are represented by an asterisk (*), positions with strongly conserved amino acids are indicated by a colon (:), positions with weakly conserved amino acids are indicated are indicated by period (.), and nonconserved positions are indicated by a blank space ( ). The last line shows the sequence of filamin repeat 6 in horse with the FLNC-E753K substitution shown and highlighted in bold. The position of the FLNC-E753K substitution is indicated in bold in all of the sequences.

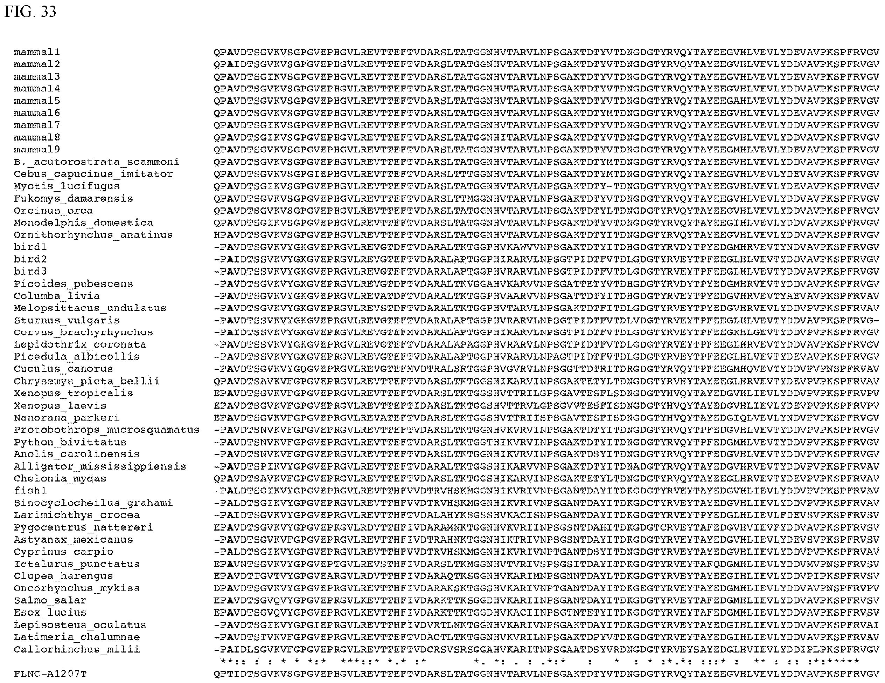

FIG. 33. Amino acid sequences (SEQ ID NO:156-205) of proteins encoded by FLNC genes, showing filamin repeat 11, which contains the equine FLNC-A1207T substitution. Species included in the analysis are described in the text. The next to the last line (labeled CLUSTAL) shows the consensus sequence, where positions with fully conserved amino acids are represented by an asterisk (*), positions with strongly conserved amino acids are indicated by a colon (:), positions with weakly conserved amino acids are indicated are indicated by period (.), and nonconserved positions are indicated by a blank space ( ). The last line shows the sequence of filamin repeat 11 in horse with the FLNC-A1207T substitution shown and highlighted in bold. The position of the FLNC-A1207T substitution is indicated in bold in all of the sequences.

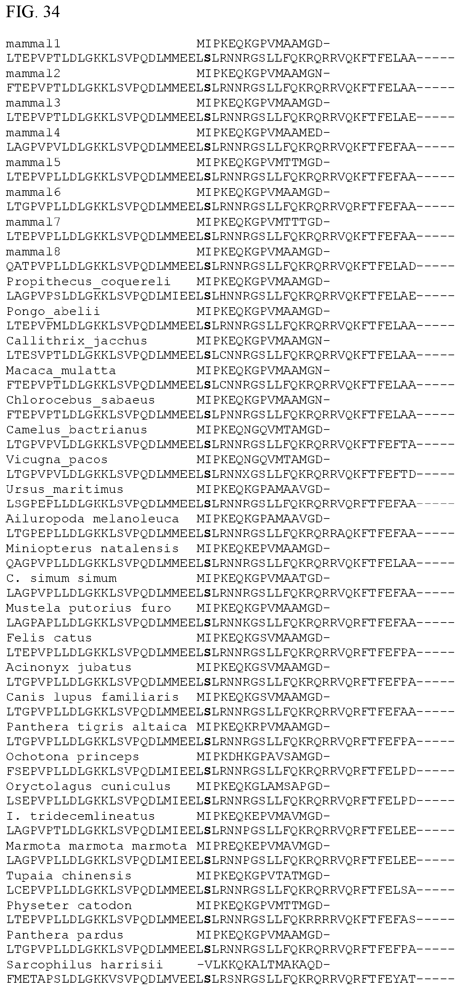

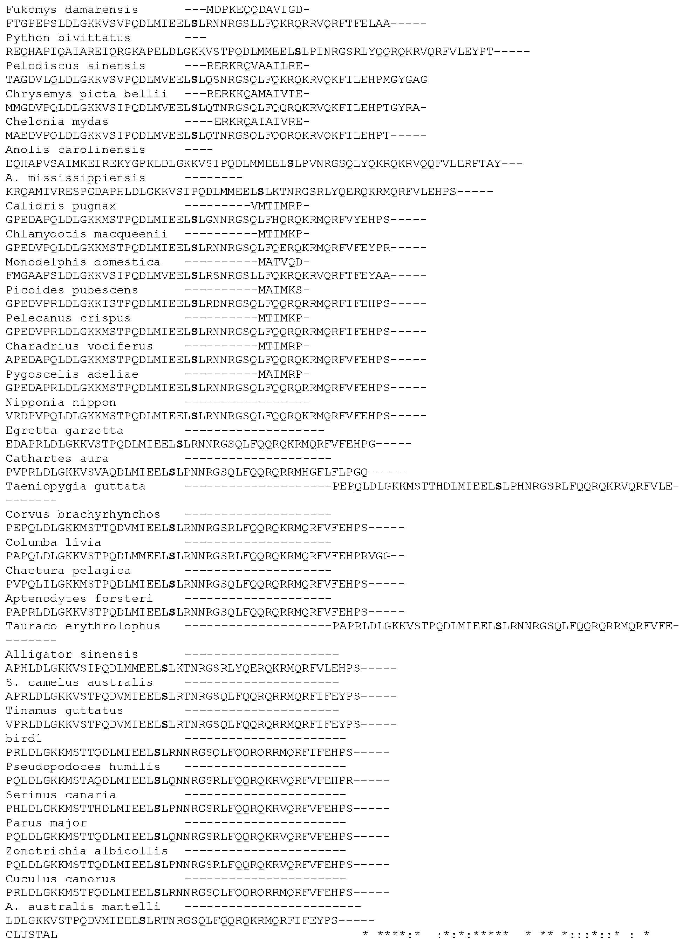

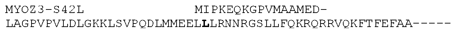

FIG. 34. Amino acid sequences (SEQ ID NO:206-271) of proteins encoded by MYOZ3 genes, centered on the position of the equine MYOZ3-S42L substitution. Species included in the analysis are described in the text. The next to the last line (labeled CLUSTAL) shows the consensus sequence, where positions with fully conserved amino acids are represented by an asterisk (*), positions with strongly conserved amino acids are indicated by a colon (:), positions with weakly conserved amino acids are indicated are indicated by period (.), and nonconserved positions are indicated by a blank space ( ). The last line shows the sequence of myozenin-3 in horse with the MYOZ3-S42L substitution shown and highlighted in bold. The position of the MYOZ3-S42L substitution is indicated in bold in all of the sequences.

DETAILED DESCRIPTION OF ILLUSTRATIVE EMBODIMENTS

The invention is as defined in the claims and to the extent that other methods are described herein, they are included merely for reference purposes.

This disclosure describes methods for detecting the presence or absence of biomarkers associated with inherited equine myopathies. These disease conditions have been variously referred to Polysaccharide Storage Myopathy, type 2 (PSSM2), Myofibrillar Myopathy (MFM), or idiopathic myopathy. The term PSSM2 is commonly used to describe horses that show exercise intolerance, a negative test result for the GYS1-R309H variant of Glycogen Synthase 1 that is associated with Polysaccharide Storage Myopathy, type 1 (PSSM1), and abnormal findings on muscle biopsy, including abnormally shaped muscle fibers, nuclei displaced to the center of muscle fibers rather than the normal position at the edge of fibers, and pools of glycogen granules of normal size in regions of disorganization that give the false appearance of a glycogen storage disease. Myofibrillar Myopathy is a subtype of PSSM2 characterized by protein aggregates displaced from the Z disc that stain positive for desmin, a protein component of the Z disc. In the absence of the immunological stain for desmin, muscle biopsies of this type are simply scored as PSSM2. In one embodiment, the method involves obtaining a physiological sample from a horse and determining whether the biomarker is present in the sample. As used herein, the phrase "physiological sample" refers to a biological sample obtained from a horse that contains nucleic acid. For example, a physiological sample can be a sample collected from an individual horse such as, for example, a cell sample, such as a blood cell, e.g., a lymphocyte, a peripheral blood cell; a sample collected from the spinal cord; a tissue sample such as cardiac tissue or muscle tissue, e.g., cardiac or skeletal muscle; an organ sample, e.g., liver or skin; a hair sample, e.g., a hair sample with roots; and/or a fluid sample, such as blood.

Examples of breeds of affected horse include, but are not limited to, Quarter Horses, Percheron Horses, Paint Horses, Draft Horses, Warmblood Horses, or related or unrelated breeds. The phrase "related breed" is used herein to refer to breeds that are related to a breed, such as Quarter Horse, Draft Horse, or Warmblood Horse. Such breeds include, but are not limited to stock breeds such as the American Paint horse, the Appaloosa, and the Palomino. The term "Draft Horse" includes many breeds including but not limited to Clydesdale, Belgian, Percheron, and Shire horses. The term "Warmblood" is also a generic term that includes a number of different breeds. "Warmblood" simply distinguishes this type of horse from the "cold bloods" (draft horses) and the "hot bloods" (Thoroughbreds and Arabians). The method described herein also may be performed using a sample obtained from a crossed or mixed breed horse.

The term "biomarker" is generally refers herein to a biological indicator, such as a particular molecular feature, that may affect, may be an indicator, and/or be related to diagnosing or predicting an individual's health. For example, in certain embodiments, the biomarker can refer to (1) a mutation in the equine myotilin (MYOT) coding region (SEQ ID NO: 1), such as a polymorphic allele of MYOT that has a substitution of a guanine (G) for an adenine (A) at nucleotide position 38,519,183 on the forward strand of SEQ ID NO: 1, (2) a mutation of the equine filamin-C (FLNC) coding region (SEQ ID NO:2 and SEQ ID NO: 3), such as a polymorphic allele of FLNC that has a substitution of an adenine (A) for a guanine (G) at nucleotide position 83,736,244 on the forward strand of SEQ ID NO:2 or a substitution of an adenine (A) for a guanine (G) at nucleotide position 83,738,769 on the forward strand of SEQ ID NO:3, or (3) a mutation of the equine myozenin-3 (MYOZ3) coding region, such as a polymorphic allele of MYOZ3 that has a substitution of an adenine (A) for a guanine (G) at nucleotide position 27,399,222 on the forward strand of SEQ ID NO:4. In each of these cases, the specified nucleotide substitution may be inferred by the detection of the complementary base on the reverse strand.

"Oligonucleotide probe" can refer to a nucleic acid segment, such as a primer, that is useful to amplify a sequence in the MYOT, FLNC, or MYOZ3 coding regions that are complementary to, and hybridizes specifically to, a particular nucleotide sequence in MYOT, FLNC, or MYOZ3, or to a nucleic acid region that flanks MYOT, FLNC, or MYOZ3.

As used herein, the term "nucleic acid" and "polynucleotide" refers to deoxyribonucleotides or ribonucleotides and polymers thereof in either single-stranded or double-stranded form. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues.

A "nucleic acid fragment" is a portion of a given nucleic acid molecule. Deoxyribonucleic acid (DNA) in the majority of organisms is the genetic material while ribonucleic acid (RNA) is involved in the transfer of information contained within DNA into proteins. The term "nucleotide sequence" refers to DNA or RNA that can be single-stranded or double-stranded, optionally containing synthetic, non-natural, or altered nucleotide bases capable of incorporation into DNA or RNA.

In some embodiments, the method can involve contacting the sample with at least one oligonucleotide probe to form a hybridized nucleic acid and then amplifying the hybridized nucleic acid. "Amplifying" utilizes methods such as the polymerase chain reaction (PCR), ligation amplification (or ligase chain reaction, LCR), strand displacement amplification, nucleic acid sequence-based amplification, and amplification methods based on the use of Qβ-replicase. These methods are well known and widely practiced in the art. Reagents and hardware for conducting PCR are commercially available. For example, in certain embodiments, exon 6 of the equine myotilin coding region (also referred to as MYOT), exons 15 and 21 of the equine filamin-C coding region (also referred to as FLNC), or exon 3 of the equine myozenin-3 coding region (also referred to as MYOZ3) or portions thereof, may be amplified by PCR. In another embodiment, at least one oligonucleotide probe is immobilized on a solid surface or a semisolid surface.

The methods described herein can be used to detect the presence or absence of a biomarker associated with equine Polysaccharide Storage Myopathy type 2 (PSSM2), also known as Myofibrillar Myopathy (MFM), in a horse (live or dead) regardless of age (e.g., an embryo, a foal, a neonatal foal, aborted foal, a breeding-age adult, or any horse at any stage of life) or sex (e.g., a mare (dam) or stallion (sire)).

As used herein, the term "presence or absence" refers to affirmatively detecting the presence of a biomarker or detecting the absence of the biomarker within the experimental limits of the detection methods used to detect the biomarker.

This disclosure further provides a method for detecting and/or diagnosing Polysaccharide Storage Myopathy type 2 (PSSM2), also known as Myofibrillar Myopathy (MFM), in a horse, the method involving detecting in a physiological sample from the horse the presence or absence of biomarkers in the sample, wherein the presence of the biomarkers is indicative of the disease. One embodiment of the method further involves contacting the sample with at least one oligonucleotide probe to form a hybridized nucleic acid and amplifying the hybridized nucleic acid. For example, in one embodiment, exon 6 of equine MYOT, exons 15 and 21 of equine FLNC, or exon 3 of equine MYOZ3 (or portions thereof) are amplified using, for example, polymerase chain reaction, strand displacement amplification, ligase chain reaction, amplification methods based on the use of Qβ-replicase and/or nucleic acid sequence-based amplification. In one embodiment of the method, the biomarkers can include (1) an equine myotilin (MYOT) coding region having an A to G substitution on the forward strand at nucleotide 38,519,183 of SEQ ID NO: 1, (2) an equine filamin-C (FLNC) coding region having a G to A substitution on the forward strand at nucleotide 83,736,244 of SEQ ID NO:2 or a G to A substitution on the forward strand at nucleotide 83,738,769 of SEQ ID NO: 3, or (3) an equine myozenin-3 (MYOZ3) coding region having a G to A substitution on the forward strand at nucleotide 27,399,222 of SEQ ID NO:4. Biomarkers can also include (1) a coding region that encodes a myotilin (MYOT) polypeptide (SEQ ID NO:9) having a Serine-to-Proline (S to P) substitution at amino acid residue 232 of SEQ ID NO:9, as shown in SEQ ID NO:10, (2) a coding region that encodes a filamin-C (FLNC) polypeptide (SEQ ID NO:11) having an Glutamic Acid-to-Lysine (E-to-K) substitution at amino acid residue 753 (equivalent to amino acid residue 836 in SEQ ID NO: 12), as shown in SEQ ID NO:13 (equivalent to SEQ ID NO:14), or an Alanine-to-Threonine (A-to-T) substitution at amino acid residue 1207 (equivalent to amino acid residue 1290 in SEQ ID NO:12), as shown in SEQ ID NO:13 (equivalent to SEQ ID NO:14), or (3) a coding region that encodes a myozenin-3 (MYOZ3) polypeptide (SEQ ID NO:15) having a Serine-to-Leucine (S-to-L) substitution at amino acid residue 42 of SEQ ID NO15, as shown in SEQ ID NO:16. The method can be used to detect Polysaccharide Storage Myopathy type 2 (PSSM2), also known as Myofibrillar Myopathy (MFM) in a horse.

This disclosure further describes a kit that includes a test for diagnosing and/or detecting the presence of equine Polysaccharide Storage Myopathy type 2 (PSSM2), also known as Myofibrillar Myopathy (MFM), in a horse. The kit generally includes packing material containing, separately packaged, at least one oligonucleotide probe capable of forming a hybridized nucleic acid with MYOT, FLNC, or MYOZ3 and instructions directing the use of the probe in accord with the methods described herein.

Horses affected with Polysaccharide Storage Myopathy type 2 (PSSM2), also known as Myofibrillar Myopathy (MFM), are typically heterozygous for the affected MYOT, FLNC, or MYOZ3 alleles. An "allele" is a variant form of a particular genomic nucleic acid sequence. In the context of the methods described herein, some alleles of the MYOT, FLNC, or MYOZ3 coding regions cause Polysaccharide Storage Myopathy type 2 (PSSM2), also known as Myofibrillar Myopathy (MFM), in horses. A "MYOT allele," "FLNC allele," or "MYOZ3 allele" refers to a normal allele of the MYOT, FLNC, or MYOZ3 loci as well as an allele carrying one or more variations that predispose a horse to develop Polysaccharide Storage Myopathy type 2 (PSSM2), also known as Myofibrillar Myopathy (MFM). The coexistence of multiple alleles at a locus is known as "genetic polymorphism." Any site at which multiple alleles exist as stable components of the population is by definition "polymorphic." An allele is defined as polymorphic if it is present at a frequency of at least 1% in the population. A "single nucleotide polymorphism (SNP)" is a DNA sequence variation that involves a change in a single nucleotide.

The methods described herein involve the use of isolated or substantially purified nucleic acid molecules. An "isolated" or "purified" nucleic acid molecule is one that, by human intervention, exists apart from its native environment and is therefore not a product of nature. An isolated nucleic acid molecule may exist in a purified form or may exist in a non-native environment. For example, an "isolated" or "purified" nucleic acid molecule, or portion thereof, is substantially free of other cellular material, or culture medium when produced by recombinant techniques, or substantially free of chemical precursors or other chemicals when chemically synthesized. In one embodiment, an "isolated" nucleic acid is free of sequences that naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. An isolated or purified nucleic acid molecule can be a fragment and/or variant of a reference nucleotide sequence expressly disclosed herein.

A "fragment" or "portion" of a sequence refers to anything less than full-length of the nucleotide sequence encoding-or the amino acid sequence of-a polypeptide. As it relates to a nucleic acid molecule, sequence, or segment when linked to other sequences for expression, a "portion" or a "fragment" refers to a sequence having, for example, at least 80 nucleotides, at least 150 nucleotides, or at least 400 nucleotides. Alternatively, when not employed for expressing-e.g., in the context of a probe or a primer-a "portion" or a "fragment" means, for example, at least 9, at least 12, at least 15, or at least 20 consecutive nucleotides. Alternatively, a fragment or a portion of a nucleotide sequence that is useful as a hybridization probe generally does not encode fragment proteins retaining biological activity. Thus, fragments or portions of a nucleotide sequence may range from at least about 6 nucleotides, about 9, about 12 nucleotides, about 20 nucleotides, about 50 nucleotides, about 100 nucleotides, or more.

A "variant" of a molecule is a sequence that is substantially similar to the sequence of the reference-e.g., native, naturally-occurring, and/or wild-type-molecule. For nucleotide sequences, a variant includes any nucleotide sequence that, because of the degeneracy of the genetic code, encodes the native amino acid sequence of a protein. Naturally occurring allelic variants such as these can be identified with the use of well-known molecular biology techniques, as, for example, with polymerase chain reaction (PCR) and/or hybridization techniques. A variant nucleotide sequence also can include a synthetically-derived nucleotide sequence such as one generated, for example, by using site-directed mutagenesis that encodes the native protein, as well as variant nucleotide sequences that encode a polypeptide having amino acid substitutions. Generally, a nucleotide sequence variant will have at least 40%, at least 50%, at least 60%, at least 70% (e.g., 71%, 72%, 73%, 74%, 75%, 76%, 77%, 78%, 79%), at least 80% (e.g., 81% 82%, 83%, 84%, 85%, 86%, 87%, 88%, 89%), or at least 90% (e.g., 91%, 92%, 93%, 94%, 95%, 96%, 97%, 98%, 99%) sequence identity to the native (endogenous) nucleotide sequence.

"Synthetic" polynucleotides are those prepared by chemical synthesis.

"Recombinant DNA molecule" is a combination of DNA sequences that are joined together using recombinant DNA technology and procedures that are used to join together DNA sequences as described, for example, in Sambrook and Russell (2001).

"Naturally-occurring," "native," or "wild-type" refers to an amino acid sequence or polynucleotide sequence that can be found in nature, without any known mutation, as distinct from being produced artificially or producing a mutated, non-wild-type phenotype. For example, a nucleotide sequence present in an organism (including a virus) that can be isolated from a source in nature and that has not been intentionally modified in the laboratory is naturally occurring. Furthermore, "wild-type" refers to a coding region or organism as found in nature without any known mutation.

A "mutant" myotilin (MYOT) polypeptide, filamin-C polypeptide (FLNC), or myozenin-3 (MYOZ3) polypeptide refers to a myotilin, filamin-C, or myozenin-3 polypeptide or a fragment thereof that is encoded by a MYOT, FLNC, or MYOZ3 coding region having a mutation, e.g., such as might occur at the MYOT, FLNC, or MYOZ3 locus. A mutation in one MYOT, FLNC, or MYOZ3 allele may lead to an alteration in the ability of the encoded polypeptide to interact with actin, alpha actinin, myotilin, filamin-c, myozenin-3, or other proteins that are structural components of the Z disc in myofibrils, or other proteins that are expressed in skeletal or cardiac muscle that are required for the integrity of myofibrils, leading to alterations in the integrity of myofibrils in a horse heterozygous for the allele. Alterations in the interactions of specific proteins can be determined by methods known to the art. Mutations in MYOT, FLNC, or MYOZ3 may be disease-causing in a horse heterozygous for the mutant MYOT, FLNC, or MYOZ3 allele, e.g., a horse heterozygous for a mutation leading to a mutant MYOT, FLNC, or MYOZ3 polypeptide such as substitution mutations in exon 6 of MYOT, exons 15 and 21 of FLNC, or exon 3 of MYOZ3, such as those designated herein as MYOT-S232P, FLNC-E753K, FLNC-A1207T, or MYOZ3-S42L.

A "somatic mutation" is a mutation that occurs only in certain tissues, e.g., in liver tissue, and are not inherited in the germline. A "germline" mutation can be found in any of a body’s tissues and are inherited. The present MYOT, FLNC, and MYOZ3 mutations are germline mutations.

"Homology" refers to the percent identity between two polynucleotide sequences or two amino acid sequences. Two sequences are "homologous" to each other when the sequences exhibit at least 70% (e.g., 71%, 72%, 73%, 74%, 75%, 76%, 77%, 78%, 79%), at least 80% (e.g., 81% 82%, 83%, 84%, 85%, 86%, 87%, 88%, 89%), or at least 90% (e.g., 91%, 92%, 93%, 94%, 95%, 96%, 97%, 98%, 99%) contiguous sequence identity over a defined length of the sequences.

The following terms are used to describe the sequence relationships between two or more nucleic acids or polynucleotides: "reference sequence," "comparison window," "sequence identity," "percentage of sequence identity," and "substantial identity."

As used herein, "reference sequence" refers to a sequence used as a basis for sequence comparison. A reference sequence may be a subset or the entirety of a specified sequence. For example, a reference sequence may be a segment of a full length cDNA or coding region sequence, or the complete cDNA or coding region sequence.

As used herein, "comparison window" refers to a contiguous and specified segment of a polynucleotide sequence, wherein the polynucleotide sequence in the comparison window may reflect one or more additions and/or deletions (i.e., gaps) compared to the reference sequence (which does not exhibit the additions and/or deletions) for optimal alignment of the two sequences. Generally, the comparison window is at least 20 contiguous nucleotides in length, and optionally can be 30, 40, 50, 100, or longer. To avoid a high similarity to a reference sequence due to inclusion of gaps in the polynucleotide sequence, a gap penalty is typically introduced and is subtracted from the number of matches. Methods of alignment of sequences for comparison are well known in the art. Thus, the determination of percent identity between any two sequences can be accomplished using a mathematical algorithm.

Computer implementations of these mathematical algorithms can be used for comparing sequences to determine sequence identity. Such implementations include, but are not limited to: Clustal Omega (online at EMBL-EBI), COBALT (online at ncbi.nlm.hih.gov), the ALIGN program (Version 2.0), and GAP, BESTFIT, BLAST, FASTA, and TFASTA in the Wisconsin Genetics Software Package, Version 8 (available from the Genetics Computer Group (GCG) Madison, WI, USA). Alignments using these programs can be performed using the default parameters.

Software for performing BLAST analyses is publicly available through the National Center for Biotechnology Information (see the World Wide Web at ncbi.nlm.nih.gov). This algorithm involves first identifying high scoring pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached.

In addition to calculating percent sequence identity, the BLAST algorithm also performs a statistical analysis of the similarity between two sequences. One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, less than about 0.01, or even less than about 0.001.

To obtain gapped alignments for comparison purposes, Gapped BLAST (in BLAST 2.0) can be utilized. Alternatively, PSI-BLAST (in BLAST 2.0) can be used to perform an iterated search that detects distant relationships between molecules. When using BLAST, Gapped BLAST, PSI-BLAST, the default parameters of the respective programs (e.g., BLASTN for nucleotide sequences, BLASTX for proteins) can be used. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix. See the World Wide Web at ncbi.nlm.nih.gov. Alignment may also be performed manually by visual inspection. For purposes of the methods described herein, comparison of nucleotide sequences for determination of percent sequence identity to the promoter sequences disclosed herein is preferably made using the BlastN program (version 2.3.0 or later) with its default parameters or any equivalent program. By "equivalent program" is intended any sequence comparison program that, for any two sequences in question, generates an alignment having identical nucleotide of amino acid residue matches and an identical percent sequence identity when compared to the corresponding alignment generated by a BLAST program.

A used herein, "sequence identity" or "identity" in the context of two nucleic acid or polypeptide sequences refers to a specified percentage of residues in the two sequences that are the same when aligned for maximum correspondence over a specified comparison window, as measured by sequence comparison algorithms or by visual inspection. When percentage of sequence identity is used in reference to a protein, it is recognized that residue positions that are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Methods for making this adjustment are well known to those of skill in the art. Typically, this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, CA).

A used herein, "percentage of sequence identity" refers to the value determined by comparing two optimally aligned sequences over a comparison window, wherein the portion of the polynucleotide sequence in the comparison window may comprise additions or deletions (i.e. gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

The term "substantial identity," in the context of polynucleotide sequences, means that a polynucleotide sequence possesses at least 70% (e.g., 71%, 72%, 73%, 74%, 75%, 76%, 77%, 78%, 79%), at least 80% (e.g., 81% 82%, 83%, 84%, 85%, 86%, 87%, 88%, 89%), or at least 90% (e.g., 91%, 92%, 93%, 94%, 95%, 96%, 97%, 98%, 99%) sequence identity compared to a reference sequence using one of the alignment programs described using standard parameters. These values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 70%, or at least 80%, 90%, or even at least 95%.

Another indication that nucleotide sequences are substantially identical is if two molecules hybridize to each other under stringent conditions (see below). Generally, stringent conditions are selected to be about 5°C lower than the thermal melting point ($T_m$) for the specific sequence at a defined ionic strength and pH. However, stringent conditions encompass temperatures in the range of about 1°C to about 20°C, depending upon the desired degree of stringency as otherwise qualified herein. Nucleic acids that do not hybridize to each other under stringent conditions are still substantially identical if the polypeptides they encode are substantially identical. This may occur, e.g., when a copy of a nucleic acid is created using the maximum codon degeneracy permitted by the genetic code. One indication that the two nucleic acid sequences are substantially identical is when the polypeptide encoded by the first nucleic acid is immunologically cross reactive with the polypeptide encoded by the second nucleic acid.

The term "substantial identity," in the context of a polypeptide, indicates that a polypeptide possesses a sequence with at least 70% (e.g., 71%, 72%, 73%, 74%, 75%, 76%, 77%, 78%, 79%), at least 80% (e.g., 81% 82%, 83%, 84%, 85%, 86%, 87%, 88%, 89%), or at least 90% (e.g., 91%, 92%, 93%, 94%, 95%, 96%, 97%, 98%, 99%) amino acid sequence identity to the reference sequence over a specified comparison window. An indication that two polypeptide sequences are substantially identical is that one polypeptide is immunologically reactive with antibodies raised against the second polypeptide.

Thus, a polypeptide is substantially identical to a second polypeptide when, for example, the two polypeptides differ only by a conservative substitution. For sequence comparison, typically one amino acid sequence acts as a reference sequence to which test amino acid sequences are compared. When using a sequence comparison algorithm, test and reference amino acid sequences are input into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

As noted above, another indication that two nucleic acid sequences are substantially identical is that two molecules hybridize to each other under stringent conditions. The phrase "hybridizing specifically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridizations are sequence dependent, and are different under different environmental parameters. Longer sequences hybridize specifically at higher temperatures. The $T_m$ is the temperature (under defined ionic strength and pH) at which 50% of the target sequence hybridizes to a perfectly matched probe. Specificity is typically the function of post-hybridization washes, the critical factors being the ionic strength and temperature of the final wash solution. For DNA-DNA hybrids, the $T_m$ can be approximated from the equation of Meinkoth and Wahl:

$$T_m = 81.5°C + 16.6\ (\log M) + 0.41(\%GC) - 0.61\ (\%\ form) - 500/L$$

where M is the molarity of monovalent cations, %GC is the percentage of guanosine and cytosine nucleotides in the DNA, % form is the percentage of formamide in the hybridization solution, and L is the length of the hybrid in base pairs. $T_m$ is reduced by about 1°C for each 1% of mismatching; thus, $T_m$, hybridization, and/or wash conditions can be adjusted to hybridize to sequences of the desired identity. For example, if sequences with >90% identity are sought, the $T_m$ can be decreased 10°C. Generally, stringent conditions are selected to be about 5°C lower than the thermal melting point ($T_m$) for the specific sequence and its complement at a defined ionic strength and pH. However, severely stringent conditions can utilize a hybridization and/or wash at 1°C, 2°C, 3°C, or 4°C lower than the thermal melting point ($T_m$); moderately stringent conditions can utilize a hybridization and/or wash at 6°C, 7°C, 8°C, 9°C, or 10°C lower than the thermal melting point ($T_m$); low stringency conditions can utilize a hybridization and/or wash at 11°C, 12°C, 13°C, 14°C, 15°C, or 20°C lower than the thermal melting point ($T_m$). Using the equation, hybridization and wash compositions, and desired T, those of ordinary skill will understand that variations in the stringency of hybridization and/or wash solutions are inherently described. If the desired degree of mismatching results in a T of less than 45°C (aqueous solution) or 32°C (formamide solution), it is preferred to increase the SSC concentration (20xSSC = 3.0 M NaCl, 0.3 M trisodium citrate) so that a higher temperature can be used. Generally, highly stringent hybridization and wash conditions are selected to be about 5°C lower than the thermal melting point ($T_m$) for the specific sequence at a defined ionic strength and pH.

An example of highly stringent wash conditions is 0.15 M NaCl at 72°C for about 15 minutes. An example of stringent wash conditions is a 0.2xSSC wash at 65°C for about 15 minutes. Often, a high stringency wash is preceded by a low stringency wash to remove background probe signal. An example medium stringency wash for a duplex of, e.g., more than 100 nucleotides is 4-6xSSC at 40°C for 15 minutes. For short probes (e.g., about 10 to 50 nucleotides), stringent conditions typically involve salt concentrations of less than about 1.5 M, more preferably about 0.01 M to 1.0 M, $Na^+$ ion concentration (or other salts) at pH 7.0 to 8.3, and the temperature is typically at least about 30°C and at least about 60°C for long probes (e.g., >50 nucleotides). Stringent conditions may also be achieved with the addition of destabilizing agents such as formamide. In general, a signal to noise ratio of 2× (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code. Very stringent conditions are selected to be equal to the $T_m$ for a particular probe. An example of stringent conditions for hybridization of complementary nucleic acids which have more than 100 complementary residues on a filter in a Southern or Northern blot is 50% formamide, e.g., hybridization in 50% formamide, 1 M NaCl, 1% SDS at 37°C; and a wash in 0.1×SSC at 60°C to 65°C. Exemplary low stringency conditions include hybridization with a buffer solution of 30 to 35% formamide, 1 M NaCl, 1% SDS (sodium dodecyl sulfate) at 37°C, and a wash in 1x to 2xSSC at 50°C to 55°C. Exemplary moderate stringency conditions include hybridization in 40% to 45% formamide, 1.0 M NaCl, 1% SDS at 37°C, and a wash in 0.5x to 1xSSC at 55°C to 60°C.

The term "variant" polypeptide refers to a polypeptide derived from the native protein by deletion (so-called truncation) and/or addition of one or more amino acids to the N-terminal and/or C-terminal end of the native protein, deletion and/or addition of one or more amino acids at one or more sites in the native protein, and/or substitution of one or more amino acids at one or more sites in the native protein. Such variants may result from, for example, genetic polymorphism or human manipulation. Methods for such manipulations are generally known in the art. A variant MYOT, FLNC, or MYOZ3 polypeptide may be altered in various ways including, for example, being altered to exhibit one or more amino acid substitutions, one or more deletions, one or more truncations, and/or one or more insertions. For example, an amino acid sequence can be prepared by one or more mutations in the DNA encoding the MYOT, FLNC, or MYOZ3 polypeptide. Guidance regarding appropriate amino acid substitutions that do not affect biological activity of the protein of interest is well known in the art. Conservative substitutions, such as exchanging one amino acid with another having similar properties, are preferred.

Thus, the nucleotide sequences used to practice the methods described herein can include both naturally-occurring sequences or mutant forms. Likewise, the polypeptides referred to herein can include naturally-occurring polypeptides as well as variations and modified forms thereof. Such variants may continue to possess the desired activity. The deletions, insertions, or substitutions of the polypeptide sequence encompassed herein are not expected to produce radical changes in the characteristics of the polypeptide. However, when it is difficult to predict the exact effect of the substitution, deletion, or insertion in advance of doing so, the effect can be evaluated by routine screening assays.

An individual substitution, deletion, or addition that alters, adds, or deletes a single amino acid or a small percentage of amino acids (typically less than 5%, more typically less than 1%) in an encoded sequence are "conservatively modified variations."

"Conservatively modified variations" of a particular nucleic acid sequence refers to those nucleic acid sequences that encode identical or essentially identical amino acid sequences, or where the nucleic acid sequence does not encode an amino acid sequence, to essentially identical sequences. Because of the degeneracy of the genetic code, a large number of functionally identical nucleic acids encode any given polypeptide. For instance, the codons CGT, CGC, CGA, CGG, AGA, and AGG all encode the amino acid arginine. Thus, at every position where an arginine is specified by a codon, the codon can be altered to any of the corresponding codons described without altering the encoded protein. Such nucleic acid variations are "silent variations," which are one species of "conservatively modified variations." Every nucleic acid sequence described herein that encodes a polypeptide also describes every possible silent variation, except where otherwise noted. One of skill will recognize that each codon in a nucleic acid (except ATG, which is ordinarily the only codon for methionine, and TGG, which is ordinarily the only codon for tryptophan) can be modified to yield a functionally identical molecule by standard techniques. Accordingly, each "silent variation" of a nucleic acid that encodes a polypeptide is implicit in each described sequence.

Known methods of PCR include, but are not limited to, methods using paired primers, nested primers, single specific primers, degenerate primers, gene-specific primers, vectorspecific primers, partially mismatched primers, and the like.

The terms "heterologous DNA sequence," "exogenous DNA segment," or "heterologous nucleic acid" refer to a sequence that originates from a source foreign to the particular host cell or, if from the same source, is modified from its original form. Thus, a heterologous coding region in a host cell includes a coding region that is endogenous to the particular host cell but has been modified through, for example, the use of single-stranded mutagenesis. The terms also include non-naturally-occurring multiple copies of a naturally occurring DNA sequence. Thus, the terms refer to a DNA segment that is foreign or heterologous to the cell, or homologous to the cell but in a position within the host cell nucleic acid in which the element is not ordinarily found. Exogenous DNA segments, when expressed, yield exogenous polypeptides.

A "homologous" DNA sequence is a DNA sequence that is naturally associated with a host cell into which it is introduced.

"Genome" refers to the complete genetic material of an organism.

"Coding sequence" refers to a DNA or RNA sequence that codes for a specific amino acid sequence and excludes non-coding (e.g., regulatory) nucleotide sequences. For example, a DNA "coding sequence" or a "sequence encoding" a particular polypeptide is a DNA sequence that is transcribed and translated into a polypeptide in vitro or in vivo when placed under the control of appropriate regulatory elements. The boundaries of the coding sequence are determined by a start codon at the 5'-terminus and a translation stop codon at the 3'-terminus. A coding sequence can include, but is not limited to, prokaryotic sequences, cDNA from eukaryotic mRNA, genomic DNA sequences from eukaryotic (e.g., mammalian) DNA, and/or synthetic DNA sequences. A transcription termination sequence will usually be located 3' to the coding sequence. It may constitute an "uninterrupted coding sequence,"-i.e., lacking an intron, such as in cDNA or it may include one or more introns bounded by appropriate splice junctions. An "intron" is a sequence of RNA that is contained in the primary transcript but that is removed through cleavage and re-ligation of the RNA within the cell to create the mature mRNA that can be translated into a protein.

The terms "open reading frame" and "ORF" refer to the nucleotide sequence between translation initiation and termination codons of a coding sequence. The terms "initiation codon" and "termination codon" refer to a unit of three adjacent nucleotides ("codon") in a coding sequence that specifies initiation and chain termination, respectively, of protein synthesis (mRNA translation).

The term "RNA transcript" refers to the product resulting from RNA polymerase catalyzed transcription of a DNA sequence. When the RNA transcript is a perfect complementary copy of the DNA sequence, it is referred to as a primary transcript or it may be an RNA sequence derived from post transcriptional processing of the primary transcript and is referred to as the mature RNA. "Messenger RNA" (mRNA) refers to the RNA that is without introns and can be translated into protein by the cell. "cDNA" refers to a single- or double-stranded DNA that is complementary to and derived from mRNA.

The term "regulatory sequence" refers to a nucleotide sequence that includes, for example, a promoter, an enhancer, and/or other expression control elements (e.g., polyadenylation signals). Such regulatory sequences are known to those skilled in the art. The design of an expression vector may depend on such factors as the choice of the host cell to be transfected and/or the amount of fusion protein to be expressed.

The term "DNA control elements" refers collectively to promoters, ribosome binding sites, polyadenylation signals, transcription termination sequences, upstream regulatory domains, enhancers, and the like, that collectively provide for the transcription and translation of a coding sequence in a host cell. Not all of these control sequences need always be present in a recombinant vector so long as the desired coding region is capable of being transcribed and translated.

A control element, such as a promoter, "directs the transcription" of a coding sequence in a cell when RNA polymerase binds to the promoter and transcribes the coding sequence into mRNA, which is then translated into the polypeptide encoded by the coding sequence.

A cell has been "transformed" by exogenous DNA when the exogenous DNA has been introduced inside the cell membrane. Exogenous DNA may or may not be integrated (covalently linked) into chromosomal DNA making up the genome of the cell. In prokaryotes and yeasts, for example, the exogenous DNA may be maintained on an episomal element, such as a plasmid. With respect to other eukaryotic cells, a stably transformed cell is one in which the exogenous DNA has become integrated into the chromosome so that it is inherited by daughter cells through chromosome replication. This stability is demonstrated by the ability of the eukaryotic cell to establish cell lines or clones having a population of daughter cells containing the exogenous DNA.

"Operably linked" refers to the association of nucleic acid sequences on single nucleic acid fragments so that the function of one is affected by the other, e.g., an arrangement of elements wherein the components so described are configured so as to perform their usual function. For example, a regulatory DNA sequence is said to be "operably linked to" a DNA sequence that codes for an RNA or a polypeptide if the two sequences are situated such that the regulatory DNA sequence affects expression of the coding DNA sequence (i.e., that the coding sequence or functional RNA is under the transcriptional control of the promoter). Coding sequences can be operably linked to regulatory sequences in sense or antisense orientation. Control elements operably linked to a coding sequence are capable of effecting the expression of the coding sequence. The control elements need not be contiguous with the coding sequence, so long as they function to direct the expression thereof. Thus, for example, intervening untranslated yet transcribed sequences can be present between a promoter and the coding sequence and the promoter can still be considered "operably linked" to the coding sequence.

"Transcription stop fragment" refers to nucleotide sequences that contain one or more regulatory signals, such as polyadenylation signal sequences, capable of terminating transcription. Examples include the 3' non-regulatory regions of the genes encoding myotilin, filamin-C, and myozenin-3 (MYOT, FLNC, and MYOZ3).

"Translation stop fragment" or "translation stop code" or "stop codon" refers to nucleotide sequences that contain one or more regulatory signals, such as one or more termination codons in all three frames, capable of terminating translation. Insertion of a translation stop fragment adjacent to or near the initiation codon at the 5' end of the coding sequence will result in no translation or improper translation. The change of at least one nucleotide in a nucleic acid sequence can result in an interruption of the coding sequence of the gene, e.g., a premature stop codon. Such sequence changes can cause a mutation in the polypeptide encoded by the MYOT, FLNC, or MYOZ3 genes. For example, if the mutation is a nonsense mutation, the mutation results in the generation of a premature stop codon, causing the generation of a truncated MYOT, FLNC, or MYOZ3 polypeptide.

Nucleic Acids

Nucleotide sequences that are subjected to the methods described herein can be obtained from any prokaryotic or eukaryotic source. For example, they can be obtained from a mammalian, such as equine, cellular source. Alternatively, nucleic acid molecules can be obtained from a library, such as the CHORI-241 Equine BAC library or a similar resource available elsewhere.

As discussed above, the terms "isolated and/or purified" refer to a nucleic acid-e.g. a DNA or RNA molecule-that has been isolated from its natural cellular environment and from association with other components of the cell, such as nucleic acid or polypeptide, so that it can be sequenced, replicated, and/or expressed. For example, an "isolated nucleic acid" may be a DNA molecule that is complementary or hybridizes to a sequence in a coding region of interest-e.g., a nucleic acid sequence encoding an equine filamin-C protein, and remains stably bound under stringent conditions (as defined by methods well known in the art). Thus, an RNA or a DNA is "isolated" in that it is free from at least one contaminating nucleic acid with which it is normally associated in the natural source of the RNA or DNA and in one embodiment is substantially free of any other mammalian RNA or DNA. The phrase "free from at least one contaminating source nucleic acid with which it is normally associated" includes the case where nucleic acid is reintroduced into the source or natural cell but is in a different chromosomal location or is otherwise flanked by nucleic acid sequences not normally found in the source cell, e.g., in a vector or plasmid.

As used herein, the term "recombinant nucleic acid," e.g., "recombinant DNA sequence or segment" refers to a nucleic acid, e.g., to DNA that has been derived or isolated from any appropriate cellular source, that may be substantially chemically altered in vitro, so that its sequence is not naturally occurring, or corresponds to naturally occurring sequences that are not positioned as they would be positioned in a genome that has not been transformed with exogenous DNA. An example of preselected DNA "derived" from a source would be a DNA sequence that is identified as a useful fragment within a given organism, and which is then chemically synthesized in essentially pure form. An example of such DNA "isolated" from a source would be a useful DNA sequence that is excised or removed from the source by chemical means, e.g., by the use of restriction endonucleases, so that it can be further manipulated, e.g. amplified, for use in the methods described herein. Thus, recovery or isolation of a given fragment of DNA from a restriction digest can employ separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. Therefore, "recombinant DNA" includes completely synthetic DNA sequences, semi-synthetic DNA sequences, DNA sequences isolated from biological sources, and DNA sequences derived from RNA, as well as mixtures thereof.

Nucleic acid molecules having base substitutions (i.e., variants) are prepared by a variety of methods known in the art. These methods include, but are not limited to, isolation from a natural source (in the case of naturally occurring sequence variants) or preparation by oligonucleotide-mediated (or site-directed) mutagenesis, PCR mutagenesis, and cassette mutagenesis of an earlier prepared variant or non-variant version of the nucleic acid molecule.

Nucleic Acid Amplification Methods

DNA present in a physiological sample may be amplified by any means known to the art. Examples of suitable amplification techniques include, but are not limited to, polymerase chain reaction (including, for RNA amplification, reverse-transcriptase polymerase chain reaction), ligase chain reaction, strand displacement amplification, transcription-based amplification, selfsustained sequence replication (or "3 SR"), the Qβ-replicase system, nucleic acid sequence-based amplification (or "NASBA"), the repair chain reaction (or "RCR"), and boomerang DNA amplification (or "BDA").

The bases incorporated into the amplification product may be natural or modified bases (modified before or after amplification), and the bases may be selected to optimize subsequent electrochemical detection steps.

Polymerase chain reaction (PCR) may be performed according to known techniques. In general, PCR involves, first, treating a nucleic acid sample (e.g., in the presence of a heat stable DNA polymerase) with one oligonucleotide primer for each strand of the specific sequence to be detected under hybridizing conditions so that an extension product of each primer is synthesized that is complementary to each nucleic acid strand, with the primers sufficiently complementary to each strand of the specific sequence to hybridize therewith so that the extension product synthesized from each primer, when it is separated from its complement, can serve as a template for synthesis of the extension product of the other primer, and then treating the sample under denaturing conditions to separate the primer extension products from their templates if the sequence or sequences to be detected are present. These steps are cyclically repeated until the desired degree of amplification is obtained. Detection of the amplified sequence may be carried out by adding to the reaction product an oligonucleotide probe capable of hybridizing to the reaction product (e.g., an oligonucleotide probe), the probe carrying a detectable label, and then detecting the label in accordance with known techniques. Where the nucleic acid to be amplified is RNA, amplification may be carried out by initial conversion to DNA by reverse transcriptase in accordance with known techniques.

Strand displacement amplification (SDA) may be performed according to known techniques. For example, SDA may be carried out with a single amplification primer or a pair of amplification primers, with exponential amplification being achieved with the latter. In general, SDA amplification primers comprise, in the 5' to 3' direction, a flanking sequence (the DNA sequence of which is noncritical), a restriction site for the restriction enzyme employed in the reaction, and an oligonucleotide sequence (e.g., an oligonucleotide probe) that hybridizes to the target sequence to be amplified and/or detected. The flanking sequence, which serves to facilitate binding of the restriction enzyme to the recognition site and provides a DNA polymerase priming site after the restriction site has been nicked, is about 15 to 20 nucleotides in length in one embodiment. The restriction site may be functional in the SDA reaction: the oligonucleotide probe portion may be about 13 to 15 nucleotides in length.

Ligase chain reaction (LCR) also may be performed according to known techniques. In general, the reaction is carried out with two pairs of oligonucleotide probes: one pair binds to one strand of the sequence to be detected; the other pair binds to the other strand of the sequence to be detected; each pair together completely overlaps the strand to which it corresponds. The reaction is carried out by, first, denaturing (e.g., separating) the strands of the sequence to be detected, then reacting the strands with the two pairs of oligonucleotide probes in the presence of a heat stable ligase so that each pair of oligonucleotide probes is ligated together, then separating the reaction product, and then cyclically repeating the process until the sequence has been amplified to the desired degree. Detection may then be carried out in like manner as described above with respect to PCR.

In some embodiments, each exon of the MYOT, FLNC, or MYOZ3 coding region is amplified by PCR using primers based on the known sequence. The amplified exons are then sequenced using, for example, an automated sequencer. In this manner, the exons of the MYOT, FLNC, or MYOZ3 coding region from horses suspected of having Polysaccharide Storage Myopathy type 2 (PSSM2), also known as Myofibrillar Myopathy (MFM), in their pedigree are then sequenced until a mutation is found. Examples of such mutations include those in exon 6 of the MYOT DNA, exons 15 and 21 of the FLNC DNA, or exon 3 of the MYOZ3 DNA. For example, mutations in the MYOT gene include an adenine (A) to guanine (G) substitution on the forward strand at nucleotide base chr14:38,519,183 in exon 6 (FIG. 1); two mutations in the FLNC gene include a guanine (G) to adenine (A) substitution on the forward strand at nucleotide base chr4:83736244 in exon 15 and a guanine (G) to adenine (A) substitution on the forward strand at nucleotide base chr4:83738769 in exon 21 (FIG. 2 and FIG 3); mutations in the MYOZ3 gene include a guanine (G) to adenine (A) substitution on the forward strand at nucleotide base chr14:27,399,222 (FIG. 4). Using this technique, additional mutations causing equine Polysaccharide Storage Myopathy type 2 (PSSM2), also known as Myofibrillar Myopathy (MFM), can be identified. Thus, the methods described herein may be used to detect and/or identify an alteration within the wild-type MYOT, FLNC, or MYOZ3 locus. "Alteration of" a specified locus encompasses all forms of mutations including, for example, a deletion, an insertion, and/or a point mutation in the coding and noncoding regions. A deletion can involve the deletion of all or any portion of the coding region. A point mutation may result in an aberrant stop codon, a frameshift mutation, an amino acid substitution, and/or an alteration in pre-mRNA processing (splicing) that produces a protein with an altered amino acid sequence. Point mutational events may occur in regulatory regions, such as in the promoter of the gene, leading to decreased expression of the mRNA. A point mutation also may interfere with proper RNA processing, leading to decreased expression of the MYOT, FLNC, or MYOZ3 translation products, decreased mRNA stability, and/or decreased translation efficiency. Polysaccharide Storage Myopathy type 2 (PSSM2), also known as Myofibrillar Myopathy (MFM), is a disease caused by point mutations at nucleic acid chr14:38,519,183 (MYOT), chr4:83736244 and chr4:83738769 (FLNC), and chr14:27,399,222 (MYOZ3). Horses predisposed to or having Polysaccharide Storage Myopathy type 2 (PSSM2), also known as Myofibrillar Myopathy (MFM), need only have one mutated MYOT, FLNC, or MYOZ3 allele.

Techniques that are useful in performing the methods described herein include, but are not limited to direct DNA sequencing, PFGE analysis, allele-specific oligonucleotide (ASO), dot blot analysis, and/or denaturing gradient gel electrophoresis.

There are several methods that can be used to detect DNA sequence variation. Direct DNA sequencing, either manual or automated (e.g., fluorescent or semiconductor-based sequencing), can detect sequence variation. Another approach is the single-stranded conformation polymorphism assay (SSCA). This method does not detect all sequence changes, especially if the DNA fragment size is greater than 200 bp, but can be used to detect most DNA sequence variation. SSCA allows for increased throughput compared to direct sequencing for mutation detection on a research basis. The fragments that have shifted mobility on SSCA gels are then sequenced to determine the exact nature of the DNA sequence variation. Other approaches based on the detection of mismatches between the two complementary DNA strands include clamped denaturing gel electrophoresis (CDGE), heteroduplex analysis (HA), and chemical mismatch cleavage (CMC). Once a mutation is known, an allele specific detection approach such as allele specific oligonucleotide (ASO) hybridization can be utilized to rapidly screen large numbers of other samples for that same mutation. Such a technique can utilize probes that are labeled with gold nanoparticles to yield a visual color result.

Detecting point mutations may be accomplished by molecular cloning and then sequencing one or more MYOT, FLNC, or MYOZ3 alleles. Alternatively, the coding region sequences can be amplified directly from a genomic DNA preparation from equine tissue, using known techniques. The DNA sequence of the amplified sequences can then be determined.

Exemplary methods for a more complete, yet still indirect, test for confirming the presence of a mutant allele include, for example, single stranded conformation analysis (SSCA), denaturing gradient gel electrophoresis (DDGE), an RNase protection assay, allele-specific oligonucleotides (ASOs), the use of a protein that recognizes nucleotide mismatches (e.g., the *E. coli* mutS protein), and allele-specific PCR. For allele-specific PCR, primers are used that hybridize at their 3' ends to a particular MYOT, FLNC, or MYOZ3 mutation. If the particular mutation is not present, an amplification product is not observed. Allele-specific PCR may also be carried out using quantitative PCR or real-time PCR using a specialized instrument that is capable of detecting and quantifying the appearance of amplification products during each amplification cycle. An Amplification Refractory Mutation System (ARMS) can also be used. Insertions and deletions of genes can also be detected by cloning, sequencing, and amplification. In addition, restriction fragment length polymorphism (RFLP) probes for the target locus or a surrounding marker locus can be used to score alteration of an allele or an insertion in a polymorphic fragment. Other techniques for detecting insertions or deletions as known in the art can also be used.

In the first three methods (i.e., SSCA, DGGE, and RNase protection assay), a new electrophoretic band appears. SSCA detects a band that migrates differently because the sequence change causes a difference in single-strand, intramolecular base pairing. RNase protection involves cleaving the mutant polynucleotide into two or more smaller fragments. DGGE detects differences in migration rates of mutant sequences compared to wild-type sequences using a denaturing gradient gel. In an allele-specific oligonucleotide assay, an oligonucleotide is designed that detects a specific sequence, and the assay is performed by detecting the presence or absence of a hybridization signal. In the mutS assay, the protein binds only to sequences that contain a nucleotide mismatch in a heteroduplex between mutant and wild-type sequence.

As used herein, a "nucleotide mismatch" refers to a hybridized nucleic acid duplex in which the two strands are not 100% complementary. Lack of total homology may be due to a deletion, an insertion, an inversion, and/or a substitution. Mismatch detection can be used to detect point mutation in the coding region or its mRNA product. While these techniques are less sensitive than sequencing, they are simpler to perform on a large number of samples. An example of a mismatch cleavage technique is the RNase protection method. In the context of detecting a MYOT-, FLNC-, or MYOZ3-associated mismatch, the method involves the use of a labeled riboprobe that is complementary to the horse wild-type MYOT, FLNC, or MYOZ3 coding region coding sequence. The riboprobe and either mRNA or DNA isolated from tissue are annealed (i.e., hybridized) and subsequently digested with the enzyme RNase A, which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen that is smaller than the full length duplex RNA for the riboprobe and the mRNA or DNA. The riboprobe need not be the full length of the MYOT, FLNC, or MYOZ3 mRNA or coding region but can be a segment of either. If the riboprobe includes only a segment of the MYOT, FLNC, or MYOZ3 mRNA or DNA, it may be desirable to use a number of probes to screen the whole mRNA sequence for mismatches.

In a similar fashion, DNA probes can be used to detect a mismatch through enzymatic and/or chemical cleavage.

Alternatively, a mismatch can be detected by shifts in the electrophoretic mobility of mismatched duplexes relative to matched duplexes. With either riboprobes or DNA probes, the cellular mRNA or DNA that might contain a mutation can be amplified using PCR before hybridization.

Nucleic acid analysis via microchip technology

A DNA sequence of the MYOT, FLNC, or MYOZ3 coding regions that has been amplified by PCR may be screened using an allele-specific probe. Allele-specific probes are nucleic acid oligomers, each of which contains a region of the MYOT, FLNC, or MYOZ3 coding region harboring a known mutation. For example, one oligomer may be about 30 nucleotides in length, corresponding to a portion of the MYOT, FLNC, or MYOZ3 coding region sequence. Using a battery of such allele-specific probes, a PCR amplification product can be screened to identify the presence of a previously identified mutation in the MYOT, FLNC, or MYOZ3 coding region. Hybridizing an allele-specific probe with an amplified MYOT, FLNC, or MYOZ3 sequence can be performed, for example, on a nylon filter. Hybridizing to a particular probe under stringent hybridization conditions indicates the presence of the same mutation in the tissue as in the allele-specific probe.

An alteration of MYOT, FLNC, or MYOZ3 mRNA expression can be detected by any technique known in the art. Exemplary techniques include, for example, Northern blot analysis, PCR amplification, and/or RNase protection. Decreased mRNA expression indicates an alteration of the wild-type MYOT, FLNC, or MYOZ3 locus.

Alteration of wild-type MYOT, FLNC, or MYOZ3 coding region also can be detected by screening for alteration of a wild-type MYOT, FLNC, or MYOZ3 polypeptide such as, for example, the wild-type MYOT, FLNC, or MYOZ3 protein or a portion the wild-type MYOT, FLNC, or MYOZ3 protein. For example, a monoclonal antibody immunoreactive with wild-type MYOT, FLNC, or MYOZ3 (or to a specific portion of the MYOT, FLNC, or MYOZ3 protein) can be used to screen a tissue. Lack of cognate antigen would indicate a mutation. An antibody specific for a product of a mutant allele also can be used to detect a mutation in the MYOT, FLNC, or MYOZ3 coding region. Such an immunological assay can be performed using conventional methods. Exemplary methods include, for example, Western blot analysis, an immunohistochemical assay, an ELISA assay, and/or any method for detecting an altered MYOT, FLNC, or MYOZ3 polypeptide. In some embodiments, a functional assay can be used such as, for example, protein binding determination. In addition, an assay can be used that detects MYOT, FLNC, or MYOZ3 biochemical function. Finding a mutant MYOT, FLNC, or MYOZ3 polypeptide indicates a mutation at the MYOT, FLNC, or MYOZ3 locus.

A mutant MYOT, FLNC, or MYOZ3 coding region or translation product can be detected in a variety of physiological samples collected from a horse. Examples of appropriate samples include a cell sample, such as a blood cell (e.g., a lymphocyte, a peripheral blood cell), a sample collected from the spinal cord, a tissue sample such as cardiac tissue or muscle tissue (e.g. cardiac or skeletal muscle) an organ sample (e.g., liver or skin), a hair sample, especially a hair sample with the hair bulb (roots) attached, and/or a fluid sample (e.g., blood).

The methods described herein are applicable to any equine disease in which MYOT, FLNC, or MYOZ3 has a role. The method may be particularly useful for, for example, a veterinarian, a Breed Association, and/or individual breeders, so they can decide upon an appropriate course of treatment, and/or to determine if an animal is a suitable candidate as a brood mare or sire.

Oligonucleotide Probes

As described above, the method may be used to detect the presence and/or absence of a polymorphism in equine DNA. In particular, mutations in the MYOT gene include an adenine (A) to guanine (G) substitution on the forward strand at nucleotide base chr14:38,519,183 in exon 6 (FIG. 1); two mutations in the FLNC gene include a guanine (G) to adenine (A) substitution on the forward strand at nucleotide base chr4:83736244 in exon 15 and a guanine (G) to adenine (A) substitution on the forward strand at nucleotide base chr4:83738769 in exon 21 (FIG. 2 and FIG. 3); mutations in the MYOZ3 gene include a guanine (G) to adenine (A) substitution on the forward strand at nucleotide base chr14:27,399,222 (FIG. 4). These substitutions result in: (1) a serine (S) at codon 232 in the myotilin (MYOT) protein (SEQ ID NO:9) being replaced by a proline (P), as shown in SEQ ID NO: 10, (2) a glutamic acid (E) at codon 753 in the filamin-C (FLNC) protein (SEQ ID NO:11, equivalent to SEQ ID NO:12) being replaced by a lysine (K), as shown in SEQ ID NO:13 (equivalent to SEQ ID NO:14), and an alanine (A) at codon 1207 in the filamin-C (FLNC) protein (SEQ ID NO:11, equivalent to SEQ ID NO:12) being replaced by a threonine (T), as shown in SEQ ID NO:13 (equivalent to SEQ ID NO:14), and (3) a serine (S) at codon 42 in the myozenin-3 (MYOZ3) protein (SEQ ID NO:15) being replaced by a leucine (L), as shown in SEQ ID NO:16.

A primer pair may be used to determine the nucleotide sequence of a particular MYOT, FLNC, or MYOZ3 allele using PCR. A pair of single-stranded DNA primers can be annealed to sequences within or surrounding the FLNC coding region in order to prime amplifying DNA synthesis of the MYOT, FLNC, or MYOZ3 coding region itself. A complete set of primers allows one to synthesize all of the nucleotides of the MYOT, FLNC, or MYOZ3 coding sequence. In some embodiments, a set of primers can allow synthesis of both intron and exon sequences. In some embodiments, allele-specific primers can be used. Such primers anneal only to particular MYOT, FLNC, or MYOZ3 mutant alleles, and thus will only amplify product efficiently in the presence of the mutant allele as a template.

The first step of the process involves contacting a physiological sample obtained from a horse, which sample contains nucleic acid, with an oligonucleotide probe to form a hybridized DNA. The oligonucleotide probe can be any probe having from about 4 or 6 bases up to about 80 or 100 bases or more. In one embodiment, the oligonucleotide probe can have between about 10 and about 20 bases.

The primers themselves can be synthesized using conventional techniques and, in some cases, can be made using an automated oligonucleotide synthesizing machine. Given the MYOT genomic sequence as partially set forth in SEQ ID NO: 1, the FLNC genomic sequence as partially set forth in SEQ ID NO:2 and SEQ ID NO:3, and the MYOZ3 genomic sequence as partially set forth in SEQ ID NO:4, one can design a set of oligonucleotide primers to probe any portion of the MYOT, FLNC, or MYOZ3 coding sequences. The primers may be designed to hybridize entirely to coding sequence (exons), to noncoding sequence (introns or other noncoding sequences), or to regions spanning the junction of coding and noncoding sequences in genomic DNA.

An oligonucleotide probe may be prepared according to conventional techniques to have any suitable base sequence. Suitable bases for preparing the oligonucleotide probe may be selected from naturally-occurring bases such as adenine, cytosine, guanine, uracil, and thymine. An oligonucleotide probe also can incorporate one or more non-naturally-occurring or "synthetic" nucleotide bases. Exemplary synthetic bases include, for example, 7-deaza-guanine, 8-oxo-guanine, 6-mercaptoguanine, N4-acetylcytidine, 5-(carboxyhydroxyethyl)uridine, 2'-O-methylcytidine, 5-(carboxymethylaminomethyl)-2-thiouridine, 5-carboxymethylaminomethyluridine, dihydrouridine, 2'-O-methylpseudouridine, β,D-galactosylqueuosine, 2'-O-methylguanosine, inosine, N6-isopentenyladenosine, 1-methyladenosine, 1-methylpseudouridine, 1-methylguanosine, 1-methylinosine, 2,2-dimethylguanosine, 2-methyladenosine, N2-methylguanosine, 3-methylcytidine, 5-methylcytidine, N6-methyladenosine, 7-methylguanosine, 5-methylaminomethyluridine, 5-methoxyaminomethyl-2-thiouridine, β,D-mannosylqueuosine, 5-methloxycarbonylmethyluridine, 5-methoxyuridine, 2-methylthio-N6-isopentenyladenosine, N-((9-β-D-ribofuranosyl-2-methylthiopurine-6-yl)carbamoyl)threonine, N-((9-β-D-ribofuranosylpurine-6-yl)N-methyl-carbamoyl)threonine, uridine-5-oxyacetic acid methylester, uridine-5-oxyacetic acid, wybutoxosine, pseudouridine, queuosine, 2-thiocytidine, 5-methyl-2-thiouridine, 2-thiouridine, 5-methyluridine, N-((9-beta-D-ribofuranosylpurine-6-yl)carbamoyl)threonine, 2'-O-methyl-5-methyluridine, 2'-O-methyluridine, wybutosine, and/or 3-(3-amino-3-carboxypropyl)uridine. Any oligonucleotide backbone may be employed, including DNA, RNA (although RNA may be less preferred than DNA in certain circumstances), modified sugars such as carbocycles, and sugars containing 2' substitutions (e.g., fluoro or methoxy). The oligonucleotides may be oligonucleotides wherein at least one, or all, of the internucleotide bridging phosphate residues is a modified phosphate such as, for example, a methyl phosphate, a methyl phosphonotlioate, a phosphoroinorpholidate, a phosphoropiperazidate, and/or a phospholioramidate-for example, every other one of the internucleotide bridging phosphate residues may be modified. The oligonucleotide may be a "peptide nucleic acid" such as described in Nielsen et al., Science, 254, 1497-1500 (1991).

The oligonucleotide probe should possess a sequence at least a portion of which is capable of binding to a known portion of the sequence of the nucleic acid in the physiological sample.

In some embodiments, the nucleic acid in the sample may be contacted with a plurality of oligonucleotide probes having different base sequences (e.g., where there are two or more target nucleic acids in the sample, or where a single target nucleic acid is hybridized to two or more probes in a "sandwich" assay).

The oligonucleotide probes provided herein may be useful for a number of purposes. For example, the oligonucleotide probes can be used to detect PCR amplification products and/or to detect mismatches with the FLNC coding region or mRNA.

Hybridization Methodology

The nucleic acid from the physiological sample may be contacted with the oligonucleotide probe in any conventional manner. For example, the sample nucleic acid may be solubilized in solution and contacted with the oligonucleotide probe by solubilizing the oligonucleotide probe in solution with the sample nucleic acid under condition that permit hybridization. Suitable hybridization conditions are well known to those skilled in the art. Alternatively, the sample nucleic acid may be solubilized in solution with the oligonucleotide probe immobilized on a solid or semisolid support, whereby the sample nucleic acid may be contacted with the oligonucleotide probe by immersing the solid or semisolid support having the oligonucleotide probe immobilized thereon in the solution containing the sample nucleic acid.

Certain embodiments of the methods described herein relate to mutations in the MYOT, FLNC, or MYOZ3 coding regions or the diagnosis of Polysaccharide Storage Myopathy type 2 (PSSM2), also known as Myofibrillar Myopathy (MFM), the detection of a predisposition for Polysaccharide Storage Myopathy type 2 (PSSM2), also known as Myofibrillar Myopathy (MFM), or to the detection of a mutant MYOT, FLNC, or MYOZ3 allele in a horse.

Mutations in the equine MYOT, FLNC, or MYOZ3 coding regions (encoding the skeletal muscle proteins myotilin, filamin-C, or myozenin-3) are present in many populations of horses affected by Polysaccharide Storage Myopathy type 2 (PSSM2), also known as Myofibrillar Myopathy (MFM). The differences in the genomic DNA between horses affected by Polysaccharide Storage Myopathy type 2 (PSSM2), also known as Myofibrillar Myopathy (MFM), include point mutations at nucleic acid chr14:38,519,183 (MYOT), chr4:83736244 and chr4:83738769 (FLNC), and chr14:27,399,222 (MYOZ3).

Scientific Narrative

FIG. 1 shows the equine genomic sequence from the reference assembly (EquCab2) around the position of the adenine (A) to guanine (G) substitution at chr14:38,519,183 in MYOT. The reverse complement sequence is shown, so the substitution appears as a thymine (T) to cytosine (C) substitution in SEQ ID NO:1.

FIG. 2 shows the equine genomic sequence from the reference assembly (EquCab2) around the position of the guanine (G) to adenine (A) substitution at chr4:83736244 in FLNC (SEQ ID NO:2). The forward strand sequence is shown.

FIG. 3 shows the equine genomic sequence from the reference assembly (EquCab2) around the position of the guanine (G) to adenine (A) substitution at chr4:83738769 in FLNC (SEQ ID NO:3). The forward strand sequence is shown.

FIG. 4 shows the equine genomic sequence from the reference assembly (EquCab2) around the position of the guanine (G) to adenine (A) substitution at chr14:27,399,222 in MYOZ3. The reverse complement sequence is shown, so the substitution appears as a cytosine (C) to thymine (T) substitution in SEQ ID NO:4.

FIG. 5 shows the equine MYOT coding sequence (SEQ ID NO:5, also known as XM_014730661.1), with exon 6 indicated in bold. The position of the thymine (T) to cytosine (C) substitution at position 664 in SEQ ID NO:5 (chr14:38,519,183 in SEQ ID NO:1) is underlined.

FIG. 6 shows the alignment of two models of the equine FLNC coding sequence. SEQ ID NO:6, also known as Ensembl CDS 00000012220, is shown aligned to SEQ ID NO:7, also known as XM_014739030.1. Exons 15 and 21 are shown in bold. The position of the guanine (G) to adenine (A) substitution at chr4:83736244 in exon 15 is underlined, as is the position of the guanine (G) to adenine (A) substitution at chr4:83738769 in exon 21. The guanine (G) to adenine (A) base substitution in exon 15 at nucleotide position chr4:83736244 corresponds to base 2257 in SEQ ID NO:6 and 2506 in SEQ ID NO:7. The guanine (G) to adenine (A) base substitution in exon 21 at nucleotide position chr4:83738769 corresponds to base 3619 in SEQ ID NO:6 and 3868 in SEQ ID NO:7. The two models for the CDS of equine FLNC differ slightly. First, the two models differ at the 5' end. The horse genome assembly EquCab 2.0 contains a gap in the assembly near the 5' end of the FLNC gene. This results in a model in which the initiation codon of SEQ ID NO:6 is an ACG rather than the more typical ATG as in SEQ ID NO:7. Second, SEQ ID NO:7 contains a 63 base insertion from positions 1478 to 1540. It is likely that this is an annotation error in one of the models. Third, SEQ ID NO:6 and SEQ ID NO:7 differ with respect to the sequence starting at position 5652 in SEQ ID NO:6 and position 5415 in SEQ ID NO:7. The three-base sequence at this position is GAG in SEQ ID NO:6 and TGA in SEQ ID NO:7. Finally, SEQ ID NO:6 contains a 29 base insertion from positions 7856 to 7884. It is likely that this is an annotation error in one of the models. Note that the two base substitutions found in horses with Polysaccharide Storage Myopathy type 2 (PSSM2) or Myofibrillar Myopathy (MFM) do not occur in any of the areas of disagreement between the two models.

FIG. 7 shows the equine MYOZ3 coding sequence (SEQ ID NO:8, derived from XM_014730574.1), with exon 6 indicated in bold. The position of the thymine (T) to cytosine (C) substitution at position 125 in SEQ ID NO:8 (chr14:27,399,222 in SEQ ID NO:4) is underlined.

FIG. 8 shows two protein sequences derived from the translation of the equine MYOT coding sequence shown in FIG. 5. The wild-type or common protein sequence is shown as SEQ ID NO:9, while the MYOT-S232P variant sequence derived from the sequence bearing the thymine (T) to cytosine (C) substitution shown at chr14:38,519,183 in SEQ ID NO:1 (FIG. 1) and position 664 in SEQ ID NO:5 (FIG. 5) is shown as SEQ ID NO:10.

FIG. 9 shows an alignment of protein sequences derived from the translation of the equine FLNC sequences shown in FIG. 6 (SEQ ID NO:6 and SEQ ID NO:7). The entire FLNC coding nucleotide sequences shown in FIG. 6 were translated to give the wild-type or common amino acid sequences (SEQ ID NO:11, also known as F6ZWZ3, and SEQ ID NO:12, also known as XP_014594516.1). FIG. 9 shows translations of sequences bearing the guanine (G) to adenine (A) substitution at chr4:83736244 in FLNC (SEQ ID NO:2) shown in FIG. 2 and the guanine (G) to adenine (A) substitution at chr4:83738769 in FLNC (SEQ ID NO:3) shown in FIG.3. These substitution positions are also shown in FIG. 6 as position 2257 in SEQ ID NO:6 (corresponding to position 2506 in SEQ ID NO:7) and position 3619 in SEQ ID NO:6 (corresponding to position 3868 in SEQ ID NO: 7). The protein sequences derived from translation of the sequences with the substitutions are presented as SEQ ID NO:13 and SEQ ID NO:14.

The guanine (G) to adenine (A) substitution at chr4:83736244 in FLNC (SEQ ID NO:2) changes the glutamic acid (E) at position 753 in SEQ ID NO:11 and at position 836 in SEQ ID NO:12 to a lysine (K) at the corresponding positions in SEQ ID NO:13 and SEQ ID NO:14. This variant is referred to as FLNC-E753K.

The guanine (G) to adenine (A) substitution at chr4:83738769 in FLNC (SEQ ID NO:3) changes the alanine (A) at position 1207 in SEQ ID NO:11 and at position 1290 in SEQ ID NO:12 to a threonine (T) at the corresponding positions in SEQ ID NO:13 and SEQ ID NO:14.. This variant is referred to as FLNC-A1207T.

The differences between the two models for the coding sequence of equine FLNC described in the discussion of FIG. 6 above produce differences in the amino acid sequences of the predicted proteins, as shown in SEQ ID NO: 13 and SEQ ID NO: 14 in FIG. 9. As noted above, the two predicted protein sequences do not differ in the regions encoded by exon 15 or exon 21, which contain the sites of the FLNC-E753K and FLNC-A1207T substitutions.

FIG. 10 shows two protein sequences derived from the translation of the equine MYOZ3 coding sequence shown in FIG. 7. The wild-type or common protein sequence is shown as SEQ ID NO: 15, while the MYOZ3-S42L variant sequence derived from the sequence bearing the cytosine (C) to thymine (T) substitution shown at chr14:38,519,183 in SEQ ID NO:4 (FIG. 4) and position 125 in SEQ ID NO:8 (FIG. 7) is shown as SEQ ID NO: 16.

FIG. 11 shows MYOT exon 6 and flanking genomic DNA sequence from which PCR primers to amplify genomic DNA containing the site of the MYOT-S232P mutation would be most appropriately derived. Genomic coordinates are as in FIG. 1. Exon 6 from chr14:38,519,913 to chr14:38,519,061 is shown broken into codons in the correct reading frame for the wild-type allele (SEQ ID NO:17) and the MYOT-S232P allele (SEQ ID NO:18). Only the reference sequence from the assembly is shown for the flanking sequences. The site of a A to G mutation site at nucleotide position chr14:38,519,183 is shown in bold (T to C in the reverse complement as shown). This changes the underlined three base codon from one coding for a serine (TCT) to one coding for a proline (CCT). Example primers used experimentally to amplify genomic DNA containing the mutation site are shown in lower case (SEQ ID NO: 19 and SEQ ID NO:20).

FIG. 12 shows FLNC exon 15 and flanking genomic DNA sequence from which PCR primers to amplify genomic DNA containing the site of the FLNC-E753K mutation would be most appropriately derived. Genomic coordinates are as in FIG. 2. Exon 15 from chr4:83,736,133 to chr4:83,736,256 is shown broken into codons in the correct reading frame for the wild-type allele (SEQ ID NO:21) and the FLNC-E753K allele (SEQ ID NO:22). Only the reference sequence from the assembly is shown for the flanking sequences. The site of a G to A mutation site at nucleotide position chr4:83,736,244 is shown in bold. This mutation changes the underlined three base codon from one coding for a glutamic acid (GAG) to one coding for a lysine (AAG). Example primers used experimentally to amplify genomic DNA containing the mutation site are shown in lower case (SEQ ID NO:23 and SEQ ID NO:24).

FIG. 13 shows FLNC exon 21 and flanking genomic DNA sequence from which PCR primers to amplify genomic DNA containing the site of the FLNC-A1207T mutation would be most appropriately derived. Genomic coordinates are as in FIG. 3. Exon 21 from chr4:83,738,223 to chr4:83,738,820 is shown broken into codons in the correct reading frame for the wild-type allele (SEQ ID NO:25) and the FLNC-A1207T allele (SEQ ID NO:26). Only the reference sequences from the assembly are shown for the flanking sequences. The exon sequences are broken into codons in the correct reading frame. The site of a G to A mutation site at nucleotide position chr4:83,738,769 is shown in bold. This mutation changes the underlined three base codon from one coding for an alanine (GCT) to one coding for a threonine (ACT). Example primers used experimentally to amplify genomic DNA containing the mutation site are shown in lower case (SEQ ID NO:27 and SEQ ID NO:28).

FIG. 14 shows MYOZ3 exon 3 and flanking genomic DNA sequence from which PCR primers to amplify genomic DNA containing the site of the MYOZ3-S42L mutation would be most appropriately derived. Genomic coordinates are as in FIG. 4. Exon 3 from chr14:27,399,285 to chr14:27,399,131 is shown broken into codons in the correct reading frame for the wild-type allele (SEQ ID NO:29) and the MYOZ3-S42L allele (SEQ ID NO:30). Only the reference sequences from the assembly are shown for the flanking sequences. The exon sequences are broken into codons in the correct reading frame. The site of a G to A mutation site at nucleotide position chr14:27,399,222 is shown in bold (C to T in the reverse complement as shown). This mutation changes the underlined three base codon from one coding for a serine (TCG) to one coding for a leucine (TTG). Example primers used experimentally to amplify genomic DNA containing the mutation site are shown in lower case (SEQ ID NO:31 and SEQ ID NO:32).

Genomic DNA obtained from horses can be genotyped by amplifying a region containing a variant in the MYOT, FLNC, or MYOZ3 using Polymerase Chain Reaction (PCR), then sequencing the amplified DNA using Sanger sequencing. The results can be scored as homozygous for the common or wild-type allele, heterozygous for a nucleotide substitution, or homozygous for the nucleotide substitution.

FIG. 15 shows traces from Sanger DNA sequencing of amplified MYOT genomic DNA using primers shown in FIG. 11 (SEQ ID NO:19 and SEQ ID NO:20). The sequence of the forward strand is shown. The arrows in the figure indicate nucleotide position chr14:38,519,183, the site of a substitution of a guanine (G) for an adenine (A) in this position that creates the MYOT-S232P variant. The traces show, from left to right, results for a horse homozygous for the wild-type or common allele, results for a horse heterozygous for the substitution, and results for a horse homozygous for the substitution.

FIG. 16 shows traces from Sanger DNA sequencing of amplified FLNC genomic DNA using primers shown in FIG. 12 (SEQ ID NO:23 and SEQ ID NO:24). The sequence of the forward strand is shown. The arrows in the figure indicate nucleotide position chr4:83,736,244, the site of a substitution of an adenine (A) for a guanine (G) in this position that creates the FLNC-E753K variant. The traces show, from left to right, results for a horse homozygous for the wild-type or common allele, results for a horse heterozygous for the substitution, and results for a horse homozygous for the substitution.

FIG. 17 shows traces from Sanger DNA sequencing of amplified FLNC genomic DNA using primers shown in FIG. 13 (SEQ ID NO:27 and SEQ ID NO:28). The sequence of the forward strand is shown. The arrows in the figure indicate nucleotide position chr4:83,738,769, the site of a substitution of an adenine (A) for a guanine (G) in this position that creates the FLNC-A1207T variant. The traces show, from left to right, results for a horse homozygous for the wild-type or common allele, results for a horse heterozygous for the substitution, and results for a horse homozygous for the substitution.

FIG. 18 shows traces from Sanger DNA sequencing of amplified MYOZ3 genomic DNA using primers shown in FIG. 14 (SEQ ID NO:31 and SEQ ID NO:32). The sequence of the reverse strand is shown. The arrows in the figure indicate nucleotide position chr14:27,399,222, the site of a substitution of an thymine (T) for a cytosine (C) in this position that creates the MYOZ3-S42L variant. The traces show, from left to right, results for a horse homozygous for the wild-type or common allele, results for a horse heterozygous for the substitution, and results for a horse homozygous for the substitution.

FIG. 19 shows horse MYOT exon 6 and flanking genomic DNA sequence from which allele-specific PCR primers to amplify genomic DNA containing the site of the MYOT-S232P mutation would be most appropriately derived. Genomic coordinates are as in FIG. 1. Exon 6 from chr14:38,519,913 to chr14:38,519,061 is shown broken into codons in the correct reading frame for the wild-type allele (SEQ ID NO:17) and the MYOT-S232P allele (SEQ ID NO:18). Only the reference sequence from the assembly is shown for the flanking sequences. The site of a A to G mutation site at nucleotide position chr14:38,519,183 is shown in bold (T to C in the reverse complement as shown). This changes the underlined three base codon from one coding for a serine (TCT) to one coding for a proline (CCT). Example primers used experimentally to amplify genomic DNA containing the mutation site are shown in lower case. SEQ ID NO:33 is the common primer that is not allele-specific; the allele-specific primers SEQ ID NO:34 and SEQ ID NO:35 preferentially amplify the wild-type and MYOT-S232P alleles, respectively.

Two separate allele-specific real time reactions were prepared and were run together on the same PCR plate using the Strategene MX3000P real time PCR machine. The forward allele-specific primers, SEQ ID NO:34 (5'-TTGCATCCTGATCATTCACATCTCCCCTTGACGA-3'), which was used to detect the A-allele, and SEQ ID NO:35 (5'-TTGCATCCTGATCATTCACATCTCCCCTTGACGG-3'), which was used to detect the G-allele, were separately combined with the reverse common primer SEQ ID NO:33 (5'-GCACATGATAAGAATTGTCCATGGGGTACTCTGCA-3') in PCR reaction mix that contained 0.25 uM forward primer; 0.25 uM reverse primer; 1.5 mM $Mg_2Cl$; 50 mM KCl; 10 mM Tris-HCl (pH 8.3); 5% DMSO (v/v), 0.2 mM each of dATP, dCTP, dGTP and dTTP; 6.25 uM SYTO 21; and 0.5 unit of Amplitaq Gold (ThermoFisher). Reactions were carried out for $95^0$C for 10 min, 40 amplification cycles at $95^0$C for 15s, 60° C for 30 s and 720C for 30s. The CCD camera was set to capture the fluorescent signal during polymerization at 720C. At the end of the PCR amplification, a melting curve analysis was performed by heating the PCR extension product to $95^0$C for 1 min and then cooling to $55^0$C for 1 min before heating up to $95^0$C again at a rate of $0.3^0$C per second. The fluorescent signal was captured during the heating up of the PCR extension product from $55^0$C to $95^0$C.

The threshold cycles (Ct) of two separate allele-specific real time reactions were determined by the real time PCR machine. When an individual is homozygous for the A allele, i.e. A/A, there is a wide separation between the A-allele amplification curve and the G-allele amplification curve. The separation can be represented by ΔCt, i.e. subtracting the Ct value of the A-allele amplification curve from that of the G-allele amplification curve. When an individual is homozygous for the G allele, i.e. C/C, the ΔCt value will decrease to a negative value. The ΔCt values were determined and matched with their genotypes. A genotype of A/A, A/G and G/G were concluded if ΔCt was >5, -2< ΔCt >2, and <-5 respectively.

| Genotype | ΔCt |
|---|---|
| Normal | >5 |
| Heterozygous for mutation | -2 to 2 |
| Homozygous for mutation (affected) | <-5 |

FIG. 20 shows horse FLNC exon 15 and flanking genomic DNA sequence from which allele-specific PCR primers to amplify genomic DNA containing the site of the FLNC-E753K mutation would be most appropriately derived. Genomic coordinates are as in FIG. 2. Exon 15 from chr4:83,736,133 to chr4:83,736,256 is shown broken into codons in the correct reading frame for the wild-type allele (SEQ ID NO:21) and the FLNC-E753K allele (SEQ ID NO:22). Only the reference sequence from the assembly is shown for the flanking sequences. The site of a G to A mutation site at nucleotide position chr4:83,736,244 is shown in bold. This mutation changes the underlined three base codon from one coding for a glutamic acid (GAG) to one coding for a lysine (AAG). Example primers used experimentally to amplify genomic DNA containing the mutation site are shown in lower case. SEQ ID NO:36 is the common primer that is not allele-specific; the allele-specific primers SEQ ID NO:37 and SEQ ID NO:38 preferentially amplify the wild-type and FLNC-E753K alleles, respectively. Note that both allele-specific primers span the exon-intron boundary Note also that additional mismatches have been introduced into both allele-specific primers.

Two separate allele-specific real time reactions were prepared and were run together on the same PCR plate using the Strategene MX3000P real time PCR machine. The forward allele-specific primers, SEQ ID NO:37 (5'-GGCTGGTGCACCTTGCCCCGCGTC-3'), which was used to detect the G-allele, and SEQ ID NO:38 (5'-GGCTGGTGCACCTTGCCCCGCGTT-3'), which was used to detect the A-allele, were separately combined with the reverse common primer SEQ ID NO:36 (5'-TGTCGCTGGGCCCTGGTCACTGCTC-3') in PCR reaction mix that contained 0.25 uM forward primer; 0.25 uM reverse primer; 1.5 mM $Mg_2Cl$; 50 mM KCl; 10 mM Tris-HCl (pH 8.3); 5% DMSO (v/v), 0.2 mM each of dATP, dCTP, dGTP and dTTP; 6.25 uM SYTO 21; and 0.5 unit of Amplitaq Gold (ThermoFisher). Reactions were carried out for $95^0$C for 10 min, 40 amplification cycles at $95^0$C for 15s, 60° C. for 30 s and $72^0$C for 30s. The CCD camera was set to capture the fluorescent signal during polymerization at $72^0$C. At the end of the PCR amplification, a melting curve analysis was performed by heating the PCR extension product to $95^0$C for 1 min and then cooling to $55^0$C for 1 min before heating up to $95^0$C again at a rate of $0.3^0$C per second. The fluorescent signal was captured during the heating up of the PCR extension product from $55^0$C to $95^0$C.

The threshold cycles (Ct) of two separate allele-specific real time reactions were determined by the real time PCR machine. When an individual is homozygous for the G allele, i.e. G/G, there is a wide separation between the G-allele amplification curve and the A-allele amplification curve. The separation can be represented by ΔCt, i.e. subtracting the Ct value of the G-allele amplification curve from that of the A-allele amplification curve. When an individual is homozygous for the A allele, i.e. A/A, the ΔCt value will decrease to a negative value. The ΔCt values were determined and matched with their genotypes. A genotype of G/G, G/A and A/A were concluded if ΔCt was >5, -2< ΔCt >2, and <-5 respectively..

| Genotype | ΔCt |
|---|---|
| Normal | >5 |
| Heterozygous for mutation | -2 to 2 |
| Homozygous for mutation (affected) | <-5 |

FIG. 21 shows horse FLNC exon 21 and flanking genomic DNA sequence from which allele-specific PCR primers to amplify genomic DNA containing the site of the FLNC-A1207T mutation would be most appropriately derived. Genomic coordinates are as in FIG. 3. Exon 21 from chr4:83,738,223 to chr4:83,738,820 is shown broken into codons in the correct reading frame for the wild-type allele (SEQ ID NO:25) and the FLNC-A1207T allele (SEQ ID NO:26). Only the reference sequences from the assembly are shown for the flanking sequences. The site of a G to A mutation site at nucleotide position chr4:83,738,769 is shown in bold. This mutation changes the underlined three base codon from one coding for an alanine (GCT) to one coding for a threonine (ACT). Example primers used experimentally to amplify genomic DNA containing the mutation site are shown in lower case. SEQ ID NO:39 is the common primer that is not allele-specific; the allele-specific primers SEQ ID NO:40 and SEQ ID NO:41 preferentially amplify the wild-type and FLNC-A1207T alleles, respectively. Note that additional mismatches have been introduced into both allele-specific primers.

Two separate allele-specific real time reactions were prepared and were run together on the same PCR plate using the Strategene MX3000P real time PCR machine. The forward allele-specific primers, SEQ ID NO:40 (5'-ACCCGCGTCCATGTGCAGCGCG-3'), which was used to detect the G-allele, and SEQ ID NO:41 (5'-ACCCGCGTCCATGTGCAGCGCA-3'), which was used to detect the A-allele, were separately combined with the reverse common primer SEQ ID NO:39 (5-CCAGGGCTGTCCCCAAGTCCTCCC-3) in PCR reaction mix that contained 0.25 uM forward primer; 0.25 uM reverse primer; 1.5 mM $Mg_2Cl$; 50 mM KCl; 10 mM Tris-HCl (pH 8.3); 5% DMSO (v/v), 0.2 mM each of dATP, dCTP, dGTP and dTTP; 6.25 uM SYTO 21; and 0.5 unit of Amplitaq Gold (ThermoFisher). Reactions were carried out for $95^0$C for 10 min, 40 amplification cycles at $95^0$C for 15s, 60° C. for 30 s and $72^0$C for 30s. The CCD camera was set to capture the fluorescent signal during polymerization at 720C. At the end of the PCR amplification, a melting curve analysis was performed by heating the PCR extension product to $95^0$C for 1 min and then cooling to $55^0$C for 1 min before heating up to $95^0$C again at a rate of $0.3^0$C per second. The fluorescent signal was captured during the heating up of the PCR extension product from $55^0$C to $95^0$C.

The threshold cycles (Ct) of two separate allele-specific real time reactions were determined by the real time PCR machine. When an individual is homozygous for the G allele, i.e. G/G, there is a wide separation between the G-allele amplification curve and the A-allele amplification curve. The separation can be represented by ΔCt, i.e. subtracting the Ct value of the G-allele amplification curve from that of the A-allele amplification curve. When an individual is homozygous for the A allele, i.e. A/A, the ΔCt value will decrease to a negative value. The ΔCt values were determined and matched with their genotypes. A genotype of G/G, G/A and A/A were concluded if ΔCt was >5, -2< ΔCt >2, and <-5 respectively.

| Genotype | ΔCt |
|---|---|
| Normal | >5 |

(continued)

| Genotype | ΔCt |
|---|---|
| Heterozygous for mutation | -2 to 2 |
| Homozygous for mutation (affected) | <-5 |

FIG. 22 shows horse MYOZ3 exon 3 and flanking genomic DNA sequence from which allele-specific PCR primers to amplify genomic DNA containing the site of the MYOZ3-S42L mutation would be most appropriately derived. Genomic coordinates are as in FIG. 4. Exon 3 from chr14:27,399,285 to chr14:27,399,131 is shown broken into codons in the correct reading frame for the wild-type allele (SEQ ID NO:29) and the MYOZ3-S42L allele (SEQ ID NO:30). Only the reference sequences from the assembly are shown for the flanking sequences. The site of a G to A mutation site at nucleotide position chr14:27,399,222 is shown in bold (C to T in the reverse complement as shown). This mutation changes the underlined three base codon from one coding for a serine (TCG) to one coding for a leucine (TTG). Example primers used experimentally to amplify genomic DNA containing the mutation site are shown in lower case. SEQ ID NO:42 is the common primer that is not allele-specific; the allele-specific primers SEQ ID NO:43 and SEQ ID NO:44 preferentially amplify the wild-type and MYOZ3-S42L alleles, respectively. Note that additional mismatches have been introduced into both allele-specific primers.

Two separate allele-specific real time reactions were prepared and were run together on the same PCR plate using the Strategene MX3000P real time PCR machine. The forward allele-specific primers, SEQ ID NO:43 (5'-GCCCCAGGACCTGATGATGGAAGAGCTCTC -3'), which was used to detect the C-allele, and SEQ ID NO:44 (5'-GCCCCAGGACCTGATGATGGAAGAGCTCTT-3'), which was used to detect the T-allele, were separately combined with the reverse common primer SEQ ID NO:42 (5'-GGCCAGAGGTCCTCCCCTGGCT-3') in PCR reaction mix that contained 0.25 uM forward primer; 0.25 uM reverse primer; 2.5 mM $Mg_2Cl$; 50 mM KCl; 10 mM Tris-HCl (pH 8.3); 5% DMSO (v/v), 0.2 mM each of dATP, dCTP, dGTP and dTTP; 6.25 uM SYTO 21; and 0.5 unit of Amplitaq Gold (Thermo-Fisher). Reactions were carried out for $95^0$C for 10 min, 40 amplification cycles at $95^0$C for 15s, 60° C. for 30 s and $72^0$C for 30s. The CCD camera was set to capture the fluorescent signal during polymerization at $72^0$C. At the end of the PCR amplification, a melting curve analysis was performed by heating the PCR extension product to 950C for 1 min and then cooling to $55^0$C for 1 min before heating up to $95^0$C again at a rate of $0.3^0$C per second. The fluorescent signal was captured during the heating up of the PCR extension product from $55^0$C to $95^0$C.

The threshold cycles (Ct) of two separate allele-specific real time reactions were determined by the real time PCR machine. When an individual is homozygous for the C allele, i.e. C/C, there is a wide separation between the C-allele amplification curve and the T-allele amplification curve. The separation can be represented by ΔCt, i.e. subtracting the Ct value of the C-allele amplification curve from that of the T-allele amplification curve. When an individual is homozygous for the Tallele, i.e. T/T, the ΔCt value will decrease to a negative value. The ΔCt values were determined and matched with their genotypes. A genotype of C/c, C/T and T/T were concluded if ΔCt was >5, -2< ΔCt >2, and <-5 respectively.

| Genotype | ΔCt |
|---|---|
| Normal | >5 |
| Heterozygous for mutation | -2 to 2 |
| Homozygous for mutation (affected) | <-5 |

The human orthologs of the equine MYOT, FLNC, and MYOZ3 genes and the human proteins that these genes encode are richly annotated with experimental data derived from genetic and biochemical studies. It is informative to compare the amino acid substitutions in MYOT, FLNC, and MYOZ3 found in horses to the information on protein domains and clinically significant variation in the human myotilin (MYOT), filamin-C (FLNC), and myozenin-3 (MYOZ3) proteins. In order to do this, the equine protein models used in this disclosure must be compared to the canonical or reference sequence of the human proteins in a public database that captures data from the published literature, such as UniProt.

FIG. 23 shows the alignment of the sequence of a portion of the human MYOT protein with the horse protein sequence SEQ ID NO:9 shown in FIG. 8. The top line (indicated as Human) corresponds to a portion of the human myotilin protein (MYOT) from UniProt Q9UBF9. The second line shows the alignment of the human sequence to the horse sequence SEQ ID NO:9. A single conservative amino acid substitution is seen at amino acid 232. The last line, indicated as MYOT-S232P, shows the position of the S232P nonconservative substitution, at the same position as the conservative substitution between human and horse. The numbering of the amino acid positions in the horse myotilin protein model presented as SEQ ID NO:9 in FIG. 8 corresponds precisely to the numbering of the human amino acid positions in the human myotilin model UniProt Q9UBF9.

FIG. 24 shows the alignment of the sequence of filamin repeat 6 of the human FLNC protein with the horse protein sequences SEQ ID NO:11 and SEQ ID NO:12 shown in FIG. 9. The top line (indicated as Human) corresponds to filamin repeat 6 of human filamin-C protein (FLNC) from UniProt Q14315. The second line shows the alignment of the human sequence to the horse sequences SEQ ID NO:11 and SEQ ID NO:12, which are identical over this region. A single conservative amino acid substitution between human and horse is seen at amino acid 766 in the human sequence. The third line (indicated as ENS) corresponds to filamin repeat 6 of horse filamin-C protein (FLNC) with the numbering of amino acid positions as in SEQ ID NO:11. The fourth line (indicated as XP) corresponds to filamin repeat 6 of horse filamin-C protein (FLNC) with the numbering of amino acid positions as in SEQ ID NO:12. The last line (indicated as E753K) shows the position of the E753K substitution. The equine FLNC-E753K missense allele corresponds to amino acid position 793 in the human FLNC protein, located in filamin repeat 6.

FIG. 25 shows the alignment of the sequence of filamin repeat 11 of the human FLNC protein with the horse protein sequences SEQ ID NO:11 and SEQ ID NO:12 shown in FIG. 9. The top line (indicated as Human) corresponds to filamin repeat 11 of human filamin-C protein (FLNC) from UniProt Q14315. The second line shows the alignment of the human sequence to the horse sequences SEQ ID NO:11 and SEQ ID NO:12, which are identical over this region. Two conservative amino acid substitutions between human and horse are seen at amino acids 1248 and 1332 in the human sequence. The third line (indicated as ENS) corresponds to filamin repeat 11 of horse filamin-C protein (FLNC) with the numbering of amino acid positions as in SEQ ID NO:11. The fourth line (indicated as XP) corresponds to filamin repeat 11 of horse filamin-C protein (FLNC) with the numbering of amino acid positions as in SEQ ID NO:12. The last line (indicated as A1207T) shows the position of the A1207T substitution. The equine FLNC-A1207T missense allele corresponds to amino acid position 1247 in the human FLNC protein, located in filamin repeat 11.

The immunoglobulin-like filamin repeats found in filamin C (as well as in the paralogs filamin A and filamin B) are antiparallel beta sheets. FIG. 26 shows the general structure of antiparallel and parallel beta sheets. Beta sheets are held together by hydrogen bonding between N-H groups in the backbone of one strand and the C=O groups in the backbone of the adjacent strand. In an antiparallel beta sheet, the adjacent strands have opposite polarity with respect to the N- and C-termini. In a parallel beta sheet, the adjacent strands have the same polarity with respect to the N- and C-termini. Comparison of the two structures shows that R groups are in close opposition in an antiparallel beta sheet, while R groups in a parallel beta sheet occupy the space between the N-H group and the C=O group of the adjacent strand.

The structure of the antiparallel beta sheet in immunoglobulin-like filamin repeats makes it possible to understand why the equine FLNC-E753K and FLNC-A1207T alleles potentially disrupt the protein structure of filamin C. In the protein encoded by FLNC-E753K, a negatively-charged amino acid, glutamic acid, is replaced by a positively-charged amino acid, lysine. The R groups are relatively large and of comparable size. The R group of the negatively charged glutamic acid in the wild-type filamin C is in close proximity to an unknown R group on the adjacent stand. If the opposite R group is positively charged, the interaction of the R groups in the wild-type protein would stabilize the antiparallel beta sheet of filamin repeat 6. Substitution of the R group of the positively charged amino acid lysine in this position would be expected to be destabilizing.

In the protein encoded by FLNC-A1207T, a hydrophobic amino acid, alanine, is replaced by a polar uncharged amino acid, threonine. The R group of alanine is among the smallest R groups found in amino acids, and would show hydrophobic interactions with seven other amino acids (valine, isoleucine, leucine, methionine, phenylalanine, tyrosine, and tryptophan) with larger R groups. Substitution of the R group of the larger polar uncharged amino acid threonine in this position would be expected to be destabilizing. In addition, the human FLNC-A1539T allele, associated with a dominant pathogenic phenotype, has the same amino acid substitution in a comparable position in filamin repeat 14.

FIG. 27 shows the alignment of the sequence of a portion of the human MYOZ3 protein with the horse protein sequence SEQ ID NO: 15 shown in FIG. 10. The top line (indicated as Human) corresponds to a portion of the human myozenin-3 protein (MYOZ3) from UniProt Q8TDC0. The second line shows the alignment of the human sequence to the horse sequence SEQ ID NO: 15. Five nonconservative substitutions are seen at positions 14, 17, 18, 22, and 66. The last line, indicated as MYOZ3-S42L, shows the position of the S42L nonconservative substitution. The numbering of the amino acid positions in the horse myozenin-3 protein model presented as SEQ ID NO: 15 in FIG. 10 corresponds precisely to the numbering of the human amino acid positions in the human myozenin-3 model UniProt Q8TDC0.

FIG. 28 shows features of the human MYOT protein. The top line shows a linear representation of the 498 amino acid human myotilin protein. The locations of pathogenic amino acid substitutions summarized in TABLE 1 below are indicated. The second line shows the amino acids encoded by exon 6 (228 to 272), with the position of the equine MYOT-S323P mutation indicated. The third line shows the region (79 to 150) that has been shown to interact with alpha-actinin (ACTN1). The fourth line shows the region (215 to 498) that has been shown to interact with actin (ACTA1). The last line shows the region (215 to 493) that has been shown to interact with filamin-C (FLNC).

TABLE 1. Pathogenic variants of human MYOT and the associated diseases.

| MYOT substitution | Disease |
|---|---|
| R6H | Limb-Girdle Muscular Dystrophy, type 1A |
| S39F | Spheroid Body Myopathy |
| S55F | Limb-Girdle Muscular Dystrophy, type 1A; Myofibrillar Myopathy, type 3 |
| T57I | Limb-Girdle Muscular Dystrophy, type 1A |
| S60C | Myofibrillar Myopathy, type 3; Distal Myopathy |
| S60F | Myofibrillar Myopathy, type 3; Distal Myopathy |
| S95I | Myofibrillar Myopathy, type 3 |
| R405K | Limb-Girdle Muscular Dystrophy, type 1A |

All of the pathogenic MYOT alleles listed in TABLE 1 are amino acid substitutions inherited as dominant variants-i.e., individuals heterozygous for the variant and a normal allele are affected. In contrast, mice homozygous for a knock-out allele of MYOT (i.e., a mutation that has been created in vitro that completely eliminates the expression of MYOT) appear normal, with normal viability, fertility, and lifespan. Their muscle capacity does not appear to differ from wild-type mice. They show normal muscle sarcomeric and sarcolemmal integrity. There are no alterations in the heart or other organs of newborn or adult mice homozygous for the MYOT knockout allele. These results suggest that, in mouse, MYOT either plays no role in muscle development or function, or that the MYOT protein is redundant, and proteins encoded by other regions of the genome are capable of fulfilling the role of myotilin when it is absent.

Because human alleles of MYOT with single amino acid substitutions are pathogenic, myotilin may normally be involved in muscle development and function, but that other proteins may substitute for myotilin when it is absent.

Myotilin is one of a group of structural proteins in muscle that are important for the integrity of sarcomeres. The amino terminus of myotilin is unique-i.e., it does not share significant sequence similarity with other proteins-and is rich in serine residues. The carboxy terminus of myotilin is highly conserved within the family, consisting of immunoglobulin (Ig)-like domains similar in amino acid sequence to immunoglobulin (Ig)-like domains in the muscle proteins myosin-binding protein C, titin, palladin, and myopallidin. The immunoglobulin (Ig)-like domains of these proteins are known to bind to actin, myosin, or both, and in myotilin, are involved in homodimer formation. Myotilin is expressed in skeletal and cardiac muscle, where it co-localizes with the actin binding protein alpha-actinin in sarcomeric I bands. It binds F-actin and filamin and interacts directly with alpha-actinin. The regions of the myotilin protein that interact with other proteins have been defined in yeast two-hybrid experiments.

FIG. 29 shows features of the human FLNC protein. The top line shows a linear representation of the 2725 amino acid human filamin-C protein (UniProt Q14315) with key features indicated. The actin binding domain with domains CH1 and CH2 is located at the amino terminus. Most of the molecule consists of filamin repeats, numbered 1-24. There are two hinge domains, H1 and H2. Between filamin repeat 19 and the partial filamin repeat 20 is an 82 amino acid region not found in filamin-A or filamin-B that is required for localization to the Z disc and for interaction with myotilin. The carboxy-terminal region including H2 and filamin repeat 24 is required for dimerization. The locations of pathogenic amino acid substitutions found in human patients and summarized in TABLE 2 below are indicated (human variants). The locations of amino acid substitutions found in horses with Polysaccharide Storage Myopathy type 2 (PSSM2), also known as Myofibrillar Myopathy (MFM), are shown in the second line (equine variants). The substitution shown in FIG. 24 is indicated as E753K while the substitution shown in FIG. 25 is indicated as A1207T. The amino acid positions affected by the E753K and A1207T variants in horse correspond to positions 793 and 1247 in the human FLNC sequence represented by UniProt Q14315.

Filamin-C is one of a group of structural proteins in muscle that are important for the development and integrity of sarcomeres. Filamin-C consists of an amino-terminal actin-binding domain, 24 filamin repeats that are structurally similar to immunoglobulin repeats, and a carboxy-terminal dimerization domain. The paralogs filamin-A and filamin-B are actin-binding proteins expressed in a wide variety of tissues whose structure is very similar to filamin C. One unique feature of filamin-C is the insertion of a segment of 82 amino acids between filamin repeat 19 and the partial filamin repeat 20. This segment is required for the targeting of filamin-C to the Z disc and its interaction with myotilin.

Mice homozygous for an allele of FLNC that is missing the last eight exons, encoding a protein that is missing the segment beginning in filamin repeat 20, die shortly after birth due to respiratory failure. They exhibit defects in primary myogenesis including variations in muscle fiber size with centrally located nuclei. Mice heterozygous for the truncated FLNC allele are viable and fertile, and do not exhibit a gross defect in muscle development or function. The protein product of the truncated FLNC allele is expressed at very low levels in both homozygotes and heterozygotes. These results demonstrate that filamin-C is required for the normal development of muscle fibers, and that a hypomorphic (partial loss-of-function) allele is recessive.

Mutations in the FLNC coding region have been shown to cause various myopathies in humans. Most of these diseases are produced by missense alleles (amino acid substitutions), with one example of an in-frame deletion that removes four amino acids. They are inherited as dominant mutations and are fully penetrant-i.e., there are no unaffected individuals heterozygous for the mutant allele. In humans, various mutations in FLNC cause Myofibrillar Myopathy 5, Familial Hypertrophic Cardiomyopathy 26, Distal Myopathy 4, and Familial Restrictive Cardiomyopathy 5. Some of the specific mutations and the disease states produced are summarized in TABLE 2.

TABLE 2. Pathogenic variants of human FLNC and the associated diseases.

| FLNC substitution | Disease |
|---|---|
| V123A | Familial Hypertrophic Cardiomyopathy 26 |
| A193T | Distal Myopathy 4 |
| M251T | Distal Myopathy 4 |
| V930-T933del | Myofibrillar Myopathy 5 |
| A1539T | Familial Hypertrophic Cardiomyopathy 26 |
| S1624L | Familial Restrictive Cardiomyopathy 5 |
| I2160F | Familial Hypertrophic Cardiomyopathy 26 |
| H2315N | Familial Hypertrophic Cardiomyopathy 26 |
| W2710X | Myofibrillar Myopathy 5 |

The in-frame deletion V930-T933del and all of the amino acid substitutions listed in TABLE 2 are pathogenic FLNC alleles inherited as dominant variants-i.e., individuals heterozygous for the variant and a normal allele are affected. Filamin-C is known to function as a dimer. If normal and mutant alleles of filamin-C are expressed at comparable levels, an individual heterozygous for a missense allele is expected to have only 25% fully normal dimers, with 50% of the dimers having one normal and one mutant protein, and 25% having two mutant proteins. This explains why missense alleles are identified as dominant pathogenic variants in humans, while a loss-of-function allele is a recessive lethal mutation in mice.

FIG. 29 also shows the position of the equine FLNC-E753K and FLNC-A1207T alleles found in horses mapped against the amino acid sequence of the human FLNC protein. The evidence used to assign the horse missense alleles to appropriate positions in the human FLNC protein is shown in FIG. 24 and FIG 25. The equine FLNC-E753K missense allele corresponds to amino acid position 793 in the human FLNC protein, located in filamin repeat 6. The equine FLNC-A1207T missense allele corresponds to amino acid position 1247 in the human FLNC protein, located in filamin repeat 11.

FIG. 30 shows features of the human MYOZ3 protein. The top line shows a linear representation of the 251 amino acid human myozenin-3 protein (UniProt Q8TDC0). No pathogenic human alleles are known. The location of the equine MYOZ3-S42L is shown. The second line shows a region of the human MYOZ3 protein shown to bind the alpha-actinin (ACTN1), calcineurin, and telethonin (TCAP) proteins. Calcineurin is a calcium- and calmodulin-dependent serine/threonine protein phosphatase made up of one calmodulin-binding catalytic subunit encoded by three different genes (PPP3CA, PPP3CB, and PPP3CC) and a one regulatory subunit encoded by two different genes (PPP3R1 and PPP3R2). The third line shows a region of the human MYOZ3 protein shown to bind filamin-C (FLNC) protein. The fourth line shows a second region of the human MYOZ3 protein shown to bind alpha-actinin (ACTN1) protein.

Myozenin-3, originally called calsarcin-3, is expressed solely in skeletal muscle. It was identified biochemically as a protein that coimmunoprecipitated with cacineurin, telethonin (TCAP), alpha-actinin-2 (ACTN2), and filamin-C (FLNC). Myozenin-3 interacts with LIM Domain-binding 3 (LDB3) as determined by a yeast two-hybrid assay. Myozenin-3 is localized to the Z disc and may serve to link its various binding proteins at the Z disc.

There is no information on clinically significant alleles of human MYOZ3, and there are currently no mouse knockout alleles of Myoz3 or other mouse models.

In order to assess the effects of amino acid substitutions resulting from mutations detected in horses with Polysaccharide Storage Myopathy type 2 (PSSM2), also known as Myofibrillar Myopathy, described in this disclosure as MYOT-S232P, FLNC-E753K, FLNC-A1207T, and MYOZ3-S42L, are pathogenic, the predicted sequences of myotilin (MYOT), filamin-C (FLNC), and myozenin-3 (MYOZ3) from diverse organisms were retrieved from GenBank using BLASTP searches with query sequences derived from canonical human sequences. The retrieved sequences were grouped into clusters of identical sequences if any amino acid sequences retrieved from different species were identical. The clustered sequences were aligned using CLUSTAL OMEGA with the default parameters. Amino acids observed in particular positions in the aligned sequences may be fully conserved (no change in the amino acid found at this position is observed in any species), highly conserved (with only highly conservative substitutions, such as serine (S) for threonine (T)), moderately conserved (such as serine (S) for arginine (R)), or not conserved (with nonconservative substitutions such as serine (S) for proline (P)). If substitutions like MYOT-S232P, FLNC-E753K, FLNC-A1207T, and MYOZ3-S42L occur in positions that are poorly conserved across different species, they are not likely to be pathogenic. If, on the other hand, substitutions like MYOT-S232P, FLNC-E753K, FLNC-A1207T, and MYOZ3-S42L occur in positions that are highly conserved across species, and these specific substitutions are not seen in natural populations, it is likely that these substitutions negatively affect muscle function and therefore reproductive fitness, and when they have occurred in natural populations, they have been eliminated by natural selection.

FIG. 31 shows the amino acid sequences of proteins encoded by MYOT genes, centered on the position of the equine MYOT-S232P substitution. The next to the last line (labeled CLUSTAL) shows the consensus sequence, where positions with fully conserved amino acids are represented by an asterisk (*), positions with strongly conserved amino acids are indicated by a colon (:), positions with weakly conserved amino acids are indicated are indicated by period (.), and nonconserved positions are indicated by a blank space ( ). The last line shows the sequence of myotilin in horse with the MYOT-S232P substitution shown and highlighted in bold. The position of the MYOT-S232P substitution is indicated in bold in all of the sequences.

The 99 species in the alignment shown in FIG. 31 are: primate1 [Human (*Homo sapiens*), Chimpanzee *(Pan troglodytes),* Bonobo *(Pan paniscus),* Western lowland gorilla (*Gorilla gorilla gorilla*), Sumatran orangutan (*Pongo abelii*)], primate2 [Crab-eating macaque (*Macaca fascicularis*)*,* Rhesus macaque (*Macaca mulatta*)*,* Drill (*Mandrillus leucophaeus*), Olive baboon (*Papio anubis*), Sooty mangabey (*Cercocebus atys*), Golden snub-nosed monkey (*Rhinopithecus roxellana*)], mammal1 [Horse (*Equus caballus*), Siberian tiger (*Panthera tigris altaica*), Cheetah (*Acinonyx jubatus*), Cat *(Felis catus),* Cape golden mole (*Chrysochloris asiatica*)*,* Ferret (*Mustelaputorius furo*)]*,* mammal2 [Cattle (*Bos taurus*), Domestic yak (*Bos mutus*)]*,* mammal3 [Alpaca (*Vicugnapacos*), Bactrian camel (*Camelus ferus*)], mammal4 [Brandt's bat (*Myotis brandtii*)*,* Little brown bat (*Myotis lucifugus*), Big brown bat (*Eptesicus fuscus*)], mammal5 [Bottle-nosed dolphin (*Tursiops truncates*), Orca (*Orcinus orca*)], mammal6 [Polar bear (*Ursus maritimus*), Giant panda (*Ailuropoda melanoleuca*)], mammal7 [Black flying fox (*Pteropus alecto*), Large flying fox (*Pteropus vampyrus*)], mammal8 [Alpine marmot (*Marmota marmota marmota*), Thirteen-lined ground squirrel (*Ictidomys tridecemlineatus*)]*,* White-headed capuchin (*Cebus capucinus imitator*), Green monkey (*Chlorocebus sabaeus*), Gray mouse lemur (*Microcebus murinus*), Northern greater galago (*Otolemur garnettii*), Water buffalo (*Bubalus bubalis*), Norway rat (*Rattus norvegicus*), Naked mole-rat (*Heterocephalus glaber*), Mouflon (*Ovis aries musimon*), North American beaver (*Castor canadensis*), Dog (*Canis lupus familiaris*), Natal long-fingered bat (*Miniopterus natalensis*), Black flying fox (*Pteropus alecto*), Sperm whale *(Physeter catodon),* Florida manatee (*Trichechus manatus latirostris*), Lesser hedgehog tenrec (*Echinops telfairi*), European hedgehog (*Erinaceus europaeus*), Nine-banded armadillo (*Dasypus novemcinctus*), Sunda pangolin (*Manis javanica*), Aardvark (*Orycteropus afer afer*)*,* Gray short-tailed opossum (*Monodelphis domestica*), Tasmanian devil (*Sarcophilus harrisii*), Platypus (*Ornithorhynchus anatinus*), bird1 [Swan goose (*A user cygnoides domesticus*), Mallard (*Anas platyrhynchos*)], bird2 [Blue-crowned manakin (*Lepidothrix coronata*), Golden-collared manakin (*Manacus vitellinus*)], bird3 [Adelie penguin (*Pygoscelis adeliae*)*,* Japanese quail (*Coturnix japonica*), Common cuckoo (*Cuculus canorus*), Great cormorant (*Phalacrocorax carbo*), American flamingo (*Phoenicopterus ruber ruber*), Red-legged seriema (*Cariama cristata*), Turkey vulture (*Cathartes aura*), Grey crowned crane (*Balearica regulorum gibbericeps*), White-tailed tropicbird (*Phaethon lepturus*), Chuck-will's-widow (*Caprimulgus carolinensis*), Red-throated loon (*Gavia stellata*), Red-crested turaco (*Tauraco erythrolophus*)]*,* bird4 [White-throated sparrow (*Zonotrichia albicollis*), Medium ground finch (*Geospiza fortis*)], bird5 [Atlantic canary (*Serinus canaria*), European starling *(Sturnus vulgaris),* Ground tit *(Pseudopodoces humilis),* Rock dove (*Columba livia*), Ruff (*Calidris pugnax*), Zebra finch (*Taeniopygia guttata*), Rifleman (*Acanthisitta chloris*)]*,* Red junglefowl (*Gallus gallus*), White-tailed eagle (*Haliaeetus albicilla*), White-throated tinamou (*Tinamus guttatus*), Emperor penguin (*Aptenodytes forsteri*), Turkey (*Meleagris gallopavo*), Golden eagle (*Aquila chrysaetos canadensis*), Sunbittern (*Eurypyga helias*), Speckled mousebird (*Colius striatus*), Budgerigar (*Melopsittacus undulatus*), Collared flycatcher (*Ficedula albicollis*), Southern ostrich (*Struthio camelus australis*), North island brown kiwi (*Apteryx australis mantelli*), Northern carmine bee-eater (*Merops nubicus*), Dalmatian pelican (*Pelecanus crispus*), Little egret (*Egretta garzetta*)*,* Barn owl (*Tyto alba*), Peregrine falcon (*Falco peregrinus*), Kea (*Nestor notabilis*), Downy woodpecker (*Picoides pubescens*), Hoatzin (*Opisthocomus hoazin*).

In most of the species shown in FIG. 31, the amino acid affected by the equine MYOT-S232P variant is a serine (S). The sole exception is the primate1 cluster [Human (*Homo sapiens*), Chimpanzee *(Pan troglodytes),* Bonobo *(Pan paniscus),* Western lowland gorilla (*Gorilla gorilla gorilla*), Sumatran orangutan (*Pongo abelii*)]. The closely related species in the primate1 cluster have a conservative substitution of a threonine (T) for a serine (S) in this position. The equine variant MYOT-S232P is a nonconservative substitution in a highly conserved position, with sequence conservation extending to birds, which diverged from mammals approximately 300 million years ago. Many other positions in the aligned sequences show no conservation over this evolutionary distance. This is evidence that the MYOT-S232P variant is pathogenic, and has been eliminated from populations by natural selection when it has occurred.

FIG. 32 shows the amino acid sequences of filamin repeat 6 of FLNC genes, showing filamin repeat 6, which contains the equine FLNC-E753K substitution. The next to the last line (labeled CLUSTAL) shows the consensus sequence, where positions with fully conserved amino acids are represented by an asterisk (*), positions with strongly conserved amino acids are indicated by a colon (:), positions with weakly conserved amino acids are indicated are indicated by period (.), and nonconserved positions are indicated by a blank space ( ). The last line shows the sequence of filamin repeat 6 in horse with the FLNC-E753K substitution shown and highlighted in bold. The position of the FLNC-E753K substitution is indicated in bold in all of the sequences.

The 124 species in the alignment shown in Figure 32 are: mammal 1 [Human (*Homo sapiens*), Common chimpanzee *(Pan troglodytes),* Bonobo *(Pan paniscus),* Western lowland gorilla (*Gorilla gorilla gorilla*), Sumatran orangutan (*Pongo abelii*), Southern pig-tailed macaque (*Macaca nemestrina*), Olive baboon (*Papio anubis*), White-headed capuchin (*Cebus capucinus imitator*), Coquerel's sifaka (*Propithecus coquereli*), Bolivian Squirrel Monkey (*Saimiri boliviensis boliviensis*), Sooty mangabey (*Cercocebus atys*), Angola colobus (*Colobus angolensis palliatus*), Green monkey (*Chlorocebus sabaeus*), Common marmoset *(Callithrix jacchus*), Black snub-nosed monkey (*Rhinopithecus bieti*), Crab-eating macaque (*Macaca fascicularis*), Drill (*Mandrillus leucophaeus*), Rhesus macaque (*Macaca mulatta*)*,* Nancy Ma's night monkey (*Aotus nancymaae*), Golden snub-nosed monkey (*Rhinopithecus roxellana*), Northern greater galago (*Otolemur garnettii*), Damara mole rat (*Fukomys damarensis*), Cape elephant shrew (*Elephantulus edwardii*), Malayan flying fox (*Pteropus vampyrus*), Black flying fox (*Pteropus alecto*), Big brown bat (*Eptesicus fuscus*), Natal long-fingered bat (*Miniopterus natalensis*), Egyptian fruit bat (*Rousettus aegyptiacus*), David's myotis (*Myotis davidii*), Alpaca (*Vicugna pacos*), Goat (*Capra hircus*), Tibetan antelope (*Pantholops hodgsonii*), Mouflon (*Ovis aries musimon*), Dromedary (*Camelus dromedarius*), Bactrian camel (*Camelus bactrianus*), Cattle (*Bos taurus*)*,* Yak (*Bos mutus*), Water buffalo (*Bubalus bubalis*), Giant panda (*Ailuropoda melanoleuca*), Polar bear (*Ursus maritimus*), Dog (*Canis lupus familiaris*), Cat *(Felis catus),* Siberian Tiger (*Panthera tigris altaica*), Wild boar (*Sus scrofa*), White rhinoceros (*Ceratotherium simum simum*), Weddell seal (*Leptonychotes weddellii*), West Indian manatee (*Trichechus manatus latirostris*), Minke whale (*Balaenoptera acutorostrata scammoni*)*,* Baiji (*Lipotes vexillifer*), Killer whale (*Orcinus orca*), Sperm whale *(Physeter catodon),* Aardvark (*Orycteropus afer afer*)*,* Sunda pangolin (*Manis javanica*)]*,* mammal2 [Horse (*Equus caballus*), Donkey (*Equus asinus*), Przewalski's horse (*Equusprzewalskii*)]*,* mammal3 [Gray mouse lemur (*Microcebus murinus*), Brown rat (*Rattus norvegicus*), Upper Galilee Mountains blind mole-rat (*Nannospalax galili*), Ord's kangaroo rat (*Dipodomys ordii*)*,* Lesser hedgehog tenrec (*Echinops telfairi*), Platypus (*Ornithorhynchus anatinus*)]*,* mammal4 [Mouse (*Mus musculus*), Prairie vole (*Microtus ochrogaster*)]*,* mammal5 [Little brown bat (*Myotis lucifugus*), Brandt's bat (*Myotis brandtii*)]*,* Philippine tarsier (*Carlito syrichta*), Chinese hamster (*Cricetulus griseus*), Star-nosed mole (*Condylura cristata*), marsupial 1 [Gray short-tailed opossum (*Monodelphis domestica*), Tasmanian devil (*Sarcophilus harrisii*)], bird1 [Atlantic canary (*Serinus canaria*), Ground tit *(Pseudopodoces humilis),* Common starling *(Sturnus vulgaris),* Great tit (*Parus major*), Medium ground finch (*Geospiza fortis*), American crow (*Corvus brachyrhynchos*)], bird2 [Peregrine falcon (*Falco peregrinus*), Saker falcon (*Falco cherrug*)*,* Downy woodpecker (*Picoides pubescens*)]*,* bird3 [Blue-fronted amazon (*Amazona aestiva*), Budgerigar (*Melopsittacus undulatus*)]*,* Bald eagle (*Haliaeetus leucocephalus*)*,* Chinese goose (*Anser cygnoides domesticus*), Common cuckoo (*Cuculus canorus*), Rock dove (*Columba livia*), Collared flycatcher (*Ficedula albicollis*), Blue-crowned manakin (*Lepidothrix coronata*), Burmese python (*Python bivittatus*), Taiwan habu (*Protobothrops mucrosquamatus*), Green sea turtle (*Chelonia mydas*), Painted turtle (*Chrysemys picta bellii*)*,* Schlegel's Japanese gecko (*Gekkojaponicas*), Carolina anole *(Anolis carolinensis),* American alligator (*Alligator mississippiensis*), Western clawed frog (*Xenopus tropicalis*), African clawed frog (*Xenopus laevis*), High Himalaya frog (*Nanorana parkeri*), fish1 [Zebrafish (*Danio rerio*), Horned golden-line barbel (*Sinocyclocheilus rhinocerous*)*,* Golden-line barbel (*Sinocyclocheilus grahami*), Common carp (*Cyprinus carpio*)], fish 2 [Zebra mbuna (*Maylandia zebra*), Lake Tanganyika cichlid (*Neolamprologus brichardi*)*,* Burton's mouthbrooder (*Haplochromis burtoni*), Nile tilapia (*Oreochromis niloticus*)]*,* fish 3 [Channel catfish *(Ictalurus punctatus*), Red-bellied piranha (*Pygocentrus nattereri*)]*,* fish4 [Rainbow trout (*Oncorhynchus mykiss*), Atlantic salmon (*Salmo salar*)]*,* Spotted gar (*Lepisosteus oculatus*)*,* Tongue sole (*Cynoglossus semilaevis*)*,* Turquoise killifish (*Nothobranchiusfurzeri*), Large yellow croaker (*Larimichthys crocea*), Asian arowana (*Scleropages formosus*)*,* Barramundi (*Rates calcarifer*), Atlantic herring (*Clupea harengus*), Lake Victoria cichlid (*Pundamilia nyererei*), Bicolor damselfish (*Stegastes partitus*), Eyeless goldenline fish (*Sinocyclocheilus anshuiensis*), Northern pike (*Esox lucius*), Mexican tetra (*Astyanax mexicanus*), West Indian Ocean coelacanth (*Latimeria chalumnae*), Australian ghostshark (*Callorhinchus milii*) *.*

In most of the species shown in FIG. 32, the amino acid affected by the equine FLNC-E753K variant is a glutamic acid (E). Only two species, Carolina anole *(Anolis carolinensis),* and Australian ghostshark (*Callorhinchus milii*), have an amino acid substitution at this position. In both cases, it is a conservative substitution of an aspartic acid (D) for a glutamic acid (E), the conservative substitution of one negatively charged amino acid for another. This is distinctly different from the substitution of lysine (K) for glutamic acid (E) found in the FLNC-E753K variant, a nonconservative substitution of a positively charged amino acid for a negatively charged one. The equine variant FLNC-E753K is a nonconservative substitution in a highly conserved position, with sequence conservation extending to cartilaginous fishes, which diverged from mammals over 500 million years ago. This is evidence that the FLNC-E753K variant is pathogenic, and has been eliminated from populations by natural selection when it has occurred.

FIG. 33 shows the amino acid sequences encoded by FLNC genes, showing filamin repeat 11, which contains the equine FLNC-A1207T substitution. Species included in the analysis are described in the text. The next to the last line (labeled CLUSTAL) shows the consensus sequence, where positions with fully conserved amino acids are represented by an asterisk (*), positions with strongly conserved amino acids are indicated by a colon (:), positions with weakly conserved amino acids are indicated are indicated by period (.), and nonconserved positions are indicated by a blank space ( ). The last line shows the sequence of filamin repeat 11 in horse with the FLNC-A1207T substitution shown and highlighted in bold. The position of the FLNC-A1207T substitution is indicated in bold in all of the sequences.

The 106 species in the alignment shown in Figure 33 are: mammall [Human (*Homo sapiens*), Common chimpanzee *(Pan troglodytes),* Bonobo *(Pan paniscus),* Western lowland gorilla (*Gorilla gorilla gorilla*), Sumatran orangutan (*Pongo abelii*), Olive baboon (*Papio anubis),* Angola colobus (*Colobus angolensis palliatus*), Black snub-nosed monkey (*Rhinopithecus bieti*)*,* Sooty mangabey (*Cercocebus atys*), Green monkey (*Chlorocebus sabaeus*), Drill (*Mandrillus leucophaeus*)*,* Crab-eating macaque (*Macaca fascicularis*)*,* Rhesus macaque (*Macaca mulatta*)*,* Nancy Ma's night monkey (*Aotus nancymaae*), Southern pig-tailed macaque (*Macaca nemestrina*)*,* Philippine tarsier (*Carlito syrichta*), Ord's kangaroo rat (*Dipodomys ordii*)], mammal2 [Horse (*Equus caballus*), Przewalski's horse (*Equus przewalskii*)*,* Donkey (*Equus asinus*), Gray mouse lemur (*Microcebus murinus*), Coquerel's sifaka (*Propithecus coquereli*)], mammal3, [Malayan flying fox (*Pteropus vampyrus*), Big brown bat (*Eptesicusfuscus*), David's myotis (*Myotis davidii*)*,* Black flying fox (*Pteropus alecto*), Mouse (*Mus musculus*), Prairie vole (*Microtus ochrogaster*), Aardvark (*Orycteropus afer afer*)*,* Brandt's bat (*Myotis brandlii*)*,* Bolivian Squirrel Monkey (*Saimiri boliviensis boliviensis*), Polar bear (*Ursus maritimus*), Chinese hamster (*Cricetulus griseus*), Brown rat (*Rattus norvegicus*), Giant panda (*Ailuropoda melanoleuca*), Sunda pangolin (*Manisjavanica*), Egyptian fruit bat (*Rousettus aegyptiacus*)]*,* mammal4 [Baiji (*Lipotes vexillifer*)*,* Siberian Tiger (*Panthera tigris altaica*), Cat *(Felis catus),* Wild boar (*Sus scrofa*), Sperm whale *(Physeter catodon),* Star-nosed mole (*Condylura cristata*), Tasmanian devil (*Sarcophilus harrisii*)*,* Alpaca (*Vicugna pacos*)*,* Bactrian camel (*Camelus bactrianus*), Dromedary (*Camelus dromedarius*)]*,* mammal5 [Cattle (*Bos taurus*)*,Yak* (*Bos mutus*), Water buffalo (*Bubalus bubalis*), Mouflon (*Ovis aries musimon*), Goat (*Capra hircus*)]*,* mammal6 [Common marmoset (*Callithrix jacchus*)*,* Natal long-fingered bat (*Miniopterus natalensis*)*,* Lesser hedgehog tenrec (*Echinops telfairi*)], mammal7 [Dog (*Canis lupus familiaris*), Weddell seal (*Leptonychotes weddellii*)], mammals [West Indian manatee (*Trichechus manatus latirostris*), White rhinoceros (*Ceratotherium simum simum*)]*,* mammal9 [Northern greater galago (*Otolemur garnettii*), Upper Galilee Mountains blind mole-rat (*Nannospalax galili*)], Coquerel's sifaka (*Balaenoptera acutorostrata scammoni*), White-headed capuchin (*Cebus capucinus imitator*), Little brown bat (*Myotis lucifugus*), Damara mole-rat (*Fukomys damarensis*), Killer whale (*Orcinus orca*), Gray short-tailed opossum (*Monodelphis domestica*), Platypus (*Ornithorhynchus anatinus*), bird1 [Peregrine falcon (*Falco peregrinus*), Saker falcon (*Falco cherrug*)]*,* bird2 [Ground tit *(Pseudopodoces humilis),* Great tit (*Parus major*)], bird3 [Medium ground finch (*Geospiza fortis*)*,* Atlantic canary (*Serinus canaria*)]*,* Downy woodpecker (*Picoides pubescens*), Rock dove (*Columba livia*), Budgerigar (*Melopsittacus undulatus*), Common starling *(Sturnus vulgaris),* American crow (*Corvus brachyrhynchos*), Blue-crowned manakin (*Lepidothrix coronata*), Collared flycatcher (*Ficedula albicollis*), Common cuckoo (*Cuculus canorus*), Painted turtle (*Chrysemys picta bellii*), Western clawed frog (*Xenopus tropicalis*), African clawed frog (*Xenopus laevis*), High Himalaya frog (*Nanorana parkeri*), Taiwan habu (*Protobothrops mucrosquamatus*), Burmese python (*Python bivittatus*), Carolina anole *(Anolis carolinensis),* American alligator (*Alligator mississippiensis*), Green sea turtle (*Chelonia mydas*), fish1 [Eyeless goldenline fish (*Sinocyclocheilus anshuiensis*), Golden-line barbel (*Sinocyclocheilus rhinocerous*)]*,* Golden-line barbel (*Sinocyclocheilus grahami*), Large yellow croaker (*Larimichthys crocea*)*,* Red-bellied piranha (*Pygocentrus nattereri*)*,* Mexican tetra (*Astyanax mexicanus*), Common carp (*Cyprinus carpio*), Channel catfish (*Ictalurus punctatus*), Atlantic herring (*Clupea harengus*)*,* Rainbow trout (*Oncorhynchus mykiss*), Atlantic salmon (*Salmo salar*), Northern pike (*Esox lucius*), Spotted gar (*Lepisosteus oculatus*), West Indian Ocean coelacanth (*Latimeria chalumnae*), Australian ghostshark (*Callorhinchus milii*)*.*

In all of the species presented in FIG. 33, the amino acid affected by the equine FLNC-A1207T variant is an alanine (A). Only the equine FLNC-A1207T variant has a substitution of a threonine (T) at this position; this is a nonconservative substitution of a polar uncharged amino acid for a hydrophobic one. The sequence conservation at this position extends to cartilaginous fishes, which diverged from mammals over 500 million years ago. This is evidence that the FLNC-A1207T variant is pathogenic, and has been eliminated from populations by natural selection when it has occurred.

FIG. 34 shows the amino acid sequences of proteins encoded by MYOZ3 genes, centered on the position of the equine MYOZ3-S42L substitution. The next to the last line (labeled CLUSTAL) shows the consensus sequence, where positions with fully conserved amino acids are represented by an asterisk (*), positions with strongly conserved amino acids are indicated by a colon (:), positions with weakly conserved amino acids are indicated are indicated by period (.), and nonconserved positions are indicated by a blank space ( ). The last line shows the sequence of myozenin-3 in horse with the MYOZ3-S42L substitution shown and highlighted in bold. The position of the MYOZ3-S42L substitution is indicated in bold in all of the sequences.

The 88 species in the alignment shown in Figure 34 are: mammal 1 [Human (*Homo sapiens*), Bonobo *(Pan paniscus),* Western lowland gorilla (*Gorilla gorilla gorilla*), Northern white-cheeked gibbon (*Nomascus leucogenys*), Nancy Ma's night monkey (*Aotus nancymaae*)], mammal2 [Olive baboon (*Papio anubis*), Rhesus macaque (*Macaca mulatta*), Crab-eating macaque (*Macaca fascicularis*), Black snub-nosed monkey (*Rhinopithecus bieti*), Sooty mangabey (*Cercocebus atys*), Angola colobus (*Colobus angolensis palliates*), Golden snub-nosed monkey (*Rhinopithecus roxellana*)], mammal3 [White-headed capuchin (*Cebus capucinus imitator*), Black-capped squirrel monkey (*Saimiri boliviensis boliviensis*)], mammal4 [Horse (*Equus caballus*), Donkey (*Equus asinus*)], mammal5 [Bottle-nosed dolphin (*Tursiops truncates*), Domestic yak (*Bos mutus*), Baiji (*Lipotes vexillifer*)], mammal6 [Weddell seal (*Leptonychotes weddellii*), Walrus (*Odobenus rosmarus divergens*)], mammal7 [Cattle (*Bos taurus*), Water buffalo (*Bubalus bubalis*), Killer whale (*Orcinus orca*), American bison (*Bison bison bison*), Tibetan antelope (*Pantholops hodgsonii*), Goat (*Capra hircus*)], mammals [Large flying fox (*Pteropus vampyrus*), Black flying fox (*Pteropus alecto*)], bird1 [Common starling *(Sturnus vulgaris),* Medium ground finch (*Geospiza fortis*), Collared flycatcher (*Ficedula albicollis*)], Coquerel's sifaka (*Propithecus coquereli*), Sumatran orangutan (*Pongo abelii*), Common marmost (*Callithrix jacchus*), Rhesus macaque (*Macaca mulatto*), Green monkey (*Chlorocebus sabaeu*s), Bactrian camel (*Camelus bactrianus*), Alpaca (*Vicugna pacos*), Polar bear (*Ursus maritimus*), Giant panda (*Ailuropoda melanoleuca*), Natal long-fingered bat (*Miniopterus natalensis*), White rhinoceros (*Ceratotherium simum simum*), Ferret (*Mustela putorius furo*), Cat *(Felis catus),* Cheetah (*Acinonyx jubatus*), Dog (*Canis lupus familiaris*), Siberian tiger (*Panthera tigris altaica*), American pika (*Ochotona princeps*), European rabbit (*Oryctolagus cuniculus*), Thirteen-lined ground squirrel (*Ictidomys tridecemlineatus*), Alpine marmot (*Marmota marmota marmota*), Chinese tree shrew (*Tupaia chinensis*), Sperm whale *(Physeter catodon),* Leopard (*Panthera pardus*), Tasmanian devil (*Sarcophilus harrisii*), Damaraland mole-rat (*Fukomys damarensis*), Burmese python (*Python bivittatus*), Chinese softshell turtle (*Pelodiscus sinensis*), Painted turtle (*Chrysemys picta bellii*), Green turtle (*Chelonia mydas*), Carolina anole *(Anolis carolinensis),* American alligator (*Alligator mississippiensis*), Ruff (*Calidrispugnax*), MacQueen's bustard (*Chlamydotis macqueenii*), Gray short-tailed opossum (*Monodelphis domestica*), Downy woodpecker (*Picoides pubescens*), Dalmatian penguin (*Pelecanus crispus*), Killdeer (*Charadrius vociferus*), Adelie penguin (*Pygoscelis adeliae*), Crested ibis (*Nipponia nippon*), Little egret (*Egretta garzetta*), Turkey vulture (*Cathartes aura*), Zebra finch (*Taeniopygia guttata*), American crow (*Corvus brachyrhynchos*), Rock dove (*Columba livia*), Chimney swift (*Chaetura pelagica*), Emperor penguin (*Aptenodytesforsteri*), Red-crested turaco (*Tauraco erythrolophus*), Chinese alligator (*Alligator sinensis*), Ostrich (*Struthio camelus australis*), White-throated tinamou (*Tinamus guttatus*), Ground tit *(Pseudopodoces humilis),* Atlantic canary (*Serinus canaria*), Great tit (*Parus major*), White-throated sparrow (*Zonotrichia albicollis*), Common cuckoo (*Cuculus canorus*), North Island brown kiwi (*Apteryx australis mantelli*).

In all of the species presented in FIG. 34, the amino acid affected by the equine MYOZ3-S42L variant is a serine (S). Only the equine MYOZ3-S42L variant has a substitution of a leucine (L) at this position; this is a nonconservative substitution of a hydrophobic amino acid for a polar uncharged one. The sequence conservation at this position extends to birds, which diverged from mammals 300 million years ago. This is evidence that the MYOZ3-S42L variant is pathogenic, and has been eliminated from populations by natural selection when it has occurred.

Phenotypic effects of the MYOT-S232P, FLNC-E753K, FLNC-A1207T, and MYOZ3-S42L variants

The MYOT-S232P variant (hereafter abbreviated as P2) was discovered by analysis of whole genome sequencing data from six Quarter Horses diagnosed via muscle biopsy with Polysaccharide Storage Myopathy type 2 (PSSM2). All six individuals were heterozygous, that is, n/P2. The FLNC-E753K and FLNC-A1207T variants (hereafter abbreviated as P3a and P3b, respectively) were discovered by analysis of whole genome sequencing data from two Thoroughbreds diagnosed via muscle biopsy with either Polysaccharide Storage Myopathy type 2 (PSSM2) or Myofibrillar Myopathy (MFM). Both individuals were heterozygous, that is n/P3a n/P3b. Subsequent genotyping of additional cases shows that the two FLNC variants are inherited together as a single haplotype (hereafter abbreviated P3). The MYOZ3-S42L variant (hereafter abbreviated as P4) was discovered by analysis of whole genome sequencing data from two horses, a Paso Fino and a Quarter Horse. In both cases, one parent had contributed the P2 variant and the other parent had contributed the P4 variant. Both n/P2 n/P4 horses were symptomatic, while both owners reported that both parents in both cases were apparently asymptomatic.

All three variants (treating FLNC-E753K + FLNC-A1207T as a single haplotype designated P3) behave as semidominant variants with incomplete penetrance. Homozygotes have been observed for each variant (P2/P2, P3/P3, and P4/P4); in each case, the phenotype of homozygous individuals is more severe than that of heterozygotes, with an earlier age of onset and more severe symptoms.

All possible compound heterozygotes with two variants (n/P2 n/P3, n/P2 n/P4, and n/P3 n/P4) have been identified. In addition, some horses homozygous for one variant and heterozygous for a second variant (P2/P2 n/P3, n/P2 P3/P3, P3/P3 n/P4, and n/P3 P4/P4) and have been identified. Some of these horses have been severely affected, with one P2/P2 n/P3 being euthanized following recumbency as a yearling.

The following account of symptoms is a generalization; the symptoms produced by the P2, P3, and P4 variants are similar.

One of the earliest symptoms is a change in behavior apparently associated with pain. Owners note a difference in temperament, with horses reacting badly to being ridden or even saddled. Common behaviors include biting at the flanks or even at the rider or trainer, and bucking, rearing, and other displays of resistance that trainers often blame on lack of discipline from the owner.

Another early symptom is stifle problems. The stifle is the largest joint in the horse's body, equivalent to the human knee, but in contrast to the human knee, the equine stifle is held at an angle when the horse is standing still. Stifle problems commonly result from injury or arthritis, degenerative joint disease, or injury. In stifle problems resulting from Polysaccharide Storage Myopathy type 2 (PSSM2), there will be no radiographic findings. Stifle problems are one example of shifting lameness. A horse with Polysaccharide Storage Myopathy type 2 (PSSM2) will exhibit lameness that appears first in one limb, then another. There will be no radiographic findings.

Changes in gait are often apparent. These include stiffness in the hindquarters and limited range of motion of the hind legs ("short-gaited"). At canter, disunited canter ("cross-firing") and "bunny hopping" (bringing both hind legs forward at the same time) are seen. "Rope walking" (placing one foot directly in front of the other along the centerline as if walking a tightrope) is sometimes seen in all for legs or in the rear legs only.

Other gait changes resulting from weakness in the hind limbs are described by horse owners as "heavy on the forehand, not able to come from behind." This means that the horse's gait is altered in such a way that it appears to be pulling itself forward with its front hooves instead of pushing from the rear. Farriers note this as a pattern of wear in the front hooves for unshod horses.

Muscle wasting in the hindquarters (pelvic girdle and proximal limb) and in the topline (shoulder girdle) becomes evident as symptoms progress. Owners report that they are able to partially reverse this symptom by dietary supplementation with complete protein (whey or soy) or with essential amino acids typically limiting in plant protein (lysine, methionine, and threonine). Events that cause negative nitrogen balance, such as viral infections or an injury requiring stitches, can trigger a rapid loss of muscle mass, quickly reversing gains made through dietary supplementation.

Some horses exhibit "divots," focal muscle atrophy that can sometimes be reversed through dietary supplementation with complete protein or essential amino acids typically limiting in plant protein. The locations of focal muscle atrophy are typically asymmetric, appearing on one side only. Some horses exhibit a washboard-like pattern of focal muscle atrophy.

There are reports of respiratory difficulty in the end stages of Polysaccharide Storage Myopathy type 2 (PSSM2), and in one case, a necropsy revealed that the diaphragm was affected. The end stage of symptoms typically involves recumbency, with either all four limbs affected or the hind limbs only. In the latter case, the horse may attempt to retain an upright stance by supporting its hindquarters on a fence or wall.

Many owners report that as symptoms progress, veterinarians are perplexed by the appearance of these symptoms of muscle wasting while blood work shows levels of serum creatine kinase (CK) and aspartate aminotransferase (AST) that are frequently in the normal range.

There is no evidence of cardiomyopathy.

Muscle wasting in the hindquarters (pelvic girdle and proximal limb) and topline (shoulder girdle) in Polysaccharide Storage Myopathy type 2 (PSSM2) or Myofibrillar Myopathy (MFM) appears similar to human cases of Limb-Girdle Muscular Dystrophy, a genetically diverse group of disorders with similar clinical features. Human patients with Limb-Girdle Muscular Dystrophy also develop gait abnormalities as symptoms progress.

In the preceding description and following claims, the term "and/or" means one or all of the listed elements or a combination of any two or more of the listed elements; the terms "comprises," "comprising," and variations thereof are to be construed as open ended-i.e., additional elements or steps are optional and may or may not be present; unless otherwise specified, "a," "an," "the," and "at least one" are used interchangeably and mean one or more than one; and the recitations of numerical ranges by endpoints include all numbers subsumed within that range (e.g., 1 to 5 includes 1, 1.5, 2, 2.75, 3, 3.80, 4, 5, etc.).

In the preceding description, particular embodiments may be described in isolation for clarity. Unless otherwise expressly specified that the features of a particular embodiment are incompatible with the features of another embodiment, certain embodiments can include a combination of compatible features described herein in connection with one or more embodiments.

For any method disclosed herein that includes discrete steps, the steps may be conducted in any feasible order. And, as appropriate, any combination of two or more steps may be conducted simultaneously.

This is illustrated by the following examples. To the extent that the Examples contains subject matter that is not within the scope of the invention as defined by the present claims, the subject matter is included merely for reference purposes.

EXAMPLES

Example 1 - Method of Detecting DNA Mutations Associated with Equine Polysaccharide Storage Myopathy type 2 (PSSM2), also known as Myofibrillar Myopathy (MFM).

The complete DNA sequence of the horse MYOT, FLNC, and MYOZ3 coding regions were obtained from the current version of the public horse genome assembly (EquCab2).

Using the MYOT, FLNC, and MYOZ3 sequences, PCR primers are developed that can amplify the sites of genomic DNA containing MYOT-S232P, FLNC-E753K, FLNC-A1207T, and MYOZ3-S42L mutations. For example, a PCR primer pair that has been successfully and reliably used to amplify the region including MYOT-S232P from isolated horse DNA samples lies in the region around exon 6 (FIG. 11). These sequences are 5'-TATGACAATGGAAAGGGAATTC- 3' (SEQ ID NO:19) and 5'-TTCTCAAGCTGTGGAGCAAG- 3' (SEQ ID NO:20). A PCR primer pair that has been successfully and reliably used to amplify the region including FLNC-E753K from isolated horse DNA samples lies in the region around exon 15 (FIG. 12). These sequences are 5'-GGCAGTCACCCTGAGAAAGT-3' (SEQ ID NO:23) and 5'-ACTTGATGCCAATGCTCAC-3' (SEQ ID NO:24). A PCR primer pair that has been successfully and reliably used to amplify the region including FLNC-A1207T from isolated horse DNA samples lies in the region around exon 21 (FIG. 13). These sequences are 5'-GGTGCTGATCCACAACAATG-3' (SEQ ID NO:27) and 5'-CCCAAGTCCTCCCTTCAGAC-3' (SEQ ID NO:28). A PCR primer pair that has been successfully and reliably used to amplify the region including MYOZ3-S42L from isolated horse DNA samples lies in the region around exon 3 (FIG. 14). These sequences are 5'-CAGGTTTCTCACACACAATGG-3' (SEQ ID NO:31) and 5'-AGGCATTCTGCATTTTCCAC- 3' (SEQ ID NO:32). Many other primer pairs are also possible.

Using the above PCR primers to amplify the two regions, the genotype of any horse (A/A, A/G, or G/G for the DNA sequence of the forward strand at chr14:38,519,183, and S/S, S/P, or P/P for the amino acid sequence of the MYOT-S232P variant, G/G, G/A, or A/A for the DNA sequence of the forward strand at chr4:83,736,244, and E/E, E/K, and K/K for the amino acid sequence of the FLNC-E753K variant, G/G, G/A, or A/A for the DNA sequence of the forward strand at chr4:83,738,769, and A/A, A/T, or T/T for the amino acid sequence of the FLNC-A1207T variant, or G/G, G/A, or A/A for the DNA sequence of the forward strand at chr14:27,399,222, and S/S, S/L, or L/L for the amino acid sequence of the MYOZ3-S42L variant) can be obtained. In this method, the amplified DNA may be cloned and then sequenced or sequenced directly without cloning. Alternatively, the appearance of amplified product in the presence of primers specific to the wild type or mutant allele may be monitored in real time using a qPCR instrument designed for this purpose. Many other methods of detecting the nucleotides at the positions of the horse MYOT, FLNC, and MYOZ3 sequence are possible.

DNA testing based on now provides veterinarians and veterinary pathologists with a means to more accurately determine if a horse with the clinical signs of Polysaccharide Storage Myopathy type 2 (PSSM2), also known as Myofibrillar Myopathy (MFM), has the heritable and common form of the disease that can be specifically attributed to the MYOT-S232P, FLNC-E753K, FLNC-A1207T, or MYOZ3-S42L coding region mutations. All that is needed are a tissue sample containing the individual's DNA (typically hair root or blood) and appropriate PCR and sequence analysis technology to detect the four distinct nucleotide changes. Such PCR primers are based in MYOT exon 6 and the flanking intron sequences, as shown in FIG. 11, FLNC exons 15 and 21 and their flanking intron sequences as shown in FIG. 12 and FIG. 13, and MYOZ3 exon 3 and the flanking intron sequences, as shown in FIG. 14, or in other DNA sequences of these genes.

Also, DNA testing provides owners and breeders with a means to determine if any horse can be expected to produce offspring with this form of Polysaccharide Storage Myopathy type 2 (PSSM2), also known as Myofibrillar Myopathy (MFM). Abbreviating the MYOT-S232P allele as P2, the FLNC-E753K + FLNC-A1207T allele as P3, and the MYOZ3-S42L allele as P4, and the wild-type alleles (MYOT-S232, FLNC-E753 + FLNC-A1207, and MYOZ3-S42) as n; a P2/P2, P3/P3, or P4/P4 horse would produce an affected foal 100% of the time, while an n/P2, n/P3, or n/P4 horse would produce an affected foal 50% of the time when mated to an n/n horse. Mating of an n/P2 horse to an n/P2 horse, an n/P3 horse to an n/P3 horse, or an n/P4 horse to an n/P4 horse would produce an affected foal 75% of the time. Breeding programs could incorporate this information in the selection of parents that could eventually reduce and even eliminate this form of Polysaccharide Storage Myopathy type 2 (PSSM2), also known as Myofibrillar Myopathy (MFM), in their herds.

The foregoing detailed description and examples have been given for clarity of understanding only. No unnecessary limitations are to be understood therefrom.

Unless otherwise indicated, all numbers expressing quantities of components, molecular weights, and so forth used in the specification and claims are to be understood as being modified in all instances by the term "about." Accordingly, unless otherwise indicated to the contrary, the numerical parameters set forth in the specification and claims are approximations that may vary depending upon the desired properties sought to be obtained. At the very least, and not as an attempt to limit the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. All numerical values, however, inherently contain a range necessarily resulting from the standard deviation found in their respective testing measurements.

All headings are for the convenience of the reader and should not be used to limit the meaning of the text that follows the heading, unless so specified.

## Claims

1. A method for detecting the presence or absence of biomarkers in a horse, the method comprising:
detecting in a biological sample from a horse, the biological sample comprising a nucleic acid that includes the coding regions for myotilin (MYOT), filamin-C (FLNC) and myozenin-3 (MYOZ3), the presence or absence of a base substitution corresponding to guanine (G) substituted for an adenine (A) at nucleotide chr14:38519183 of the forward strand of SEQ ID NO: 1, a base substitution corresponding to an adenine (A) substituted for a guanine (G) at nucleotide chr4:83736244 of the forward strand of SEQ ID NO:2, a base substitution corresponding to an adenine (A) substituted for a guanine (G) at nucleotide chr4:83738769 of the forward strand of SEQ ID NO:3, and a base substitution corresponding to an adenine (A) substituted for a guanine (G) at nucleotide chr14:27399222 of SEQ ID NO:4, or the complement thereof, wherein the presence of any of said substitutions in said nucleic acid is indicative of inherited equine myopathy in said horse.

2. The method of claim 1, further comprising:

   contacting the nucleic acid with at least one oligonucleotide probe to form a hybridized nucleic acid; and
   amplifying the hybridized nucleic acid.

3. The method of claim 2, wherein exon 6 of the equine myotilin coding region (MYOT), exons 15 and 21 of the equine filamin-C coding region (FLNC), and exon 3 of the equine myozenin-3 coding region (MYOZ3), or a portion thereof is amplified.

4. The method of claim 2, wherein the hybridized nucleic acid is amplified using polymerase chain reaction, strand displacement amplification, ligase chain reaction, or nucleic acid sequence-based amplification.

5. The method of claim 2, wherein at least one oligonucleotide probe is immobilized on a solid surface or a semisolid surface.

6. A method according to any of Claims 1-5, wherein the nucleic acid is genomic DNA.

7. A method according to any of Claims 1-5, wherein the nucleic acid is RNA.

8. A method according to Claim 7 wherein the method comprises converting the RNA into DNA by reverse transcriptase.

9. A method according to Claim 8 wherein the method comprises detecting the presence or absence of a biomarker that comprises an equine MYOT polynucleotide of SEQ ID NO: 5 having a cytosine (C) substituted for thymine (T) at nucleotide position 694, an equine FLNC polynucleotide of SEQ ID NO: 6 having an adenine (A) substituted for guanine (G) at nucleotide position 2257, an equine FLNC polynucleotide of SEQ ID NO: 6 having an adenine (A) substituted for guanine (G) at nucleotide position 3619, and an equine MYOZ3 polynucleotide of SEQ ID NO: 8 having a thymine (T) substituted for cytosine (C) at nucleotide position 125; in all cases the presence of the specified nucleotide can be inferred from detecting the nucleotide present at the complement thereof, wherein the presence of any of said biomarkers is indicative of inherited equine myopathy in said horse.

10. A method for detecting the presence or absence of biomarkers in a horse, the method comprising:
detecting in a biological sample from a horse, the biological sample comprising a nucleic acid encoding a myotilin polypeptide, a filamin-C polypeptide, and a myozenin-3 polypeptide, the presence or absence of a nucleic acid that encodes a myotilin polypeptide having a proline residue (P) substituted for serine (S) at position 232 of SEQ ID NO: 10, a nucleic acid that encodes a filamin-C polypeptide having a lysine residue (K) substituted for glutamate (E) at position 753, a nucleic acid that encodes a filamin-C polypeptide having a threonine residue (T) substituted for alanine (A) at position 1207 of SEQ ID NO: 13, and a nucleic acid that encodes a myozenin-3 polypeptide having a leucine residue (L) substituted for serine (S) at position 42 of SEQ ID NO: 16, wherein the presence of any of said nucleic acids in said sample is indicative of inherited equine myopathy in said horse.

**Patentansprüche**

1. Verfahren zum Nachweis des Vorhandenseins oder der Abwesenheit von Biomarkern in einem Pferd, das Verfahren umfassend:
Nachweisen in einer biologischen Probe eines Pferdes, wobei die biologische Probe eine Nukleinsäure umfasst, die die codierenden Regionen für Myotilin (MYOT), Filamin-C (FLNC) und Myozenin-3 (MYOZ3) einschließt, des Vorhandenseins oder der Abwesenheit einer Basensubstitution, die einem Guanin (G) anstelle eines Adenins (A) am Nukleotid chrl4: 38519183 des Vorwärtsstrangs von SEQ ID NO:1 entspricht, einer Basensubstitution, die auf einem Adenin (A) anstelle eines Guanins (G) am Nukleotid chr4:83736244 des Vorwärtsstrangs von SEQ ID NO:2 basiert, einer Basensubstitution, die einem Adenin (A) entspricht, das ein Guanin (G) am Nukleotid chr4:83738769 des Vorwärtsstrangs von SEQ ID NO:3 ersetzt, und einer Basensubstitution, die einem Adenin (A) entspricht, das ein Guanin (G) am Nukleotid chr4:27399222 von SEQ ID NO:4 ersetzt, oder dem Komplement davon, wobei das Vorhandensein einer der Substitutionen in der Nukleinsäure auf eine vererbte equine Myopathie bei dem Pferd hinweist.

2. Verfahren nach Anspruch 1, ferner umfassend:

   Inkontaktbringen der Nukleinsäure mit mindestens einer Oligonukleotidsonde zur Formung einer hybridisierten Nukleinsäure; und
   Amplifizieren der hybridisierten Nukleinsäure.

3. Verfahren nach Anspruch 2, wobei Exon 6 der equinen Myotilin codierenden Region (MYOT), Exon 15 und 21 der equinen Filamin-C codierenden Region (FLNC) und Exon 3 der equinen Myozenin-3 codierenden Region (MYOZ3) oder ein Abschnitt davon amplifiziert wird.

4. Verfahren nach Anspruch 2, wobei die hybridisierte Nukleinsäure unter Verwendung von Polymerasekettenreaktion, Strangverdrängungsamplifikation, Ligasekettenreaktion oder Nukleinsäuresequenz-basierter Amplifikation amplifiziert wird.

5. Verfahren nach Anspruch 2, wobei mindestens eine Oligonukleotidsonde auf einer festen Oberfläche oder einer halbfesten Oberfläche immobilisiert ist.

6. Verfahren nach einem der Ansprüche 1 bis 5, wobei die Nukleinsäure genomische DNA ist.

7. Verfahren nach einem der Ansprüche 1 bis 5, wobei die Nukleinsäure RNA ist.

8. Verfahren nach Anspruch 7, wobei das Verfahren die Umwandlung der RNA in DNA durch reverse Transkriptase umfasst.

9. Verfahren nach Anspruch 8, wobei das Verfahren das Nachweisen des Vorhandenseins oder der Abwesenheit eines Biomarkers umfasst, der ein equines MYOT-Polynukleotid der SEQ ID NO: 5 mit einem Cytosin (C) als Ersatz für Thymin (T) an der Nukleotidposition 694, ein equines FLNC-Polynukleotid der SEQ ID NO: 6 mit einem Adenin (A) als Ersatz für Guanin (G) an der Nukleotidposition 2257, ein equines FLNC-Polynukleotid der SEQ ID NO: 6, das an der Nukleotidposition 3619 ein Adenin (A) als Ersatz für Guanin (G) aufweist, und ein equines MYOZ3-Polynukleotid der SEQ ID NO: 8, das an der Nukleotidposition 125 ein Thymin (T) als Ersatz für Cytosin (C) aufweist, umfasst; in allen Fällen kann das Vorhandensein des spezifizierten Nukleotids aus dem Nachweis des am Komplement vorhandenen Nukleotids abgeleitet werden, wobei das Vorhandensein eines der Biomarker auf eine vererbte equine Myopathie bei dem Pferd hinweist.

10. Verfahren zum Nachweis des Vorhandenseins oder der Abwesenheit von Biomarkern in einem Pferd, das Verfahren umfassend:
Nachweisen des Vorhandenseins oder der Abwesenheit einer Nukleinsäure, die für ein Myotilinpolypeptid, ein Filamin-C-Polypeptid und ein Myozenin-3-Polypeptid codiert, in einer biologischen Probe eines Pferdes, wobei die biologische Probe eine Nukleinsäure aufweist, die für ein Myotilinpolypeptid codiert, das einen Pralinrest (P) als Ersatz für Serin (S) an Position 232 von SEQ ID NO: 10, eine Nukleinsäure, die ein Filamin-C-Polypeptid codiert, bei dem ein Lysinrest (K) durch Glutamat (E) an Position 753 ersetzt ist, eine Nukleinsäure, die ein Filamin-C-Polypeptid codiert, bei dem ein Threoninrest (T) durch Alanin (A) an Position 1207 von SEQ ID NO: 13 codiert, und eine Nukleinsäure, die ein Myozenin-3-Polypeptid codiert, bei dem an Position 42 von SEQ ID NO:16 ein Leucinrest (L) durch Serin (S) ersetzt ist, umfasst, wobei das Vorhandensein einer der Nukleinsäuren in der Probe ein Hinweis auf eine vererbte equine Myopathie bei dem Pferd ist.

## Revendications

1. Procédé de détection de la présence ou de l'absence de biomarqueurs chez un cheval, le procédé comprenant : la détection dans un échantillon biologique provenant d'un cheval, de l'échantillon biologique comprenant un acide nucléique qui comporte les régions codantes pour la myotiline (MYOT), la filamine-C (FLNC) et la myozénine-3 (MYOZ3), la présence ou l'absence d'une substitution de base correspondant à une guanine (G) substituée à une adénine (A) au nucléotide chrl4:38519183 du brin avant de SEQ ID NO: 1, une substitution de base correspondant à une adénine (A) substituée à une guanine (G) au nucléotide chr4:83736244 du brin avant de SEQ ID NO:2, une substitution de base correspondant à une adénine (A) substituée à une guanine (G) au nucléotide chr4:83738769 du brin avant de SEQ ID NO:3, et une substitution de base correspondant à une adénine (A) substituée à une guanine (G) au nucléotide chrl4:27399222 de SEQ ID NO:4, ou son complément, dans lequel la présence de l'une quelconque desdites substitutions dans ledit acide nucléique indique une myopathie équine héréditaire chez ledit cheval.

2. Procédé selon la revendication 1, comprenant en outre :

   la mise en contact de l'acide nucléique avec au moins une sonde oligonucléotidique pour former un acide nucléique hybridé ; et
   l'amplification de l'acide nucléique hybridé.

3. Procédé selon la revendication 2, dans lequel l'exon 6 de la région codante de la myotiline équine (MYOT), les exons 15 et 21 de la région codante de la filamine C équine (FLNC) et l'exon 3 de la région codante de la myozénine-3 équine (MYOZ3), ou une partie de ceux-ci, sont amplifiés.

4. Procédé selon la revendication 2, dans lequel l'acide nucléique hybridé est amplifié à l'aide d'une réaction en chaîne par polymérase, d'une amplification par déplacement de brin, d'une réaction en chaîne par ligase ou d'une amplification basée sur une séquence d'acide nucléique.

5. Procédé selon la revendication 2, dans lequel au moins une sonde oligonucléotidique est immobilisée sur une surface solide ou une surface semi-solide.

6. Procédé selon l'une quelconque des revendications 1 à 5, dans lequel l'acide nucléique est de l'ADN génomique.

7. Procédé selon l'une quelconque des revendications 1 à 5, dans lequel l'acide nucléique est l'ARN.

8. Procédé selon la revendication 7, dans lequel le procédé comprend la conversion de l'ARN en ADN par transcriptase inverse.

9. Procédé selon la revendication 8, dans lequel le procédé comprend la détection de la présence ou de l'absence d'un biomarqueur qui comprend un polynucléotide MYOT équin de SEQ ID NO :5 ayant une cytosine (C) substituée à la thymine (T) à la position nucléotidique 694, un polynucléotide FLNC équin de SEQ ID NO :6 ayant une adénine (A) substituée à la guanine (G) à la position nucléotidique 2257, un polynucléotide FLNC équin de SEQ ID NO : 6 ayant une adénine (A) substituée à la guanine (G) à la position nucléotidique 3619, et un polynucléotide MY0Z3 équin de SEQ ID NO : 8 ayant une thymine (T) substituée à la cytosine (C) à la position nucléotidique 125 ; dans tous les cas, la présence du nucléotide spécifié peut être déduite de la détection du nucléotide présent au niveau du complément de celui-ci, dans lequel la présence de l'un quelconque desdits biomarqueurs étant indicative d'une myopathie équine héréditaire chez ledit cheval.

10. Procédé de détection de la présence ou de l'absence de biomarqueurs chez un cheval, le procédé comprenant : la détection dans un échantillon biologique provenant d'un cheval, de l'échantillon biologique comprenant un acide nucléique codant pour un polypeptide de myotiline, un polypeptide de filamine-C et un polypeptide de myozénine-3, la présence ou l'absence d'un acide nucléique codant pour un polypeptide de myotiline ayant un résidu de proline (P) substitué par de la sérine (S) à la position 232 de SEQ ID NO : 10, un acide nucléique codant pour un polypeptide de filamine-C ayant un résidu de lysine (K) substitué par du glutamate (E) à la position 753, un acide nucléique codant pour un polypeptide de filamine-C ayant un résidu de thréonine (T) substitué par de l'alanine (A) à la position 1207 de SEQ ID NO : 13, et un acide nucléique codant pour un polypeptide de myozénine-3 ayant un résidu de leucine (L) substitué par de la sérine (S) à la position 42 de SEQ ID NO : 16, dans lequel la présence de l'un quelconque desdits acides nucléiques dans ledit échantillon indique une myopathie équine héréditaire chez ledit cheval.

FIG. 1

```
38,520,183    CTACTGCCAA CACAAGTGAC TTCATGTCTA TTCCTGTTTT ACGAAAGGAC
              AATCTGTACT GTGTGCTCCC ATTTCAAACA CACCAGGGGG TCATATCATC
              CTTTGTTGTA AAAGAGTAAT CTGAAAAATG AACCACTATC AATTCGAAGT
              CTAGCTAATT TTCATAAATC ATGTCTGTTG AACAGTCTTA TTAATTCTGA
              GGATTTCTTG CTGGAGTATT AGAAGGTTAG TAGAACTAAC CACAATTGTA
              CAAATACAGC CAACATAGAA TTGTTCCTTT CAACTACAAC TACTTAAGTC
              TGCTTCATTT CAACCTAACA ATTGCATATT AACATTATCA TTTTGTTTTA
              AATTCGAGCA GCACAATTCA GAACACGCAC GACTACAAGT TCCTACATCA
              CAAGTGAGGT AAAAATTTTT TAATTTTAAA GACATATATT TTGCTATGTA
              AAATGTTTTC CGTTTAAAAG TGTACTATTT CGAAAGCCCT CAAGTTTCCT
              ACATAACTTT TATAAAGAAC AACGTGAATT TTCTAATGTC CAACATCATG
              ATCAAAATTT TTATCTGTTA ATCATCAGAC CTATCTCGGA GGAAGTTTTT
              AAAATGTTTT GGTCAAATAA GCCAGGACCA TGTTTCCATG TTACACAAGT
              TTCCTATGAC AATGGAAACC CAATTCTTAT TATACAATCT TTGCACATGA
              TAAGAATTGT CCATGGGGTA CTCTGCATTT TAACAGATCA GCTTCTTCAT
              TAATTCGTTG CACTGTAAAA AGGAAACAAA GATGTATAAT TTGGGCCAGA
              TGTTTCTAAA TTGCTACCAT TTCTACCAGT CATCAAATAT ACCACAGCTA
              AATATAGCCT CTCTCCATTG TTATTACACT ATCAGATCAA ACCCACTCTA
              GGGTTTTAAA TTTGATGACT AAACTATATC TGTGTGAAAG GAAGAGCAAT
              GATAATTTTC CGCATGGTGA TGATTAAACT ATTTTTGCAG AAGCAGATCA
38,519,183    C
              CTTCAAGGGG AGATGTGAAT GATCAGGATG CAATCCAGGA GAAGTTTTAC
              CCACCTCGTT TCATTCAAGT GCCAGAAAAC ATGTCAATTG ACGAAGGAAG
              ATTCTGCAGA ATGGACTTCA AAGTAAGAGA AGAGTTCAAA AGTACTGGAG
              GAAAATTAAC AATGTGATAC TAAGTTTTGA AAAATGTGCT CTTCCTCTTC
              ATAGCTTGCT CCACAGCTTG AGAAGCAGCA CGTACAGTGG AGAAGAACAC
              CAATTCTAGA GCCAAGCTTA CCTGGATTTG GATCCTGGCT CTGCTACTTT
              CCAGCTGTGT AACCTCAGAC AAGTCACTTA CCCTGTGCTT TCCTTTTCTT
              TTCTATTTTT TTTTAAAGAT TGGCACCTGA ACTAACATCT GTTTGCAATC
              TTTTTTTTTC TTCTTCTTCT TCTTCTTCTT CGCCCCAAAG CCCCCTAGCA
              CATAGTTGTA TGTTCTAGTT GTGAGTGCCT CTGGTTGTGC TATGTGGGAC
              GTCACCTCAG CACAGCCTGA CCAGCGGTGC CACGTCTGCA CTCAGGATCC
              GAACCGGCAA AACCCTGGGC CGCCACAGAG CACGTGAACT TAACCGCTTG
              GCCACGGGGG CAGCCCCCTA TCCTGTGCTT TTCTCATCTG CAAAACAAGA
              ATAACAAATG TACCTAACTC ATAAAGTTGT TTGAGGATTA ACTGAGTAAA
              TAGGCATAAG GTACTCACAA TAGTGCCTAC AACCTAATAA ATACTCTATA
              TAAGTGAAGC TATCATAATA GGATTTCTAA CGCAGAATGC CTCTTTAATT
              CTCCTCCAAT AAGAAATTCA ATCAACCCTC CTTTCTCTTG CCCTAGAAGA
              ATTTTTCGGG CTAGAAAGGC CTACATTTTT ATTACCCTCA TTTTGGAGAA
              CTTTTTCTTA GAAAATGTTT TCCCGAATTC ATTATGAATG AAAGGCAGTG
              CATGGATTTA ATTGTTAAGG CTTACATAAA AACACTTAAA CTGCCATTTG   38,518,183

              (SEQ ID NO:1)
```

FIG. 2

```
83,735,244     ACTATGGAAA ACTTGAAATT TTGTTTTCTT ACTGATAATA ATCTTAAAAA
               CATATTTTAC CAAAAACGTA TTTTTATACT AATAATTTGT AGTTAAAAGA
               TGGTTTGATT GAATAAGACA TTGGATTCCC AGGTCAGTTT CTCAGACTGG
               CGTCCCTTGA TCCCTAGAGG TTAATGAACA TATTCTTGGG TCCAAAAGTA
               TCTAATTCTT TAAAAGCTTC ATCATTCAAT TTGAGGAAGA TTGATTTAGC
               CCAGCCTCTC CCCTGCCAAC ATATTTCAGA GACCTAGGCT GGGAGACCAG
               GCCATGCAGA CGGCCTAGAG CCCTGTCTCC TGGCTCCCAG GGTGCGCCCT
               GGCCACCAGA ATGCAGAGCA CTAGGCTGTG TGGCCTGAGG TCTATGGCGG
               TTGGGGCGCT GGGTGTTGGC GGTGGGTAGG GAGGACAGCC CCAAGCCAGC
               AGAGGGCGTC GGTGCTCTGA GAAGGCCAGA GTGCTCTAAG TACCTTGCTC
               GGATGATGCC CACAGGACGC CGACGGCTGC CCTATCGACA TCAACGTCTT
               CACCACTGGC AACGGCATCT TCCGCTGCTC CTACGTGCCC ACCAAGCCCA
               TTAAACACAC CATCATCATC TCTTGGGGAG GTGTCAACGT GCCCAAGAGC
               CCCTTCCGGG TGCGTCCTCT GAACCCTGCC TGGTGCCCAC TGCCCAGGGG
               TCCCAGAGGG AGGGTGAAGC CCTATGCAGG AGATGTCGGC TGTCGCTGGG
               CCCTGGTCAC TGCTCCCCAC CTCAAGGCCC CCACGAAGGA GCTAAGATTG
               ATCCCATTAA GGCTGAGGCG CCTAGTGCTG GGGAAGAGGA TGGGCTTTGA
               GGGAGGGGCA CCATGCTGAG CATTGACCCC TTCCCACAGG TAAACGTGGG
               AGAGGGCAGT CACCCTGAGA AAGTGAAGGT GTACGGCCCT GGCGTGGAGA
               AGACGGGCCT CAAGGCCAAC GAGCCCACCT ATTTCACCGT GGACTGCAGC
83,736,244     A
               AGGCGGGGCA AGGTGCACCA GCCAGGCGGG GGAGTAGGAG ACGGGGCCTG
               GGGCGGGGTC TGGGTGGGTA GGGCTAGTAC TCCTGGCAGA GCTGTTTCCT
               GGCAGAGCTC TAGTGGGACT GCTCAGGTGG GGCAGGTTGT TGAGCCCCCA
               TCTCATGGCC TCCTGTCTGC CTACCCTCAG GCGATGTGAG CATTGGCATC
               AAGTGCGCCC CCGGCGTGGT GGGCCCCGCA GAGGCTGACA TTGACTTTGA
               CATCATCAAG AATGACAATG ACACCTTCAC AGTCAAGTAC ACACCCCCAG
               GGGCCGGCCG CTACACCATC ATGGTGCTGT TTGCCAACCA GGTACCCTGC
               CCTCAGCTTT TGATACTCAC TAGCGGCCTA CACCTCCCAA TTCTAAACCA
               GCAGCTTGAG ATGCTGTCAT CTGGGGACAA TTCCACAGCC ATTGACTGAG
               CACGTTCCTG TGTGTGGGGA GCATGCCTCA GAAGAAAAAA AAGGGGCTCT
               TTCAGAGACT GCCCTTGTCC ACCATGACGA AGCCTTTTCT CCATGCAGGA
               GATCCCTGCC AGCCCCTTCC ACATCAAGGT GGACCCATCC CATGATGCCA
               GCAAGGTCAA GGCTGAGGGC CCTGGGCTGA ACCGCACAGG TAGGTGTCTG
               GGCAGGGGCT GGGCTGGGCT GGGGTTTGCG CTAGGTGGCT GCCAGGCCCT
               CACCACTGTC TCTGGGTGGG CTCCCCAGGT GTAGAAGTTG GGAAGCCCAC
               TCACTTCACG GTGCTGACCA AGGGAGCTGG CAAGGCTAAG CTGGACGTGC
               ACTTTGCCGG GGCCGGCAAG GGTGAGGCTG TGCGGGACTT TGAGATCATC
               GACAACCACG ACTACTCCTA TACCGTCAAG TACACCGCCG TCCAGCAGGT
               GGGCTCTGCC CCCTCCCATG CCCCTGCCTG TCCTGGCTAA TGGGGGTCCT
               GGGTCTCCTT CCTCACATAG GGCCAGTTCT GGATCTTGGC CCCTTTCCTG   83,737,244
```

(SEQ ID NO:2)

FIG. 3

```
83,737,769     CCACCCGGTA CCTGGCAGCC CCTTCACTGT GGAGGGTGTC CTGCCCCCCG
               ACCCCTCCAA GGTGAGGAGA TAGAAGCTGA TGGGAGCTGG GAGTTAGGGG
               CTTGTAGGGA AGATGGATGA GTCACAGGGG CAGAGGTCAG AGGGCCTTAT
               GTCCTGGAGT GGGCACAGCC AAGGACCCAG GGCCAGGGCG ATCCCCCACC
               CCAGGACCTC CTTTTGAGAC AGCTTAGTTA GTAAGAACCT GGCCTTGGAT
               CAGACCTGGG CTTGAATCCC AAGTCTACTA ACTGGGACAA ATTATTTAAT
               CTCTCTGCTT CCGTTTCTGC ACCTGGAATT GTTATAGGGC CTCAGTCTGT
               AGTAGCTGCT ACAGTTGAAG ATGCCTGGCT GGGTGGGGGG CCATGAAGGC
               CAAGCACGGG TGAGGCCAAG TGTAGGGAAC TCACATACCT GCTCTTCCCT
               CTAGGTTTGT GCTTATGGCC CTGGTCTCAA GGGTGGGCTG GTAGGCAGCC
               CAGCGCCGTT CTCCATCGAC ACCAAGGGGG CTGGCACCGG TGGCCTGGGG
               CTGACTGTGG AGGGCCCCTG TGAGGCCAAG ATCGAGTGCC AGGACAATGG
               TGATGGCTCA TGTGCGGTCA GCTACCTGCC CACGGAGCCG GGCGAGTACA
               CCATCAACAT CCTGTTCGCC GAAGCCCACA TCCCCGGCTC ACCCTTCAAG
               GCTACCATCC GGCCCGTGTT CGACCCGAGC AAGGTGCGGG CCAGTGGGCC
               AGGCCTGGAG CGTGGCAAGG CTGGTGAGGC AGCCACCTTC ACTGTGGACT
               GCTCGGAGGC GGGCGAGGCT GAGCTGACCA TCGAGATCCT GTCAGACGCT
               GGCGTCAAGG CCGAGGTGCT GATCCACAAC AATGCTGACG GCACCTACCA
               CATCACCTAC AGCCCCGCCT TCCCCGGCAC CTACACTATT ACCATCAAGT
               ACGGTGGGCA CCCCGTACCC AAATTCCCCA CCCGCGTCCA TGTGCAGCCC
83,738,769     A
               CTATCGACAC CAGTGGAGTC AAGGTCTCGG GGCCTGGTGT GGAGCCGCAC
               GGTGAGTGAG AGAGGAGCCA GGAGCCCGTC AGGGAGCCAG GGGAGGCAGC
               AGAAGGGACG GAAGCAAGCC TGAGTGCTTA GCAGAGCACC ATAGTCTGAA
               GGGAGGACTT GGGGACAGCC CTGGCGTCCT TAGGGCTCAG ACACCCCAGA
               GAAGCAGCCA AGGGTGCAGG CGGGGCGTGG AGCTTTGTCT CTGCTCTTGT
               GACACAATTG CATCTGCCCA TGGACATGTT TCTGGGGAGA TTCTTAACTT
               TCATTAGCTT AAGGGGTCCC TGATCTGAGA AAGCTAAGAA CCCTGCTCTA
               GGCAGTGCAG AGGGAAAGCT TTGGGGTAGG GGGTGACGAG AGCCAGGAGA
               CAGCTCAGCT CCATTCAGTA AATGAGAGTG GATCACCTGC TGGGGGCCAG
               ACCCTGGGTT AGGCATGAGG GTGGGCGAGG GACGTAAGAC CCAGACTCAC
               CCATGAGCGG CCCTCAGCCT GGTGGGGCAA CTGACGTGAA CAGAATGTCA
               CCATATGGTG AAGCGAGCAA AGGCAGGGGC TGTGGGAGAC AGGGCCTGGA
               GCTCACAGGG GTGCAGAGGG TGGCCTGGCT CACGCTAGGA AGCAGTCAGT
               GGAAAGGAAG AGCAGCCTTG GAGTCGGGGA GACGAAAAGA GGGGTGAGCA
               ACTGTGTGAA GGTTGATCCC AGCCCATGCT GAGCTCTCTT CTCCTGGGAC
               CCAGGTGTCC TGCGTGAGGT GACCACTGAG TTCACTGTGG ATGCAAGATC
               CCTAACAGCC ACAGGTGGGA ACCATGTGAC GGCTCGTGTG CTCAACCCCT
               CGGGTGCTAA GACGGACACC TACGTGACGG ACAACGGGGA TGGCACCTAC
               CGAGTGCAAT ACACAGCCTA TGAAGAGGGT GAGGGGCCGG CTGGGCGGGG
               ATGGGGGATC TGATCACAGC ATTCTCAGGG CAGGAGGGTG CTGGGGCCGC   83,739,769

               (SEQ ID NO:3)
```

FIG. 4

27,400,222 TGTTCCAGTT TTTATCTCCA GGTTAGTTTT CTCCCATGAA CACTGGACCC
ACACATTCAG CTCCCTTCTC ACATTTGAGT GTCTACACAG CTCTTCAAAT
CTTATATGCC CAATTTTGGA GACCATTAGG AGAGTTGAAC ATGGACTGGG
CATTATATAA AACAAAATTT ATTCACTTGG AAGGGTAGAG ACCTTTAATT
GATAAAAGCA AGTTATAAAG CATGATACTA TGATTCCATT TTGGTTAAAA
ACTCATATAG GAATATATAG TCATTGAAAA AGTATGAAAT GATTTACCTG
AAAGTGTTAA TAGTGGCAAT CTCTGGGTGG GAGGCAATTC TTTTCTTTTT
CTCTTCTATC TGTATTTTCT AACTTTCTTC AATGCACATA TGTGCTATTC
TTATAATAAT AAGAGGCTAT TTTGAATTGA AAAGACCTCG AGTCTAAAAC
TGAACTCGAT CTTTCCCGCA CCTTCTCCTC CTCCAGCCGT CCCCACCTCA
GGAGGTGGCT CCATCCTTCC AGTGGCTCAG GCCAGAGCCT CTCCGTAGAC
TCCCACCCTC TCTCTCTCCT AGCCCATACC CATGGCTGAC CTTTAAGGGA
TATCTAGAAT CTGATCATTT CTCACTACTT CCACCACTAT GGGCAAAGAG
CTGCACAGGA AATTCACGGA ATAAATACAA ATGGCCAAAG AACGTCTGAA
ATGGGGCTCA GTCTCACTGA GTGTCAAACA CATGCAAGTT AAACTCTGAG
GTGTCATTTC TGCCCATCAG GTGGGCAAAG ATTCGGCTGA ATGATGACTC
TCAGTGTCGG TAAGTGTGGG GAGAAACCAG GTTTCTCACA CACAATGGAC
AGTGTGCTGA GGAGGCCTCC TGAGAGGCGG CAGCCTAGGA AGCCTGCGGG
TTTGGTAGTA AGAGCTGCCT CTGGCCTCTC CTGGCAGTCC CTGTGCTGGA
CCTGGGCAAG AAGCTGAGCG TGCCCCAGGA CCTGATGATG GAAGAGCTGT
27,399,222 T
GCTCCGCAAC AACCGGGGAT CCCTCCTCTT CCAGAAGAGG CAGCGCCGCG
TGCAGAAATT CACCTTTGAG TTTGCAGCCA GCCAGCGGGC GGTGAGTAAG
CCCCCACTGT GCTCACAGGG AAACTGAGGC CCAGAGAGAG CCAGTGGTTA
GTCTAAAGCC CAACAGTGAG CCAGGGGAGG ACCTCTGGCC CCTGGGGAGT
CCCTCAAGCT CCAGCTGGGG AAGACTGTGA TGTTCCCATC GGACTGAGCC
CCGCCCTGCC GGGTGATCCT AGAAAAGGGT TACCTCTTTA GGCGTCTCCC
CAGATGTGAA ACATGAAGTG GCATGGGCAG GAGGAGCTGT GGAGTGAAGG
ACTCTGGCTC CTATAAAAAG GGCTGAAGCA TAGTCATGTG CTCTGGGCTG
AATTTACAGC AAGCCGGATT TAGGTTAGAC TTGAGCTTGA TCTAAGGTGG
AAAATGCAGA ATGCCTTTTT CTCTTCTTCC CACTGGAAGA AGGAAAAGGC
CACGGCACAG TCTGCTGGTA GAGAAAGGAG GCGAGAGCTG TTCATCCTCA
GGCTGCAAAG AGGCAGGAGG CAGTTTCAGG TACTTCTAAG GAACTCCCGC
ACATTCCATC CAGATGCTGC TTAGTTTCTG GACTAGACAG GGAGCGGCTG
AGAGGAGTCA TGTGGCCGAA TGATAAAGAC GACAAACACC TGCCTCCCTT
TGTGCCCAGG ACGGTGCTAG CTAGCGGCTT TGCATCTATC ATCTCACTTA
ATGCTCACAG AGTGAGTTAT CGTCATCACC TCCATTTTAC AGAGGAGGAA
ACTGAGGCTC AGGGAAGTCA AGTATCTGTG CAAAATACAA TATCTGTCAA
TATTATAATA GCAACTAAGA CGCAGAGCAT TTCTCTGTCC AGGCGCTGGG
CACCCGGCCC CCCCATCCCC GGGGAGGTGG GCGTTAGGAT TAGGCCACGC
CCTTTTATTT TCTTGGAGGG AGACCCTGGT CCCTTTCCCC GCTGAGCCCT 27,398,222

(SEQ ID NO:4)

FIG. 5

1 ATGTTTAATT ACGAACGTCC AAAGCACTTC ATCCAATCCC AAAACCCATG
51 TGGCTCCAGA CTGCAGCCTC CTGGACCGGA AACCTCCAGC TACTCTAGCC
101 AGACCAAACA GTCTTCCATT ATCATCCAGC CCCGCCAGTG CACAGAGCAA
151 AGATTTTCTG CCTCCTCAAC AATGAGCTCT CATATCACCA TGTCCTCCTC
201 TGCTTTCCCT GCTTCTTCCC AGCAGCTTGC TGGCTCCAAT CCAGGCCAAA
251 GGGTTACAGC CACTTATAAC CAGTCCCCAG CCAGCTTCCT CAGCTCCATA
301 TTACCATCAC AGCCTGATTA CAGTAGCAGT AAAATCCCTT CCACTGTGGA
351 CTCCAACTAT CAACAACCTT CATTTGGCCA ACCTGTAAAT GCTAAGCCAT
401 CCCAAAGTGC GAATGCTAAG CCCATACCAA GGACTCCTGA CCATGAAATC
451 CAAGGATCAA AGGAAGCTCT GATTCAAGAT TTGGAGAGAA AGCTGAAGTG
501 CAAGGACAGC CTTCTTCATA ACGGAAATCA ACGGCTAACA TACGAGGAGA
551 AGATGGCTCG CAGATTGCTA GGACCACAGA ATGCAGCTGC GGTGTTTCAA
601 GCTCAAAATG ACAGTGAAGC ACAAGATTCA CAGCAGCACA ATTCAGAACA
651 CGCACGACTA CAAGTTCCTA CATCACAAGT GAG**AAGCAGA TCATCTTCAA**
701 **GGGGAGATGT GAATGATCAG GATGCAATCC AGGAGAAGTT TTACCCACCT**
751 **CGTTTCATTC AAGTGCCAGA AAACATGTCA ATTGACGAAG GAAGATTCTG**
801 **CAGAATGGAC TTCAAAG**TGA GCGGACTGCC AGCTCCTGAT GTGTCATGGT
851 ATCTAAATGG AAGACCAGTT CAATCAGACG ATTTTCACAA AATGATAGTG
901 TCTGAAAAGG GTTTTCATTC ACTCATCTTT GAAGTTGTGA GAGCCTCAGA
951 TGCAGGGGCT TATGCCTGTG TTGCCAGGAA CAGAGCAGGA GAAGCCACCT
1001 TTACTGTGCA GCTGGACGTC CTGGCAAAAG AACATAGAAG AGCACCAATG
1051 TTTATCTACA AACCACAAAG CAAAAAAGTT TTTGAGGGAG AGTCCGTGAA
1101 GCTAGAATGC CAAATCTCAG CTATACCTCC ACCAAAGCTT TTCTGGAAAA
1151 GAAACAATGA AATGGTGCAT TTCAATACTG ATCGAATCAG CTTATATCAT
1201 GATAACTCTG GAAGAGTCAC CTTACTGATA AAAGATGTAA ACAAGAAAGA
1251 TGCTGGGTGG TATACTGTGT CTGCAGTTAA CGAAGCTGGA GTAACCTCAT
1301 GTAACACGAG ACTAGATGTT ACAGCCCGTC CAAACCAAAC TCTTCCAGCT
1351 CCTAAGCAGT TACGTGTTCG ACCAACTTTC AGCAAGTATT TAGCACTTAA
1401 CGGGAGAGGC TTGAATGTGA AACAAGCTTT TAATCCTGAA GGAGAGTTTC
1451 AGCGCCTGGC AGCTCAATCT GGACTCTATG AAAGTGAAGA ACTTTAA (SEQ ID NO:5)

FIG. 6

```
ENS          ------------------------------------------------------------

XM   1       ATGGTTTTTATCCACTTTACTCTGGCCACCAGTTGGCTGAGAATATATGTGGTGGAAAGT    60

ENS          ------------------------------------------------------------

XM  61       ACTGGAAGATCACCTACCACTGAGGATCACTGGTTTGACTTGGATTTAGAAGCCAAGATG   120

ENS          ------------------------------------------------------------

XM 121       GAGGTCACAGTCTGGAAAGACCCTGGCGGAAAGCCGCCCCGTGGGAAGGAAGATTCCAGC   180

ENS          ------

XM 181       AGAATC                                                         186

ENS   1      ACGTTCACGCGCCGGTGCAACGAGCACCTCACGTGCGTGGTCAAGCGCCTGACCGACCTG    60
             ||||||||||||||||||||||||||||||||||||||||||||||||||||||||||||
XM  187      ACGTTCACGCGCCGGTGCAACGAGCACCTCACGTGCGTGGTCAAGCGCCTGACCGACCTG   246

ENS 61       CAGCGAGACCTCAGCGACGGGCTACGCCTCATCGCGCTGCTCGAGGTACTCAGCCAGAAG   120
             ||||||||||||||||||||||||||||||||||||||||||||||||||||||||||||
XM  247      CAGCGAGACCTCAGCGACGGGCTACGCCTCATCGCGCTGCTCGAGGTACTCAGCCAGAAG   306

ENS 121      CGCATGTATCGCAAGTTCCACCCGCGTCCCAACTTCCGTCAGATGAAGCTGGAGAACGTG   180
             ||||||||||||||||||||||||||||||||||||||||||||||||||||||||||||
XM  307      CGCATGTATCGCAAGTTCCACCCGCGTCCCAACTTCCGTCAGATGAAGCTGGAGAACGTG   366

ENS 181      TCCGTGGCCCTCGAGTTCCTCGAGCGCGAGCACATCAAGCTTGTGTCCATCGACAGCAAG   240
             ||||||||||||||||||||||||||||||||||||||||||||||||||||||||||||
XM  367      TCCGTGGCCCTCGAGTTCCTCGAGCGCGAGCACATCAAGCTTGTGTCCATCGACAGCAAG   426

ENS 241      GCCATCGTGGATGGGAACCTGAAGCTGATCCTGGGGCTGATCTGGACGTTGATCCTGCAC   300
             ||||||||||||||||||||||||||||||||||||||||||||||||||||||||||||
XM  427      GCCATCGTGGATGGGAACCTGAAGCTGATCCTGGGGCTGATCTGGACGTTGATCCTGCAC   486

ENS 301      TACTCCATCTCCATGCCCATGTGGGAAGATGAGGATGATGAAGATGCCCGCAAACAGACG   360
             ||||||||||||||||||||||||||||||||||||||||||||||||||||||||||||
XM  487      TACTCCATCTCCATGCCCATGTGGGAAGATGAGGATGATGAAGATGCCCGCAAACAGACG   546

ENS 361      CCCAAGCAGCGTCTGCTTGGCTGGATCCAGAACAAGGTGCCCCAGCTGCCCATCACTAAC   420
             ||||||||||||||||||||||||||||||||||||||||||||||||||||||||||||
XM  547      CCCAAGCAGCGTCTGCTTGGCTGGATCCAGAACAAGGTGCCCCAGCTGCCCATCACTAAC   606

ENS 421      TTCAACCGCGACTGGCAGGATGGCAAAGCTCTGGGTGCCCTGGTGGACAACTGTGCCCCT   480
             ||||||||||||||||||||||||||||||||||||||||||||||||||||||||||||
XM  607      TTCAACCGCGACTGGCAGGATGGCAAAGCTCTGGGTGCCCTGGTGGACAACTGTGCCCCT   666

ENS 481      GGCCTCTGCCCTGACTGGGAGGCCTGGGATCCCAACCAGCCTGTGGAGAACGCCCGGGAG   540
             ||||||||||||||||||||||||||||||||||||||||||||||||||||||||||||
XM  667      GGCCTCTGCCCTGACTGGGAGGCCTGGGATCCCAACCAGCCTGTGGAGAACGCCCGGGAG   726

ENS 541      GCCATGCAGCAGGCGGACGACTGGCTCGGGGTGCCCCAGGTGATTGCCCCCGAGGAGATT   600
             ||||||||||||||||||||||||||||||||||||||||||||||||||||||||||||
XM  727      GCCATGCAGCAGGCGGACGACTGGCTCGGGGTGCCCCAGGTGATTGCCCCCGAGGAGATT   786

ENS 601      GTGGACCCCAATGTAGATGAGCATTCTGTCATGACCTACCTGTCCCAGTTCCCCAAGGCC   660
             ||||||||||||||||||||||||||||||||||||||||||||||||||||||||||||
```

```
XM  787     GTGGACCCCAATGTAGATGAGCATTCTGTCATGACCTACCTGTCCCAGTTCCCCAAGGCC   846

ENS 661     AAGCTCAAACCTGGTGCCCCTGTTCGCTCCAAGCAGCTGAACCCCAAGAAGGCCATTGCC   720

||||||||||||||||||||||||||||||||||||||||||||||||||||||||||||
XM  847     AAGCTCAAACCTGGTGCCCCTGTTCGCTCCAAGCAGCTGAACCCCAAGAAGGCCATTGCC   906

ENS 721     TATGGGCCTGGCATTGAGCCCCAGGGCAACACCGTGCTGCAGCCTGCCCACTTCACCGTG   780
            ||||||||||||||||||||||||||||||||||||||||||||||||||||||||||||
XM  907     TATGGGCCTGGCATTGAGCCCCAGGGCAACACCGTGCTGCAGCCTGCCCACTTCACCGTG   966

ENS 781     CAGACCGTGGATGCCGGTGTGGGCGAGGTGCTGGTCTACATTGAGGATCCTGAGGGCCAC   840
            ||||||||||||||||||||||||||||||||||||||||||||||||||||||||||||
XM  967     CAGACCGTGGATGCCGGTGTGGGCGAGGTGCTGGTCTACATTGAGGATCCTGAGGGCCAC  1026

ENS 841     ACCGAGGAGGCCAAGGTGGTTCCCAACAATGACAAGGACCGCACCTATGCTGTCTCCTAC   900
            ||||||||||||||||||||||||||||||||||||||||||||||||||||||||||||
XM  1027    ACCGAGGAGGCCAAGGTGGTTCCCAACAATGACAAGGACCGCACCTATGCTGTCTCCTAC  1086

ENS 901     GTGCCTAAGGTTGCTGGGTTGCACAAGGTGACTGTGCTCTTTGCTGGCCAGAACATCGAA   960
            ||||||||||||||||||||||||||||||||||||||||||||||||||||||||||||
XM  1087    GTGCCTAAGGTTGCTGGGTTGCACAAGGTGACTGTGCTCTTTGCTGGCCAGAACATCGAA  1146

ENS 961     CGCAGCCCCTTTGAGGTGAATGTGGGCATGGCCCTTGGGGATGCCAACAAGGTGTCAGCC  1020
            ||||||||||||||||||||||||||||||||||||||||||||||||||||||||||||
XM  1147    CGCAGCCCCTTTGAGGTGAATGTGGGCATGGCCCTTGGGGATGCCAACAAGGTGTCAGCC  1206

ENS 1021    CGTGGCCCTGGCCTGGAGCCTGTGGGCAATGTGGCCAACAAACCTACCTACTTTGACATC  1080
            ||||||||||||||||||||||||||||||||||||||||||||||||||||||||||||
XM  1207    CGTGGCCCTGGCCTGGAGCCTGTGGGCAATGTGGCCAACAAACCTACCTACTTTGACATC  1266

ENS 1081    TACACTGCAGGGGCCGGCACTGGTGATGTTGCCGTGGTGATCGTGGACCCGCAGGGCCGG  1140
            ||||||||||||||||||||||||||||||||||||||||||||||||||||||||||||
XM  1267    TACACTGCAGGGGCCGGCACTGGTGATGTTGCCGTGGTGATCGTGGACCCGCAGGGCCGG  1326

ENS 1141    CGGGACACAGTGGAGGTGGCCCTGGAGGACAAGGGCGACAGCACGTTCCGCTGCACATAC  1200
            ||||||||||||||||||||||||||||||||||||||||||||||||||||||||||||
XM  1327    CGGGACACAGTGGAGGTGGCCCTGGAGGACAAGGGCGACAGCACGTTCCGCTGCACATAC  1386

ENS 1201    AGGCCTGTGATGGAGGGGCCCCACACAGTGCATGTGGCCTTCGCTGGTGCCCCCATCACC  1260
            ||||||||||||||||||||||||||||||||||||||||||||||||||||||||||||
XM  1387    AGGCCTGTGATGGAGGGGCCCCACACAGTGCATGTGGCCTTCGCTGGTGCCCCCATCACC  1446

ENS 1261    CGCAGTCCTTTCCCCGTCCATGTGGCAGAAG                               1291
            |||||||||||||||||||||||||||||||
XM  1447    CGCAGTCCTTTCCCCGTCCATGTGGCAGAAG                               1477

ENS         ------------------------------------------------------------

XM  1478    AGCCCTTGCCACCGCTCGCCCCTTCCGTGCCCATCGTCCACCAGGCCAAGAGAGTGGTGC  1537

ENS         ---

XM  1538    CAC                                                           1540

ENS 1292    CCTGTAACCCCAATGCCTGCCGCGCCTCTGGGCGGGGCCTGCAGCCCAAGGGTGTGCGGG  1351
            ||||||||||||||||||||||||||||||||||||||||||||||||||||||||||||
XM  1541    CCTGTAACCCCAATGCCTGCCGCGCCTCTGGGCGGGGCCTGCAGCCCAAGGGTGTGCGGG  1600

ENS 1352    TGAAAGAGGTGGCTGACTTCAAGGTGTTCACCAAGGGCGCTGGCAGCGGAGAGCTCAAGG  1411
            ||||||||||||||||||||||||||||||||||||||||||||||||||||||||||||
```

XM 1601 TGAAAGAGGTGGCTGACTTCAAGGTGTTCACCAAGGGCGCTGGCAGCGGAGAGCTCAAGG 1660

ENS 1412 TCACAGTCAAGGGGCCAAAGGGCACAGAGGAGCTGGTGAAGGTGCGAGAGGCTGGGGACG 1471
||||||||||||||||||||||||||||||||||||||||||||||||||||||||||||
XM 1661 TCACAGTCAAGGGGCCAAAGGGCACAGAGGAGCTGGTGAAGGTGCGAGAGGCTGGGGACG 1720

ENS 1472 GTGTGTTCGAGTGTGAGTACTACCCTGTGGTGCCTGGGAAGTATGTGGTGACCATCACGT 1531
||||||||||||||||||||||||||||||||||||||||||||||||||||||||||||
XM 1721 GTGTGTTCGAGTGTGAGTACTACCCTGTGGTGCCTGGGAAGTATGTGGTGACCATCACGT 1780

ENS 1532 GGGGCGGCTATGCCATCCCCCGCAGTCCCTTTGAGGTACAGGTGAGCCCAGAGGCAGGAG 1591
||||||||||||||||||||||||||||||||||||||||||||||||||||||||||||
XM 1781 GGGGCGGCTATGCCATCCCCCGCAGTCCCTTTGAGGTACAGGTGAGCCCAGAGGCAGGAG 1840

ENS 1592 CGCAGAAGGTACGGGCCTGGGGGCCTGGTTTGGAAACTGGCCAGGTGGGCAAGTCAGCTG 1651
||||||||||||||||||||||||||||||||||||||||||||||||||||||||||||
XM 1841 CGCAGAAGGTACGGGCCTGGGGGCCTGGTTTGGAAACTGGCCAGGTGGGCAAGTCAGCTG 1900

ENS 1652 ACTTTGTGGTGGAGGCCATTGGCACGGAGGTGGGGACACTGGGCTTCTCCATTGAGGGGC 1711
||||||||||||||||||||||||||||||||||||||||||||||||||||||||||||
XM 1901 ACTTTGTGGTGGAGGCCATTGGCACGGAGGTGGGGACACTGGGCTTCTCCATTGAGGGGC 1960

ENS 1712 CTTCACAGGCCAAGATCGAGTGTGATGACAAGGGGGATGGCTCCTGCGATGTACGGTACT 1771
||||||||||||||||||||||||||||||||||||||||||||||||||||||||||||
XM 1961 CTTCACAGGCCAAGATCGAGTGTGATGACAAGGGGGATGGCTCCTGCGATGTACGGTACT 2020

ENS 1772 GGCCCACTGAGCCCGGGGAGTACGCCGTGCATGTCATCTGCGATGATGAGGACATCCGAG 1831
||||||||||||||||||||||||||||||||||||||||||||||||||||||||||||
XM 2021 GGCCCACTGAGCCCGGGGAGTACGCCGTGCATGTCATCTGCGATGATGAGGACATCCGAG 2080

ENS 1832 ACTCGCCCTTCATTGCCCACATCCAGCCAGCCCCACCTGACTGCTTCCCGGACAAGGTGA 1891
||||||||||||||||||||||||||||||||||||||||||||||||||||||||||||
XM 2081 ACTCGCCCTTCATTGCCCACATCCAGCCAGCCCCACCTGACTGCTTCCCGGACAAGGTGA 2140

ENS 1892 AGGCCTTTGGGCCTGGCCTGGAGCCCACTGGCTGCATCGTGGACAAGCCTGCGGAGTTCA 1951
||||||||||||||||||||||||||||||||||||||||||||||||||||||||||||
XM 2141 AGGCCTTTGGGCCTGGCCTGGAGCCCACTGGCTGCATCGTGGACAAGCCTGCGGAGTTCA 2200

ENS 1952 CCATTGATGCCTCTGCAGCTGGCAAGGGAGACCTGAAGCTCTATGCCCAGGACGCCGACG 2011
||||||||||||||||||||||||||||||||||||||||||||||||||||||||||||
XM 2201 CCATTGATGCCTCTGCAGCTGGCAAGGGAGACCTGAAGCTCTATGCCCAGGACGCCGACG 2260

ENS 2012 GCTGCCCTATCGACATCAACGTCTTCACCACTGGCAACGGCATCTTCCGCTGCTCCTACG 2071
||||||||||||||||||||||||||||||||||||||||||||||||||||||||||||
XM 2261 GCTGCCCTATCGACATCAACGTCTTCACCACTGGCAACGGCATCTTCCGCTGCTCCTACG 2320

ENS 2072 TGCCCACCAAGCCCATTAAACACACCATCATCATCTCTTGGGGAGGTGTCAACGTGCCCA 2131
||||||||||||||||||||||||||||||||||||||||||||||||||||||||||||
XM 2321 TGCCCACCAAGCCCATTAAACACACCATCATCATCTCTTGGGGAGGTGTCAACGTGCCCA 2380

ENS 2132 AGAGCCCCTTCCGG**GTAAACGTGGGAGAGGGCAGTCACCCTGAGAAAGTGAAGGTGTACG** 2191
||||||||||||||||||||||||||||||||||||||||||||||||||||||||||||
XM 2381 AGAGCCCCTTCCGG**GTAAACGTGGGAGAGGGCAGTCACCCTGAGAAAGTGAAGGTGTACG** 2440

ENS 2192 **GCCCTGGCGTGGAGAAGACGGGCCTCAAGGCCAACGAGCCCACCTATTTCACCGTGGACT** 2251
||||||||||||||||||||||||||||||||||||||||||||||||||||||||||||
XM 2441 **GCCCTGGCGTGGAGAAGACGGGCCTCAAGGCCAACGAGCCCACCTATTTCACCGTGGACT** 2500

ENS 2252 **GCAGCGAGGCGGGGCAAG**GCGATGTGAGCATTGGCATCAAGTGCGCCCCCGGCGTGGTGG 2311
||||||||||||||||||||||||||||||||||||||||||||||||||||||||||||
XM 2501 **GCAGCGAGGCGGGGCAAG**GCGATGTGAGCATTGGCATCAAGTGCGCCCCCGGCGTGGTGG 2560

```
ENS 2312    GCCCCGCAGAGGCTGACATTGACTTTGACATCATCAAGAATGACAATGACACCTTCACAG  2371
            ||||||||||||||||||||||||||||||||||||||||||||||||||||||||||||
XM  2561    GCCCCGCAGAGGCTGACATTGACTTTGACATCATCAAGAATGACAATGACACCTTCACAG  2620

ENS 2372    TCAAGTACACACCCCCAGGGGCCGGCCGCTACACCATCATGGTGCTGTTTGCCAACCAGG  2431
            ||||||||||||||||||||||||||||||||||||||||||||||||||||||||||||
XM  2621    TCAAGTACACACCCCCAGGGGCCGGCCGCTACACCATCATGGTGCTGTTTGCCAACCAGG  2680

ENS 2432    AGATCCCTGCCAGCCCCTTCCACATCAAGGTGGACCCATCCCATGATGCCAGCAAGGTCA  2491
            ||||||||||||||||||||||||||||||||||||||||||||||||||||||||||||
XM  2681    AGATCCCTGCCAGCCCCTTCCACATCAAGGTGGACCCATCCCATGATGCCAGCAAGGTCA  2740

ENS 2492    AGGCTGAGGGCCCTGGGCTGAACCGCACAGGTGTAGAAGTTGGGAAGCCCACTCACTTCA  2551
            ||||||||||||||||||||||||||||||||||||||||||||||||||||||||||||
XM  2741    AGGCTGAGGGCCCTGGGCTGAACCGCACAGGTGTAGAAGTTGGGAAGCCCACTCACTTCA  2800

ENS 2552    CGGTGCTGACCAAGGGAGCTGGCAAGGCTAAGCTGGACGTGCACTTTGCCGGGGCCGGCA  2611
            ||||||||||||||||||||||||||||||||||||||||||||||||||||||||||||
XM  2801    CGGTGCTGACCAAGGGAGCTGGCAAGGCTAAGCTGGACGTGCACTTTGCCGGGGCCGGCA  2860

ENS 2612    AGGGTGAGGCTGTGCGGGACTTTGAGATCATCGACAACCACGACTACTCCTATACCGTCA  2671
            ||||||||||||||||||||||||||||||||||||||||||||||||||||||||||||
XM  2861    AGGGTGAGGCTGTGCGGGACTTTGAGATCATCGACAACCACGACTACTCCTATACCGTCA  2920

ENS 2672    AGTACACCGCCGTCCAGCAGGGCAACATGGCAGTGACAGTGACCTATGGTGGGGACCCCG  2731
            ||||||||||||||||||||||||||||||||||||||||||||||||||||||||||||
XM  2921    AGTACACCGCCGTCCAGCAGGGCAACATGGCAGTGACAGTGACCTATGGTGGGGACCCCG  2980

ENS 2732    TCCCCAAGAGTCCCTTTGTGGTGAATGTGGCACCTCCGCTGGACCTCAGCAAAGTCAAAG  2791
            ||||||||||||||||||||||||||||||||||||||||||||||||||||||||||||
XM  2981    TCCCCAAGAGTCCCTTTGTGGTGAATGTGGCACCTCCGCTGGACCTCAGCAAAGTCAAAG  3040

ENS 2792    TTCAAGGCCTCAACAGTAAGGTGGCTGTGGGGCAAGAACAGGCATTCTCTGTGAACACAC  2851
            ||||||||||||||||||||||||||||||||||||||||||||||||||||||||||||
XM  3041    TTCAAGGCCTCAACAGTAAGGTGGCTGTGGGGCAAGAACAGGCATTCTCTGTGAACACAC  3100

ENS 2852    GAGGGGCTGGTGGTCAGGGCCAGCTGGATGTGCGGATGACCTCACCCTCCCGACGACCGA  2911
            ||||||||||||||||||||||||||||||||||||||||||||||||||||||||||||
XM  3101    GAGGGGCTGGTGGTCAGGGCCAGCTGGATGTGCGGATGACCTCACCCTCCCGACGACCGA  3160

ENS 2912    TCCCCTGCAAGCTGGAGCCTGGGGGCGGAGCTGAAGCCCAGGCTGTGCGCTACATGCCTC  2971
            ||||||||||||||||||||||||||||||||||||||||||||||||||||||||||||
XM  3161    TCCCCTGCAAGCTGGAGCCTGGGGGCGGAGCTGAAGCCCAGGCTGTGCGCTACATGCCTC  3220

ENS 2972    CTGAGGAGGGTCCCTACAAGGTGGACATTACCTACGACGGCCACCCGGTACCTGGCAGCC  3031
            ||||||||||||||||||||||||||||||||||||||||||||||||||||||||||||
XM  3221    CTGAGGAGGGTCCCTACAAGGTGGACATTACCTACGACGGCCACCCGGTACCTGGCAGCC  3280

ENS 3032    CCTTCACTGTGGAGGGTGTCCTGCCCCCCGACCCCTCCAAGGTTTGTGCTTATGGCCCTG  3091
            ||||||||||||||||||||||||||||||||||||||||||||||||||||||||||||
XM  3281    CCTTCACTGTGGAGGGTGTCCTGCCCCCCGACCCCTCCAAGGTTTGTGCTTATGGCCCTG  3340

ENS 3092    GTCTCAAGGGTGGGCTGGTAGGCAGCCCAGCGCCGTTCTCCATCGACACCAAGGGGGCTG  3151
            ||||||||||||||||||||||||||||||||||||||||||||||||||||||||||||
XM  3341    GTCTCAAGGGTGGGCTGGTAGGCAGCCCAGCGCCGTTCTCCATCGACACCAAGGGGGCTG  3400

ENS 3152    GCACCGGTGGCCTGGGGCTGACTGTGGAGGGCCCCTGTGAGGCCAAGATCGAGTGCCAGG  3211
            ||||||||||||||||||||||||||||||||||||||||||||||||||||||||||||
XM  3401    GCACCGGTGGCCTGGGGCTGACTGTGGAGGGCCCCTGTGAGGCCAAGATCGAGTGCCAGG  3460
```

```
ENS 3212    ACAATGGTGATGGCTCATGTGCGGTCAGCTACCTGCCCACGGAGCCGGGCGAGTACACCA  3271
            ||||||||||||||||||||||||||||||||||||||||||||||||||||||||||||
XM  3461    ACAATGGTGATGGCTCATGTGCGGTCAGCTACCTGCCCACGGAGCCGGGCGAGTACACCA  3520

ENS 3272    TCAACATCCTGTTCGCCGAAGCCCACATCCCCGGCTCACCCTTCAAGGCTACCATCCGGC  3331
            ||||||||||||||||||||||||||||||||||||||||||||||||||||||||||||
XM  3521    TCAACATCCTGTTCGCCGAAGCCCACATCCCCGGCTCACCCTTCAAGGCTACCATCCGGC  3580

ENS 3332    CCGTGTTCGACCCGAGCAAGGTGCGGGCCAGTGGGCCAGGCCTGGAGCGTGGCAAGGCTG  3391
            ||||||||||||||||||||||||||||||||||||||||||||||||||||||||||||
XM  3581    CCGTGTTCGACCCGAGCAAGGTGCGGGCCAGTGGGCCAGGCCTGGAGCGTGGCAAGGCTG  3640

ENS 3392    GTGAGGCAGCCACCTTCACTGTGGACTGCTCGGAGGCGGGCGAGGCTGAGCTGACCATCG  3451
            ||||||||||||||||||||||||||||||||||||||||||||||||||||||||||||
XM  3641    GTGAGGCAGCCACCTTCACTGTGGACTGCTCGGAGGCGGGCGAGGCTGAGCTGACCATCG  3700

ENS 3452    AGATCCTGTCAGACGCTGGCGTCAAGGCCGAGGTGCTGATCCACAACAATGCTGACGGCA  3511
            ||||||||||||||||||||||||||||||||||||||||||||||||||||||||||||
XM  3701    AGATCCTGTCAGACGCTGGCGTCAAGGCCGAGGTGCTGATCCACAACAATGCTGACGGCA  3760

ENS 3512    CCTACCACATCACCTACAGCCCCGCCTTCCCCGGCACCTACACTATTACCATCAAGTACG  3571
            ||||||||||||||||||||||||||||||||||||||||||||||||||||||||||||
XM  3761    CCTACCACATCACCTACAGCCCCGCCTTCCCCGGCACCTACACTATTACCATCAAGTACG  3820

ENS 3572    GTGGGCACCCCGTACCCAAATTCCCCACCCGCGTCCATGTGCAGCCCGCTATCGACACCA  3631
            ||||||||||||||||||||||||||||||||||||||||||||||||||||||||||||
XM  3821    GTGGGCACCCCGTACCCAAATTCCCCACCCGCGTCCATGTGCAGCCCGCTATCGACACCA  3880

ENS 3632    GTGGAGTCAAGGTCTCGGGGCCTGGTGTGGAGCCGCACGGTGTCCTGCGTGAGGTGACCA  3691
            ||||||||||||||||||||||||||||||||||||||||||||||||||||||||||||
XM  3881    GTGGAGTCAAGGTCTCGGGGCCTGGTGTGGAGCCGCACGGTGTCCTGCGTGAGGTGACCA  3940

ENS 3692    CTGAGTTCACTGTGGATGCAAGATCCCTAACAGCCACAGGTGGGAACCATGTGACGGCTC  3751
            ||||||||||||||||||||||||||||||||||||||||||||||||||||||||||||
XM  3941    CTGAGTTCACTGTGGATGCAAGATCCCTAACAGCCACAGGTGGGAACCATGTGACGGCTC  4000

ENS 3752    GTGTGCTCAACCCCTCGGGTGCTAAGACGGACACCTACGTGACGGACAACGGGGATGGCA  3811
            ||||||||||||||||||||||||||||||||||||||||||||||||||||||||||||
XM  4001    GTGTGCTCAACCCCTCGGGTGCTAAGACGGACACCTACGTGACGGACAACGGGGATGGCA  4060

ENS 3812    CCTACCGAGTGCAATACACAGCCTATGAAGAGGGCGTACATTTGGTGGAGGTGCTGTATG  3871
            ||||||||||||||||||||||||||||||||||||||||||||||||||||||||||||
XM  4061    CCTACCGAGTGCAATACACAGCCTATGAAGAGGGCGTACATTTGGTGGAGGTGCTGTATG  4120

ENS 3872    ATGACGTAGCTGTGCCCAAGAGTCCCTTCCGAGTGGGTGTGACCGAGGGCTGTGACCCCA  3931
            ||||||||||||||||||||||||||||||||||||||||||||||||||||||||||||
XM  4121    ATGACGTAGCTGTGCCCAAGAGTCCCTTCCGAGTGGGTGTGACCGAGGGCTGTGACCCCA  4180

ENS 3932    CGCGTGTTCGGGCCTATGGACCAGGCCTGGAGGGTGGCTTGGTCAACAAGGCCAACCGCT  3991
            ||||||||||||||||||||||||||||||||||||||||||||||||||||||||||||
XM  4181    CGCGTGTTCGGGCCTATGGACCAGGCCTGGAGGGTGGCTTGGTCAACAAGGCCAACCGCT  4240

ENS 3992    TCACTGTGGAGACCAGGGGAGCAGGCACTGGGGGCCTAGGCCTAGCCATCGAGGGCCCCT  4051
            ||||||||||||||||||||||||||||||||||||||||||||||||||||||||||||
XM  4241    TCACTGTGGAGACCAGGGGAGCAGGCACTGGGGGCCTAGGCCTAGCCATCGAGGGCCCCT  4300

ENS 4052    CGGAAGCCAAGATGTCCTGCAAAGACAACAAGGATGGCAGCTGCACCGTAGAGTACATCC  4111
            ||||||||||||||||||||||||||||||||||||||||||||||||||||||||||||
XM  4301    CGGAAGCCAAGATGTCCTGCAAAGACAACAAGGATGGCAGCTGCACCGTAGAGTACATCC  4360

ENS 4112    CCTTCACCCCTGGAGACTATGACGTCAATATCACCTTTGGGGGGCGGCCCATCCCAGGGA  4171
```

```
               ||||||||||||||||||||||||||||||||||||||||||||||||||||||||||||
XM  4361       CCTTCACCCCTGGAGACTATGACGTCAATATCACCTTTGGGGGGCGGCCCATCCCAGGGA  4420

ENS 4172       GCCCGTTCCGGGTGCCAGTGAAGGATGTGGTGGACCCCGGGAAAGTGAAATGCTCAGGAC  4231
               ||||||||||||||||||||||||||||||||||||||||||||||||||||||||||||
XM  4421       GCCCGTTCCGGGTGCCAGTGAAGGATGTGGTGGACCCCGGGAAAGTGAAATGCTCAGGAC  4480

ENS 4232       CAGGGCTGGGGGCCGGTGTCAGGGCCCGGGTACCCCAGACCTTCACGGTGGACTGCAGCC  4291
               ||||||||||||||||||||||||||||||||||||||||||||||||||||||||||||
XM  4481       CAGGGCTGGGGGCCGGTGTCAGGGCCCGGGTACCCCAGACCTTCACGGTGGACTGCAGCC  4540

ENS 4292       AGGCTGGCCGGGCCCCCCTGCAGGTGGCTGTGCTGGGCCCCACAGGTGTGGCTGAGCCTG  4351
               ||||||||||||||||||||||||||||||||||||||||||||||||||||||||||||
XM  4541       AGGCTGGCCGGGCCCCCCTGCAGGTGGCTGTGCTGGGCCCCACAGGTGTGGCTGAGCCTG  4600

ENS 4352       TGGAGATACGTGACAATGGAGATGGCACCCATGCTGTCCACTACACCCCGGCCACTGATG  4411
               ||||||||||||||||||||||||||||||||||||||||||||||||||||||||||||
XM  4601       TGGAGATACGTGACAATGGAGATGGCACCCATGCTGTCCACTACACCCCGGCCACTGATG  4660

ENS 4412       GTCCATACACGGTAGCCGTCAAGTATGCCGACCAGGAAGTGCCACGCAGCCCCTTCAAGA  4471
               ||||||||||||||||||||||||||||||||||||||||||||||||||||||||||||
XM  4661       GTCCATACACGGTAGCCGTCAAGTATGCCGACCAGGAAGTGCCACGCAGCCCCTTCAAGA  4720

ENS 4472       TCAAGGTGCTTCCAGCCCATGATGCCAGCAAGGTGCGGGCCAGTGGCCCTGGCCTCAACG  4531
               ||||||||||||||||||||||||||||||||||||||||||||||||||||||||||||
XM  4721       TCAAGGTGCTTCCAGCCCATGATGCCAGCAAGGTGCGGGCCAGTGGCCCTGGCCTCAACG  4780

ENS 4532       CCGCTGGCATCCCTGCCAGTCTGCCTGTGGAGTTCACCATCGATGCCCGGGATGCTGGTG  4591
               ||||||||||||||||||||||||||||||||||||||||||||||||||||||||||||
XM  4781       CCGCTGGCATCCCTGCCAGTCTGCCTGTGGAGTTCACCATCGATGCCCGGGATGCTGGTG  4840

ENS 4592       AGGGCTTGCTTACCGTCCAGATCCTGGACCCCGAGGGTAAGCCCAAGAAGGCCAACATCC  4651
               ||||||||||||||||||||||||||||||||||||||||||||||||||||||||||||
XM  4841       AGGGCTTGCTTACCGTCCAGATCCTGGACCCCGAGGGTAAGCCCAAGAAGGCCAACATCC  4900

ENS 4652       GAGACAATGGGGATGGCACATACACCGTGTCCTACCTGCCAGACATGAGTGGCCGGTACA  4711
               ||||||||||||||||||||||||||||||||||||||||||||||||||||||||||||
XM  4901       GAGACAATGGGGATGGCACATACACCGTGTCCTACCTGCCAGACATGAGTGGCCGGTACA  4960

ENS 4712       CCATCACCATCAAGTACGGCGGTGACGAGATCCCCTACTCACCCTTCCGCATCCATGCCC  4771
               ||||||||||||||||||||||||||||||||||||||||||||||||||||||||||||
XM  4961       CCATCACCATCAAGTACGGCGGTGACGAGATCCCCTACTCACCCTTCCGCATCCATGCCC  5020

ENS 4772       TGCCCACTGGGGACGCCAGCAAGTGCCTCGTCACAGTGTCCATTGGAGGCCATGGCCTGG  4831
               ||||||||||||||||||||||||||||||||||||||||||||||||||||||||||||
XM  5021       TGCCCACTGGGGACGCCAGCAAGTGCCTCGTCACAGTGTCCATTGGAGGCCATGGCCTGG  5080

ENS 4832       GTGCCTGCCTAGGCCCCCGCATCCAGATCGGGGAGGAGACGGTGATCACAGTGGACGCCA  4891
               ||||||||||||||||||||||||||||||||||||||||||||||||||||||||||||
XM  5081       GTGCCTGCCTAGGCCCCCGCATCCAGATCGGGGAGGAGACGGTGATCACAGTGGACGCCA  5140

ENS 4892       AGGCAGCAGGCAAGGGGAAGGTAACATGCACAGTGTCCACGCCGGATGGGGCAGAGCTCG  4951
               ||||||||||||||||||||||||||||||||||||||||||||||||||||||||||||
XM  5141       AGGCAGCAGGCAAGGGGAAGGTAACATGCACAGTGTCCACGCCGGATGGGGCAGAGCTCG  5200

ENS 4952       ACGTGGACGTGGTTGAGAACCATGACGGTACCTTTGACATCTACTACACAGCGCCCGAGC  5011
               ||||||||||||||||||||||||||||||||||||||||||||||||||||||||||||
XM  5201       ACGTGGACGTGGTTGAGAACCATGACGGTACCTTTGACATCTACTACACAGCGCCCGAGC  5260

ENS 5012       CGGGCAAGTACGTCATCACCATCCGCTTTGGAGGCGAGCACATCCCCAACAGTCCCTTCC  5071
               ||||||||||||||||||||||||||||||||||||||||||||||||||||||||||||
```

```
XM  5261    CGGGCAAGTACGTCATCACCATCCGCTTTGGAGGCGAGCACATCCCCAACAGTCCCTTCC  5320

ENS 5072    ATGTGCTGGCGTGTGACCCCATGCCCCACGTGGAGGAGCCCTCTGACGTGTTGCAGCTGC  5131
            ||||||||||||||||||||||||||||||||||||||||||||||||||||||||||||
XM  5321    ATGTGCTGGCGTGTGACCCCATGCCCCACGTGGAGGAGCCCTCTGACGTGTTGCAGCTGC  5380

ENS 5132    ACCGGCCCAGCGCCTACCCCACACACTGGGCCACAGAGGAGCCAGTGGTGCCTGTGGAGC  5191
            ||||||||||||||||||||||||||||||||||||||||||||||||||||||||||||
XM  5381    ACCGGCCCAGCGCCTACCCCACACACTGGGCCACAGAGGAGCCAGTGGTGCCTGTGGAGC  5440

ENS 5192    CAATGGAGTCTATGTTGAGGCCCTTCAACCTGGTCATCCCCTTCACCGTGCAGAAAGGGG  5251
            ||||||||||||||||||||||||||||||||||||||||||||||||||||||||||||
XM  5441    CAATGGAGTCTATGTTGAGGCCCTTCAACCTGGTCATCCCCTTCACCGTGCAGAAAGGGG  5500

ENS 5252    AGCTCACAGGGGAGGTACGGATGCCCTCTGGGAAAACTGCCCGGCCCAATATCACCGACA  5311
            ||||||||||||||||||||||||||||||||||||||||||||||||||||||||||||
XM  5501    AGCTCACAGGGGAGGTACGGATGCCCTCTGGGAAAACTGCCCGGCCCAATATCACCGACA  5560

ENS 5312    ACAAGGATGGCACCATCACAGTGAGGTACGCGCCCACTGAGAAAGGCCTGCACCAGATGG  5371
            ||||||||||||||||||||||||||||||||||||||||||||||||||||||||||||
XM  5561    ACAAGGATGGCACCATCACAGTGAGGTACGCGCCCACTGAGAAAGGCCTGCACCAGATGG  5620

ENS 5372    GGATCAAGTATGATGGCAACCACATCCCTGGTGA-CCCCCTGCAGTTCTATGTGGATGCC  5430
            |||||||||||||||||||||||||||||||| || |||||||||||||||||||||||||
XM  5621    GGATCAAGTATGATGGCAACCACATCCCTGG-GAGCCCCCTGCAGTTCTATGTGGATGCC  5679

ENS 5431    ATCAACAGCCGCCATGTCAGTGCCTACGGGCCAGGCCTGAGCCATGGCATGGTCAACAAG  5490
            ||||||||||||||||||||||||||||||||||||||||||||||||||||||||||||
XM  5680    ATCAACAGCCGCCATGTCAGTGCCTACGGGCCAGGCCTGAGCCATGGCATGGTCAACAAG  5739

ENS 5491    CCGGCCACCTTCACCATTGTCACCAAGGATGCTGGGGAAGGGGGTCTGTCACTGGCCGTG  5550
            ||||||||||||||||||||||||||||||||||||||||||||||||||||||||||||
XM  5740    CCGGCCACCTTCACCATTGTCACCAAGGATGCTGGGGAAGGGGGTCTGTCACTGGCCGTG  5799

ENS 5551    GAGGGCCCGTCCAAAGCAGAGATCACCTGCAAGGACAACAAGGATGGCACCTGCACGGTG  5610
            ||||||||||||||||||||||||||||||||||||||||||||||||||||||||||||
XM  5800    GAGGGCCCGTCCAAAGCAGAGATCACCTGCAAGGACAACAAGGATGGCACCTGCACGGTG  5859

ENS 5611    TCCTACCTACCCACAGCGCCTGGAGACTACAGCATCATCGTGCGCTTTGATGACAAGCAC  5670
            ||||||||||||||||||||||||||||||||||||||||||||||||||||||||||||
XM  5860    TCCTACCTACCCACAGCGCCTGGAGACTACAGCATCATCGTGCGCTTTGATGACAAGCAC  5919

ENS 5671    ATCCCGGGGAGCCCCTTCACAGCCAAGATCACAGGCGATGACTCGATGAGGACGTCACAG  5730
            ||||||||||||||||||||||||||||||||||||||||||||||||||||||||||||
XM  5920    ATCCCGGGGAGCCCCTTCACAGCCAAGATCACAGGCGATGACTCGATGAGGACGTCACAG  5979

ENS 5731    CTAAACGTGGGCACCTCCACGGATGTGTCACTGAAGATCACCGAGAGTGACCTGAGCCTG  5790
            ||||||||||||||||||||||||||||||||||||||||||||||||||||||||||||
XM  5980    CTAAACGTGGGCACCTCCACGGATGTGTCACTGAAGATCACCGAGAGTGACCTGAGCCTG  6039

ENS 5791    CTGACCGCCAGCATCCGTGCCCCCTCGGGCAACGAGGAGCCCTGCCTGCTGAAGCGCCTG  5850
            ||||||||||||||||||||||||||||||||||||||||||||||||||||||||||||
XM  6040    CTGACCGCCAGCATCCGTGCCCCCTCGGGCAACGAGGAGCCCTGCCTGCTGAAGCGCCTG  6099

ENS 5851    CCCAACCGGCACATTGGCATCTCCTTCACCCCCAAGGAAGTTGGGGAGCATGTGGTGAGC  5910
            ||||||||||||||||||||||||||||||||||||||||||||||||||||||||||||
XM  6100    CCCAACCGGCACATTGGCATCTCCTTCACCCCCAAGGAAGTTGGGGAGCATGTGGTGAGC  6159

ENS 5911    GTGCGCAAGAGTGGGAAGCACGTCACCAATAGCCCCTTCAAGATCCTGGTGGGGCCTTCT  5970
            ||||||||||||||||||||||||||||||||||||||||||||||||||||||||||||
XM  6160    GTGCGCAAGAGTGGGAAGCACGTCACCAATAGCCCCTTCAAGATCCTGGTGGGGCCTTCT  6219
```

```
ENS 5971    GAGATCGGGGACGCTAGCAAGGTGCGGGTCTGGGGCAAGGGCCTGTCCGAGGGACAAACC  6030
            ||||||||||||||||||||||||||||||||||||||||||||||||||||||||||||
XM  6220    GAGATCGGGGACGCTAGCAAGGTGCGGGTCTGGGGCAAGGGCCTGTCCGAGGGACAAACC  6279

ENS 6031    TTCCAGGTGGCGGAGTTCATCGTGGACACTCGTAATGCAGGTTATGGGGGCCTGGGGCTG  6090
            ||||||||||||||||||||||||||||||||||||||||||||||||||||||||||||
XM  6280    TTCCAGGTGGCGGAGTTCATCGTGGACACTCGTAATGCAGGTTATGGGGGCCTGGGGCTG  6339

ENS 6091    AGTATTGAAGGCCCTAGCAAGGTGGACATCAACTGTGAGGACATGGAGGATGGCACATGC  6150
            ||||||||||||||||||||||||||||||||||||||||||||||||||||||||||||
XM  6340    AGTATTGAAGGCCCTAGCAAGGTGGACATCAACTGTGAGGACATGGAGGATGGCACATGC  6399

ENS 6151    AAAGTCACCTACTGCCCCACTGAACCCGGCACCTACATCATCAACATCAAGTTTGCTGAC  6210
            ||||||||||||||||||||||||||||||||||||||||||||||||||||||||||||
XM  6400    AAAGTCACCTACTGCCCCACTGAACCCGGCACCTACATCATCAACATCAAGTTTGCTGAC  6459

ENS 6211    AAGCATGTGCCAGGAAGCCCCTTCACTGTGAAGGTTACCGGTGAGGGCCGCATGAAGGAA  6270
            ||||||||||||||||||||||||||||||||||||||||||||||||||||||||||||
XM  6460    AAGCATGTGCCAGGAAGCCCCTTCACTGTGAAGGTTACCGGTGAGGGCCGCATGAAGGAA  6519

ENS 6271    AGCATCACCCGGCGCAGGCAGGCACCTTCCATCGCCACCATTGGCAGCACCTGTGACCTC  6330
            ||||||||||||||||||||||||||||||||||||||||||||||||||||||||||||
XM  6520    AGCATCACCCGGCGCAGGCAGGCACCTTCCATCGCCACCATTGGCAGCACCTGTGACCTC  6579

ENS 6331    AACCTCAAGATCCCAGGGAACTGGTTCCAGATGGTGTCTGCCCAGGAGCGCCTGACACGC  6390
            ||||||||||||||||||||||||||||||||||||||||||||||||||||||||||||
XM  6580    AACCTCAAGATCCCAGGGAACTGGTTCCAGATGGTGTCTGCCCAGGAGCGCCTGACACGC  6639

ENS 6391    ACCTTCACGCGCAGCAGCCACACGTACACCCGCACAGAGCGCACGGAGATCAGCAAGACC  6450
            ||||||||||||||||||||||||||||||||||||||||||||||||||||||||||||
XM  6640    ACCTTCACGCGCAGCAGCCACACGTACACCCGCACAGAGCGCACGGAGATCAGCAAGACC  6699

ENS 6451    CGGGGCGGAGAGACCAAGCGTGAGGTGCGGGTGGAGGAGTCCACCCAGGTTGGCGGAGAC  6510
            ||||||||||||||||||||||||||||||||||||||||||||||||||||||||||||
XM  6700    CGGGGCGGAGAGACCAAGCGTGAGGTGCGGGTGGAGGAGTCCACCCAGGTTGGCGGAGAC  6759

ENS 6511    CCCTTCCCCGCGGTCTTCGGGGACTTCCTGGGCCGCGAACGCCTGGGCTCCTTTGGCAGC  6570
            ||||||||||||||||||||||||||||||||||||||||||||||||||||||||||||
XM  6760    CCCTTCCCCGCGGTCTTCGGGGACTTCCTGGGCCGCGAACGCCTGGGCTCCTTTGGCAGC  6819

ENS 6571    ATCACTCGGCAGCAGGAGGGTGAGGCCAGCTCTCAAGACATGACCGCACAGGTGACCAGC  6630
            ||||||||||||||||||||||||||||||||||||||||||||||||||||||||||||
XM  6820    ATCACTCGGCAGCAGGAGGGTGAGGCCAGCTCTCAAGACATGACCGCACAGGTGACCAGC  6879

ENS 6631    CCATCGGGCAAGACAGAAGCCGCAGAGATCGTCGAGGGGGAGGACAGTGCATACAGCGTG  6690
            ||||||||||||||||||||||||||||||||||||||||||||||||||||||||||||
XM  6880    CCATCGGGCAAGACAGAAGCCGCAGAGATCGTCGAGGGGGAGGACAGTGCATACAGCGTG  6939

ENS 6691    CGCTTTGTGCCCCAGGAGATGGGGCCCCATACTGTCACTGTCAAGTACCGTGGCCAGCAC  6750
            ||||||||||||||||||||||||||||||||||||||||||||||||||||||||||||
XM  6940    CGCTTTGTGCCCCAGGAGATGGGGCCCCATACTGTCACTGTCAAGTACCGTGGCCAGCAC  6999

ENS 6751    GTGCCCGGCAGCCCCTTTCAGTTCACTGTGGGGCCACTGGGTGAAGGTGGTGCCCACAAG  6810
            ||||||||||||||||||||||||||||||||||||||||||||||||||||||||||||
XM  7000    GTGCCCGGCAGCCCCTTTCAGTTCACTGTGGGGCCACTGGGTGAAGGTGGTGCCCACAAG  7059

ENS 6811    GTGCGGGCTGGAGGCACAGGGCTGGAGCGAGGTGTGGCTGGCGTGCCAGCTGAGTTCAGC  6870
            ||||||||||||||||||||||||||||||||||||||||||||||||||||||||||||
XM  7060    GTGCGGGCTGGAGGCACAGGGCTGGAGCGAGGTGTGGCTGGCGTGCCAGCTGAGTTCAGC  7119
```

```
ENS 6871    ATCTGGACCCGAGAAGCAGGTGCCGGGGGCCTGTCGATTGCTGTGGAGGGTCCCAGCAAG  6930
            ||||||||||||||||||||||||||||||||||||||||||||||||||||||||||||
XM  7120    ATCTGGACCCGAGAAGCAGGTGCCGGGGGCCTGTCGATTGCTGTGGAGGGTCCCAGCAAG  7179

ENS 6931    GCAGAGATTGCATTTGAGGACCGCAAAGACGGCTCCTGTGGGGTCTCCTATGTTGTCCAG  6990
            ||||||||||||||||||||||||||||||||||||||||||||||||||||||||||||
XM  7180    GCAGAGATTGCATTTGAGGACCGCAAAGACGGCTCCTGTGGGGTCTCCTATGTTGTCCAG  7239

ENS 6991    GAACCAGGTGACTATGAGGTCTCCATCAAGTTCAATGATGAGCACATCCCAGACAGCCCC  7050
            ||||||||||||||||||||||||||||||||||||||||||||||||||||||||||||
XM  7240    GAACCAGGTGACTATGAGGTCTCCATCAAGTTCAATGATGAGCACATCCCAGACAGCCCC  7299

ENS 7051    TTTGTGGTGCCTGTGGCCTCCCTCTCAGACGATGCTCGCCGCCTCACTGTCACCAGCCTC  7110
            ||||||||||||||||||||||||||||||||||||||||||||||||||||||||||||
XM  7300    TTTGTGGTGCCTGTGGCCTCCCTCTCAGACGATGCTCGCCGCCTCACTGTCACCAGCCTC  7359

ENS 7111    CAGGAGACGGGGCTCAAGGTGAACCAGCCAGCGTCCTTTGCGGTGCAGCTGAATGGTGCG  7170
            ||||||||||||||||||||||||||||||||||||||||||||||||||||||||||||
XM  7360    CAGGAGACGGGGCTCAAGGTGAACCAGCCAGCGTCCTTTGCGGTGCAGCTGAATGGTGCG  7419

ENS 7171    CGGGGCGTGATCGATGCTAGGGTGCACACGCCCTCGGGTGCGGTGGAGGAGTGCTACGTC  7230
            ||||||||||||||||||||||||||||||||||||||||||||||||||||||||||||
XM  7420    CGGGGCGTGATCGATGCTAGGGTGCACACGCCCTCGGGTGCGGTGGAGGAGTGCTACGTC  7479

ENS 7231    TCCGAGCTGGACAGTGACAAGCACACCATCCGCTTCATCCCCCACGAGAATGGCGTCCAC  7290
            ||||||||||||||||||||||||||||||||||||||||||||||||||||||||||||
XM  7480    TCCGAGCTGGACAGTGACAAGCACACCATCCGCTTCATCCCCCACGAGAATGGCGTCCAC  7539

ENS 7291    TCCATCGATGTCAAGTTCAACGGTGCCCACATCCCTGGCAGTCCCTTCAAGATCCGTGTT  7350
            ||||||||||||||||||||||||||||||||||||||||||||||||||||||||||||
XM  7540    TCCATCGATGTCAAGTTCAACGGTGCCCACATCCCTGGCAGTCCCTTCAAGATCCGTGTT  7599

ENS 7351    GGGGAGCAGAGCCAAGCTGGGGACCCAGGCTTGGTGTCAGCCTATGGTCCCGGGCTGGAG  7410
            ||||||||||||||||||||||||||||||||||||||||||||||||||||||||||||
XM  7600    GGGGAGCAGAGCCAAGCTGGGGACCCAGGCTTGGTGTCAGCCTATGGTCCCGGGCTGGAG  7659

ENS 7411    GGAGGCACTACAGGTGTATCATCAGAGTTCATTGTCAACACCCTGAACGCGGGCTCAGGG  7470
            ||||||||||||||||||||||||||||||||||||||||||||||||||||||||||||
XM  7660    GGAGGCACTACAGGTGTATCATCAGAGTTCATTGTCAACACCCTGAACGCGGGCTCAGGG  7719

ENS 7471    GCCTTGTCTGTCACCATCGATGGCCCCTCCAAGGTGCAGCTGGACTGTCGGGAATGTCCT  7530
            ||||||||||||||||||||||||||||||||||||||||||||||||||||||||||||
XM  7720    GCCTTGTCTGTCACCATCGATGGCCCCTCCAAGGTGCAGCTGGACTGTCGGGAATGTCCT  7779

ENS 7531    GAGGGCCACGTGGTCACTTACACTCCCATGGCCCCTGGCAACTACCTCATCGCCATCAAG  7590
            ||||||||||||||||||||||||||||||||||||||||||||||||||||||||||||
XM  7780    GAGGGCCACGTGGTCACTTACACTCCCATGGCCCCTGGCAACTACCTCATCGCCATCAAG  7839

ENS 7591    TATGGTGGCCCGCAGCACATCGTGGGCAGCCCCTTCAAGGCCAAAGTCACAGGTCCCCGG  7650
            ||||||||||||||||||||||||||||||||||||||||||||||||||||||||||||
XM  7840    TATGGTGGCCCGCAGCACATCGTGGGCAGCCCCTTCAAGGCCAAAGTCACAGGTCCCCGG  7899

ENS 7651    CTATCGGGAGGCCACAGCCTTCACGAAACATCCACGGTTCTGGTGGAGACTGTGACCAAG  7710
            ||||||||||||||||||||||||||||||||||||||||||||||||||||||||||||
XM  7900    CTATCGGGAGGCCACAGCCTTCACGAAACATCCACGGTTCTGGTGGAGACTGTGACCAAG  7959

ENS 7711    TCATCCTCAAGCCGGGGCTCCAGCTACAGCTCCATCCCCAAGTTCTCCTCGGATGCCAGC  7770
            ||||||||||||||||||||||||||||||||||||||||||||||||||||||||||||
XM  7960    TCATCCTCAAGCCGGGGCTCCAGCTACAGCTCCATCCCCAAGTTCTCCTCGGATGCCAGC  8019

ENS 7771    AAGGTGGTGACGCGGGGCCCCGGGCTGTCCCAGGCCTTTGTGGGCCAGAAGAACTCCTTC  7830
```

```
             ||||||||||||||||||||||||||||||||||||||||||||||||||||||||||||
XM  8020     AAGGTGGTGACGCGGGGCCCCGGGCTGTCCCAGGCCTTTGTGGGCCAGAAGAACTCCTTC  8079

ENS 7831     ACCGTGGACTGCAGCAAAGCAGGCA                                     7855
             |||||||||||||||||||||||||
XM  8080     ACCGTGGACTGCAGCAAAGCAGGCA                                     8104

ENS 7856     TGCTGAGGGGGAGAGGGGCAGCCTCCCAG                                 7884

XM           -----------------------------

ENS 7885     GGCACCAACATGATGATGGTGGGTGTGCACGGGCCCAAGACCCCCTGTGAGGAGGTGTAT  7944
             ||||||||||||||||||||||||||||||||||||||||||||||||||||||||||||
XM  8101     GGCACCAACATGATGATGGTGGGTGTGCACGGGCCCAAGACCCCCTGTGAGGAGGTGTAT  8160

ENS 7945     GTGAAGCACATGGGGAACCGGGTGTACAACGTCACCTACACCGTCAAGGAGAAAGGAGAC  8004
             ||||||||||||||||||||||||||||||||||||||||||||||||||||||||||||
XM  8161     GTGAAGCACATGGGGAACCGGGTGTACAACGTCACCTACACCGTCAAGGAGAAAGGAGAC  8220

ENS 8005     TACATCCTCATCGTCAAGTGGGGCGACGAAAGTGTCCCCGGAAGCCCCTTCAAAGTCAAT  8064
             ||||||||||||||||||||||||||||||||||||||||||||||||||||||||||||
XM  8221     TACATCCTCATCGTCAAGTGGGGCGACGAAAGTGTCCCCGGAAGCCCCTTCAAAGTCAAT  8280

ENS 8065     GTGCCCTGA  8073 (SEQ ID NO:6)
             |||||||||
XM  8281     GTGCCCTGA  8289 (SEQ ID NO:7)
```

FIG. 7

1 ATGATCCCCA AGGAGCAGAA GGGGCCCGTG ATGGCTGCCA TGGAGGACCT
51 CGCTGGACCA G**TCCCTGTGC TGGACCTGGG CAAGAAGCTG AGCGTGCCCC**
101 **AGGACCTGAT GATGGAAGAG CTGTCGCTCC GCAACAACCG GGGATCCCTC**
151 **CTCTTCCAGA AGAGGCAGCG CCGCGTGCAG AAATTCACCT TTGAGTTTGC**
201 **AGCCAGCCAG CGGGCG**ACCG TGGCCGGAAG CGCCAAGGGG AAGGTGCCTG
251 GAGCAGCAGA GCCCGGGACG GTCACCAACG GCCCCGAGGG GCAGAACTAC
301 CGCTCGGAGC TCCACATCTT CCCGGCCTCG CCCGGGGGCC CCGAGGACGC
351 GCAGCCCGCA GCCTCCGGGG CAAAGAGCGC CCGCAGCCCC AGCGCCCTGG
401 CGCCAGGCTA CGCGGAGCCC CTGAAGAGCG TCCCGCCCGA GAAGTTCAAC
451 CACACGGCCA TCCCCAAGGG CTACCGCTGC CCGTGGCAGG AGTTCATCAG
501 CTACCGGGAC TACCAGAGCG ACGGCCGAAG TCACACCCCT AGCCCGGCCG
551 AGTATCGGAA TTTCAACAAG ACCCCGGTGC CCTTTGGAGG ACCCCTGGTG
601 GGGGAGGCCG TGCCCAGGGC AGGCACCTCC TTCATCCCAG AGCTCACCAG
651 CGGCTTGGAA CTCCTCCGCC TGAGGCCCAG CTTCAACAGA GTGGCCCAGG
701 GCTGGGTCCG CAACCTCCCG GAGTCCGAGG ATCTGTAG (SEQ ID NO:8)

FIG. 8

| | | | | | | |
|---|---|---|---|---|---|---|
| 1 | MFNYERPKHF | IQSQNPCGSR | LQPPGPETSS | YSSQTKQSSI | IIQPRQCTEQ | |
| 51 | RFSASSTMSS | HITMSSSAFP | ASSQQLAGSN | PGQRVTATYN | QSPASFLSSI | |
| 101 | LPSQPDYSSS | KIPSTVDSNY | QQPSFGQPVN | AKPSQSANAK | PIPRTPDHEI | |
| 151 | QGSKEALIQD | LERKLKCKDS | LLHNGNQRLT | YEEKMARRLL | GPQNAAAVFQ | |
| 201 | AQNDSEAQDS | QQHNSEHARL | QVPTSQVR**SR** | **SSSRGDVNDQ** | **DAIQEKFYPP** | |
| 251 | **RFIQVPENMS** | **IDEGRFCRMD** | **FK**VSGLPAPD | VSWYLNGRPV | QSDDFHKMIV | |
| 301 | SEKGFHSLIF | EVVRASDAGA | YACVARNRAG | EATFTVQLDV | LAKEHRRAPM | |
| 351 | FIYKPQSKKV | FEGESVKLEC | QISAIPPPKL | FWKRNNEMVH | FNTDRISLYH | |
| 401 | DNSGRVTLLI | KDVNKKDAGW | YTVSAVNEAG | VTSCNTRLDV | TARPNQTLPA | |
| 451 | PKQLRVRPTF | SKYLALNGRG | LNVKQAFNPE | GEFQRLAAQS | GLYESEEL | (SEQ ID NO:9) |

| | | | | | | |
|---|---|---|---|---|---|---|
| 1 | MFNYERPKHF | IQSQNPCGSR | LQPPGPETSS | YSSQTKQSSI | IIQPRQCTEQ | |
| 51 | RFSASSTMSS | HITMSSSAFP | ASSQQLAGSN | PGQRVTATYN | QSPASFLSSI | |
| 101 | LPSQPDYSSS | KIPSTVDSNY | QQPSFGQPVN | AKPSQSANAK | PIPRTPDHEI | |
| 151 | QGSKEALIQD | LERKLKCKDS | LLHNGNQRLT | YEEKMARRLL | GPQNAAAVFQ | |
| 201 | AQNDSEAQDS | QQHNSEHARL | QVPTSQVR**SR** | **SPSRGDVNDQ** | **DAIQEKFYPP** | |
| 251 | **RFIQVPENMS** | **IDEGRFCRMD** | **FK**VSGLPAPD | VSWYLNGRPV | QSDDFHKMIV | |
| 301 | SEKGFHSLIF | EVVRASDAGA | YACVARNRAG | EATFTVQLDV | LAKEHRRAPM | |
| 351 | FIYKPQSKKV | FEGESVKLEC | QISAIPPPKL | FWKRNNEMVH | FNTDRISLYH | |
| 401 | DNSGRVTLLI | KDVNKKDAGW | YTVSAVNEAG | VTSCNTRLDV | TARPNQTLPA | |
| 451 | PKQLRVRPTF | SKYLALNGRG | LNVKQAFNPE | GEFQRLAAQS | GLYESEEL | (SEQ ID NO:10) |

FIG. 9

ENS 1 TFTRRCNEHLTCVVKRLTDLQRDLSDGLRLIALLEVLSQKRMYRKFHPRPNFRQMKLENV 60
XP 63 TFTRRCNEHLTCVVKRLTDLQRDLSDGLRLIALLEVLSQKRMYRKFHPRPNFRQMKLENV 122

ENS 61 SVALEFLEREHIKLVSIDSKAIVDGNLKLILGLIWTLILHYSISMPMWEDEDDEDARKQT 120
XP 123 SVALEFLEREHIKLVSIDSKAIVDGNLKLILGLIWTLILHYSISMPMWEDEDDEDARKQT 182

ENS 121 PKQRLLGWIQNKVPQLPITNFNRDWQDGKALGALVDNCAPGLCPDWEAWDPNQPVENARE 180
XP 183 PKQRLLGWIQNKVPQLPITNFNRDWQDGKALGALVDNCAPGLCPDWEAWDPNQPVENARE 242

ENS 181 AMQQADDWLGVPQVIAPEEIVDPNVDEHSVMTYLSQFPKAKLKPGAPVRSKQLNPKKAIA 240
XP 243 AMQQADDWLGVPQVIAPEEIVDPNVDEHSVMTYLSQFPKAKLKPGAPVRSKQLNPKKAIA 302

ENS 241 YGPGIEPQGNTVLQPAHFTVQTVDAGVGEVLVYIEDPEGHTEEAKVVPNNDKDRTYAVSY 300
XP 303 YGPGIEPQGNTVLQPAHFTVQTVDAGVGEVLVYIEDPEGHTEEAKVVPNNDKDRTYAVSY 362

ENS 301 VPKVAGLHKVTVLFAGQNIERSPFEVNVGMALGDANKVSARGPGLEPVGNVANKPTYFDI 360
XP 363 VPKVAGLHKVTVLFAGQNIERSPFEVNVGMALGDANKVSARGPGLEPVGNVANKPTYFDI 422

ENS 361 YTAGAGTGDVAVVIVDPQGRRDTVEVALEDKGDSTFRCTYRPVMEGPHTVHVAFAGAPIT 420
XP 423 YTAGAGTGDVAVVIVDPQGRRDTVEVALEDKGDSTFRCTYRPVMEGPHTVHVAFAGAPIT 482

ENS 421 RSPFPVHVAEA---------------------CNPNACRASGRGLQPKGVRVKEVADFKV 459
XP 483 RSPFPVHVAEEPLPPLAPSVPIVHQAKRVVPPCNPNACRASGRGLQPKGVRVKEVADFKV 542

ENS 460 FTKGAGSGELKVTVKGPKGTEELVKVREAGDGVFECEYYPVVPGKYVVTITWGGYAIPRS 519
XP 543 FTKGAGSGELKVTVKGPKGTEELVKVREAGDGVFECEYYPVVPGKYVVTITWGGYAIPRS 602

ENS 520 PFEVQVSPEAGAQKVRAWGPGLETGQVGKSADFVVEAIGTEVGTLGFSIEGPSQAKIECD 579
XP 603 PFEVQVSPEAGAQKVRAWGPGLETGQVGKSADFVVEAIGTEVGTLGFSIEGPSQAKIECD 662

ENS 580 DKGDGSCDVRYWPTEPGEYAVHVICDDEDIRDSPFIAHIQPAPPDCFPDKVKAFGPGLEP 639
XP 663 DKGDGSCDVRYWPTEPGEYAVHVICDDEDIRDSPFIAHIQPAPPDCFPDKVKAFGPGLEP 722

ENS 640 TGCIVDKPAEFTIDASAAGKGDLKLYAQDADGCPIDINVFTTGNGIFRCSYVPTKPIKHT 699
XP 723 TGCIVDKPAEFTIDASAAGKGDLKLYAQDADGCPIDINVFTTGNGIFRCSYVPTKPIKHT 782

ENS 700 IIISWGGVNVPKSPFR**VNVGEGSHPEKVKVYGPGVEKTGLKANEPTYFTVDCSEAGQ**GDV 759
XP 783 IIISWGGVNVPKSPFR**VNVGEGSHPEKVKVYGPGVEKTGLKANEPTYFTVDCSEAGQ**GDV 842

ENS 760 SIGIKCAPGVVGPAEADIDFDIIKNDNDTFTVKYTPPGAGRYTIMVLFANQEIPASPFHI 819
XP 843 SIGIKCAPGVVGPAEADIDFDIIKNDNDTFTVKYTPPGAGRYTIMVLFANQEIPASPFHI 902

ENS 820 KVDPSHDASKVKAEGPGLNRTGVEVGKPTHFTVLTKGAGKAKLDVHFAGAGKGEAVRDFE 879
XP 903 KVDPSHDASKVKAEGPGLNRTGVEVGKPTHFTVLTKGAGKAKLDVHFAGAGKGEAVRDFE 962

ENS 880 IIDNHDYSYTVKYTAVQQGNMAVTVTYGGDPVPKSPFVVNVAPPLDLSKVKVQGLNSKVA 939
XP 963 IIDNHDYSYTVKYTAVQQGNMAVTVTYGGDPVPKSPFVVNVAPPLDLSKVKVQGLNSKVA 1022

ENS 940 VGQEQAFSVNTRGAGGQGQLDVRMTSPSRRPIPCKLEPGGGAEAQAVRYMPPEEGPYKVD 999
XP 1023 VGQEQAFSVNTRGAGGQGQLDVRMTSPSRRPIPCKLEPGGGAEAQAVRYMPPEEGPYKVD 1082

ENS 1000 ITYDGHPVPGSPFTVEGVLPPDPSK**VCAYGPGLKGGLVGSPAPFSIDTKGAGTGGLGLTV** 1059
XP 1083 ITYDGHPVPGSPFTVEGVLPPDPSK**VCAYGPGLKGGLVGSPAPFSIDTKGAGTGGLGLTV** 1142

ENS 1060 **EGPCEAKIECQDNGDGSCAVSYLPTEPGEYTINILFAEAHIPGSPFKATIRPVFDPSKVR** 1119
XP 1143 **EGPCEAKIECQDNGDGSCAVSYLPTEPGEYTINILFAEAHIPGSPFKATIRPVFDPSKVR** 1202

ENS 1120 **ASGPGLERGKAGEAATFTVDCSEAGEAELTIEILSDAGVKAEVLIHNNADGTYHITYSPA** 1179
XP 1203 **ASGPGLERGKAGEAATFTVDCSEAGEAELTIEILSDAGVKAEVLIHNNADGTYHITYSPA** 1262

ENS 1180 **FPGTYTITIKYGGHPVPKFPTRVHVQPAIDTSGVKVSGPGVEPH**GVLREVTTEFTVDARS 1239
XP 1263 **FPGTYTITIKYGGHPVPKFPTRVHVQPAIDTSGVKVSGPGVEPH**GVLREVTTEFTVDARS 1322

ENS 1240 LTATGGNHVTARVLNPSGAKTDTYVTDNGDGTYRVQYTAYEEGVHLVEVLYDDVAVPKSP 1299
XP 1323 LTATGGNHVTARVLNPSGAKTDTYVTDNGDGTYRVQYTAYEEGVHLVEVLYDDVAVPKSP 1382

ENS 1300 FRVGVTEGCDPTRVRAYGPGLEGGLVNKANRFTVETRGAGTGGLGLAIEGPSEAKMSCKD 1359
XP 1383 FRVGVTEGCDPTRVRAYGPGLEGGLVNKANRFTVETRGAGTGGLGLAIEGPSEAKMSCKD 1442

ENS 1360 NKDGSCTVEYIPFTPGDYDVNITFGGRPIPGSPFRVPVKDVVDPGKVKCSGPGLGAGVRA 1419
XP 1443 NKDGSCTVEYIPFTPGDYDVNITFGGRPIPGSPFRVPVKDVVDPGKVKCSGPGLGAGVRA 1502

ENS 1420 RVPQTFTVDCSQAGRAPLQVAVLGPTGVAEPVEIRDNGDGTHAVHYTPATDGPYTVAVKY 1479
XP 1503 RVPQTFTVDCSQAGRAPLQVAVLGPTGVAEPVEIRDNGDGTHAVHYTPATDGPYTVAVKY 1562

ENS 1480 ADQEVPRSPFKIKVLPAHDASKVRASGPGLNAAGIPASLPVEFTIDARDAGEGLLTVQIL 1539
XP 1563 ADQEVPRSPFKIKVLPAHDASKVRASGPGLNAAGIPASLPVEFTIDARDAGEGLLTVQIL 1622

ENS 1540 DPEGKPKKANIRDNGDGTYTVSYLPDMSGRYTITIKYGGDEIPYSPFRIHALPTGDASKC 1599
XP 1623 DPEGKPKKANIRDNGDGTYTVSYLPDMSGRYTITIKYGGDEIPYSPFRIHALPTGDASKC 1682

ENS 1600 LVTVSIGGHGLGACLGPRIQIGEETVITVDAKAAGKGKVTCTVSTPDGAELDVDVVENHD 1659
XP 1683 LVTVSIGGHGLGACLGPRIQIGEETVITVDAKAAGKGKVTCTVSTPDGAELDVDVVENHD 1742

ENS 1660 GTFDIYYTAPEPGKYVITIRFGGEHIPNSPFHVLACDPMPHVEEPSDVLQLHRPSAYPTH 1719
XP 1743 GTFDIYYTAPEPGKYVITIRFGGEHIPNSPFHVLACDPMPHVEEPSDVLQLHRPSAYPTH 1802

ENS 1720 WATEEPVVPVEPMESMLRPFNLVIPFTVQKGELTGEVRMPSGKTARPNITDNKDGTITVR 1779
XP 1803 WATEEPVVPVEPMESMLRPFNLVIPFTVQKGELTGEVRMPSGKTARPNITDNKDGTITVR 1862

ENS 1780 YAPTEKGLHQMGIKYDGNHIPGDPLQFYVDAINSRHVSAYGPGLSHGMVNKPATFTIVTK 1839
XP 1863 YAPTEKGLHQMGIKYDGNHIPGSPLQFYVDAINSRHVSAYGPGLSHGMVNKPATFTIVTK 1922

ENS 1840 DAGEGGLSLAVEGPSKAEITCKDNKDGTCTVSYLPTAPGDYSIIVRFDDKHIPGSPFTAK 1899
XP 1923 DAGEGGLSLAVEGPSKAEITCKDNKDGTCTVSYLPTAPGDYSIIVRFDDKHIPGSPFTAK 1982

ENS 1900 ITGDDSMRTSQLNVGTSTDVSLKITESDLSLLTASIRAPSGNEEPCLLKRLPNRHIGISF 1959
XP 1983 ITGDDSMRTSQLNVGTSTDVSLKITESDLSLLTASIRAPSGNEEPCLLKRLPNRHIGISF 2042

ENS 1960 TPKEVGEHVVSVRKSGKHVTNSPFKILVGPSEIGDASKVRVWGKGLSEGQTFQVAEFIVD 2019
XP 2043 TPKEVGEHVVSVRKSGKHVTNSPFKILVGPSEIGDASKVRVWGKGLSEGQTFQVAEFIVD 2102

ENS 2020 TRNAGYGGLGLSIEGPSKVDINCEDMEDGTCKVTYCPTEPGTYIINIKFADKHVPGSPFT 2079
XP 2103 TRNAGYGGLGLSIEGPSKVDINCEDMEDGTCKVTYCPTEPGTYIINIKFADKHVPGSPFT 2162

ENS 2080 VKVTGEGRMKESITRRRQAPSIATIGSTCDLNLKIPGNWFQMVSAQERLTRTFTRSSHTY 2139
XP 2163 VKVTGEGRMKESITRRRQAPSIATIGSTCDLNLKIPGNWFQMVSAQERLTRTFTRSSHTY 2222

ENS 2140 TRTERTEISKTRGGETKREVRVEESTQVGGDPFPAVFGDFLGRERLGSFGSITRQQEGEA 2199
XP 2223 TRTERTEISKTRGGETKREVRVEESTQVGGDPFPAVFGDFLGRERLGSFGSITRQQEGEA 2282

ENS 2200 SSQDMTAQVTSPSGKTEAAEIVEGEDSAYSVRFVPQEMGPHTVTVKYRGQHVPGSPFQFT 2259
XP 2283 SSQDMTAQVTSPSGKTEAAEIVEGEDSAYSVRFVPQEMGPHTVTVKYRGQHVPGSPFQFT 2342

ENS 2260 VGPLGEGGAHKVRAGGTGLERGVAGVPAEFSIWTREAGAGGLSIAVEGPSKAEIAFEDRK 2319
XP 2343 VGPLGEGGAHKVRAGGTGLERGVAGVPAEFSIWTREAGAGGLSIAVEGPSKAEIAFEDRK 2402

ENS 2320 DGSCGVSYVVQEPGDYEVSIKFNDEHIPDSPFVVPVASLSDDARRLTVTSLQETGLKVNQ 2379

```
XP   2403   DGSCGVSYVVQEPGDYEVSIKFNDEHIPDSPFVVPVASLSDDARRLTVTSLQETGLKVNQ  2462

ENS  2380   PASFAVQLNGARGVIDARVHTPSGAVEECYVSELDSDKHTIRFIPHENGVHSIDVKFNGA  2439
XP   2463   PASFAVQLNGARGVIDARVHTPSGAVEECYVSELDSDKHTIRFIPHENGVHSIDVKFNGA  2522

ENS  2440   HIPGSPFKIRVGEQSQAGDPGLVSAYGPGLEGGTTGVSSEFIVNTLNAGSGALSVTIDGP  2499
XP   2523   HIPGSPFKIRVGEQSQAGDPGLVSAYGPGLEGGTTGVSSEFIVNTLNAGSGALSVTIDGP  2582

ENS  2500   SKVQLDCRECPEGHVVTYTPMAPGNYLIAIKYGGPQHIVGSPFKAKVTGPRLSGGHSLHE  2559
XP   2583   SKVQLDCRECPEGHVVTYTPMAPGNYLIAIKYGGPQHIVGSPFKAKVTGPRLSGGHSLHE  2642

ENS  2560   TSTVLVETVTKSSSSRGSSYSSIPKFSSDASKVVTRGPGLSQAFVGQKNSFTVDCSKAGM  2619
XP   2643   TSTVLVETVTKSSSSRGSSYSSIPKFSSDASKVVTRGPGLSQAFVGQKNSFTVDCSKA--  2700

ENS  2620   LRGRGAASQGTNMMMVGVHGPKTPCEEVYVKHMGNRVYNVTYTVKEKGDYILIVKWGDES  2679
XP   2701   ---------GTNMMMVGVHGPKTPCEEVYVKHMGNRVYNVTYTVKEKGDYILIVKWGDES  2751

ENS  2680   VPGSPFKVNVP  2690 (SEQ ID NO:11)
XP   2752   VPGSPFKVNVP  2762 (SEQ ID NO:12)
```

```
ENS     1   TFTRRCNEHLTCVVKRLTDLQRDLSDGLRLIALLEVLSQKRMYRKFHPRPNFRQMKLENV  60
XP     63   TFTRRCNEHLTCVVKRLTDLQRDLSDGLRLIALLEVLSQKRMYRKFHPRPNFRQMKLENV  122

ENS    61   SVALEFLEREHIKLVSIDSKAIVDGNLKLILGLIWTLILHYSISMPMWEDEDDEDARKQT  120
XP    123   SVALEFLEREHIKLVSIDSKAIVDGNLKLILGLIWTLILHYSISMPMWEDEDDEDARKQT  182

ENS   121   PKQRLLGWIQNKVPQLPITNFNRDWQDGKALGALVDNCAPGLCPDWEAWDPNQPVENARE  180
XP    183   PKQRLLGWIQNKVPQLPITNFNRDWQDGKALGALVDNCAPGLCPDWEAWDPNQPVENARE  242

ENS   181   AMQQADDWLGVPQVIAPEEIVDPNVDEHSVMTYLSQFPKAKLKPGAPVRSKQLNPKKAIA  240
XP    243   AMQQADDWLGVPQVIAPEEIVDPNVDEHSVMTYLSQFPKAKLKPGAPVRSKQLNPKKAIA  302

ENS   241   YGPGIEPQGNTVLQPAHFTVQTVDAGVGEVLVYIEDPEGHTEEAKVVPNNDKDRTYAVSY  300
XP    303   YGPGIEPQGNTVLQPAHFTVQTVDAGVGEVLVYIEDPEGHTEEAKVVPNNDKDRTYAVSY  362

ENS   301   VPKVAGLHKVTVLFAGQNIERSPFEVNVGMALGDANKVSARGPGLEPVGNVANKPTYFDI  360
XP    363   VPKVAGLHKVTVLFAGQNIERSPFEVNVGMALGDANKVSARGPGLEPVGNVANKPTYFDI  422

ENS   361   YTAGAGTGDVAVVIVDPQGRRDTVEVALEDKGDSTFRCTYRPVMEGPHTVHVAFAGAPIT  420
XP    423   YTAGAGTGDVAVVIVDPQGRRDTVEVALEDKGDSTFRCTYRPVMEGPHTVHVAFAGAPIT  482

ENS   421   RSPFPVHVAEA---------------------CNPNACRASGRGLQPKGVRVKEVADFKV  459
XP    483   RSPFPVHVAEEPLPPLAPSVPIVHQAKRVVPPCNPNACRASGRGLQPKGVRVKEVADFKV  542

ENS   460   FTKGAGSGELKVTVKGPKGTEELVKVREAGDGVFECEYYPVVPGKYVVTITWGGYAIPRS  519
XP    543   FTKGAGSGELKVTVKGPKGTEELVKVREAGDGVFECEYYPVVPGKYVVTITWGGYAIPRS  602

ENS   520   PFEVQVSPEAGAQKVRAWGPGLETGQVGKSADFVVEAIGTEVGTLGFSIEGPSQAKIECD  579
XP    603   PFEVQVSPEAGAQKVRAWGPGLETGQVGKSADFVVEAIGTEVGTLGFSIEGPSQAKIECD  662

ENS   580   DKGDGSCDVRYWPTEPGEYAVHVICDDEDIRDSPFIAHIQPAPPDCFPDKVKAFGPGLEP  639
XP    663   DKGDGSCDVRYWPTEPGEYAVHVICDDEDIRDSPFIAHIQPAPPDCFPDKVKAFGPGLEP  722

ENS   640   TGCIVDKPAEFTIDASAAGKGDLKLYAQDADGCPIDINVFTTGNGIFRCSYVPTKPIKHT  699
```

| | | | |
|---|---|---|---|
| XP | 723 | TGCIVDKPAEFTIDASAAGKGDLKLYAQDADGCPIDINVFTTGNGIFRCSYVPTKPIKHT | 782 |
| | | | |
| ENS | 700 | IIISWGGVNVPKSPFR**VNVGEGSHPEKVKVYGPGVEKTGLKANEPTYFTVDCSKAGQ**GDV | 759 |
| XP | 783 | IIISWGGVNVPKSPFR**VNVGEGSHPEKVKVYGPGVEKTGLKANEPTYFTVDCSKAGQ**GDV | 842 |
| | | | |
| ENS | 760 | SIGIKCAPGVVGPAEADIDFDIIKNDNDTFTVKYTPPGAGRYTIMVLFANQEIPASPFHI | 819 |
| XP | 843 | SIGIKCAPGVVGPAEADIDFDIIKNDNDTFTVKYTPPGAGRYTIMVLFANQEIPASPFHI | 902 |
| | | | |
| ENS | 820 | KVDPSHDASKVKAEGPGLNRTGVEVGKPTHFTVLTKGAGKAKLDVHFAGAGKGEAVRDFE | 879 |
| XP | 903 | KVDPSHDASKVKAEGPGLNRTGVEVGKPTHFTVLTKGAGKAKLDVHFAGAGKGEAVRDFE | 962 |
| | | | |
| ENS | 880 | IIDNHDYSYTVKYTAVQQGNMAVTVTYGGDPVPKSPFVVNVAPPLDLSKVKVQGLNSKVA | 939 |
| XP | 963 | IIDNHDYSYTVKYTAVQQGNMAVTVTYGGDPVPKSPFVVNVAPPLDLSKVKVQGLNSKVA | 1022 |
| | | | |
| ENS | 940 | VGQEQAFSVNTRGAGGQGQLDVRMTSPSRRPIPCKLEPGGGAEAQAVRYMPPEEGPYKVD | 999 |
| XP | 1023 | VGQEQAFSVNTRGAGGQGQLDVRMTSPSRRPIPCKLEPGGGAEAQAVRYMPPEEGPYKVD | 1082 |
| | | | |
| ENS | 1000 | ITYDGHPVPGSPFTVEGVLPPDPSK**VCAYGPGLKGGLVGSPAPFSIDTKGAGTGGLGLTV** | 1059 |
| XP | 1083 | ITYDGHPVPGSPFTVEGVLPPDPSK**VCAYGPGLKGGLVGSPAPFSIDTKGAGTGGLGLTV** | 1142 |
| | | | |
| ENS | 1060 | **EGPCEAKIECQDNGDGSCAVSYLPTEPGEYTINILFAEAHIPGSPFKATIRPVFDPSKVR** | 1119 |
| XP | 1143 | **EGPCEAKIECQDNGDGSCAVSYLPTEPGEYTINILFAEAHIPGSPFKATIRPVFDPSKVR** | 1202 |
| | | | |
| ENS | 1120 | **ASGPGLERGKAGEAATFTVDCSEAGEAELTIEILSDAGVKAEVLIHNNADGTYHITYSPA** | 1179 |
| XP | 1203 | **ASGPGLERGKAGEAATFTVDCSEAGEAELTIEILSDAGVKAEVLIHNNADGTYHITYSPA** | 1262 |
| | | | |
| ENS | 1180 | **FPGTYTITIKYGGHPVPKFPTRVHVQPTIDTSGVKVSGPGVEPH**GVLREVTTEFTVDARS | 1239 |
| XP | 1263 | **FPGTYTITIKYGGHPVPKFPTRVHVQPTIDTSGVKVSGPGVEPH**GVLREVTTEFTVDARS | 1322 |
| | | | |
| ENS | 1240 | LTATGGNHVTARVLNPSGAKTDTYVTDNGDGTYRVQYTAYEEGVHLVEVLYDDVAVPKSP | 1299 |
| XP | 1323 | LTATGGNHVTARVLNPSGAKTDTYVTDNGDGTYRVQYTAYEEGVHLVEVLYDDVAVPKSP | 1382 |
| | | | |
| ENS | 1300 | FRVGVTEGCDPTRVRAYGPGLEGGLVNKANRFTVETRGAGTGGLGLAIEGPSEAKMSCKD | 1359 |
| XP | 1383 | FRVGVTEGCDPTRVRAYGPGLEGGLVNKANRFTVETRGAGTGGLGLAIEGPSEAKMSCKD | 1442 |
| | | | |
| ENS | 1360 | NKDGSCTVEYIPFTPGDYDVNITFGGRPIPGSPFRVPVKDVVDPGKVKCSGPGLGAGVRA | 1419 |
| XP | 1443 | NKDGSCTVEYIPFTPGDYDVNITFGGRPIPGSPFRVPVKDVVDPGKVKCSGPGLGAGVRA | 1502 |
| | | | |
| ENS | 1420 | RVPQTFTVDCSQAGRAPLQVAVLGPTGVAEPVEIRDNGDGTHAVHYTPATDGPYTVAVKY | 1479 |
| XP | 1503 | RVPQTFTVDCSQAGRAPLQVAVLGPTGVAEPVEIRDNGDGTHAVHYTPATDGPYTVAVKY | 1562 |
| | | | |
| ENS | 1480 | ADQEVPRSPFKIKVLPAHDASKVRASGPGLNAAGIPASLPVEFTIDARDAGEGLLTVQIL | 1539 |
| XP | 1563 | ADQEVPRSPFKIKVLPAHDASKVRASGPGLNAAGIPASLPVEFTIDARDAGEGLLTVQIL | 1622 |
| | | | |
| ENS | 1540 | DPEGKPKKANIRDNGDGTYTVSYLPDMSGRYTITIKYGGDEIPYSPFRIHALPTGDASKC | 1599 |
| XP | 1623 | DPEGKPKKANIRDNGDGTYTVSYLPDMSGRYTITIKYGGDEIPYSPFRIHALPTGDASKC | 1682 |
| | | | |
| ENS | 1600 | LVTVSIGGHGLGACLGPRIQIGEETVITVDAKAAGKGKVTCTVSTPDGAELDVDVVENHD | 1659 |
| XP | 1683 | LVTVSIGGHGLGACLGPRIQIGEETVITVDAKAAGKGKVTCTVSTPDGAELDVDVVENHD | 1742 |
| | | | |
| ENS | 1660 | GTFDIYYTAPEPGKYVITIRFGGEHIPNSPFHVLACDPMPHVEEPSDVLQLHRPSAYPTH | 1719 |
| XP | 1743 | GTFDIYYTAPEPGKYVITIRFGGEHIPNSPFHVLACDPMPHVEEPSDVLQLHRPSAYPTH | 1802 |
| | | | |
| ENS | 1720 | WATEEPVVPVEPMESMLRPFNLVIPFTVQKGELTGEVRMPSGKTARPNITDNKDGTITVR | 1779 |
| XP | 1803 | WATEEPVVPVEPMESMLRPFNLVIPFTVQKGELTGEVRMPSGKTARPNITDNKDGTITVR | 1862 |
| | | | |
| ENS | 1780 | YAPTEKGLHQMGIKYDGNHIPGDPLQFYVDAINSRHVSAYGPGLSHGMVNKPATFTIVTK | 1839 |
| XP | 1863 | YAPTEKGLHQMGIKYDGNHIPGSPLQFYVDAINSRHVSAYGPGLSHGMVNKPATFTIVTK | 1922 |
| | | | |
| ENS | 1840 | DAGEGGLSLAVEGPSKAEITCKDNKDGTCTVSYLPTAPGDYSIIVRFDDKHIPGSPFTAK | 1899 |
| XP | 1923 | DAGEGGLSLAVEGPSKAEITCKDNKDGTCTVSYLPTAPGDYSIIVRFDDKHIPGSPFTAK | 1982 |

```
ENS  1900   ITGDDSMRTSQLNVGTSTDVSLKITESDLSLLTASIRAPSGNEEPCLLKRLPNRHIGISF  1959
XP   1983   ITGDDSMRTSQLNVGTSTDVSLKITESDLSLLTASIRAPSGNEEPCLLKRLPNRHIGISF  2042

ENS  1960   TPKEVGEHVVSVRKSGKHVTNSPFKILVGPSEIGDASKVRVWGKGLSEGQTFQVAEFIVD  2019
XP   2043   TPKEVGEHVVSVRKSGKHVTNSPFKILVGPSEIGDASKVRVWGKGLSEGQTFQVAEFIVD  2102

ENS  2020   TRNAGYGGLGLSIEGPSKVDINCEDMEDGTCKVTYCPTEPGTYIINIKFADKHVPGSPFT  2079
XP   2103   TRNAGYGGLGLSIEGPSKVDINCEDMEDGTCKVTYCPTEPGTYIINIKFADKHVPGSPFT  2162

ENS  2080   VKVTGEGRMKESITRRRQAPSIATIGSTCDLNLKIPGNWFQMVSAQERLTRTFTRSSHTY  2139
XP   2163   VKVTGEGRMKESITRRRQAPSIATIGSTCDLNLKIPGNWFQMVSAQERLTRTFTRSSHTY  2222

ENS  2140   TRTERTEISKTRGGETKREVRVEESTQVGGDPFPAVFGDFLGRERLGSFGSITRQQEGEA  2199
XP   2223   TRTERTEISKTRGGETKREVRVEESTQVGGDPFPAVFGDFLGRERLGSFGSITRQQEGEA  2282

ENS  2200   SSQDMTAQVTSPSGKTEAAEIVEGEDSAYSVRFVPQEMGPHTVTVKYRGQHVPGSPFQFT  2259
XP   2283   SSQDMTAQVTSPSGKTEAAEIVEGEDSAYSVRFVPQEMGPHTVTVKYRGQHVPGSPFQFT  2342

ENS  2260   VGPLGEGGAHKVRAGGTGLERGVAGVPAEFSIWTREAGAGGLSIAVEGPSKAEIAFEDRK  2319
XP   2343   VGPLGEGGAHKVRAGGTGLERGVAGVPAEFSIWTREAGAGGLSIAVEGPSKAEIAFEDRK  2402

ENS  2320   DGSCGVSYVVQEPGDYEVSIKFNDEHIPDSPFVVPVASLSDDARRLTVTSLQETGLKVNQ  2379
XP   2403   DGSCGVSYVVQEPGDYEVSIKFNDEHIPDSPFVVPVASLSDDARRLTVTSLQETGLKVNQ  2462

ENS  2380   PASFAVQLNGARGVIDARVHTPSGAVEECYVSELDSDKHTIRFIPHENGVHSIDVKFNGA  2439
XP   2463   PASFAVQLNGARGVIDARVHTPSGAVEECYVSELDSDKHTIRFIPHENGVHSIDVKFNGA  2522

ENS  2440   HIPGSPFKIRVGEQSQAGDPGLVSAYGPGLEGGTTGVSSEFIVNTLNAGSGALSVTIDGP  2499
XP   2523   HIPGSPFKIRVGEQSQAGDPGLVSAYGPGLEGGTTGVSSEFIVNTLNAGSGALSVTIDGP  2582

ENS  2500   SKVQLDCRECPEGHVVTYTPMAPGNYLIAIKYGGPQHIVGSPFKAKVTGPRLSGGHSLHE  2559
XP   2583   SKVQLDCRECPEGHVVTYTPMAPGNYLIAIKYGGPQHIVGSPFKAKVTGPRLSGGHSLHE  2642

ENS  2560   TSTVLVETVTKSSSSRGSSYSSIPKFSSDASKVVTRGPGLSQAFVGQKNSFTVDCSKAGM  2619
XP   2643   TSTVLVETVTKSSSSRGSSYSSIPKFSSDASKVVTRGPGLSQAFVGQKNSFTVDCSKA--  2700

ENS  2620   LRGRGAASQGTNMMMVGVHGPKTPCEEVYVKHMGNRVYNVTYTVKEKGDYILIVKWGDES  2679
XP   2701   ---------GTNMMMVGVHGPKTPCEEVYVKHMGNRVYNVTYTVKEKGDYILIVKWGDES  2751

ENS  2680   VPGSPFKVNVP  2690 (SEQ ID NO:13)
XP   2752   VPGSPFKVNVP  2762 (SEQ ID NO:14)
```

FIG. 10

1 MIPKEQKGPV MAAMEDLAGP V**PVLDLGKKL SVPQDLMMEE L<u>S</u>LRNNRGSL**
51 **LFQKRQRRVQ KFTFEFAASQ RA**TVAGSAKG KVPGAAEPGT VTNGPEGQNY
101 RSELHIFPAS PGGPEDAQPA ASGAKSARSP SALAPGYAEP LKSVPPEKFN
151 HTAIPKGYRC PWQEFISYRD YQSDGRSHTP SPAEYRNFNK TPVPFGGPLV
201 GEAVPRAGTS FIPELTSGLE LLRLRPSFNR VAQGWVRNLP ESEDL (SEQ ID NO:15)

1 MIPKEQKGPV MAAMEDLAGP V**PVLDLGKKL SVPQDLMMEE L<u>L</u>LRNNRGSL**
51 **LFQKRQRRVQ KFTFEFAASQ RA**TVAGSAKG KVPGAAEPGT VTNGPEGQNY
101 RSELHIFPAS PGGPEDAQPA ASGAKSARSP SALAPGYAEP LKSVPPEKFN
151 HTAIPKGYRC PWQEFISYRD YQSDGRSHTP SPAEYRNFNK TPVPFGGPLV
201 GEAVPRAGTS FIPELTSGLE LLRLRPSFNR VAQGWVRNLP ESEDL (SEQ ID NO:16)

FIG. 11

```
          5´-tatgacaat ggaaagggaa ttc-3´ (SEQ ID NO:19)
            CTATGACAAT GGAAACCCAA TTCTTATTAT ACAATCTTTG CACATGATAA GAATTGTCCA

            TGGGGTACTC TGCATTTTAA CAGATCAGCT TCTTCATTAA TTCGTTGCAC TGTAAAAAGG

            AAACAAAGAT GTATAATTTG GGCCAGATGT TTCTAAATTG CTACCATTTC TACCAGTCAT

            CAAATATACC ACAGCTAAAT ATAGCCTCTC TCCATTGTTA TTACACTATC AGATCAAACC

            CACTCTAGGG TTTTAAATTT GATGACTAAA CTATATCTGT GTGAAAGGAA GAGCAATGAT

            AATTTTCCGC ATGGTGATGA TTAAACTATT TTTGCAG

38,519,913  normal   A AGC AGA TCA TCT TCA AGG GGA GAT GTG AAT GAT CAG GAT GCA
38,519,913  S232P    A AGC AGA TCA CCT TCA AGG GGA GAT GTG AAT GAT CAG GAT GCA

            normal     ATC CAG GAG AAG TTT TAC CCA CCT CGT TTC ATT CAA GTG CCA
            S232P      ATC CAG GAG AAG TTT TAC CCA CCT CGT TTC ATT CAA GTG CCA

            normal     GAA AAC ATG TCA ATT GAC GAA GGA AGA TTC TGC AGA ATG GAC
            S232P      GAA AAC ATG TCA ATT GAC GAA GGA AGA TTC TGC AGA ATG GAC

            normal     TTC AAA AAC ATG TCA ATT GAC GAA GGA AGA TTC TGC AGA ATG
            S232P      TTC AAA AAC ATG TCA ATT GAC GAA GGA AGA TTC TGC AGA ATG

            normal     GAC TTC AAA  (SEQ ID NO:17)     38,519,061
            S232P      GAC TTC AAA  (SEQ ID NO:18)     38,519,061


            GTAAGAGAAG  AGTTCAAAAG  TACTGGAGGA  AAATTAACAA  TGTGATACTA  AGTTTTGAAA


            AATGTGCTCT  TCCTCTTCAT  AGCTTGCTCC  ACAGCTTGAG  AAGCAGCACG  TACAGTGGAG
                                 3´-gaacgagg  tgtcgaactc  tt-5´ (SEQ ID NO:20)
```

FIG. 12

```
            GGGGAAGAGG ATGGGCTTTG AGGGAGGGGC ACCATGCTGA GCATTGACCC CTTCCCACAG

                                     5´-ggc agt cac cct gag aaa gt-3´ (SEQ ID
NO:23)
83,736,133  normal   GTA AAC GTG GGA GAG GGC AGT CAC CCT GAG AAA GTG
83,736,133  E753K    GTA AAC GTG GGA GAG GGC AGT CAC CCT GAG AAA GTG

            normal   AAG GTG TAC GGC CCT GGC GTG GAG AAG ACG GGC CTC
            E753K    AAG GTG TAC GGC CCT GGC GTG GAG AAG ACG GGC CTC

            normal   AAG GCC AAC GAG CCC ACC TAT TTC ACC GTG GAC TGC
            E753K    AAG GCC AAC GAG CCC ACC TAT TTC ACC GTG GAC TGC

            normal   AGC GAG GCG GGG CAA G  (SEQ ID NO:21)              83,736,256
            E753K    AGC AAG GCG GGG CAA G  (SEQ ID NO:22)              83,736,256

          GTGCACCAGC CAGGCGGGGG AGTAGGAGAC GGGGCCTGGG GCGGGGTCTG GGTGGGTAGG

          GCTAGTACTC CTGGCAGAGC TGTTTCCTGG CAGAGCTCTA GTGGGACTGC TCAGGTGGGG

          CAGGTTGTTG AGCCCCCATC TCATGGCCTC CTGTCTGCCT ACCCTCAGGC GATGTGAGCA
                                                (SEQ ID NO:24) 3´-cactcgt

          TTGGCATCAA GTGCGCCCCC GGCGTGGTGG GCCCCGCAGA GGCTGACATT GACTTTGACA
          aaccgtagtt ca-5´
```

FIG. 13

```
           GAAGGCCAAG CACGGGTGAG GCCAAGTGTA GGGAACTCAC ATACCTGCTC TTCCCTCTAG

83,738,223 normal   GTT TGT GCT TAT GGC CCT GGT CTC AAG GGT GGG CTG
83,738,223 A1207T   GTT TGT GCT TAT GGC CCT GGT CTC AAG GGT GGG CTG

           normal   GTA GGC AGC CCA GCG CCG TTC TCC ATC GAC ACC AAG
           A1207T   GTA GGC AGC CCA GCG CCG TTC TCC ATC GAC ACC AAG

           normal   GGG GCT GGC ACC GGT GGC CTG GGG CTG ACT GTG GAG
           A1207T   GGG GCT GGC ACC GGT GGC CTG GGG CTG ACT GTG GAG

           normal   GGC CCC TGT GAG GCC AAG ATC GAG TGC CAG GAC AAT
           A1207T   GGC CCC TGT GAG GCC AAG ATC GAG TGC CAG GAC AAT

           normal   GGT GAT GGC TCA TGT GCG GTC AGC TAC CTG CCC ACG
           A1207T   GGT GAT GGC TCA TGT GCG GTC AGC TAC CTG CCC ACG

           normal   GAG CCG GGC GAG TAC ACC ATC AAC ATC CTG TTC GCC
           A1207T   GAG CCG GGC GAG TAC ACC ATC AAC ATC CTG TTC GCC

           normal   GAA GCC CAC ATC CCC GGC TCA CCC TTC AAG GCT ACC
           A1207T   GAA GCC CAC ATC CCC GGC TCA CCC TTC AAG GCT ACC

           normal   ATC CGG CCC GTG TTC GAC CCG AGC AAG GTG CGG GCC
           A1207T   ATC CGG CCC GTG TTC GAC CCG AGC AAG GTG CGG GCC

           normal   AGT GGG CCA GGC CTG GAG CGT GGC AAG GCT GGT GAG
           A1207T   AGT GGG CCA GGC CTG GAG CGT GGC AAG GCT GGT GAG

           normal   GCA GCC ACC TTC ACT GTG GAC TGC TCG GAG GCG GGC
           A1207T   GCA GCC ACC TTC ACT GTG GAC TGC TCG GAG GCG GGC

           normal   GAG GCT GAG CTG ACC ATC GAG ATC CTG TCA GAC GCT
           A1207T   GAG GCT GAG CTG ACC ATC GAG ATC CTG TCA GAC GCT

                                      5´-g gtg ctg atc cac aac aat g-3´ (SEQ ID
NO:27)
           normal   GGC GTC AAG GCC GAG GTG CTG ATC CAC AAC AAT GCT
           A1207T   GGC GTC AAG GCC GAG GTG CTG ATC CAC AAC AAT GCT

           normal   GAC GGC ACC TAC CAC ATC ACC TAC AGC CCC GCC TTC
           A1207T   GAC GGC ACC TAC CAC ATC ACC TAC AGC CCC GCC TTC

           normal   CCC GGC ACC TAC ACT ATT ACC ATC AAG TAC GGT GGG
           A1207T   CCC GGC ACC TAC ACT ATT ACC ATC AAG TAC GGT GGG

           normal   CAC CCC GTA CCC AAA TTC CCC ACC CGC GTC CAT GTG
           A1207T   CAC CCC GTA CCC AAA TTC CCC ACC CGC GTC CAT GTG

           normal   CAG CCC GCT ATC GAC ACC AGT GGA GTC AAG GTC TCG
           A1207T   CAG CCC ACT ATC GAC ACC AGT GGA GTC AAG GTC TCG

           normal   GGG CCT GGT GTG GAG CCG CAC G  (SEQ ID NO:25)       83,738,820
           A1207T   GGG CCT GGT GTG GAG CCG CAC G  (SEQ ID NO:26)       83,738,820

           GTGAGTGAGA GAGGAGCCAG GAGCCCGTCA GGGAGCCAGG GGAGGCAGCA GAAGGGACGG

           AAGCAAGCCT GAGTGCTTAG CAGAGCACCA TAGTCTGAAG GGAGGACTTG GGGACAGCCC
```

(SEQ ID NO:28) 3´-catacttc cctcctgaac ccc-5´

FIG. 14

```
                  5'-caggtttctc acacacaatg g-3'  (SEQ ID NO:31)
                 GGGGAGAAAC CAGGTTTCTC ACACACAATG GACAGTGTGC TGAGGAGGCC TCCTGAGAGG
                 CGGCAGCCTA GGAAGCCTGC GGGTTTGGTA GTAAGAGCTG CCTCTGGCCT CTCCTGGCAG

27,399,285  normal   TC CCT GTG CTG GAC CTG GGC AAG AAG CTG AGC GTG CCC
27,399,285  S42L     TC CCT GTG CTG GAC CTG GGC AAG AAG CTG AGC GTG CCC

            normal      CAG GAC CTG ATG ATG GAA GAG CTG TCG CTC CGC AAC
            S42L        CAG GAC CTG ATG ATG GAA GAG CTG TTG CTC CGC AAC

            normal      AAC CGG GGA TCC CTC CTC TTC CAG AAG AGG CAG CGC
            S42L        AAC CGG GGA TCC CTC CTC TTC CAG AAG AGG CAG CGC

            normal      CGC GTG CAG AAA TTC ACC TTT GAG TTT GCA GCC AGC
            S42L        CGC GTG CAG AAA TTC ACC TTT GAG TTT GCA GCC AGC

            normal      CAG CGG GCG  (SEQ ID NO:29)     27,399,131
            S42L        CAG CGG GCG  (SEQ ID NO:30)     27,399,131

            GTGAGTAAGC CCCCACTGTG CTCACAGGGA AACTGAGGCC CAGAGAGAGC CAGTGGTTAG

            TCTAAAGCCC AACAGTGAGC CAGGGGAGGA CCTCTGGCCC CTGGGGAGTC CCTCAAGCTC

            CAGCTGGGGA AGACTGTGAT GTTCCCATCG GACTGAGCCC CGCCCTGCCG GGTGATCCTA

            GAAAAGGGTT ACCTCTTTAG GCGTCTCCCC AGATGTGAAA CATGAAGTGG CATGGGCAGG

            AGGAGCTGTG GAGTGAAGGA CTCTGGCTCC TATAAAAAGG GCTGAAGCAT AGTCATGTGC

            TCTGGGCTGA ATTTACAGCA AGCCGGATTT AGGTTAGACT TGAGCTTGAT CTAAGGTGGA
                                                (SEQ ID NO:32)    3'-cacct

            AAATGCAGAA TGCCTTTTTC TCTTCTTCCC ACTGGAAGAA GGAAAAGGCC ACGGCACAGT
            tttacgtctt acgga-5'
```

FIG. 15

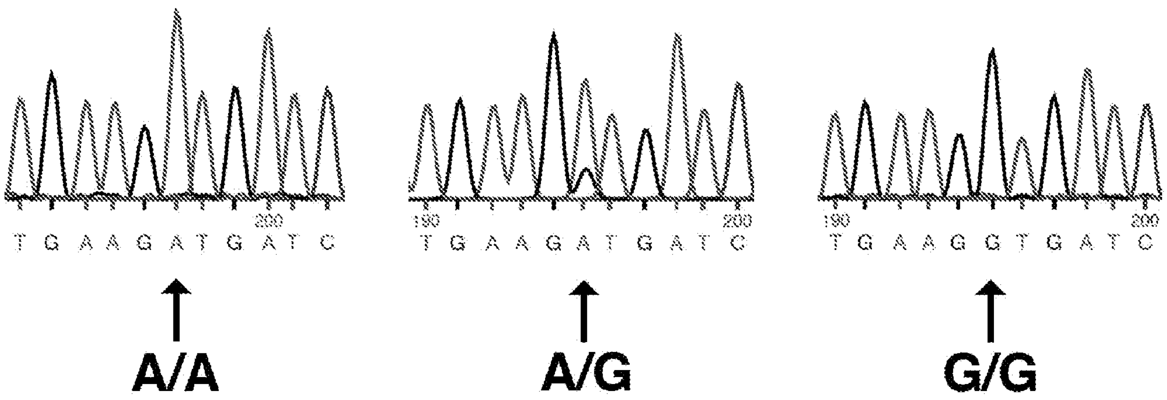
200
T G A A G A T G A T C
A/A
190 200
T G A A G A T G A T C
A/G
190 200
T G A A G G T G A T C
G/G

FIG. 16

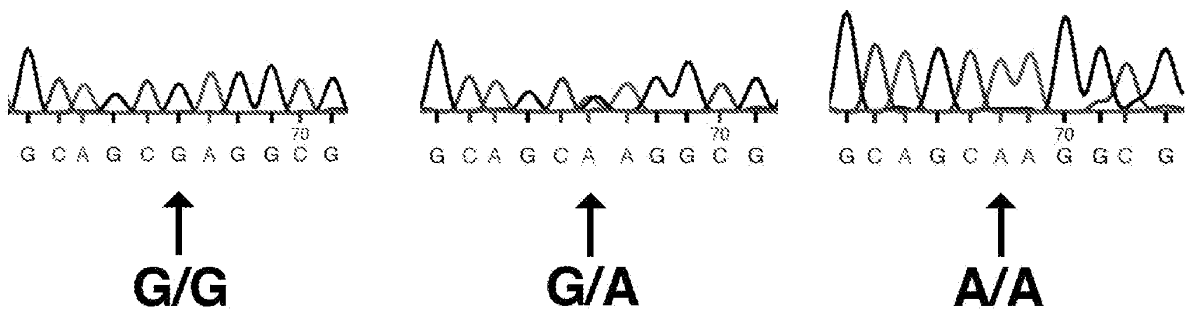
70
G C A G C G A G G C G
G/G
70
G C A G C A A G G C G
G/A
70
G C A G C A A G G C G
A/A

FIG. 17

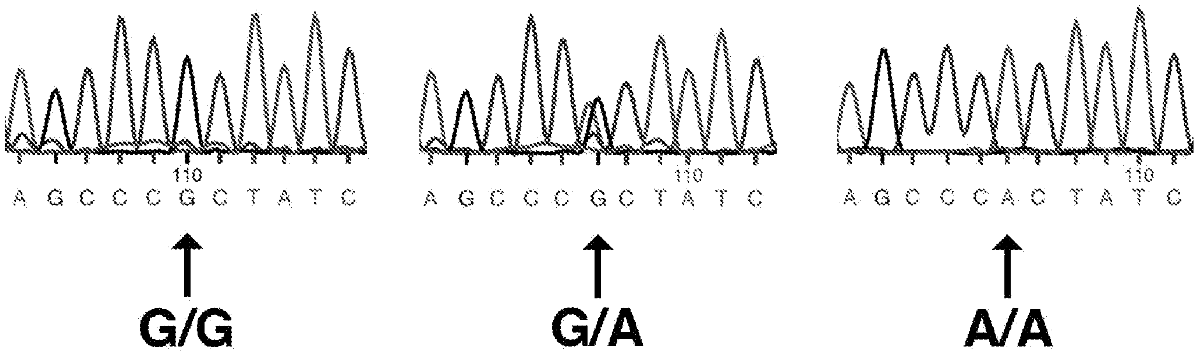
110
A G C C C G C T A T C
G/G
110
A G C C C G C T A T C
G/A
110
A G C C C A C T A T C
A/A

FIG. 18

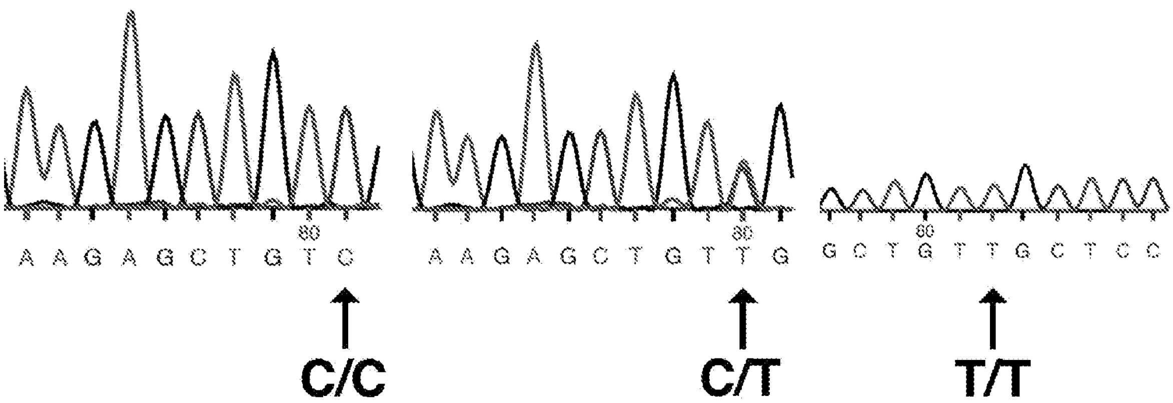
80
A A G A G C T G T C
C/C
80
A A G A G C T G T T G
C/T
80
G C T G T T G C T C C
T/T

FIG. 19

```
                                                  5’- g cacatgataa gaattgtcca
               CTATGACAAT GGAAACCCAA TTCTTATTAT ACAATCTTTG CACATGATAA GAATTGTCCA

               tggggtactc tgca-3’ (SEQ ID NO:33)
               TGGGGTACTC TGCATTTTAA CAGATCAGCT TCTTCATTAA TTCGTTGCAC TGTAAAAAGG

               AAACAAAGAT GTATAATTTG GGCCAGATGT TTCTAAATTG CTACCATTTC TACCAGTCAT

               CAAATATACC ACAGCTAAAT ATAGCCTCTC TCCATTGTTA TTACACTATC AGATCAAACC

               CACTCTAGGG TTTTAAATTT GATGACTAAA CTATATCTGT GTGAAAGGAA GAGCAATGAT

               AATTTTCCGC ATGGTGATGA TTAAACTATT TTTGCAG

38,519,913     normal    A AGC AGA TCA TCT TCA AGG GGA GAT GTG AAT GAT CAG GAT GCA ATC
                    (SEQ ID NO:34)  3’-agc agt tcc cct cta cac tta cta gtc cta cgt t-
5’

38,519,913     S232P     A AGC AGA TCA CCT TCA AGG GGA GAT GTG AAT GAT CAG GAT GCA ATC
                    (SEQ ID NO:35)  3’-ggc agt tcc cct cta cac tta cta gtc cta cgt t-
5’

               normal     CAG GAG AAG TTT TAC CCA CCT CGT TTC ATT CAA GTG CCA
               S232P      CAG GAG AAG TTT TAC CCA CCT CGT TTC ATT CAA GTG CCA

               normal     GAA AAC ATG TCA ATT GAC GAA GGA AGA TTC TGC AGA ATG
               S232P      GAA AAC ATG TCA ATT GAC GAA GGA AGA TTC TGC AGA ATG

               normal     GAC TTC AAA AAC ATG TCA ATT GAC GAA GGA AGA TTC TGC
               S232P      GAC TTC AAA AAC ATG TCA ATT GAC GAA GGA AGA TTC TGC

               normal     AGA ATG GAC TTC AAA  (SEQ ID NO:17)     38,519,061
               S232P      AGA ATG GAC TTC AAA  (SEQ ID NO:18)     38,519,061


               GTAAGAGAAG  AGTTCAAAAG  TACTGGAGGA  AAATTAACAA  TGTGATACTA  AGTTTTGAAA


               AATGTGCTCT  TCCTCTTCAT  AGCTTGCTCC  ACAGCTTGAG  AAGCAGCACG  TACAGTGGAG
```

FIG. 20

```
                  (SEQ ID NO:36) 5'- tgtcgctgg gccctggtca ctgctc-3'
           GAGGGTGAAG CCCTATGCAG GAGATGTCGG CTGTCGCTGG GCCCTGGTCA CTGCTCCCCA

           CCTCAAGGCC CCCACGAAGG AGCTAAGATT GATCCCATTA AGGCTGAGGC GCCTAGTGCT

           GGGGAAGAGG ATGGGCTTTG AGGGAGGGGC ACCATGCTGA GCATTGACCC CTTCCCACAG


83,736,133  normal   GTA AAC GTG GGA GAG GGC AGT CAC CCT GAG AAA GTG
83,736,133  E753K    GTA AAC GTG GGA GAG GGC AGT CAC CCT GAG AAA GTG

            normal   AAG GTG TAC GGC CCT GGC GTG GAG AAG ACG GGC CTC
            E753K    AAG GTG TAC GGC CCT GGC GTG GAG AAG ACG GGC CTC

            normal   AAG GCC AAC GAG CCC ACC TAT TTC ACC GTG GAC TGC
            E753K    AAG GCC AAC GAG CCC ACC TAT TTC ACC GTG GAC TGC

            normal   AGC GAG GCG GGG CAA G  (SEQ ID NO:21)          83,736,256
        (SEQ ID NO:37) 3'-ctg cgc ccc gtt c

            E753K    AGC AAG GCG GGG CAA G  (SEQ ID NO:22)          83,736,256
        (SEQ ID NO:38) 3'-ttg cgc ccc gtt c


         GTGCACCAGC CAGGCGGGGG AGTAGGAGAC GGGGCCTGGG GCGGGGTCTG GGTGGGTAGG
         cacgtggtcg g-5' (SEQ ID NO:37)
         cacgtggtcg g-5' (SEQ ID NO:38)

         GCTAGTACTC CTGGCAGAGC TGTTTCCTGG CAGAGCTCTA GTGGGACTGC TCAGGTGGGG
```

FIG. 21

```
             GAAGGCCAAG CACGGGTGAG GCCAAGTGTA GGGAACTCAC ATACCTGCTC TTCCCTCTAG

83,738,223  normal   GTT TGT GCT TAT GGC CCT GGT CTC AAG GGT
83,738,223  A1207T   GTT TGT GCT TAT GGC CCT GGT CTC AAG GGT

            normal   GGG CTG GTA GGC AGC CCA GCG CCG TTC TCC ATC GAC ACC AAG GGG
            A1207T   GGG CTG GTA GGC AGC CCA GCG CCG TTC TCC ATC GAC ACC AAG GGG

            normal   GCT GGC ACC GGT GGC CTG GGG CTG ACT GTG GAG GGC CCC TGT GAG
            A1207T   GCT GGC ACC GGT GGC CTG GGG CTG ACT GTG GAG GGC CCC TGT GAG

            normal   GCC AAG ATC GAG TGC CAG GAC AAT GGT GAT GGC TCA TGT GCG GTC
            A1207T   GCC AAG ATC GAG TGC CAG GAC AAT GGT GAT GGC TCA TGT GCG GTC

            normal   AGC TAC CTG CCC ACG GAG CCG GGC GAG TAC ACC ATC AAC ATC CTG
            A1207T   AGC TAC CTG CCC ACG GAG CCG GGC GAG TAC ACC ATC AAC ATC CTG

            normal   TTC GCC GAA GCC CAC ATC CCC GGC TCA CCC TTC AAG GCT ACC ATC
            A1207T   TTC GCC GAA GCC CAC ATC CCC GGC TCA CCC TTC AAG GCT ACC ATC

            normal   CGG CCC GTG TTC GAC CCG AGC AAG GTG CGG GCC AGT GGG CCA GGC
            A1207T   CGG CCC GTG TTC GAC CCG AGC AAG GTG CGG GCC AGT GGG CCA GGC

            normal   CTG GAG CGT GGC AAG GCT GGT GAG GCA GCC ACC TTC ACT GTG GAC
            A1207T   CTG GAG CGT GGC AAG GCT GGT GAG GCA GCC ACC TTC ACT GTG GAC

            normal   TGC TCG GAG GCG GGC GAG GCT GAG CTG ACC ATC GAG ATC CTG TCA
            A1207T   TGC TCG GAG GCG GGC GAG GCT GAG CTG ACC ATC GAG ATC CTG TCA

            normal   GAC GCT GGC GTC AAG GCC GAG GTG CTG ATC CAC AAC AAT GCT GAC
            A1207T   GAC GCT GGC GTC AAG GCC GAG GTG CTG ATC CAC AAC AAT GCT GAC

            normal   GGC ACC TAC CAC ATC ACC TAC AGC CCC GCC TTC CCC GGC ACC TAC
            A1207T   GGC ACC TAC CAC ATC ACC TAC AGC CCC GCC TTC CCC GGC ACC TAC

            normal   ACT ATT ACC ATC AAG TAC GGT GGG CAC CCC GTA CCC AAA TTC CCC
            A1207T   ACT ATT ACC ATC AAG TAC GGT GGG CAC CCC GTA CCC AAA TTC CCC

                 5’-acc cgc gtc cat gtg cag cgc g-3’ (SEQ ID NO:40)
            normal   ACC CGC GTC CAT GTG CAG CCC GCT ATC GAC ACC AGT GGA GTC AAG

                 5’-acc cgc gtc cat gtg cag cgc a-3’ (SEQ ID NO:41)
            A1207T   ACC CGC GTC CAT GTG CAG CCC ACT ATC GAC ACC AGT GGA GTC AAG

            normal   GTC TCG GGG CCT GGT GTG GAG CCG CAC G  (SEQ ID NO:25)83,738,820
            A1207T   GTC TCG GGG CCT GGT GTG GAG CCG CAC G  (SEQ ID NO:26)83,738,820

            GTGAGTGAGA GAGGAGCCAG GAGCCCGTCA GGGAGCCAGG GGAGGCAGCA GAAGGGACGG
AAGCAAGCCT

            GAGTGCTTAG CAGAGCACCA TAGTCTGAAG GGAGGACTTG GGGACAGCCC TGGCGTCCTT
AGGGCTCAGA
                        (SEQ ID NO:39)  3’-c cctcctgaac ccctgtcggg acc-5’
```

FIG. 22

```
            GGGGAGAAAC CAGGTTTCTC ACACACAATG GACAGTGTGC TGAGGAGGCC TCCTGAGAGG

            CGGCAGCCTA GGAAGCCTGC GGGTTTGGTA GTAAGAGCTG CCTCTGGCCT CTCCTGGCAG

27,399,285  normal   TC CCT GTG CTG GAC CTG GGC AAG AAG CTG AGC
27,399,285  S42L     TC CCT GTG CTG GAC CTG GGC AAG AAG CTG AGC

                 5'-g ccc cag gac ctg atg atg gaa gag ctc tc-3' (SEQ ID NO:43)
            normal   GTG CCC CAG GAC CTG ATG ATG GAA GAG CTG TCG CTC CGC AAC

                 5'-g ccc cag gac ctg atg atg gaa gag ctc tt-3' (SEQ ID NO:44)
            S42L     GTG CCC CAG GAC CTG ATG ATG GAA GAG CTG TTG CTC CGC AAC

            normal      AAC CGG GGA TCC CTC CTC TTC CAG AAG AGG CAG CGC
            S42L        AAC CGG GGA TCC CTC CTC TTC CAG AAG AGG CAG CGC

            normal      CGC GTG CAG AAA TTC ACC TTT GAG TTT GCA GCC AGC
            S42L        CGC GTG CAG AAA TTC ACC TTT GAG TTT GCA GCC AGC

            normal      CAG CGG GCG  (SEQ ID NO:29)     27,399,131
            S42L        CAG CGG GCG  (SEQ ID NO:30)     27,399,131

            GTGAGTAAGC CCCCACTGTG CTCACAGGGA AACTGAGGCC CAGAGAGAGC CAGTGGTTAG

            TCTAAAGCCC AACAGTGAGC CAGGGGAGGA CCTCTGGCCC CTGGGGAGTC CCTCAAGCTC
            (SEQ ID NO:42) 3'-tcg gtcccctcct ggagaccgg-5'

            CAGCTGGGGA AGACTGTGAT GTTCCCATCG GACTGAGCCC CGCCCTGCCG GGTGATCCTA
```

FIG. 23

```
Human 207    AQDSQQHNSEHARLQVPTSQVRSRSTSRGDVNDQDAIQEKFYPPRFIQVPE  257    (SEQ ID
NO:272)
             AQDSQQHNSEHARLQVPTSQVRSRS+SRGDVNDQDAIQEKFYPPRFIQVPE        (SEQ ID
NO:273)
Horse 207    AQDSQQHNSEHARLQVPTSQVRSRSSSRGDVNDQDAIQEKFYPPRFIQVPE  257    (SEQ ID
NO:274)
MYOT-S232P                             P
```

FIG. 24

```
Human 759    GEGSHPERVKVYGPGVEKTGLKANEPTYFTVDCSEAGQGDVSIGIKCAPGVVGPAEADID  818
             GEGSHPE+VKVYGPGVEKTGLKANEPTYFTVDCSEAGQGDVSIGIKCAPGVVGPAEADID
ENS   719    GEGSHPEKVKVYGPGVEKTGLKANEPTYFTVDCSEAGQGDVSIGIKCAPGVVGPAEADID  778
XP    802    GEGSHPEKVKVYGPGVEKTGLKANEPTYFTVDCSEAGQGDVSIGIKCAPGVVGPAEADID  861
FLNC-E753K                                     K

Human 819    FDIIKNDNDTFTVKYTPPGAGRYTIMVLFANQEIPASPFHIKV  861     (SEQ ID NO:275)
             FDIIKNDNDTFTVKYTPPGAGRYTIMVLFANQEIPASPFHIKV          (SEQ ID NO:276)
ENS   779    FDIIKNDNDTFTVKYTPPGAGRYTIMVLFANQEIPASPFHIKV  821     (SEQ ID NO:277)
XP    862    FDIIKNDNDTFTVKYTPPGAGRYTIMVLFANQEIPASPFHIKV  904     (SEQ ID NO:278)
FLNC-E753K
```

FIG. 25

```
Human  1245    QPAVDTSGVKVSGPGVEPHGVLREVTTEFTVDARSLTATGGNHVTARVLNPSGAKTDTYV  1304
               QPA+DTSGVKVSGPGVEPHGVLREVTTEFTVDARSLTATGGNHVTARVLNPSGAKTDTYV
ENS    1205    QPAIDTSGVKVSGPGVEPHGVLREVTTEFTVDARSLTATGGNHVTARVLNPSGAKTDTYV  1264
XP     1288    QPAIDTSGVKVSGPGVEPHGVLREVTTEFTVDARSLTATGGNHVTARVLNPSGAKTDTYV  1347
FLNC-A1207T      T

Human  1305    TDNGDGTYRVQYTAYEEGVHLVEVLYDEVAVPKSPFRVGV  1344 (SEQ ID NO:279)
               TDNGDGTYRVQYTAYEEGVHLVEVLYD+VAVPKSPFRVGV       (SEQ ID NO:280)
ENS    1265    TDNGDGTYRVQYTAYEEGVHLVEVLYDDVAVPKSPFRVGV  1304 (SEQ ID NO:281)
XP     1348    TDNGDGTYRVQYTAYEEGVHLVEVLYDDVAVPKSPFRVGV  1387 (SEQ ID NO:282)
FLNC-A1207T
```

FIG. 26

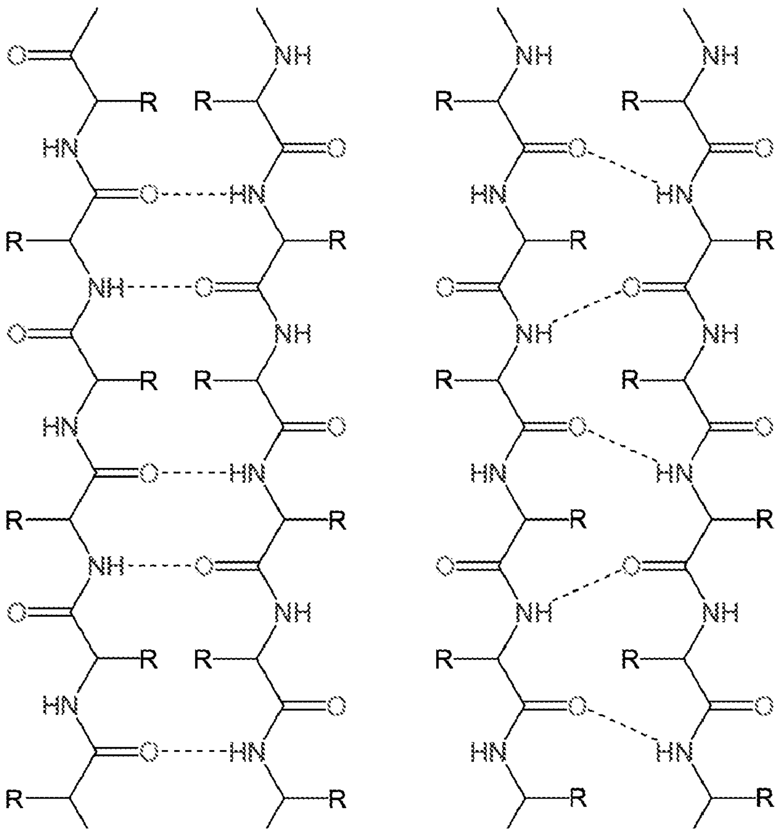

**antiparallel** **parallel**

FIG. 27

```
Human    1    MIPKEQKGPVMAAMGDLTEPVPTLDLGKKLSVPQDLMMEELSLRNNRGSLLFQKRQRRVQKFTFELAA
68
              MIPKEQKGPVMAAM DL  PVP LDLGKKLSVPQDLMMEELSLRNNRGSLLFQKRQRRVQKFTFE AA
Horse    1    MIPKEQKGPVMAAMEDLAGPVPVLDLGKKLSVPQDLMMEELSLRNNRGSLLFQKRQRRVQKFTFEFAA
68
MYOZ3-S42L                                             L
```

FIG. 28

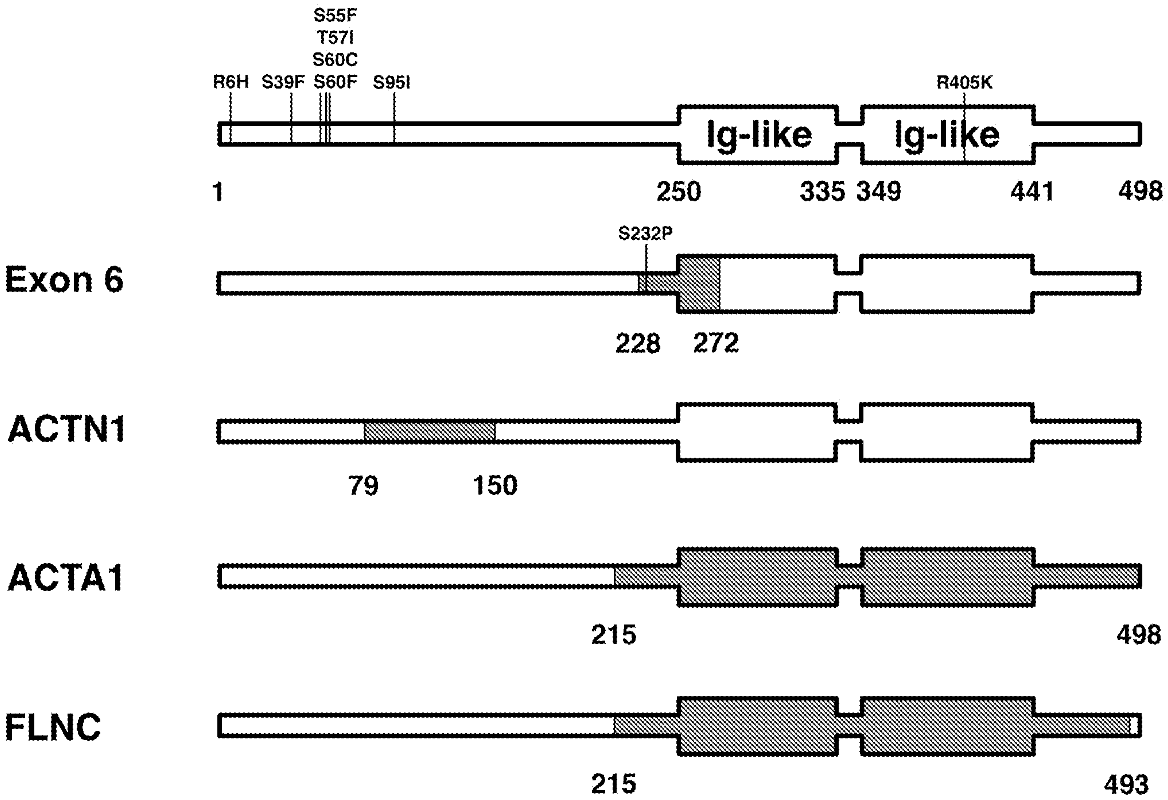
S55F
T57I
S60C
R6H
S39F
S60F
S95I
R405K
Ig-like
Ig-like
1
250
335
349
441
498
S232P
Exon 6
228
272
ACTN1
79
150
ACTA1
215
498
FLNC
215
493

FIG. 29

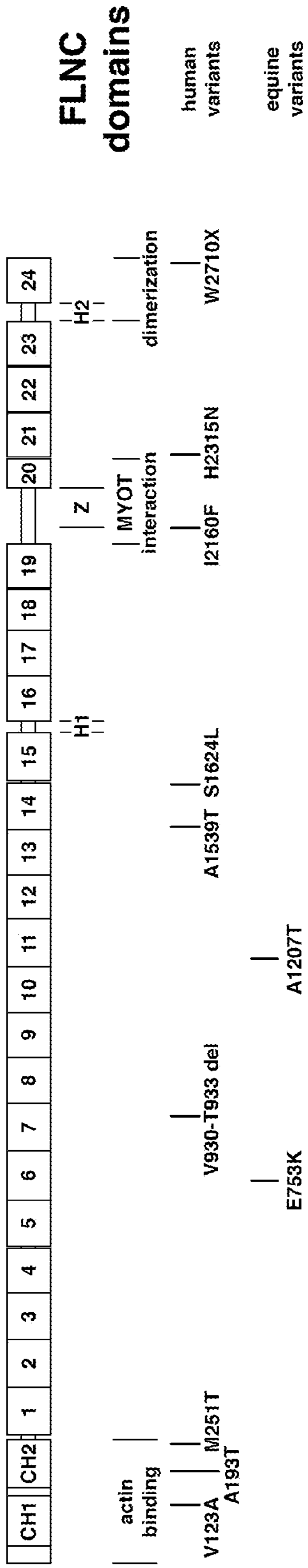
FLNC domains
CH1
CH2
1
2
3
4
5
6
7
8
9
10
11
12
13
14
15
16
17
18
19
20
21
22
23
24
H1
Z
H2
actin binding
MYOT interaction
dimerization
human variants
V123A
A193T
M251T
V930-T933 del
A1539T
S1624L
I2160F
H2315N
W2710X
equine variants
E753K
A1207T

FIG. 30

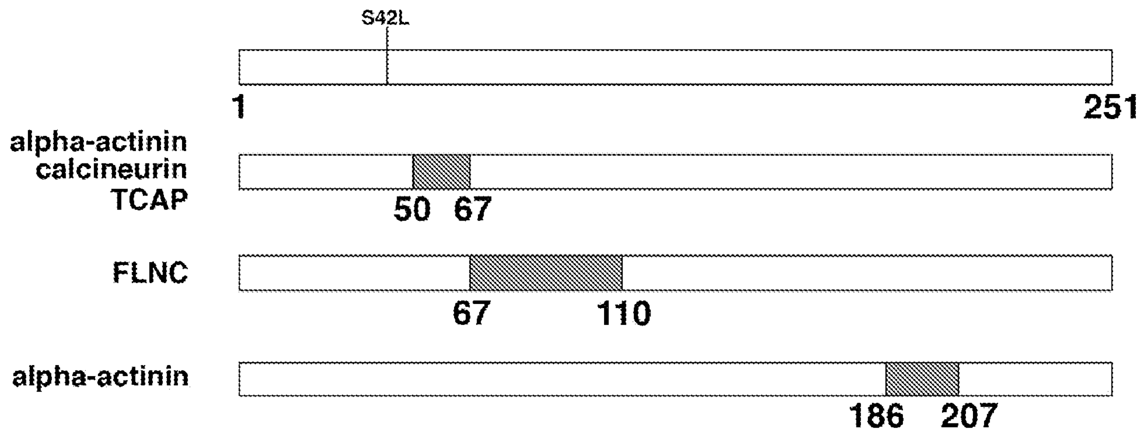
S42L
1
251
alpha-actinin
calcineurin
TCAP
50 67
FLNC
67
110
alpha-actinin
186 207

FIG. 31

```
primate1                   -GAQDSQQHNSEHARLQVPTSQVRSRSTSRGDVNDQDAIQEKFYPPRFIQVPEN
primate2                   -GAQDSQQHNSEHARLQVPTSQVRSRSSSRGDVNDQDAIQEKFYPPRFIQVPEN
mammal1                    --AQDSPQHNSEHARLQVPTSQVRSRSSSRGDVNDQDAIQEKFYPPRFIQVPEN
mammal2                    -AQDSAQQHNIEHARLQVPTSQVRSRSSSRGDVNDQDAIQEKFYPPRFIQVPEN
mammal3                    -AQDSPQQHHSEHARLQVPTSQVRSRSSSRGDVNDQDAIQEKFYPPRFIQVPEN
mammal4                    --AQDSPQHNSEHARLQVPTSQVRSRSSSRGGVNDEDAIQEKFYPPRFIQVPEN
mammal5                    -AQDPAQQHNSEHARLQLPTSQVRSRSSSRGDVNDQDAIQEKFYPPRFIQVPEN
mammal6                    --AQDSPQHNSEHARLQVPTSQVRSRSSSRGTLNEQDAIQEKFYPPRFIQVPEN
mammal7                    -AQDSPQQHNSDHVRLQVPTSQIRSRSSSRGDVNDQDAIQEKFYPPRFIQVPEN
mammal8                    -AQDSPQHHNPEHARLQVPTSQVRSRSSSRGDVNDQDAIQEKFYPPRFIQVPEN
Cebus_capucinus_imitator   ---QDSQQHNSEYARLQVPTSQVRSRSSSRGTVNDQDAIQEKFYPPRFIQVPEN
Chlorocebus_sabaeus        -GAQDSQQHNSEYARLQVPTSQVRSRSSSRGDANDQDAIQEKFYPPRFIQVPEN
Microcebus_murinus         -AQDLPQQHNSEHARLQVPTSQVRSRSSSRGDVNDQDAIQEKFYPPRFIQVPEN
Otolemur_garnettii         --AQDSPQHNSEHARLQVPTPQVRSRSSSRGDVNDQDAIQEKFYPPRFIQVPEN
Bubalus_bubalis            -AQDSAQQHNIEHARLQVPTSQVRSRSSSRGDMNDQDAIQEKFYPPRFIQVPEN
Rattus_norvegicus          --VQDSSQHNPEHARLQVPTSQVRSRSSSRADANDQDAIQEKFYPPRFIQVPEN
Heterocephalus_glaber      --AQDSPQHNAEHARLQVPTAQVRSRSSSRGDVNDQDAIQEKFYPPRFIQVPEN
Ovis_aries_musimon         --AQDSAQHNIEHARLQVPTSQVRSRSSSRGDMSDQDAIQEKFYPPRFIQVPEN
Castor_canadensis          --QDSPQQHNSEHAWLQVPTSQVRSRSSSRGDVNDQDAIQEKFYPPRFIQVPEN
Canis_lupus_familiaris     -AQDSPQQHNLEHARLQVPTSQVRSRSSSRGDVNDQDAIQEKFYPPRFIQVPEN
Miniopterus_natalensis     -AQD-SPQHNSAHARLQVPTSQIRSRSSSRGDVNDEDAIQEKFYPPRFIQVPEN
Pteropus_alecto            AQD-SPQHNSDHVRLQVPTSQIRSRSSSRGDVNDQDAIQEKFYPPRFIQVPEN
Physeter_catodon           -AQDPAQQHNSEHARLQVPTSQVRSRSSSRGDVNDQDAIQEKFYPPRFIQVPE-
T_manatus_latirostris      --AQDSPQHTSEHARLQVPTSQVRSRSSSRGDVNDQDAIQEKFYPPRFIQVPEN
Echinops_telfairi          --AQDSPQHNSEHARLHVPTAQVRSRSSSRGDVNDQDAIQEKFYPPRFIQVPEN
Erinaceus_europaeus        --VQESSQHNSEHARLQVPTPQIRSRSSSRGDVNDQDAIQEKFYPPRFIQVPEN
Dasypus_novemcinctus       --AQDSPKYNSEHARLQVPTSQVRSRSSSRGDANDQDAIQEKFYPPRFIQVPEN
Manis_javanica             --AQDSPQHNSENARLQVPTSQIRSRSSSRGDVNDQDAIQEKFYPPRFIQVPEH
Orycteropus_afer_afer      --AQDSLQHNSEHARLQVPTSQIRSRSSSRGGVNDQDAIQEKFYPPRFIQVPEN
Monodelphis_domestica      --SQDSAQHNSENARLQVPVPQIRSRSSSRGDTNDQESILEKFYPPRFVQVPEN
Sarcophilus_harrisii       --SQNSAQHNSENARLQVPTPQIRSRSSSRGDTNDQESILEKFYPPRFVQVPEN
Ornithorhynchus_anatinus   --AQDFSQPNSENVRLQVPSPQVRSRSSSRGAVNDQDSIQEKFYPPRFVQVPEN
bird1                      ---QNAQHQNAENIRLQVPTTHVRSRPSSRGDERGHDAIQEKFFQPRFTQVPED
bird2                      ---QNAQHQNAENVRLQVPTTHVRSRPSSRGDERGHDSIQEKFFQPRFTQVPED
bird3                      ---QNAQHQNAENIRLQVPTTHVRSRPSSRGDERGHDSIQEKFFQPRFTQVPED
bird4                      --VQNAQHQNAENIRLQVPTTHVRSRPSSRGDDRGHDSIQEKFFQPRFTQVPED
bird5                      ---QNAQHQNAENIRLQVPTTHVRSRPSSRGDDRGHDSIQEKFFQPRFTQVPED
Gallus_gallus              ---QNTQHQNAENVRLQVPTTHVRSRPSSRGDERGHDSIQEKFFQPRFTQVPED
Haliaeetus_albicilla       ---QNAQQQNAENIRLQVPTAHVRSRPSSRGDERGHDSIQEKFFQPRFTQVPED
Tinamus_guttatus           -GLQNAQQQNAENIRLQVPTTHVRSRPSSRGDEHGHDSIQEKFFQPRFTQVPED
Aptenodytes_forsteri       ---QNAQHQNAENIRLQVPTTHVRSRPSSRGDERGHDSIQEKFFQPRFTQVPED
Meleagris_gallopavo        --VQNAQHQNAENIRLQVPTTHVRSRPSSRGDERGHDSIQEKFFQPRFTQVPED
A_chrysaetos_canadensis    ---QNAQQQNAENIRLQVPTTHVRSRPSSRGDERGHDSIQEKFFQPRFTQVPED
Eurypyga_helias            ---QNAQEHNAENIRLQVPTTHVRSRPSSRGDERGHDSIQEKFFQPRFTQVPED
Colius_striatus            ---QNAQHHNAENIRLQVPTTHVRSRPSSRGDERGHDSIQEKFFQPRFTQVPED
Melopsittacus_undulatus    -------QHNAENIRLQVPTTHVRSRPSSRGDERGHDSIQEKFFQPRFTQVPED
Ficedula_albicollis        ---QSAQHQNSENIRLQVPTTHVRSRPSSRGDDRGHDSIQEKFFQPRFTQVPED
S_camelus_australis        ---QNAQHQNTENIRLQVPTTHVRSRPSSRGDERGHDSIQEKFFQPRFTQVPED
A_australis_mantelli       --AQNAQHQNAENIRLQVPTAHVRSRPSSRGDEHGHDSIQEKFFQPRFTQVPED
Merops_nubicus             ---QNAQHQNAEHVRLQVPTTHVRSRPSSRGDERGHDSIQEKFFPPRFTQVPED
Pelecanus_crispus          ---QNTQHQNAENVRLQVPTTHVRSRPSSRGDERGHDAIQEKFFQPRFTQVPED
Egretta_garzetta           ---QNAQHQNAENIRLQVPTTHVRSRPSSRGEERGQDSIQEKFFQPRFTQVPED
Tyto_alba                  ---QNAQHQNAENVRLQVPTTHIRSRPSSRGDERGHDSIQEKFFQPRFTQVPED
Falco_peregrinus           ---QNTQHQNAENIRLQVPTTHVRSRPSSRGDEHGHDSIQEKFFQPRFTQVPED
Nestor_notabilis           ---QNTQHQNAENIRLQVPTTHVRSRPSSRGDERGHDSIQEKFFQPRFTQVPED
Picoides_pubescens         ---QNAQHQNSENIRLQVPTTHVRSRPSSRGDERGHDSIQEKFFQPRFTQVPED
Opisthocomus_hoazin        ---QNAQHQNAENIRLQVPTTHVRSRPSSRGDERGQDSIQEKFFQPRFTQVPED
CLUSTAL                           .       *::*  ::*** :**.    .::* ***: *** ****
MYOT-S232P                 --AQDSPQHNSEHARLQVPTSQVRSRSPSRGDVNDQDAIQEKFYPPRFIQVPEN
```

FIG. 32

```
mammal1                         GEGSHPERVKVYGPGVEKTGLKANEPTYFTVDCSEAGQGDVSIGIKCAPGVVGPAEADIDFDIIKNDNDTFTVKYTPPGAGRYTIMVLFANQEIPASPFHIKV
mammal2                         GEGSHPEKVKVYGPGVEKTGLKANEPTYFTVDCSEAGQGDVSIGIKCAPGVVGPAEADIDFDIIKNDNDTFTVKYTPPGAGRYTIMVLFANQEIPASPFHIKV
mammal3                         GEGSHPERVKVYGPGVEKTGLKANEPTYFTVDCSEAGQGDVSIGIKCAPGVVGPAEADIDFDIIKNDNDTFTVKYTPPGAGHYTIMVLFANQEIPASPFHIKV
mammal4                         GEGSHPERVKVYGPGVEKTGLKANEPTYFTVDCSEAGQGDVSIGIKCAPGVVGPVEADIDFDIIKNDNDTFTVKYTPPGAGHYTIMVLFANQEIPASPFHIKV
mammal5                         GEGSHPERVKVYGPGVEKTGLKANEPTYFTVDCSEAGQGDVSIGIKCAPGVVGPAEADIDFDIIKNDNDTFTVKYTPPGAGQYTIMVLFANQEIPASPFHIKV
Carlito_syrichta                GEGSHPERVKVYGPGVEKTGLKANEPTYFTVDCSEAGQGDVSIGIKCAPGVVGPAEADIDFDIIKNDNDTFTVKYTPPGAGRYTIMVLFANQEIPASPFNIKV
Cricetulus_griseus              GEGSHPERVKVYGPGVEKTGLKANEPTYFTVDCSEAGQGDVSIGIKCGPGVVGPVEADIDFDIIKNDNDTFTVKYTPPGAGHYTIMVLFANQEIPASPFHIKV
Condylura_cristata              GEGSHPDKVKVYGPGVEKTGLKANEPTYFTVDCSEAGQGDVSIGIKCAPGVVGPAEADIDFDIIKNDNDTFTVKYTPPGAGHYTIMVLFANQEIPASPFHIKV
marsupial1                      GEGSHPEKVRVYGPGVEKTGLKANEPTYFTVDCSEAGQGDVSIGIKCAPGVVGPAEADIDFDIIKNDNDTFTVKYTPPGAGHYTIMVLFANQEIPASPFHIKV
bird1                           GEGSHPSRVKVHGPGVEKTGLKANEPTYFTVDCSEAGQGDVSIGIKCAPGVVGPLEADIDFDIIKNDNDTFTVKYTAPGAGLYTIMVLFANQEIPSSPFRIKV
bird2                           GEGSHPGRVKVYGPGVEKTGLKANEPTYFTVDCSEAGQGDVSIGIKCAPGVVGPLEADIDFDIIKNDNDTFTVKYTPPGAGLYTIMVLFANQEIPSSPFRIKV
bird3                           GEGSHPGRVKVYGPGVEKTGLKANEPTYFTVDCSEAGQGDVSIGIKCAPGVVGPVEADIDFDIIKNDNDTFTVKYTPPGAGLYTIMVLFANQEIPSSPFRIKV
Haliaeetus_leucocephalus        GEGSHPGRVKVYGPGVEKSGLKANEPTYFTVDCSEAGQGDVSIGIKCAPGVVGPLEADIDFDIIKNDNDTFTVKYTPPGAGLYTIMVLFANQEIPSSPFRIKV
Anser_cygnoides_domesticus      GEGSHPGRVRVHGPGVEKTGLKAGEPTYFTVDCSEAGQGDVSIGIKCAPGVVGPLEADIDFDIIKNDNDTFTVKYTPPGAGLYTIMVLFANQEIPSSPFRIKV
Cuculus_canorus                 GEGSHPGRVKVYGPGVEKTGLKANEPTYFTVDCSEAGQGDVSIGIKCAPGVVGPLEADIDFDIIKNDNDTFTVKYTPPGAGLYTIMVLFANQEIPSSPFRISV
Columba_livia                   GEGSHPGRVKVYGPGVEKTGLKANEPTYFTVDCSEAGQGDVSIGIKCAPGVVGPLEADIDFDIIKNDNDTFTVKYTPPGAGLYTIMVLFANQEIPSSPFRVKV
Ficedula_albicollis             GEGSHPSRVKVHGPGVEKTGLKANEPTYFTVDCSEAGQGDVSIGIKCAPGVVGPLEADIDFDIIKNDNDTFTVKYTPPGAGLYTIMVLFANQEIPSSPFRIKV
Lepidothrix_coronata            GEGSHPGRVKVHGPGVEKTGLKANEPTYFTVDCSEAGQGDVSIGIKCAPGVVGPLEADIDFDIIKNDNDTFTVKYTAPGAGLYTIMVLFANQEIPSSPFRIKV
Python_bivittatus               GEGSHPDKVKVYGPGVEKTGLKANEPTYFTVDCSEAGQGDVSIGIKCAPGVVGPVEADIDFDIIKNDNDTFTVKYTPPGPGRYTIMVLFASQEIPISPFHIKV
Protobothrops_mucrosquamatus    GEGSHPDKVKVYGPGVEKTGLKANEPTYFTVDCSEAGQGDVSIGIKCAPGVVGPAEADIDFDIIKNDNDTFTVKYTPPGPGRYTIMVLFATQEIPTSPFHIKV
Chelonia_mydas                  GEGSHPEKVKVYGPGVEKTGLKANEPTYFTVDCSEAGQGDVSIGIKCAPGVVGPVEADIEFDIIKNDNDTFTVKYTPPGAGRYTIMVLFANQEIPPSPFRIKV
Chrysemys_picta_bellii          GEGSHPDKVKVYGPGVEKTGLKANEPTYFTVDCSEAGQGDVSIGIKCAPGVVGPLEADIEFDIIKNDNDTFTVKYTPPGAGRYTIMVLFANQEIPTSPFRIKV
Gekko_japonicus                 GEGSHPDKVKVYGPGVEKTGLKASEPTYFTVDCSEAGQGDVSIGIKCAPGVVGPLEADIDFDIIKNDNDTFTVKYTPPGPGRYTIMVLFANQEIPISPFHIKV
Anolis_carolinensis             GEGSYPDKVKVYGPGVEKTGLKANEPTYFTVDCSDAGQGDVSIGIKCAPGVVGPVEADIDFDIIKNDNDTFTVKYTPPGPGRYTIMVLFANQEIPISPFHIKV
Alligator_mississippiensis      GEGSHPEKVRVYGPGVEKTGLKASEPTYFTVDCSEAGQGDVSIGIKCAPGVVGPAEADIDFDIIKNDNDTFTVKYTPPGAGLYTIMVLFANQEIPSSPFRIKV
Xenopus_tropicalis              GEGSHPNKVKVYGPGVEKTGLKANEPTYFTVDCSEAGQGDVSIGIKCAPGVVGPAEADIDFDIIKNDNDTFTVKYTPPGAGKYTIMVLFADQEIPTSPFRVKV
Xenopus_laevis                  GEGSHPSKVKVYGPGVEKTGLKASEPTYFTVDCSEAGQGDVSIGIKCAPGVLGPAEADIDFDIIKNDNDTFTVKYTPPGAGRYTIMVLFADQEIPISPFRVKV
Nanorana_parkeri                GEGSHPNKVKVYGPGVEKTGLKANEPTYFTVDCSEAGQGDVSIGIKCAPGVVAPAEADIDFDIIKNDNDTFTVKYTPPGAGRYTIMVLFADQEIPISPFRVKV
fish1                           GEGSHPENVKVHGPGVEKTGLKANEPTYFTVDCSEAGQGDVSIGIKCAPGVVGPAEADIDFDIIKNDNDTFTVKYTPPGAGRYTIMVLFADQEIPISPFRIKV
fish2                           GEGSHPDRVKVYGPGVEKTGLKANEPTYFTVDCSEAGQGDISIGIKCAPGVVGPAEADIDFDIIKNDNDTFTVKYTPPGPGRYTIMVLFADQEIPVSPFKVKV
fish3                           GEGSHPENVKVYGPGVEKSGLKANEPTYFTVDCSEAGQGDVSIGIKCAPGVVGPAEADIDFDIIKNDNDTFTVKYTPPGPGRYTIMVLFADQEIPISPFRIKV
fish4                           GEGCHPAKVKVYGPGVEKTGLKANEPTYFTVDCAEAGQGDISIGIKCAPGVVGPAEADIDFDIIKNDNDTFTVKYTPPGAGKYTIMVLFAEQEIPISPFRIKV
Lepisosteus_oculatus            GEGSHPENVKVYGPGIEKTGLKANEPTYFTVDCSEAGQGDVSIGIKCAPGVVGPAEADIDFDIIKNDNDTFTVKYTPPGAGRYTIMVLFADQEIPISPFRIKV
Cynoglossus_semilaevis          GEGSHPEKVKVYGPGVEKTGLKANEPTYFTVDCSEAGQGDISIGIKCAPGVVGPAEADIDFDIIKNDNDTFTVKYTPPGAGKYTIMVLFADQEIPVSPFKVKV
Nothobranchius_furzeri          GEGSHPDKVKVYGPGVEKTGLKANEPTYFTVDCSEAGQGDISIGIKCAPGVVGPAEADIDFDIIKNDNDTFTVKYTPPGAGRYTIMVLFADQEIPISPFKVKV
Larimichthys_crocea             GEGSHPENVKVYGPGVEKTGLKANEPTYFTVDCSEAGQGDVSIGIKCAPGVVGPAEADIDFDIIKNDNDTFTVKYTPPGAGQYTIMVLFADQEIPISPFRIKV
Scleropages_formosus            GEGSHPENVKVYGPGVEKTGLKANEPTYFTVDCSEAGQGDVSIGIKCAPGVVGPAEADIDFDIIKNDNDTFTVKYTPPGPGRYTIMVLFADQEIPISPFRIKV
Lates_calcarifer                GEGSHPDRVKVYGPGVEKTGLKANEPTYFTVDCSEAGQGDISIGIKCAPGVVGPAEADIDFDIIKNDNDTFTVKYTPPGPGRYTIMVLFADQEIPISPFKVKV
Clupea_harengus                 GEGCHPEKVKVYGPGVEKTGLKANEPTYFTVDCSEAGQGDISIGIKCAPGVVGPAEADIDFDIIKNDNDTFTVKYTPPGAGRYTIMVLFAEQEIPTSPYKVKV
Pundamilia_nyererei             GEGSHPDRVKVYGPGVEKTGLKANEPTYFTVDCSEAGQGDISIGIKCAPGVVGPAETDIDFDIIKNDNDTFTVKYTPPGPGRYTIMVLFADQEIPVSPFKVKV
Stegastes_partitus              GEGSHPDRVKVYGPGVEKTGLKANEPTYFTVDCGEAGQGDISIGIKCAPGVVGPAEADIDFDIIKNDNDTFTVKYTPPGPGRYTIMVLFADQEIPVSPFKVKV
Sinocyclocheilus_anshuiensis    GEGSHPENVKVHGPGVEKTGLKASEPTYFTVDCSEAGQGDVSIGIKCAPGVVGPAEADIDFDIIKNDNDTFTVKYTPPGAGRYTIMVLFADQEIPISPFRIKV
Esox_lucius                     GEGCHPDKVKVYGPGVEKTGLKANEPTYFTVDCAEAGQGDISIGIKCAPGVVGPAEADIDFDIIKNDNDTFTVKYTPPGAGKYTIMVLFAEQEIPISPFRVKV
Astyanax_mexicanus              GEGCHPDKVKVYGPGVEKTGLKSSEPTYFTVDCGEAGQGDISIGIKCAAGVVGTAEADIDFDIIKNDNDTFTVKYTPPAPGRYTVMVLFAEKEIPSSPYKVKV
Latimeria_chalumnae             GEGSHPENVKVYGPGVEKTGLKANEPTYFTVDCSEAGQGDVSIGIKCAPGVVGPAEADIDFDIIKNDNDTFTVKYTPPGAGRYTIMVLFADQEIPISPFRIKV
Callorhinchus_milii             GEGSHPNKVRVYGPGVEKAGLKANEPTYFTVDCSDAGQGDISIGIKCAPGVVGPAEADIDFDIIKNDNDTFTVKYTPPGAGRYTIMVLFADQEIPISPFRIKV
CLUSTAL                         ***.:* .*:*:***:**:***:.*********.:*****:******. **:.  *:**:*************** *. * **:***** :*** **:.:.*
FLNC-E753K                      GEGSHPEKVKVYGPGVEKTGLKANEPTYFTVDCSKAGQGDVSIGIKCAPGVVGPAEADIDFDIIKNDNDTFTVKYTPPGAGRYTIMVLFANQEIPASPFHIKV
```

FIG. 33

```
mammal1                          QPAVDTSGVKVSGPGVEPHGVLREVTTEFTVDARSLTATGGNHVTARVLNPSGAKTDTYVTDNGDGTYRVQYTAYEEGVHLVEVLYDEVAVPKSPFRVGV
mammal2                          QPAIDTSGVKVSGPGVEPHGVLREVTTEFTVDARSLTATGGNHVTARVLNPSGAKTDTYVTDNGDGTYRVQYTAYEEGVHLVEVLYDDVAVPKSPFRVGV
mammal3                          QPAVDTSGIKVSGPGVEPHGVLREVTTEFTVDARSLTATGGNHVTARVLNPSGAKTDTYVTDNGDGTYRVQYTAYEEGVHLVEVLYDEVAVPKSPFRVGV
mammal4                          QPAVDTSGVKVSGPGVEPHGVLREVTTEFTVDARSLTATGGNHVTARVLNPSGAKTDTYVTDNGDGTYRVQYTAYEEGVHLVEVLYDDVAVPKSPFRVGV
mammal5                          QPAVDTSGVKVSGPGVEPHGVLREVTTEFTVDARSLTATGGNHVTARVLNPSGAKTDTYVTDNGDGTYRVQYTAYEEGAHLVEVLYDDVAVPKSPFRVGV
mammal6                          QPAVDTSGVKVSGPGVEPHGVLREVTTEFTVDARSLTATGGNHVTARVLNPSGAKTDTYMTDNGDGTYRVQYTAYEEGVHLVEVLYDEVAVPKSPFRVGV
mammal7                          QPAVDTSGIKVSGPGVEPHGVLREVTTEFTVDARSLTATGGNHVTARVLNPSGAKTDTYVTDNGDGTYRVQYTAYEEGVHLVEVLYDDVAVPKSPFRVGV
mammal8                          QPAVDTSGIKVSGPGVEPHGVLREVTTEFTVDARSLTATGGNHITARVLNPSGAKTDTYVTDNGDGTYRVQYTAYEEGVHLVEVLYDDVAVPKSPFRVGV
mammal9                          QPAVDTSGVKVSGPGVEPHGVLREVTTEFTVDARSLTATGGNHVTARVLNPSGAKTDTYVTDNGDGTYRVQYTAYEEGMHLVEVLYDEVAVPKSPFRVGV
B._acutorostrata_scammoni        QPAVDTSGVKVSGPGVEPHGVLREVTTEFTVDARSLTATGGNHVTARVLNPSGAKTDTYMTDNGDGTYRVQYTAYEEGVHLVEVLYDDVAVPKSPFRVGV
Cebus_capucinus_imitator         QPAVDTSGVKVSGPGIEPHGVLREVTTEFTVDARSLTTTGGNHVTARVLNPSGAKTDTYMTDNGDGTYRVQYTAYEEGVHLVEVLYDEVAVPKSPFRVGV
Myotis_lucifugus                 QPAVDTSGIKVSGPGVEPHGVLREVTTEFTVDARSLTATGGNHVTARVLNPSGAKTDTY-TDNGDGTYRVQYTAYEEGVHLVEVLYDEVAVPKSPFRVGV
Fukomys_damarensis               QPAVDTSGVKVSGPGVEPHGVLREVTTEFTVDARSLTTMGGNHVTARVLNPSGAKTDTYVTDNGDGTYRVQYTAYEEGVHLVEVLYDDVAVPKSPFRVGV
Orcinus_orca                     QPAVDTSGVKVSGPGVEPHGVLREVTTEFTVDARSLTATGGNHVTARVLNPSGAKTDTYLTDNGDGTYRVQYTAYEEGVHLVEVLYDDVAVPKSPFRVGV
Monodelphis_domestica            QPAVDTSGIKVSGPGVEPHGVLREVTTEFTVDARSLTATGGNHVTARVLNPSGAKTDTYVTDNGDGTYRVQYTAYEEGIHLVEVLYDDVAVPKSPFRVGV
Ornithorhynchus_anatinus         HPAVDTSGVKVSGPGVEPHGVLREVTTEFTVDARSLTATGGNHVTARVLNPSGAKTDTYITDNGDGTYRVQYTAYEEGVHLVEVLYDDVAVPKSPFRVGV
bird1                            -PAVDTSGVKVYGKGVEPRGVLREVGTDFTVDARALTKTGGPHVKAWVVNPSGAKTDTYITDHGDGTYRVDYTPYEDGMHRVEVTYNDVAVPKSPFRVGV
bird2                            -PAIDTSSVKVYGKGVEPRGVLREVGTEFTVDARALAPTGGPHIRARVLNPSGTPIDTFVTDLGDGTYRVEYTPFEEGLHLVEVTYDDVAVPKSPFRVGV
bird3                            -PAVDTSSVKVYGKGVEPRGVLREVGTEFTVDARALAPTGGPHVRARVLNPSGTPIDTFVTDLGDGTYRVEYTPFEEGLHLVEVTYDDVAVPKSPFRVGV
Picoides_pubescens               -PAVDTSGVKVYGKGVEPRGVLREVGTDFTVDARALTKVGGAHVKARVVNPSGATTETYVTDHGDGTYRVDYTPYEDGMHRVEVTYDDVAVPKSPFRVGV
Columba_livia                    -PAVDTSGVKVYGKGVEPRGVLREVATDFTVDARALTKTGGPHVAARVVNPSGATTDTYITDHGDGTYRVEYTPYEDGVHRVEVTYAEVAVPKSPFRVAV
Melopsittacus_undulatus          -PAVDTSGVKVYGKGVEPRGVLREVSTDFTVDARALTKTGGPHVTARVLNPSGAKTDTFITDLGDGTYRVEYTPYEDGVHRVEVLYDDVPVPKSPFRVAV
Sturnus_vulgaris                 -PAVDTSSVKVYGKGVEPRGVLREVGTEFTVDARALAPTGGPHVRARVLNPSGTPIDTFVTDLVDGTYRVEYTPFEEGLHLVEVTYDDVAVPKSPFRVG-
Corvus_brachyrhynchos            -PAIDTSSVKVYGKGVEPRGVLREVGTEFMVDARALAPTGGPHIRARVLNPSGTPIDTFVTDLGDGTYRVEYTPFEEGXHLGEVTYDDVPVPKSPFRVGV
Lepidothrix_coronata             -PAVDTSSVKVYGKGVEPRGVLREVGTEFTVDARALAPAGGPHVRARVLNPSGTPIDTFVTDLGDGTYRVEYTPFEEGLHRVEVTYDDVAVPKSPFRVGV
Ficedula_albicollis              -PAVDTSSVKVYGKGVEPRGVLREVGTEFTVDARALAPTGGPHVRARVLNPAGTPIDTFVTDLGDGTYRVEYTPFEEGLHLVEVTYDEVAVPKSPFRVGV
Cuculus_canorus                  -PAVDTSGVKVYGQGVEPRGVLREVGTEFMVDTRALSRTGGPHVGVRVLNPSGGTTDTRITDRGDGTYRVQYTPFEEGMHQVEVTYDDVPVPNSPFRVAV
Chrysemys_picta_bellii           QPAVDTSAVKVFGPGVEPRGVLREVTTEFTVDARSLTKTGGSHIKARVINPSGAKTETYLTDNGDGTYRVHYTAYEEGLHRVEVTYDEVPVPKSPFRVAV
Xenopus_tropicalis               EPAVDTSGVKVFGPGVEPRGVLREVTTEFTVDARSLTKTGGSHVTTRILGPSGAVTESFLSDNGDGTYHVQYTAYEDGVHLIEVLYDDVPVPKSPFRVPV
Xenopus_laevis                   EPAVDTSGVKVFGPGVEPRGVLREVTTEFTIDARSLTKTGGSHVTTRVLGPSGVVTESFISDNGDGTYHVQYTAYEDGVHLIEVLYDEVPVPKSPFRVPV
Nanorana_parkeri                 EPAVDTSGVKVFGPGVEPRGVLREVATEFTVDARSLTKTGGSHVTTRIISPSGAVTESFISDNGDGTYHVQYTAYEDGIQLVEVLYNDVPVPKSPFRVGV
Protobothrops_mucrosquamatus     -PAVDTSNVKVFGPGVEPRGVLREVTTEFTVDARSLTKTGGNHVKVRVINPSGAKTDTYITDNGDGTYRVQYTPFEDGVHLVEVTYDDVPVPKSPFRVGV
Python_bivittatus                -PAVDTSNVKVFGPGVEPRGVLREVTTEFTVDARSLTKTGGTHIKVRVINPSGAKTDTYITDNGDGTYRVQYTPFEDGMHLVEVTYDDVPVPKSPFRVGV
Anolis_carolinensis              -PAVDTSNVKVFGPGVEPRGVLREVTTEFTVDARSLTKTGGNHIKVRVINPSGAKTDSYITDNGDGTYRVQYTPFEDGVHLVEVTYDDVPVPKSPFRVGV
Alligator_mississippiensis       -PAVDTSPIKVYGPGVEPRGVLREVTTEFTVDARALTKTGGSHIKARVVNPSGAKTDTYITDNADGTYRVQYTAYEDGVHRVEVTYDDVPVPKSPFRVAV
Chelonia_mydas                   QPAVDTSAVKVFGPGVEPRGVLREVTTEFTVDARSLTKTGGSHIKARVINPSGAKTETYLTDNGDGTYRVQYTAYEEGLHRVEVTYDEVPVPKSPFRVAV
fish1                            -PALDTSGIKVYGPGVEPRGVLREVTTHFVVDTRVHSKMGGNHIKVRIVNPSGANTDAYITDKGDGTYRVEYTAYEDGVHLIEVLYDDVPVPKSPFRVAV
Sinocyclocheilus_grahami         -PALDTSGIKVYGPGVEPRGVLREVTTHFVVDTRVHSKMGGNHIKVRIVNPSGANTDAYITDKGDCTYRVEYTAYEDGVHLIEVLYDDVPVPKSPFRVAV
Larimichthys_crocea              -PAIDTSGVKVYGPGVEPRGVLREVTTHFTVDALAHYKSGSSHVKACISNPSGANTDAYITDKGDGTYRVEYTPYEDGLHLIEVLFDEVSVPKSPFRVSV
Pygocentrus_nattereri            EPAVDTSGVKVYGPGVEPKGVLRDVTTHFIVDARAMNKTGGNHVKVRIINPSGSNTDAHITDKGDGTCRVEYTAFEDGVHVIEVFYDDVAVPKSPFRVSV
Astyanax_mexicanus               -PAVDTSGVKVFGPGVEPRGVLREVTTHFIVDTRAHNKTGGNHIKTRIVNPSGSNTDAYITDKGDGTYRVEYTAYEDGVHLIEVLYDEVSVPKSPFRVSV
Cyprinus_carpio                  -PALDTSGIKVYGPGVEPRGVLREVTTHFVVDTRVHSKMGGNHIKVRIVNPTGANTDSYITDNGDGTYRVEYTAYEDGVHLIEVLYDDVPVPKSPFRVAV
Ictalurus_punctatus              EPAVNTSGVKVYGPGVEPTGVLREVSTHFIVDARSLTKMGGNHVTVRIVSPSGSITDAYITDKGDGTYRVEYTAFQDGMHLIEVLYDDVMVPNSPFRVSV
Clupea_harengus                  EPAVDTTGVTVYGPGVEARGVLRDVTTHFIVDARAQTKSGGNHVKARIMNPSGNNTDAYLTDKGDGTYRVEYTAYEEGIHLIEVLYDDVPIPKSPFRVSV
Oncorhynchus_mykiss              DPAVDTSGVKVYGPGVEPRGVLREVTTHFIVDARAKSKTGGSHVKARIVNPTGANTDAYITDKGEGTYRVEYTAYEDGMHLIEVLYDDVAVPNSPFRVPV
Salmo_salar                      EPAVDTSGVQVYGPGVEPRGVLKEVTTHFIVDARKTTKSGGDHVKARIINPSGANTDAYITDKGDGTYRVEYTAFEDGMHLIEVLYDDVAVPKSPFRVSV
Esox_lucius                      EPAVDTSGVQVYGPGVEPRGVLKEVTTHFIVDARKTTKTGGDHVKACIINPSGTNTETYITDKGDGTYRVEYTAFEDGMHLIEVLYDDVAVPKSPFRVSV
Lepisosteus_oculatus             -PAVDTSGIKVYGPGIEPRGVLREVTTHFIVDVRTLNKTGGNHVKARIVNPSGAKTDAYITDKGDGTYRVEYTAYEDGIHLIEVLYDDVAVPKSPFRVAI
Latimeria_chalumnae              -PAVDTSTVKVFGPGVEPRGVLREVTTEFTVDACTLTKTGGNHVKVRILNPSGAKTDPYVTDKGDGTYRVEYTAYEDGIHLIEVLYDDVAVPKSPFRVAV
Callorhinchus_milii              -PAIDLSGVKVFGPGVEPRGVLREVTTEFTVDCRSVSRSGGAHVKARITNPSGAATDSYVRDNGDGTYRVEYSAYEDGLHVIEVLYDDIPLPKSPFRVGV
                                  **:: : : * * *:*  ***::* *.* :*       *. *: . : .*:*   :   *  : * :*.*: :::* :  ** :  :: :*:*****

FLNC-A1207T                      QPTIDTSGVKVSGPGVEPHGVLREVTTEFTVDARSLTATGGNHVTARVLNPSGAKTDTYVTDNGDGTYRVQYTAYEEGVHLVEVLYDDVAVPKSPFRVGV
```

FIG. 34

```
mammal1                 MIPKEQKGPVMAAMGD-
LTEPVPTLDLGKKLSVPQDLMMEELSLRNNRGSLLFQKRQRRVQKFTFELAA-----
mammal2                 MIPKEQKGPVMAAMGN-
FTEPVPTLDLGKKLSVPQDLMMEELSLRNNRGSLLFQKRQRRVQKFTFELAA-----
mammal3                 MIPKEQKGPVMAAMGD-
LTEPVPTLDLGKKLSVPQDLMMEELSLRNNRGSLLFQKRQRRVQKFTFELAE-----
mammal4                 MIPKEQKGPVMAAMED-
LAGPVPVLDLGKKLSVPQDLMMEELSLRNNRGSLLFQKRQRRVQKFTFEFAA-----
mammal5                 MIPKEQKGPVMTTMGD-
LTEPVPLLDLGKKLSVPQDLMMEELSLRNNRGSLLFQKRQRRVQKFTFEFAA-----
mammal6                 MIPKEQKGPVMAAMGD-
LTGPVPLLDLGKKLSVPQDLMMEELSLRNNRGSLLFQKRQRRVQRFTFEFAA-----
mammal7                 MIPKEQKGPVMTTTGD-
LTEPVPLLDLGKKLSVPQDLMMEELSLRNNRGSLLFQKRQRRVQKFTFEFAA-----
mammal8                 MIPKEQKGPVMAAMGD-
QATPVPLLDLGKKLSVPQDLMMEELSLRNNRGSLLFQKRQRRVQKFTFELAD-----
Propithecus_coquereli   MIPKEQKGPVMAAMGD-
LAGPVPSLDLGKKLSVPQDLMIEELSLHNNRGSLLFQKRQRRVQKFTFELAE-----
Pongo_abelii            MIPKEQKGPVMAAMGD-
LTEPVPMLDLGKKLSVPQDLMMEELSLRNNRGSLLFQKRQRRVQKFTFELAA-----
Callithrix_jacchus      MIPKEQKGPVMAAMGN-
LTESVPTLDLGKKLSVPQDLMMEELSLCNNRGSLLFQKRQRRVQKFTFELAA-----
Macaca_mulatta          MIPKEQKGPVMAAMGN-
FTEPVPTLDLGKKLSVPQDLMMEELSLCNNRGSLLFQKRQRRVQKFTFELAA-----
Chlorocebus_sabaeus     MIPKEQKGPVMAAMGN-
FTEPVPTLDLGKKLSVPQDLMMEELSLPNNRGSLLFQKRQRRVQKFTFELAA-----
Camelus_bactrianus      MIPKEQNGQVMTAMGD-
LTGPVPVLDLGKKLSVPQDLMMEELSLRNNRGSLLFQKRQRRVQKFTFEFTA-----
Vicugna_pacos           MIPKEQNGQVMTAMGD-
LTGPVPVLDLGKKLSVPQDLMMEELSLRNNXGSLLFQKRQRRVQKFTFEFTD-----
Ursus_maritimus         MIPKEQKGPAMAAVGD-
LSGPEPLLDLGKKLSVPQDLMMEELSLRNNRGSLLFQKRQRRVQRFTFEFAA-----
Ailuropoda melanoleuca  MIPKEQKGPAMAAVGD-
LTGPEPLLDLGKKLSVPQDLMMEELSLRNNRGSLLFQKRQRRAQKFTFEFAA-----
Miniopterus natalensis  MIPKEQKEPVMAAMGD-
QAGPVPLLDLGKKLSVPQDLMMEELSLRNNRGSLLFQKRQRRVQKFTFELAA-----
C. simum simum          MIPKEQKGPVMAATGD-
LAGPVPLLDLGKKLSVPQDLMMEELSLRNNRGSLLFQKRQRRVQKFTFEFAA-----
Mustela putorius furo   MIPKEQKGPVMAAMGD-
LAGPAPLLDLGKKLSVPQDLMMEELSLRNNKGSLLFQKRQRRVQRFTFEFAA-----
Felis catus             MIPKEQKGSVMAAMGD-
LTEPVPLLDLGKKLSVPQDLMMEELSLRNNRGSLLFQKRQRRVQRFTFEFPA-----
Acinonyx jubatus        MIPKEQKGSVMAAMGD-
LTGPVPLLDLGKKLSVPQDLMMEELSLRNNRGSLLFQKRQRRVQRFTFEFPA-----
Canis lupus familiaris  MIPKEQKGSVMAAMGD-
LTGPVPLLDLGKKLSVPQDLMMEELSLRNNRGSLLFQKRQRRVQRFTFEFAA-----
Panthera tigris altaica MIPKEQKRPVMAAMGD-
LTGPVPLLDLGKKLSVPQDLMMEELSLRNNRGSLLFQKRQRRVQRFTFEFPA-----
Ochotona princeps       MIPKDHKGPAVSAMGD-
FSEPVPLLDLGKKLSVPQDLMIEELSLRNNRGSLLFQKRQRRVQRFTFELPD-----
Oryctolagus cuniculus   MIPKEQKGLAMSAPGD-
LSEPVPLLDLGKKLSVPQDLMIEELSLRNNRGSLLFQKRQRRVQRFTFELPD-----
I. tridecemlineatus     MIPKEQKEPVMAVMGD-
LAGPVPTLDLGKKLSVPQDLMIEELSLRNNPGSLLFQKRQRRVQRFTFELEE-----
Marmota marmota marmota MIPREQKEPVMAVMGD-
LAGPVPLLDLGKKLSVPQDLMIEELSLRNNPGSLLFQKRQRRVQRFTFELEE-----
Tupaia chinensis        MIPKEQKGPVTATMGD-
LCEPVPLLDLGKKLSVPQDLMMEELSLRNNRGSLLFQKRQRRVQRFTFELSA-----
Physeter catodon        MIPKEQKGPVMTTMGD-
LTEPVPLLDLGKKLSVPQDLMMEELSLRNNRGSLLFQKRRRRVQKFTFEFAS-----
Panthera pardus         MIPKEQKGPVMAAMGD-
LTGPVPLLDLGKKLSVPQDLMMEELSLRNNRGSLLFQKRQRRVQRFTFEFPA-----
Sarcophilus harrisii    -VLKKQKALTMAKAQD-
FMETAPSLDLGKKVSVPQDLMVEELSLRSNRGSLLFQKRQRRVQRFTFEYAT-----
```

```
Fukomys damarensis       ---MDPKEQQDAVIGD-
FTGPEPSLDLGKKVSVPQDLMIEELSLRNNRGSLLFQKRQRRVQRFTFELAA-----
Python bivittatus        ---
REQHAPIQAIAREIQRGKAPELDLGKKVSTPQDLMMEELSLPINRGSRLYQQRQKRVQRFVLEYPT-----
Pelodiscus sinensis      ---RERKRQVAAILRE-
TAGDVLQLDLGKKVSVPQDLMVEELSLQSNRGSQLFQKRQKRVQKFILEHPMGYGAG
Chrysemys picta bellii   ---RERKKQAMAIVTE-
MMGDVPQLDLGKKVSIPQDLMVEELSLQTNRGSQLFQQRQKRVQKFILEHPTGYRA-
Chelonia mydas           ----ERKRQAIAIVRE-
MAEDVPQLDLGKKVSIPQDLMVEELSLQTNRGSQLFQQRQKRVQKFILEHPT-----
Anolis carolinensis      ----
EQHAPVSAIMKEIREKYGPKLDLGKKVSIPQDLMMEELSLPVNRGSQLYQKRQKRVQQFVLERPTAY---
A. mississippiensis      --------
KRQAMIVRESPGDAPHLDLGKKVSIPQDLMMEELSLKTNRGSRLYQERQKRMQRFVLEHPS-----
Calidris pugnax          ---------VMTIMRP-
GPEDAPQLDLGKKMSTPQDLMIEELSLGNNRGSQLFHQRQKRMQRFVYEHPS-----
Chlamydotis macqueenii   ----------MTIMKP-
GPEDVPQLDLGKKMSTPQDLMIEELSLRNNRGSQLFQERQKRMQRFVFEYPR-----
Monodelphis domestica    ----------MATVQD-
FMGAAPSLDLGKKVSIPQDLMVEELSLRSNRGSLLFQKRQKRVQRFTFEYAA-----
Picoides pubescens       ----------MAIMKS-
GPEDVPRLDLGKKISTPQDLMIEELSLRDNRGSQLFQQRQRRMQRFIFEHPS-----
Pelecanus crispus        ----------MTIMKP-
GPEDVPRLDLGKKMSTPQDLMIEELSLRNNRGSQLFQQRQRRMQRFIFEHPS-----
Charadrius vociferus     ----------MTIMRP-
APEDAPQLDLGKKMSTPQDLMIEELSLRNNRGSQLFQQRQKRMQRFVFEHPS-----
Pygoscelis adeliae       ----------MAIMRP-
GPEDAPRLDLGKKMSTPQDLMIEELSLRNNRGSQLFQQRQRRMQRFVFEHPS-----
Nipponia nippon          -----------------
VRDPVPQLDLGKKMSTPQDLMIEELSLRNNRGSQLFQQRQKRMQRFVFEHPS-----
Egretta garzetta         -------------------
EDAPRLDLGKKVSTPQDLMIEELSLRNNRGSQLFQQRQKRMQRFVFEHPG-----
Cathartes aura           --------------------
PVPRLDLGKKVSVAQDLMIEELSLPNNRGSQLFQQRQRRMHGFLFLPGQ-----
Taeniopygia guttata      --------------------PEPQLDLGKKMSTTHDLMIEELSLPHNRGSRLFQQRQKRVQRFVLE-
-------
Corvus brachyrhynchos    --------------------
PEPQLDLGKKMSTTQDVMIEELSLRNNRGSRLFQQRQKRMQRFVFEHPS-----
Columba livia            --------------------
PAPQLDLGKKVSTPQDLMMEELSLRNNRGSRLFQQRQKRMQRFVFEHPRVGG--
Chaetura pelagica        --------------------
PVPQLILGKKMSTPQDLMIEELSLRNNRGSQLFQQRQRRMQRFVFEHPS-----
Aptenodytes forsteri     --------------------
PAPRLDLGKKVSTPQDLMIEELSLRNNRGSQLFQQRQRRMQRFVFEHPS-----
Tauraco erythrolophus    --------------------PAPRLDLGKKVSTPQDLMIEELSLRNNRGSQLFQQRQRRMQRFVFE-
-------
Alligator sinensis       ---------------------
APHLDLGKKVSIPQDLMMEELSLKTNRGSRLYQERQKRMQRFVLEHPS-----
S. camelus australis     ---------------------
APRLDLGKKVSTPQDVMIEELSLRTNRGSQLFQQRQRRMQRFIFEYPS-----
Tinamus guttatus         ---------------------
VPRLDLGKKVSTPQDVMIEELSLRTNRGSQLFQQRQRRMQRFIFEYPS-----
bird1                    ----------------------
PRLDLGKKMSTTQDLMIEELSLRNNRGSQLFQQRQRRMQRFIFEHPS-----
Pseudopodoces humilis    ----------------------
PQLDLGKKMSTAQDLMIEELSLQNNRGSRLFQQRQKRVQRFVFEHPR-----
Serinus canaria          ----------------------
PHLDLGKKMSTTHDLMIEELSLPNNRGSRLFQQRQKRVQRFVFEHPS-----
Parus major              ----------------------
PQLDLGKKMSTTQDLMIEELSLQNNRGSRLFQQRQKRVQRFVFEHPS-----
Zonotrichia albicollis   ----------------------
PQLDLGKKMSTTQDLMIEELSLPNNRGSRLFQQRQKRVQRFVFEHPS-----
Cuculus canorus          ----------------------
PRLDLGKKMSTPQDLMIEELSLRNNRGSQLFQQRQRRMQRFVFEHPS-----
A. australis mantelli    ------------------------
LDLGKKVSTPQDVMIEELSLRTNRGSQLFQQRQKRMQRFIFEYPS-----
CLUSTAL                                          * ****:*  :*:*:*****  * ** *:::*::* : *
```

MYOZ3-S42L MIPKEQKGPVMAAMED-
LAGPVPVLDLGKKLSVPQDLMMEEL**L**LRNNRGSLLFQKRQRRVQKFTFEFAA-----

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62313272
- US 62421625
- WO 2008100313 A1


### Non-patent literature cited in the description


- **MYKKANEN et al.** *Research in veterinary science*, vol. 91 (3), 473-477
- **FOROUD et al.** *Neurology*, vol. 65 (12), 1936-1940
- **AURINO et al.** *Official journal of the Mediterranean Society of Myology*, 01 December 2008, 90-97
- **NIELSEN et al.** *Science*, 1991, vol. 254, 1497-1500